# EUROPEAN PATENT APPLICATION

(11) **EP 1 666 611 A1**
(43) Date of publication of application: **07.06.2006**
(21) Application number: 04292891.1
(22) Date of filing: 06.12.2004
(51) Int. Cl.: C12Q 1/68

(54) **Diagnosis of arterial diseases by identification of a mutation in the MYH11 gene or protein**

(71) Applicant: INSTITUT NATIONAL DE LA SANTE ET DE LA RECHERCHE MEDICALE (INSERM), 75013 Paris (FR)
(72) Inventor: Jeunmaitre, Xavier, 75012 Paris (FR); Khau Van Kien, Philippe, 34170 Castelnau le Lez (FR); Zhu, Li Min, 75015 Paris (FR); Mathieu, Flavie, 95330 Domont (FR)
(74) Representative: Colombet, Alain André

(57) **Abstract**

The invention relates to an *ex vivo* method of diagnosing or predicting an arterial disease, or a risk of arterial disease, in a subject, which method comprises detecting a mutation in the MYH11 gene or protein (myosin heavy polypeptide 11 of smooth muscle cells), wherein said mutation is indicative of an arterial disease or of a risk of arterial disease.

SEQUENCE LISTING

<110> INSERM

<120> Method for diagnosing and/or predicting an arterial disease

<130> BET 04P0104

<160> 15

<170> PatentIn version 3.1

<210> 1
<211> 153892
<212> DNA
<213> Homo sapiens

<220>
<221> misc_feature
<222> (1)..(153892)
<223> MYH11 genomic sequence


<220>
<221> misc_feature
<222> (153805)..(153875)
<223> exon 1


<220>
<221> misc_feature
<222> (134772)..(135133)
<223> exon 2


<220>
<221> misc_feature
<222> (120119)..(120275)
<223> exon 3


<220>
<221> misc_feature
<222> (95524)..(95551)
<223> exon 4


<220>
<221> misc_feature
<222> (83494)..(83596)
<223> exon 5


<220>
<221> misc_feature
<222> (79249)..(79341)
<223> exon 6


<220>
<221> misc_feature

<222>  (75644)..(75707)
<223>  exon 7


<220>
<221>  misc_feature
<222>  (72942)..(73040)
<223>  exon 8


<220>
<221>  misc_feature
<222>  (68433)..(68576)
<223>  exon 9


<220>
<221>  misc_feature
<222>  (60660)..(60755)
<223>  exon 10


<220>
<221>  misc_feature
<222>  (57404)..(57522)
<223>  exon 11


<220>
<221>  misc_feature
<222>  (56440)..(56592)
<223>  exon 12


<220>
<221>  misc_feature
<222>  (54691)..(54864)
<223>  exon 13


<220>
<221>  misc_feature
<222>  (53205)..(53378)
<223>  exon 14


<220>
<221>  misc_feature
<222>  (50258)..(50372)
<223>  exon 15


<220>
<221>  misc_feature
<222>  (47002)..(47195)
<223>  exon 16


<220>
<221>  misc_feature
<222>  (44911)..(45032)

<223>  exon 17

<220>
<221>  misc_feature
<222>  (44738)..(44807)
<223>  exon 18

<220>
<221>  misc_feature
<222>  (44434)..(44594)
<223>  exon 19

<220>
<221>  misc_feature
<222>  (41993)..(42101)
<223>  exon 20

<220>
<221>  misc_feature
<222>  (38624)..(38755)
<223>  exon 21

<220>
<221>  misc_feature
<222>  (38327)..(38533)
<223>  exon 22

<220>
<221>  misc_feature
<222>  (36915)..(37052)
<223>  exon 23

<220>
<221>  misc_feature
<222>  (35429)..(35552)
<223>  exon 24

<220>
<221>  misc_feature
<222>  (34313)..(34484)
<223>  exon 25

<220>
<221>  misc_feature
<222>  (32230)..(32442)
<223>  exon 26

<220>
<221>  misc_feature
<222>  (29428)..(29572)
<223>  exon 27

<220>
<221>  misc_feature
<222>  (23712)..(23918)
<223>  exon 28

<220>
<221>  misc_feature
<222>  (21752)..(21856)
<223>  exon 29

<220>
<221>  misc_feature
<222>  (21511)..(21663)
<223>  exon 30

<220>
<221>  misc_feature
<222>  (21025)..(21273)
<223>  exon 31

<220>
<221>  misc_feature
<222>  (18286)..(18498)
<223>  exon 32

<220>
<221>  misc_feature
<222>  (17703)..(17915)
<223>  exon 33

<220>
<221>  misc_feature
<222>  (17015)..(17176)
<223>  exon 34

<220>
<221>  misc_feature
<222>  (16449)..(16577)
<223>  exon 35

<220>
<221>  misc_feature
<222>  (16084)..(16172)
<223>  exon 36

<220>
<221>  misc_feature
<222>  (15179)..(15302)
<223>  exon 37

<220>
<221> misc_feature
<222> (14004)..(14212)
<223> exon 38

<220>
<221> misc_feature
<222> (12028)..(12136)
<223> exon 39

<220>
<221> misc_feature
<222> (11773)..(11945)
<223> exon 40

<220>
<221> misc_feature
<222> (5667)..(5705)
<223> exon 41

<220>
<221> misc_feature
<222> (1)..(987)
<223> exon 42

<400> 1
gtgatttata accatttatt agtgaaagtg tttttaagca cagtcagggt gtaaacagtg 60

cagcattcct gctcccctcc gtgggagcag cgtctccttt tcaattcatg tgactacaga 120

aggcacttgg tgaactgtgc gtgtctgagg tgtggaaacc aggagagggg gaaagaattc 180

tcaaaggcct gacgtgagaa gttggaaagg tttgcaggtt agggaatgaa ttgggagtgg 240

gggccggcgg cacccatttc ggtgactttc tccccatttc atgtaaacag aattgccagg 300

gaccggttac cgtggatatg tttttctaaa aactcagtgt ctgcacaatc cattgataga 360

actggaggat gtgtctgtgt ttcctgttgg gtttttctca tctcttacat catacaaact 420

tcaattttta ccttgaatac aggggtagta ggggtggtgg tggtggtggt ggttgagaca 480

gggtctctgt tgcccaggct ggagtgcaat gatgcaatta tagctcattg cagcctcgaa 540

gtcctgggct ggagcgttct tcctggctca gcctccctag tagctgggac cacaggtgtg 600

taccaccacg cccagcttat ttttaaattc ttgtatagat gaggttttac tacgttgccc 660

aggctggagg gtggtggttt ttatattcct tgtgtgaggg gtgtctgtga tatttggaat 720

ttgagaatgg atttagacaa tgctaagtac agtctgctgg gttttgcttt gttctgggtt 780

gttgttgggt tttttttgtt tgtttgtttt ggtttttggt tttcttgccg tggtgcaaaa 840

ctgtagaaag ttgcttattc actggccttg gttccattga agtctgcgtc tcgagtgtcc 900

gtttcctcct cagaaccatc tgcattttca ataactctac gtcctccaga ccttctagaa 960
ggaacgaaag aggtctcgtt tcctcgcctg tgggttgtaa gaaaacacat tatttagcaa 1020
agaaatcttc atggttggga tagatttttt ataaatctat tttaaacttg tagctatagt 1080
cctattgcaa tataaatttt ttttatggca gaaaacacac acggcacaaa taagatgcag 1140
atattcaagt tgaagtcaac aaatcattgc cagtcttaag gcatgagtga ttgaatttaa 1200
tcagctgaaa ttggttgcgg gggtgggtgg agaaggtttt tcaaagcaag acgatctgct 1260
tttcaatatg tcaggcttgg tcatttgaga agccacagga ttctggtctg ctgtggtctt 1320
tgatttagaa taggaaaaaa acgccaaaaa ccaagcaggg gattttgtct tctgacttta 1380
ttcatagccc atggggcaga gttgaagaga gaacatactg tttttcttct gtatctcctc 1440
acccccattc gccttagagg tgagttttca agatttgaat tggaagaagg tgaaatggac 1500
aagggcagcc ttgagaagga cacagagggg ctccctggtt aaaaggcgag acagcacccc 1560
ctgctggtgt gaatgttcat ggactgagtt ttccccactg cagggactgc tgcatctgtt 1620
ttgctgatcg tggaacatac cgcacatggc gtaaaacagg tgcccagtga atattagaaa 1680
aggaaattat acgtgttaat ccaaggtgga ttattttatt aaattgagat gaggtcttga 1740
tattgttgcc caggctggtc tggaactcct gagctcaagc agttctccta ccttggcctc 1800
ccaaagcgct ggcattacag gcgtgggcca ccacacctgg ccttcaaggt ggatttaaaa 1860
aagaaattta agagtctctc tcacccaggc tggagtgcag tggcatgatc ttggctcact 1920
gcaacctctg cctcctgagt tccagcagtc cttgagcctc agtattgagt agctgggact 1980
acatgcacgt gcacactgcc acgcctggct aatttttgta tttttagcag agatgggatt 2040
ttgccatgtg acccaggctg gtcttgaact cctggcctca aacgatccac ctgtcttagc 2100
ctctcaaagt gttgggatta caggtgtgtg ccagtgcacc tggcctctga ggtggatttt 2160
agctccagcc agtaggacac ccctgtctct ccttaatgct gccatctcct ctctgcgctg 2220
gttctctctt gagtttatca cctcgacttc acaggaaggg ctggtcacgt gaggggagac 2280
tttgggtagg aggaagagag tgggcagaag aaggaaaaat cagatctgcc tgctgccagc 2340
cttcaccgtt cagaagaaac acttaactct tttcagcaac cgcaacaaat aattatgttg 2400
ggctctggcc atctggccct gtttgacctg aatcggaaga caggtcttat tttaggacat 2460
caaagaagag agcagacttt tgctccccac gcaagggttg gataagaagc cataaggatg 2520
aacacaactc agtgaccaat tctttagtca aaaggaacaa ctcggctggg catggtggct 2580
cacacctgta atctcagcac tttgggaggc tgaggcgggc ggatcatgag gtcaggagat 2640
cgagaccatc ctggctaaca tggtgaaacc gtgtctctac taaaaataca aaaaattagc 2700

cgggcgtgct ggcgggtgcc tgtagtccca gctactcggg agggaggctg aggcaggaga 2760
atgacatgaa cccaggaggc ggagcttgca gtgagccgag atgtggccac tgcactccag 2820
cctaggcgac agggtgagac tccgtctcaa aaaaaaaaaa aaaaaaaaat caaggcccag 2880
agattcatga tcactgagaa gaggaattcg caaatgaccc ttttgttgac agaggatggg 2940
agagaccccg gctgggaaag cccacctggc tataaaggat gggacttccc gcagtgcagc 3000
gatcaggaat aaatacttaa agctgcagcc acttcaaagt aaacactcct agcccagctt 3060
cacagagaag cttctggact tgcagcccta ctagagaact ctccatcctt ctctctctag 3120
atgtgaccct taagtctttt tcttttttag cactgttttc caagcgcttg actcagaagg 3180
atgctcaaat tcagcgcaga ggagcagcag tttgactgtt ccctgtacgc tccccacaca 3240
gctctcttcg tcacagtttt aactccaggt tcaaaccctg atgggttggc cagttgctgc 3300
gactcatgcc tgtaatccca gcacttgggg aggccgaggt gggcagatca ctcgtgaggt 3360
cagttgttcg aggccatcct ggccaatatg gtgaaacccc gtctctacta aatatactaa 3420
attagccgtg cgtggtggca ggcgcctgga atcccagtca ctcaggaggc tgaggcagga 3480
gaattgcttg aacccaggag gtgaaggctg cagtgagcca acgttgtgcc actgcactcc 3540
agcctgggaa acagcaagac tgtctccaaa aaaaaaaaca aaaaaaaatc tgatgagctg 3600
ggaagtagtc cctaacctcc acatcagtgc tctaggcact ctcttttggg ctcttggaag 3660
agtctatttg ctaactggaa aagcttaatg gataaatttg ctcccttgac ctaagaaata 3720
cattgtgccc tgggccgttg aagctccttg cttggctgtc agcctgcaag atactctgtt 3780
ttggctgaag tgcttttctt ggaactggtt tcaaatccta gaccaaggtt gacaccaact 3840
atacctgtta caggaactaa gggacccccc taaaggtttt cttaaagggt ctgatcctgg 3900
agaatctgga attttttttt gaaatagagt ctcgctgtgt tgcccaggct ggagtgcagt 3960
ggcgtgatct tggctcactg cagcctctgc ctcccaggtt caagcgattc tcctgcctca 4020
gccttccaag taactgggac tacagacatg ccaccactcc tggctaattt ttgtattttt 4080
aggagagacg gggttttacc atgttggcca ggctggtcct gacctcaagt tatctgcccg 4140
cctcggcctc ccaaagtgct gggattatag gcatgaccca ctgtgcctgg cctagcattt 4200
ggattctatt cgtggactca gcttatccaa ggcctcggga atcatcttga gtgtaagcac 4260
ctgctagttt gcaaggactc agtttcccac tgaccagtgg gttctgcact tcagcccacc 4320
ctggaatttg gcttatccta tgatcccaat cagctggtag gagagctgct tgaagtgtcc 4380
tagaaactca tctgctcccc cacaaaactg gctcttaaca agacctcacc ctgcgcttta 4440
agtattttgg tagtgacagc aaagctgaga ttctccaaaa atgaagatgg acacaaatga 4500
gggttggttt ggcctctcca tgaggtgaag tcagatccac atggcctaac ttcacctgtg 4560

aaaagttggt catgcttgta atcccagcat ttggggaggc caaggcgcgc ggatcacctg 4620

aagtcaggag tttgagacca gcctggccaa catggtgata tcctgtctct aataaaaata 4680

caaaaattag ctgggcgagg tggcgggagc ctgtaatccc agctactcag aaggctgagg 4740

taggagaatc gcttgaaccc aggaggcaaa ggttgcggtg agccaagatt gcgccattgc 4800

actccagagc gagactccgt ctcaaaaaaa aaaaaaaaaa aaagcaaaat cccagagcag 4860

cagagacctc ttcatgcttt cttgggaagg gccctgtgtg catccttgag tcttgggtat 4920

ccactcccca gtccctgaac atgtacatgc ccgtgaagcc tccagaaaag gctctgccag 4980

gactgtgctt ggctttgcgg tggtcagctt ccaccccgtg tgctgaaaat gcagtttgta 5040

gctagacatc actcaagcca cgtgcagccc ccggcatttg tcagacatcg cagtgagctc 5100

atttccttcg cagacaagcc tgtgaaagct gaatcatggg aggactggtt gccgcgccgc 5160

agttaccgtg aactttgtga tctctgactt gtgacccaga ccagccaact agaagccaag 5220

tacgttctcc aggaatgtgc ccagatagtg gaactgtcat attcattcag tgcagtgtct 5280

tcactggaca agggaggaca gcactgctga gtgccctaac cacgtaagag tggcttttgg 5340

catgaaagga agaaaaatcc aagctacaca cacagcctct gccatctcgt caagatgcag 5400

tgataagggc gcacactgtt gggtgtcatg tcacatgtgc tgcccagagg caggaaggac 5460

agtgggtcaa gagatgaggt ctatttgtca tcctgatctt ggtttcaatg aaagctttgc 5520

acaaagatat ccaagcctcc agattttgca agaatctcgt ggaaatgtgc aagggtttaa 5580

aaattggggt gggcagaggg gcgcattggg cagaaaagaa atggatactg agacaacaca 5640

cagctgcgaa gctgaaggca tgatacctgg tgcatcactg cgaagtttcc tgtggggggg 5700

gccctctgaa acagagagag aatccccgga ggttaccatc agcaaacaag aaggagcccg 5760

gttaagtata ttcttaattg ttaaagacat ttgcttcgat ttaataatta aaggcactct 5820

tttttatttt gagatagtct ctgtcaccca ggctggagtg caggggtgcg atcttggctc 5880

actgcaacct ctacgtcctg gattcaagtg atccccttgt ctcagcctcc cgagtagctg 5940

ggattacagg caggccccac gtgcttggct aatttttgta tttttagtac agatggggtt 6000

gcactatgtt ggtcaggctt gtctccaact cctgatgtca atgatcttcc tgccttggcc 6060

tcccaaggtg ctgggatttg ggattacagg catgaaccac cgcacctggc attaaaagca 6120

cccttaagtc aacgttgcag tgaatcacct gacgccaaga gcatttggtg aggctggtag 6180

atgaaaggat aagcagtttt ttgatactct gtcacccagg atggagtgca ctggcatgat 6240

ctatctctgc tcactgcaac ctccacctcc cgggttcaag cgattctcct gcctcagcct 6300

cccgagtagc tgggattaca ggcatgcacc accgtggctg gctaattttc atatttttag 6360

tagagacgga gtttcaccat gttggccatg ctggtctcga actcctgacc tcaagtgatc 6420
cacccacctc ggcctcccaa agagctggga ttacaggtgt gagccactgc acccggctca 6480
gttttttaaa tggaaaggaa aatgtctgtc ctttagagct acttttaaga acctcctata 6540
aatcccatga ttgcatactg gttcagggaa gtaaaagcat ttgagcaagc tagtttgaga 6600
aatcagtgtt ggaacagaag ccaagaagtc ccaggcaagg aacctgcagc cgcctgtccc 6660
ttggtttatg attacttgct cttcattagc tttcgggcct ctagcaagag aggagaggtg 6720
gaggccttgt acagctcttg ccggaggtaa ctggcagtgt agccagtggt aggtaagtgg 6780
gtctggcttt agagagggct tgtgtcgaga tgcgtctggc tgatagaact tggtgaaact 6840
cgcactacag ttccttcttg gctgaggagc caggcatgct taccaacctc cctcagggct 6900
ctagtgcctg attttatgtt tttgtgcacc caaaagatgc agaaggcagg acagaccttc 6960
cacctgcatt atgtgtgtat tcgtgtcgga actggcaaaa atggtctttt tgttccactt 7020
tgcccttggc tgcccaagaa ggctcaagtg tctggggcag aacccacagg aggtgtccag 7080
tgtgtgctaa agaatcactg ggccggccgg gcatggtggc tcacgcctgt aaccccagca 7140
ctttgggaga ccgaggcagg tggatcacga ggtcaggaga tcgagaccat cccagccagt 7200
atggtgaaac cttgccacta ctaaaaatac aaaaattagc caggtgtggt ggcacgcacc 7260
tgtaatctca gctacttggg aggctgaggc aggagaatcg cttgaacctg ggaggcaaag 7320
gttgcagtga gccgagatcg cgccactgca ctccagcctg gcaacagagc tagactccgt 7380
ctaaaaataa ataataaaaa gaaaagaaat cactgggcct gagaaaggga agcagagaca 7440
gcagtggggt agggagtcag agtggctgta tccccatcgc tcagcccagc cactcaggag 7500
aggcaggggg actggttgcc agcagagcac atctgagggt ttttgttttt tgtttttttt 7560
ttggagacag agtcttgctc tgaaggctgg agtatagagg cttgatcttg gctcactgca 7620
ggctcctcct cctgggttgc aaaggagtgt cctgtctcag cctcacgagt agctgggatt 7680
acaggcatct gccaccacgc ctagctgatt tttgtgtttt tagtagagat gaagtttcac 7740
catgttggcc aggctggtct cgaactcctg acctcaggtg atcagcccac ctcagcctcc 7800
caaagtgctg ggattacagg cgtgagccac cgcgctttgc cccaggagtc ttcacagtga 7860
gaacagctct cctccactga cccctcaacc ctgggagatg cttccaggcc cctgctgtac 7920
tttgggagcc taggcctggt ttctctccta tccatggggg accaacaagc gctcaccatg 7980
cagcgagtgg ggcttttccc tgaaggcttg gtgccctgcg ccagctgctc tcttcccctt 8040
ctcagagggg tttggacttt tccctgtctc acgcagtggt ccccaaactc tagaggtgat 8100
ttcagatggc ttccgggcat gccatccgct aactgaactg ggccctgaga aagtcatttc 8160
ctttccaact ctttttaaac aagtctaatt atgatgtcag agagagtcta tttggtgcta 8220

atggctgttg tcactccctt tttagtggag aagtaggctt caggtcttgg tgatcaagtt 8280

gtggccacaa tagctagata ttgaacttga tactattttt atcatattac tttcatcgta 8340

accagttgat agaggtgatg ctggtttaaa aatatagagg aggtatgttt atttgccgtt 8400

atattcatgt atgattaaaa ctgttaaaag atcaagtcat tggtattaaa ggtagtaaga 8460

ggctcagcac atggctgtgg tggtatgaga atgaagcggg gaaaatatct aggacccgag 8520

ggataaaggt tcactctgcc tggtgctgtc ctagacactg gagatttttt ttttgagacc 8580

gagttttggt ctgtcaccca ggctgaagtg cagtggtgcg atctcagctc actgtaacct 8640

gtgtctccca ggttcaagcg attctcctgc ctcagcctcc ctagtagctg ggattacagg 8700

cacgccccac catacccggc taatttttgt gtttttagta gagacggggt ttcaccatgt 8760

tggtcaggct ggccttgaac tcctgacctc aggtgatcag cctgcgttgg cctcccaaag 8820

tgctgggatt aaggggtgag ccactgcacc cagcctgggc aggattcttt agggaaagtt 8880

gaccagattc tcccatagat gggatgtggg gtataaagga gaccaaaacg ttaagaagga 8940

ccacgaggtt tgtgatgtga attgttggca acttcatcat tttagctgct gagctggaga 9000

gaggagccag tttgggggtg aagagtttca gtcaggactt ggggtgcagc atgtttggtg 9060

tgagctgcct attagacatc catgtgggaa cgggagggtg gtctgaggtt ctaggaagaa 9120

gctagggctt ctcagagatg aactgaggag tcattagata tacatggaat tctaaagcgc 9180

caagattagt tgagacctct cacctagcgc tggggagaga tgaaaacaac taaggccgtg 9240

cagtggtcca tgcctgtaat cccagcacgt ggggaggctg aggcgggtgg atcacaagat 9300

caggagttca agaccagcct gggcaacatg gtgcaacccc atctctacta aaaatataaa 9360

aataggccaa aaatagcttg aactgggagg cagaggttgc agtgagctga gatagtgcta 9420

ctgcactcca gcctgggcaa cagagcaaga ctctgcccaa aagagaacta agttgatacc 9480

ctggggcggt ctagtggcaa gcaggtttcg aggtgaggga tcagcagagg agaggaagag 9540

ggacaacccc acaggtcggg ggacaagcag gtgtggatgg tgtcggggaa gtccagggaa 9600

gcaagtgaat taggccagag gcatgatgga ctgcgtcacg tgctgctgcc ctgtcagggg 9660

acctctggcc attagtgtca cccatgggag gcttctggga gagtgatggg tgggggcgag 9720

gggaaccagc aactcttcac atacagtttt tttttttttg agaccgagtc tcactctgtc 9780

acccaggctg gactgcaacc tctctctccc ggttttaagc cattcttgtg cctcagcctc 9840

ctgagtagct ggaactatag gcatgcacca ccacacctgg ctaattttta tattttttgg 9900

tagagacagg gtttcggcat tcacatccag ttttgtagta agggtagcaa ggtagacatg 9960

gggctaagag gttattccat ttgctgatgg agcaaatgaa agagaaaacc ttgcaaaata 10020

aggatttttg gagctatgtg cttgagtgaa caagagggtc gggtccaatg gaggtgttgg 10080

cctcggttgg tgccagaggg ttcagcatag aaatggaaaa aaaatagaag tgtacagagg 10140

tgcacggagg taggatgatg aggcagtggg agcttgtgca cgtttcatga tcatttctgc 10200

ttttgagtta agtaggaagt aaggtcccca gctgagagag aatggggagg agcgatcata 10260

agttcaaaga aaaagtgaaa agcagcagag agtgagaaga tgagggaggg gtgtgggagg 10320

acccctgagc aaggcctgct ctgtgggaaa aggtggagag tgggttttgg gttttttgag 10380

acagggtctc actctgtcca tccagggtaa gtgcattggc tagatcatgg ctcactgcag 10440

ccttgacctc tggggctcca ctgatcgtcc cacctcagcc tcctgagtag ctgggatttt 10500

aggtttgcgc catcccacct ggctgatttt tgtttgtttg ttgtagagat ggggtcttgc 10560

tgctcttgaa ctcctgagct caaagcgatt cacctgcctt ggcctcccaa agtgctggga 10620

ttataggtgt gagtcccgtg cccagccaag cttgagtttc atttgggttg tgctttcacc 10680

aaatgattgc agcaatgcaa gagggggaac cggatgttga ggatgcgcga cgggggttac 10740

tagacagctg gtttggagct gggtcagagg ctgatggcat taatggagtg aagaccccgt 10800

tggggacaga gccaaagaaa ggtggcacca ggcagacaat gggatgcctg agggtcaaat 10860

tgtgagggct cctaggagta ctggttggcc cggtagaagc agcaggatca cccagagggt 10920

caaggaaccg agaggctgcg tcgcccacct ggatgttgaa atcatggaga actgtgagga 10980

gtggtgtggg agacaggaag cccggagcta aaacctcctg ggagccaatc gggatggatg 11040

gacagggtgg tggaagcagc ccttgggagc aggggggacc cccaaggtac cagaggcatg 11100

ggagagaaaa cagcccccac tccagtgaca gcaggaattc tcagcggggt ggcagtcgta 11160

ccctcccatc tctagaggac ttctggcaac atctagagac atttttggtt gtcacattgc 11220

agggtggtgg gggactactg cagccggtgg gtagaggcca gaaatgtaag cacctgctac 11280

ttttcaggga tctttttcct tctgatgggg aatgtcggac agcccccata cccgagaata 11340

atgcagccca gatggcagca gtgctgaggg ggagaaagga ggagcagagc atgtcaggaa 11400

ggaggtgaag tggcgggggg tggtgttgca gcttcaatgg atgtcgtgaa gactcagtga 11460

tgcaatgaag gaggggctgg aggagacagt ggggatagcc tcttcctcct cctcagccgg 11520

aggaccggat gggagacggt cgatcgttaa aggatctaga aggttagctg ctaccaggca 11580

aggcagggcc cgggtctgat ctcagtgcct ggcccacact aagcttagtg attaaggaga 11640

agcaggaata aaccacagaa gggagtggcg gctgtgggca caaggggcat ttgcaggccg 11700

aaaggagccc gagcccccag tgcttttctc tggcctgaga gacggggtcc tcccgggcca 11760

cgggctcctc acctgagctt gctcttgagt gcgttcacct cgcggcccat ggcctcgttg 11820

ctctccgtgg cctcatccag ctcccgctgc agcttcctgc ggttggcgtt gatgcgctgg 11880

gactcctcct ctgcctcctc cagctgcctc ttgagctgct tgaccctggc attgcctttc 11940
tctgcctgtc gcggagagtt ggaggggtgg ttaggggagg ccggctgggg gctgggggct 12000
cgagggaggc tgggtggcag gggctacctg ctccttgtac tgctcggcca tcttgcgctc 12060
gtcctccacc tgcagcaaga tttccttcag cttcttgtct ttctgcttca gcgacttggt 12120
ggccgcctgt ttctctctgc aaacagcaag gaaaacaggt ggtttcagcg gagggtggca 12180
ccccttgtag ctggtgtgtc acctggaggt ggcatcttga gtgctttcct gagccccgta 12240
tctggactcc tctcaagaat cagtcagatg gtggaatagt attcagccat gaaaaggaat 12300
aaagtatcaa tgcatgccac agcatggatg agcctggaat tcttgccaag tgaaagaaga 12360
cacatagcag acaacatagt gtgtgattcc gttgatatca agtgtccaga atagataaat 12420
tcatagagac agaaagtaga ttatggttgc cagggactga agggagtggt tacagggcat 12480
ggggtttcta ttttttttgc ggggaggagg caaggtcttg ctgtgtgacc caggttggag 12540
tgcagtggtg ctccaatgcc tgctcttcac cctagttcat agttcactgc agccttgaac 12600
tcctggctca agcaatcctc ccacctcagc ctcccaagta gctgggacta caggcgtaca 12660
ccagcatgct cagactttta aaagtttttc atagaaacgg agtctctggc caggcgtggt 12720
ggctcatgcc tgtaatccca gtactctggg aggccgaggt gggcagatca cctgtggtca 12780
ggagttcgag accagcctgg ccaacatggt gaaaccccat ccctactaaa aaatacaatt 12840
acaagctggg tacagtggtg tgcacctgta gtcccagcta cttgggaggg tgaggcggag 12900
gttgcagtga gctgagatcg caccactgca caccagcctg ggaaacagag tgagactatg 12960
tctcaaaaaa ataaaaataa aaaagtcttg ctatgctgcc cagactggtc tcgaactcct 13020
ggactcaagt gctgctccca cctacctcag cctcccaaag tgccgggatt atgggcatga 13080
aataccacat atggcctggg gtttctgttt gagccgatga aaatgttctc aaaatgactg 13140
tggtgatcgt ttttacatgt ttgtgaatat atgaatagcc cttgaattat acactttaaa 13200
tgggtgcatt gcagggcgtg cgaattactg aaaaagctac tagaagagaa tgggtgagaa 13260
tccttagaat ccaatggtaa gaaatgtgct gtgatcatat gcctcattcg acccataatt 13320
aagaaggctt ccagtgtgtg taccaaatgg gctgccggtt ggacttttgt tttttggggt 13380
tttttttgtg tgtgtgtatg tgtgtggaag ggtcccactg tgtcacccag gctgcagtgc 13440
agtggcacaa tctcggctca ctgcaacctc cacgtgtcac gttggagtga ttctcctgcc 13500
tcagtctcct gagtagctga gataataggc atgtgccacc acactcggct aatttttggt 13560
acagacaggg tttcaccatg ttggccaggc cggtctcaaa ctcctgacct caagtgatcc 13620
acctgcctca gcctcccaaa ttgctgggat tacaggcgtg agccaccacg tccagcctgg 13680

ttggattttt gatctgtcag catgctatcc ctgcagaggc tgggaaaagt catatgtact 13740

gctgggaagg caggttaatg tcaggaggac catggagatg ctaagagcag ctcacacttt 13800

aggtgcttgc cctaggccag gaaccagcag ggcactttgc tgtgtttacg ttcagttctc 13860

acaacatacc tgcactgtag gcatcacaga gcttgcttct tacaagccag aactgatgct 13920

ggaagaggtt ccctgacttc actatgactc ctgctgtcca tcacccccct gcaaactggg 13980

ttcggaactc cacacccgca tacctggcct cctgctcgac ctgctcctcc agctgtgcaa 14040

tcttggcctc cagcgccgcg atggtggact tgaacttgga cttgacggcc ccctccatct 14100

cgtggagctt gctccggagc tccttgttct gccgctcgag ctgctgccgg gcactctcat 14160

tcttctgggc cgtgctgcgc tctgtggcca gctcgttgct gagctgctcg gcctggggag 14220

gagagtgaag gccatgaggc ggactcaggg aagcccaaga gagcgcactg agaccatgcc 14280

tcttcctgcc ttgtttggcc tctgcacgag tcccttgatc ccgggcaccc tgtcctctcc 14340

ttcatcctgt aaaactccac tcaagagctt cttccaggcc gggcgtggtg gcgcacgcct 14400

gtaatcccag cacttttgga agcagaggca ggtaaatcac ttgagctcag gagttcgaga 14460

cctgcctggc caacatggtg aaaccccgtc tctattaaaa atacaaaaat tagccgggca 14520

gtggtggcac gtgcctgtag tcccagctac ttgggaggtt gaagcaggag aatcccttga 14580

acttgggagg cagaggttgc agtgagccaa gattgcgcca ctgcactcca gcctgggcca 14640

cagagagacc ctgtctccaa aaaaaagagg tgcttccaca agtgtcagtg ccacccaggc 14700

tgagcgagtg acttgtccct tgctttctct gtcctggagt cgcctggaga atgatgcctg 14760

ttcaccctac accacaaggg gctgcaggtt actggatggt ccctagtgcc caccatggaa 14820

ctagtgctca gtgacatcac caagggctca ctggataagg tcagagcttc agctagggga 14880

aaacgcaatg aaagagcatc ccaaggcccg gactccaggg atggccgtga ggtacgggga 14940

gccagccacg ccgtggctgg aaaatgagac actgcagcgt ggagaggagt attctgaaga 15000

cagcagcctg ggcactggtg taagggccct ggatctctac tctcaggccc caccaccctc 15060

ttgtccctca atccagggcc tgcacacagg aagccgccac gcgtgtgttg actggtgcag 15120

gatcctgctg caggagagac agtaggcagc gtgactgtgg tgtccaggcg gccctcacct 15180

gctgtgtggc tttgcggacc cggtcgctca tggcctccat gttgccctgc tcctcctcca 15240

gctcctcctc cagctgggcg atccgggcct ccaggcggcg cttctcgtcc tggagtgcgt 15300

tcctggggga agggcggcca tggtgggggc ctctaccctc cccgccttta aaagatgccc 15360

cctccttgga gtcatgcctc aggggtattg ccctcatcat ctaatgggtg aaactgaggc 15420

ttggagaaga ttagaagact catctgtagt tacacagcca ggaagtggac agccgggact 15480

caggccgggt ccgtgtcagc aaagctggga ttgggatggg gaccagcaca ctcctccctg 15540

actcttcctg gcctcccctt ctccccacac agctacttat tgggaatgaa tgaaagcagc 15600
cacaccccca aacaggcatg aaagcgctga cggaaaacct aaccaccatg ggtctgtccc 15660
caatctcaga ggacgcttcg tcagcagcag cattgaaatg ggggtccagg gccaggcacg 15720
gtggctcaca cctgtaatca cagcactttg ggaggccaag gtaggaggat cacctaaggt 15780
caggagttca agactagcct ggccaacatg gtgaaacccc acctctacta aaaatataaa 15840
aattagccag gcatggtggt acacacctgt agtctcagct actcagaagg ctgaggcagg 15900
agaattgctt gaacctggga ggtggaggtt gcagtgagcc aagattgtgc cactgccctc 15960
cagcctgggt gacagaatga aactctgtct cgaaaaaatt taaaaaataa aatgggggtc 16020
gaggatgctg cctgtccccc catcctctgc ttcagagccc tcttcctcca ttcagtttcc 16080
taccttcccg acaggctact ggccagctcc tctgccagtt cctccttctc gaggtccgct 16140
tgtttgcgag ccctctcagc ggcggcgagg tcctaggtgg gagggaggaa ggctgttgtc 16200
tgccagggaa aggccaagcc ccaccaagag tccaccctga caactcagcc acccttgaaa 16260
gtacatgttc ttcctctgag ctcaggggag gccccgtgaa tacatagagg agggaagcgt 16320
gtgtctttct agacaggtgg accccagagg aggacgaaat gaaatctggg aatgcacaga 16380
ctggagctgc caaggggtgc taccgtgaca cccgcatctg aggctctcct agcaaggcga 16440
ggctttacct cttgtagctg catgaggtct gcttccaagc tcttggcttt cttctcattc 16500
tctttggctg tggcaaagat ctcatctctg gaggcacggg catcttccag ctctctttga 16560
aagtccttca tctgagcctg catgagtcaa cagggaggac aagctcagat gtccttactc 16620
ccccaagttc tgctgcccag ttcagctttg cacacccacc ccttggattt tctgcagttg 16680
accacaaaga agttcccatt gcacgagcat ggctctcagt tccaggtggc tggtggtgcg 16740
ctgggagcac cattctgggt gggctccaca gacctgcctg agaagctgag cccctgatgt 16800
gatctgaggg cagtggccaa atacctaata atgctgccct acccagggca ggagacagcc 16860
accaaatcct gaatggttcc agaaaaaccc cagctgaacc cacaccaatg gcaggtgcag 16920
gcttgcttcc tggagcccgc tctgctgact tcggtggcct gaggggaact gtgccctccc 16980
tccacccatg ccccaagctc ctagtgtcac ccacctgcag tttgcgtagc tgcttgatgg 17040
cttcctccct ccccttgatg gcagagtcgg cctgaagctc caggtctttc aggtcccctt 17100
ccagcttctt ctttgctgca gctgccaggg cacgttgctt tcgctcgtct tccagttccg 17160
tctcatactc gtgaagctgg gcgaggaata gagatgtgtg ctgccccact tgcccctggg 17220
aggtcctttg gctcacctag gcagcacatc actgcacccc ttccccagca cagccccctt 17280
gtgaggtggg catctcatcc ccagttgcag atgagaaaac ggaatcacgc cgggcgtggg 17340

tggctcatgt ctgtaatccc agcactttgg gaggccaaca ggagaatgga tcacttgagg 17400

ccaggagttc aagaccagcc tggccaacat ggtgaaacct tgtctccact gaaaaaaaaa 17460

aaaaaattag ctaggtatgg tggcatgcgc ctgtaatcca agctactcgg gaggctgagg 17520

caggaggtgg aggttgcagt gagctgagat tacgccactg cactccagcc tgggcgacag 17580

agcgagactc tgtttcaaaa aaaaataaag aaaacgaagt ttccacacca accatgagag 17640

tggtgatagg aatgaaaaag gccacccgac ctccctctgc tggcctcccc ggcagcacgc 17700

acctgtctct gcagttgcct cctcttctcc tcattctgct cgtcccgggc ttggagatcc 17760

ctttcgaact ggcccttgag cgcctgcatg ttgacttcca gccgcagttt ggcgtcctcc 17820

gtggcttgca gctcgtcctc cagctcttcc agctgcgtct tcatctcctc catctgggtc 17880

tccagggccc gcttggactt ctccagctca tggacctgcc ggcagagcgg gcagccccat 17940

tctatgaggc tcaacttcat gaagacgatt gagaaaccca ccgtgagcgg cacctcagga 18000

gatcagggag gtggctttgg cctcccacag gatgcatggc cgggactcaa gatgacccct 18060

gagagttcag accccagcct tatcctcgga ccccccaact cagacccatc ctcgactgcc 18120

attctcagcc cctcccagcc cctgcaccag tccaaaaacc tccttccatt tccgatgata 18180

gttcgctatg aaaaaggcca ggagctagcc tcgcatggac tggtgaatag cacagagggt 18240

gggcaggcga aacatggacg agaaaaacca cccagagcca cttacgttct tgcccacgtc 18300

atccttggag ctgaccaggt cttccatttc ggctttgagc attttgttgg tccgctcgag 18360

ttcctctttg gcttccaagg cctcttcaag ggcccgagcc agggacaggg ccttggtttc 18420

cttctccctg gcttctgcct cagctctgtc cctctcatcc gcgtatttgg aagagatgtt 18480

tttctcctcg gctaacaact acaacacaag acccagaggt gacttctagg catatccggg 18540

gtcagcgtca ctgaattgta aataccgggg gaagccctgt gtcctgctga atgtattgag 18600

gtgcaggtgt aagcagtgta ggttagctat gggagtaatt tatataatca tctggcgttc 18660

tgacttctac atcaacctta tgcagagcca gaaatcagct gacttgtttt tttttgttgg 18720

tttgtgtgtt tgtttgtttt agatggagtc tcactttgtc acccaggctg gagtgcaatg 18780

gtgcagtctt ggttcactgc aacctccgcc tcgtgggttc aagcgattct cctgcctcag 18840

cctccagagt agttgggatt acaggtgccc accaccacac ctggctaatt tttgtatttt 18900

aagtagagat gtggttttgc catgttgacc aggctggtct cgaactcctg accctcaagt 18960

gagcctcctg cctcggcctc ccaaagcacg gagattacag gtgtgagcca ccacacctgg 19020

ccaaatcctt gtgcttcgaa accactagac ctgcccacgt tgcttagagc acactgaggt 19080

caaagtgaga tttaaagctg ccagaagcta gttattagtt tgtttcatgg ccacaagtag 19140

ctattaatat cttacaaaag tatcctaaga gcctcagtag ggaaatggta agcatgggtg 19200

gccctgcaca catatctctc tgcttggaga agcaactcag agaacattcc atgctcacgt 19260

taatctcatc ttcagagaga acagagcaga ttataaattg taacatccaa aagccaaact 19320

tccagtattc agcaaagaca gggacctcta aatggcacag tcaacacaaa aatcccacac 19380

tatgtgtgcc actgcactac actctatgtg acaggaaaat aaaagcaacc tcaggccttc 19440

aaccagataa cgaagcattg aagatgagct gacagcccag aggagaggta gctgatggga 19500

agaggagaaa ctgggatgtt atctagaatc ctgacactag gaggtcagtc agggaggggt 19560

agggaacctg caggcatctg gcatttagcc tcactgtaca atcctcattt tatttatttt 19620

tgagacaggg ttttcactcc atcactcagg ctggggtgta gtggcgcaat cttggctcac 19680

tgcaacctcc accccaccct gggttcaagc gattctcctg cctcagcttc cagagtagca 19740

gggattacag gcgtgcgcca ccacgcccgg ctaattttgg tatttttagt agagacaaag 19800

ttgcacgatg ttggccaggc tggtctcaaa cccctgacct caagtgatcc acctgcctca 19860

gcctcccaaa gtgctaggat tacaggcgtg agccaccgtg cctggcctct caaactgatt 19920

tttagaataa ccctgagtca cttgtaaact aaatcatatt tactaaataa taatgtatct 19980

taaaagttgg tatttagggg tttgggtcat acaggggtct ccatttatcg tacaattaag 20040

atttgtgaat ctcatcacat gtaagtttta ccccaaaaga agcaaaattg tgtttaaaaa 20100

taaactctaa ttaatatgca atgttgaggt gtggaagggg aaatgtactg acatctgtaa 20160

tttgctctga aatgtaccca agagataagg catgatagat agaattagtc tttataaata 20220

aatatgtggg ccgggcatag tggctcatac ctataatccc agcactttgg gaggccaagg 20280

caggcagatc atgtgaggcc aggagttgga gaccagcctg gacaacatgg caaaacccca 20340

tctctactaa aaatacaaaa attagccaga catagtggtg tgctcctata atcccggcta 20400

catgaaaggc tgaagcagga gaatcgcttg aacccagtgg tggaggttgc ggggagctga 20460

gatcacacca ctgcactcca gcctgggtga cagagctaga tcctgtctca aaataagtat 20520

ctgataaagt tcatataata cagtgtcaat gactgaatcc aggtggtggc cttaaaggtg 20580

ttcaccgtac aattctgtca actttgctgt gtacttggaa ttttacagaa tgaaatctta 20640

gggaagagtg gtattaaaag tatagaaact gaattacttg gaatactact caataggtat 20700

tcagtaaata catttagtga ataaataagc acctcattcc ttcttcaggc tagaaactaa 20760

ggcatcacca tgtgtgcagg tgaccctcat ggtgctggta cccagaaaag tcacagtcat 20820

caaggcttct ttgccatcct tactgtgacc tggtagcgtt aatgctccat ggtcgcccaa 20880

gacaagataa gacagcctcc catggctccc cacagagtgg agaggggatg caggcacagg 20940

ccagagccac gcgtcatact ctgcagagct gattccccaa cccagcgtcc atggccagag 21000

tgggggacac cccacgccct ctacctgatc aaatttcctc tgcttctttt ccaggttgga 21060

cacgagttgc cgctggttgt ccaaatcaac aaccaggtcg tccagctcct gctgaagcct 21120

gttcttggtc ttttccagtt tatcataagc ggccgccttc tcctcgtact gctgggtgag 21180

gttctcgatc tccttctgga acctcttctt cccctcttcc agagcttcca cggtgctggc 21240

aaagtcctgc agcttcttct tcgagtcgga gagctacaag gacagcgtcc agggtagggt 21300

gagaggggga ccatgagtgg cccctgtccc tggccccaca gactctgaga agcgaagacc 21360

atgtctcctc gttggagaaa cccaatagca ggggaagctg gggggtcaag caccatcgca 21420

ccaacactcc accgcgatct gcctgcgggg gatctcagcg cagagaagtt gagaggaccc 21480

atgaaggaag caaggacacg gggcaggcac ctggatgttg agagtggaga tgtggcgctc 21540

caggttctgc ttggcctcca tctcctcgtc cagctggtct tgcaggctgt tccgctcctc 21600

ctccagctgg cgcagcttcg tagacacgtt gagcttctgc cgggtttctt cttgaagcag 21660

ctcctgcaaa agggatgcaa agaggtccca gggacctgcc ccgaggaagg ccacccccca 21720

ggtcccctgg atgatgtggc aggacactca cctgggtgtc ctggagctgg gaactgaggg 21780

acgccacgtc cttggccagc ttaatggcct tcccctcggc ctcgttaagc atccctgtga 21840

cgctctcaac ttcattctaa gggtgccaag agactggtta gtcaaagcct ctagaagggg 21900

atcctcgttg aaaggagccc tttttactca aaacacatgg gctagtactt gaggtgttca 21960

ctgattgaga aaatacccgt gaggtatggg actctgataa aaaaaaaaaa aaacacacac 22020

acacacaaaa aaaacagaat ctgtggcttg aagggaactc cgtcacctat gagttgggac 22080

cctggcccta gactctgtgg ttctaagaac ttatttgagc cccaatggta ttgactggga 22140

cctgatccca ctaaatggat cctagatccc tgccaaggtt ggtagagaca aagcagcagg 22200

tctgagagtc cagacgaggt gctctggctg gtccactctc taaggctgga gaagggagac 22260

caggatggta cttgaacgtc ccagggatgc tgtcccatcc cttccttcct cactcctact 22320

ctttgaccct gatggccaaa gccagagacg caggccctaa aggtaaaaac gtcctctctg 22380

tattctctgg cttttactcc ctagtgtctc tgcataagtc cctttgaggc tgttagccta 22440

cccctccatc tcttccattg acaaggagga tatgaatgat cttgacactg cttatatgag 22500

ggcggcaaaa gccctgctct caactgcatg tgagaaaaaa acatctcact taattcttcc 22560

ctcgcccctt ggtccctggc tgtggacatg ttaaacattt gtgaaaactt tggccacgtc 22620

cccatgagtg gcaaggcagg gtaaatggct atgccaagtg aaagaagacc aaagacaaaa 22680

agaccatgtc attcagcagc ttaaaacccc tcaacagctt cacattgccc agagtaagga 22740

tcaacataca tgtaataggt tctcaaaagc cctacatcat ctgggtacaa gctccccctc 22800

caaccccact ctgtactatc tccccctacc cccaacccca gctgggcact ccagctctac 22860

tggctgtatg tctctctcct aatttttcta cttaccacag ggcctttgca cacgctgttc 22920
cctctgcctg gtagacttat ccatgctcct tagggaagcc tttcgtggct cctcctcccc 22980
caggttaggc cccctggtta tttacacttg ggcatcatgt gccttccctt tgctgcatca 23040
tttgacattc atttgtgtga ttattcatgt ctttcccttc ccttcccttc cccttactcg 23100
gctggagtcc tactggggca gcgacagtgt ctcttcttcc ttgctgttgg atctcaggat 23160
taagcacagt gcctggcata cagcaggtgc tcaataaata cttatcaaat tggaaaagga 23220
agggtttttt ttcttttttt ttttttttcc tgagacagag ttttgccctt ttagcccagg 23280
ctggagtaca atggtgcggt ctccgcacac tgcaacctct gcctcctggg ttcaagctat 23340
tctcctgcct cagcctcccg agtagatggg actacaggtg cccacgacca cgcctggcta 23400
atttttgtat ttttagtaga gacttttcac catgttggcc agactggtct caaacccctg 23460
acctcaggtg atccacccac ctctgtctcc caaagtgctg ggattacagg catgagccac 23520
cgtgcctggc cagaaggttt ttttaatatt taattgaaca gggagatcat cgcctctcct 23580
ccaggaacgc aactagagga aaggacttgc cttgcagcaa gagagacctc agcgagccgg 23640
gaagaggctc ctccccacag aactgggcac cacccagcac tgcccaccac accaccgcgc 23700
cacctcctca cctgcagctt gtggactttg tcattgagct ccgcccgggc ccgctcccca 23760
tcgctgcact tggactgcag ctcctgcacc tgcgcctcca gcttcttctt cttatgttcc 23820
acctcctgct tggcctggcc caggacccgc agctccccgg ccaggtctgc gttctctttc 23880
tccagcgtct gcttattctt gtctaggttc gccttggcct ggcgaaggaa gcagagggga 23940
gggataacag ggaggctgtg gccgggagaa cgtttcaggc cctgcccttt cctccctcag 24000
agaggtccag gaaggcacac aacagggggc acacaatcac cagtaagaga tttggggttt 24060
ttttgttgtt attttttttt tgagatggag tcttaactct gttacccagg ctggagtgcc 24120
gtggcacaat cttggctcac tgcaacctcc gcctcccagg ttcaaactat tctcctgcct 24180
cagctgccca agtagctggg attacaggcg cccgccacca tgcccgacta atttttgtat 24240
ttttagtaga gacgaggttt caccatgtta gccaggctgc tctcgaactc ctgacctcaa 24300
gtgatccacc catcttggcc tccctaagtg ctgggattac aggcgtgagc tgccgcaccc 24360
agctgatatt ttggattcta ttttcctaaa gctcatgggt tgcaatctgg aatcccacaa 24420
gcggtatcca acctgctgac atgtcccctt tgaccctttc actactttta aaaaaatgag 24480
ttgccgacat tttaataaat gtcacataaa aatctggatt cccagcttct ttttcaaaaa 24540
actttaatgt ttgaaggcat gccatggcag ctcatacttg tcatccccag cactttggga 24600
agcaggcaga aggatcgctt aaggccagga gtacaagacc agcctgggca atgtagtgag 24660

acccccatct ctactaaaat aaaacattag ctgggtgtgg ggggcacaca ccagcaatcc 24720

cagctactca ggaggcggat ggcaggcttg cttgagccca ggaattggag gctgcagtga 24780

gctatgatca caccactgca ctccagcctg ggcaacaaag tgagaccctg tttcagaaat 24840

aagtagcttt gggaggccaa ggcaggtgga tcacttgagg tgaggagttc gagaccagcc 24900

tggccaacat ggcgaaactg catctctact aaaaatggaa aaattagccg ggcatggtgg 24960

cgcacgcctg tagtcccagc tactggggtg gctaaggcac aagtattgct tgaacctggg 25020

aggtggaggt tgcagtgaac caagatcatg ccactgcatt ccagcctggg taacagagtg 25080

agactccatc tcaatcaatc aatcaataac taacattcta tgtggtgact gtggtggtgg 25140

tagttacaca atacacattt gttaaaatta atggagcagt aaaatggata aattttacta 25200

catatgcctc agtaatccta aacttaaaaa caaaaacaaa aaaccaacaa caacgaaaga 25260

tctggaaaca gagggtcttg ctttccttca aggcaacaat ctgccacatc taagtagcgg 25320

ctggctctgc aggtggggca gtcacctttc accgcccccg tccttcacag gtgtcgtgtg 25380

attgacactg ctacttctgt cctagttaag ggaaacttat tttatggaac cagctcctgt 25440

aggcatgtga gttgccctac aggaaaaaaa aagtgtgttg gggccaggcg cggtggctca 25500

ggcctagaat cccagcactt tgggaggcct atgcaggcag gtcacttgag gtcaggagtt 25560

tgagaccagc ctggccaaca tggtgaaacc ccgtctctac taaaaatata aaaattagcc 25620

gggcatggtg gcagtcacct gtaatcccag ctgctgagga ggctgaagca ggagaattgc 25680

ctgaacccag gaggcggggg ttgcagtgag ccgagatccc gccagtgcac tccagcctgg 25740

gcgacagagt gagactttgt ctcaaaaaaa aaagcgtgtt gggcaaaagt aggacgcggt 25800

gaggggcttc tttccaggtt gtagagaagg ggaaagagaa gcgcaagtat cccgagagga 25860

cgagacccac cactcagtct tcccatagtc cctgggaggt gctggacgtt gaccggtcag 25920

ccctggatgc ccgtcctccc tccaagacat gactaagagc tgccagacct cagatgggga 25980

cagggagtct cttggtggta acaaagcagt gaagcgggga ccctagggga catgggctcc 26040

cagaaacctt agcccagcct aagaccccag aactggggcc aaaccgcacg agggcctggg 26100

gagcccaggt aggagctgat gttaaacgtt tcccccacaa gcagatggaa ccaggtctct 26160

acatcagaat tcaagttgtt agaggcaaag gcctctgttt ccctgcacct gagatgggca 26220

gaggtttgca cctctacctg ctttgtgcgg gtcagtaatg gacatgccgt ctgctggtgg 26280

tcttaataac tgcgtcttcc caaagaggcc tggctgggaa tgggcttggc ttctagctcc 26340

tgttcacgtc taatgaaccc caagcatggg gagggtcagt gattctgtct ggggcctcac 26400

tcttccaacc ataatttttt tttttttttt ttgaaactga gtctcactct gttgcccagg 26460

ctggggtgca gtggcacaat cttggctcac tgcaaccttt acctcctggg ttcaagtgat 26520

tttcatgtct cagcctccca agtagctggg attacaggcg accgccacca cgtccagcta 26580

atttttgtat ttttagtaga gacggggttt tgccatgttg gccagactgg tctcaaactc 26640

ctgacctcag gtgatctgcc tgcctcgggc tcccaaagtg ctgggattac aggcgtgagc 26700

cactgcaccc agcctaattt tatgtatttt tagtagagat ggggtttcac tatgttggtc 26760

aggctagtct tgaactcctg acttggtgat ccgcctatct cagcctccca aagtgctggg 26820

attataagca tgagccaccg cgcccggcct ttgggggcag tttctaatgg tttagcacta 26880

tccctttggt gctgccctca tgagatctgg ttgtttaaaa gtgtctggca gctccccatt 26940

ctctctcttc tgcctgctct ggtcatgtaa aacatgcctg cctccccgtt gccttccacc 27000

atgatggtaa gtttcctgag gcctccccag aagcagaagc tgctatgctt tctgtacagc 27060

ctgcagaacc gtgagccaat taaacctctc ttctttagaa attacccaat ctcagggtgg 27120

tgcagtggct cacacctgtc atctcagcac tttgggaggc caaggcaggt ggatcacctg 27180

aggtcaggag ttcgagatca gcctggccaa catggcaaaa ccccatctct actaaaaaaa 27240

aaaaaaaaaa aaaaaattag ccgggcatgg tggtgtgtgc ctgtagtccc agctacttgg 27300

gaggctgagg caggagaatc atttgaaccc aggaggtgga ggttgcagtg agccaagatc 27360

acgccactga actccagcct gagtgacaga gtaagactcc actgctcaaa aaataaacaa 27420

ataatccaat cttgggtatt tctgtagagc agtgcaacaa cggaccaata cactagtgat 27480

agaaccaaat gcaaattccc caaaattttt aggcaatgct gcacccactt aatctagcag 27540

aaccaaaagc ttccactgaa ggggatgtgg aggagctgca acgctgaggg taatgtaact 27600

caggctttca ggaaaacaat ctggccgaag gtcgcaagct aaaaaactta ccctggggaa 27660

tgaagcacag gcactacccc aaaggagaga agagcaccca agattcagca ttcggtactg 27720

ctatttacca gctcccactc ccatacaccg caaagagttc caacaacggg agagtgattt 27780

gacctagtca ttctcaaatt atgccggaga gagctgttct gccattgaaa tcaaataatg 27840

acaagtttcc tccagtcttt ttttttttta caaaagaaaa atcgctatgt tgcccaggcg 27900

atctgaaagt cctgggctca agcaatcctc cctccttggc ctcccaaagt gttaggatta 27960

cgggaggcat gagccgccat gcctggcctt cctccagtct gcagagaaat taaagtggat 28020

agaatgatcc cagttttaag tttcatttaa atctccccga aagtgttgtt acacctttgc 28080

ttatccctga agggcagtat attgataggc cccacccatc ctataggcct tcagtcctgt 28140

agaggccaga aagtggggtc taggaccaga ctgtttgggt gcaaatcatg gctctgtcac 28200

ttaccagcag tgtgaccttg ggtaagtctc ttcacctcta ggcacctagg acctcagttt 28260

ctttgcttgt aaaatgagtt taatagtaca cgtctcacag ggtggttgtg aagattaatt 28320

gacatactgt atgagaagca tttctattca ttcttttttt tttttctttt tttgagatgg 28380
cggctcactc tgtcgcccag actggagtgc agtagcacga tctgagctca ctgcaacctc 28440
ttcctcctgg gtccaagtga ttgtcctgcc tcagcctccc gagtagctaa gattacaggc 28500
acatgccacc acacccagct aattctttat atttttggta gagatggggt ttcaccatgt 28560
tggccaggct ggtctcgaac tcctgagctc aagtgatttg cccgccttgc ctcccaaatt 28620
gctgggatta cagacatggt gagccaccgt gcccagccta tttttattgt ttgattaagg 28680
tctcactgtg tcgtccaggc tagagtgcag tggcatgaca gggctcactg cagcctcaac 28740
ctcccaggct caaacgatcc tcccacctca gcctcccaaa tagctgggac cacaggaatg 28800
cactaccagg cctggccaat attttatttt attttatttt tttgagatgg aatcctgctc 28860
tgttgctcag gctggagtgc aatggcatga tctctgctca ctgcaacctc tgcctcctgg 28920
gttcaagtga ttctcctgcc tcagcctccc gagtagctgg gattacaggc atgcaccacc 28980
gcgcccggct aattttttgt atttgtagta gagatagggt ttcgccatgt tggccaggct 29040
ggtctcgaac tcctgacctc aggtgatcca cccagtttgg cctcccaaag tgctgggatt 29100
acaggcttga gccaccacgc ctggcctact tttttaaaat tttagtagaa acaaggtctc 29160
actatgttgc ccaggctggt ctccaactcc tgagctcaag cgatcctccc gcctcagcct 29220
ccccaagtac tgggattaca ggcgtgagcc acctcaccca gcctgtatgg gaagcatttt 29280
gtaaataaat ataagctgct atcatcatca ttagaatctt atgtaacttg ttttttgtct 29340
ttatgcagac cctgacctgt agtaatttga ggctgctgat gtcactctta tgtgtcatca 29400
ccaaaaagca tcaccaaaaa gcattaccct cttgaactgc tcaagctgct ctgtgagctc 29460
ctccaccgcc tgtgcgtgtt tctgcctcat ctcctggacc tgagcctcat gggaccgcgt 29520
ctcttcatcc agggccttct tcagcaccgt cacctcctgc tccctcttgg cccttggtgg 29580
gaggaacaca gtgaatggca gttgggtgga gacagagggt catctcagtg ccgtgcaaaa 29640
gaatgagagc tcttccagga cccagagctg cagtcctcat caccacccct ctctagctgt 29700
gtgaccttgg gtcattcacc taacatctct ggacctaaaa tttcctcagc tgcaaaatgt 29760
ggagcatatc acctgcctag gacatctgta atgactggca aacaaataca caaagtaata 29820
gaattgaaag gatgtctgtg aactgcaaaa ttttgcttaa atattaatgt tggctcttat 29880
ttaatgagca gttactatat gccaggccct gagctaagtt ttttatatac atcatctaat 29940
cctcagagca acccaaagag gcaggtgtgt tattatttcc attttacaaa tgaggaaact 30000
aaggctaaga gagggtaagc agttttaccc atggtgtcac agccattgtg tgatggagcc 30060
agaattagaa gctaaacctt gaccttgagt ccatggtttt gtttgtttgt ttttgagatg 30120
gagtttcact cctgttgccc aggctggagt gcgacagcgc aatcttggcc ccctgcaacc 30180

tccacctccc gggttcaagc gattctcctg cctcggcctc ctaagtagct gggattacag 30240
gtgcccacca ccacgcccgg ctaattttat atttttagta gagatggggt ttcaacatgt 30300
tggtcaggct ggtttcgaac tcctgacctc aggtgatcca cccgcctcag cctcccaaag 30360
tgctgggatt acaggtgtga gccaccgcac tcagcctggt tttttgtttt gtttgtttgt 30420
ttttcttatc gagtcttgct ccgtcgtcca gactggagta cagtggaatg atgtcagctc 30480
actgcaacct ctgcctccca ggttcaaggg attctcctgc ctcagtctcc caagtagctg 30540
ggattaccga cgcccaccac cacgcccggc taatttttgt ctttttagta gagacaggtt 30600
ttcatgatgt tggccaggct ggtctcgaac tcctgacctc aggtgatctg tctgcttcag 30660
cctcccaaag tgctgggatt acaggcatga gccaccatac ctggcccatg cactgggctc 30720
ttctgtatca aaggccaagc ggcaagggag cagaacgctg tgctctatgt gcatgtgaca 30780
ctgcacaaaa cccaaagtgg gggaagtaaa ccccagggta tatggtgttg gcacagctag 30840
catagctata tgctatgata catgctatgc caacaccaca gattgcatca aaatacattt 30900
atgtgtttac tgaatgaatc aatgacctag tttcactaga gtcaggattc gtgatctggc 30960
aggcagtttg ttattgtttg ttcatttgaa gtgaatcttc aattgaaaac caatctgatc 31020
aattttattt ttaaaattta acacaaaagt gatacacaca catacttggg aattggagct 31080
tggggactga ctgatacaca cacagacagg aaggaatttc tcaagaatgt taactggtgt 31140
gactaagggt gatttttttg tttttgtttt ttttttgaga tggagtctca ctcactgtgt 31200
cacccaggct ggagtgcgat ggtgcgatct cagctcactg caacctctgc ctcccagatt 31260
caagtgactc tactgcctca gcctcctgag tagctgggat ttacaggcat gcgccaccac 31320
gcccaggtaa tttttggatt tatagtagag acagggtttc accatgttgg ccaggctggt 31380
cttgaactcc tgacctcaag tgatccacct gtctttggcc tcccaaagtg ctgagattat 31440
aggcgtgagc ccgaccctaa aggtgatttt tttttatgtc tgcttttcta tagttttcaa 31500
attgttctct gagtatatcc aagcttacca atgaggatgg ttttgaatgc agcctgacac 31560
agagtcataa actttcttaa aacagtatga gatgtttttt ccaatttgta aaattttttt 31620
agttgatcag ctattattag tgttagtgta tcttatgtgt ggcccaagac aaatcttttt 31680
cttccaatgt ggccaaggga agccaaaaga ttgtacatcc ctggtatata ctactcttct 31740
gatgagaaat aagatgatga gggtcgggtg cagtggctca cacatgtaat accagcagtt 31800
tgggaggcgg aagtgggtgg atcacttgag gccaggagtt caagtccagc ctgggcaaca 31860
tggcaaaact ccatctctac taaaaacaaa aaattagccg ggtgtggtgg cacatacctg 31920
taatcccagc tactcaggag gctgaggcat gagaatcgct taaatctcag aggtggaggt 31980

tgcagtgagc caagatggca caactgcact ccagcctagg caacaaagcc agactgcctc 32040
aaaaaaaaaa aaaaaagaaa gaaaaaaaag aaacagccaa ccatgcttct ataagttaaa 32100
gcaaaccgcg gccaggaagg taaatgcaca ggggctgatg cacgatttgc tttgggtttg 32160
tcccctcttc tgcccccatc ccattggtgc agtgggatag caggatggtg ggattgatgg 32220
gcccctcacc tgagctcctg ctgagtggct gtgctgtcca gtgtgtcttc cagctctgtc 32280
tttagggcct ccagctcctc gccgaggtct cgcttctgct tttcagcctt gttcctggcg 32340
gcccgctctg agtccaggtc ctcctggagg tctgagatgt ggccctccag ctcccggatc 32400
ttcttcaggg cattgttctt ctgagcgatt tcatcgtcaa gccttccagg gagagaccca 32460
gcagaatgaa cccccaggtc ccttggtttc ctctcttaca atgtggccaa aaacttttct 32520
gggaccagac agttatgttg cagagacatc aaacaccttc tatccatgtc cccaggtctg 32580
cttcttgaca tgccagacta tatgagatta acataggatg tgagtgccaa ggtctgacat 32640
caaaacaggt tgtacatgtg catgggtgtg tgtacgtgtg tatgcacgtg tgtgcatgtg 32700
tgtgcaagaa tgcaaagcat tgaccccaga ctggcaaggt acctggtcac agtagggact 32760
gtgtagatgc tttctgaatg ttggacagtt cttcttccct ggcaactgtc tgttctttct 32820
gcatttgctc agtgactgag tacccgcccc atgcacagca tttggccagc cactgcttgc 32880
gggggatatt cagactgggg ctctgcatct ggtggaggtg gtgaccagag atcaaggtgg 32940
gctgcaagaa gccctgttaa atggcagaat gctgcaggag aggcaagcac tttgatttta 33000
ctgtacttgt gaaagggcag caacaagaga gaaagtgaaa tagttgggtt ggggtgagac 33060
tgcagagggt ggatgggctc tgagggtgag gctaaggaaa ctggatttcc cctagtaagc 33120
cagtgaagtt aggtttttgt tttgttgtta ttattgtttt gagacagggt cttgctctgt 33180
tgcccaggct ggagtgcagt ggcatgatca tagctcactt tagcctcaac ctcctgggct 33240
caagtgatcc tcccatctca gcctcctgag tagctgggac tacaggcaca caccaccatg 33300
cctggctcat tttttaactt tttgtaggga cagggtcttg ctatgttgcc catgttggtc 33360
tcaaactgct ggcctcaagc aatcccccca cctcggcttc ccaaagtgct gagattacag 33420
gcgtgagcca ctgcatccag ccagcaaagt tttgtgagca ggttcctgcc ccagagagat 33480
gctttccctt catcaccgta gcactgtcaa catagcacaa attggccgac aaaagagcca 33540
ggccagggga tcaatcagaa agctttgagg cttaaatccc agctctgcct cttagaagca 33600
atgacattgc tcctgttgcg ttacccctag gaggctcagc ttcctcctca aagacaccgg 33660
cagtacacct ggccaggtgt ctgcaaaggt attgcttgaa aagaacttag caccgagtga 33720
gcaggtaaat aatcattcat agtggctgct gcagtattag cattagtgta aatccagcta 33780
agagccagga agagggaaaa accagaggaa atgggtgggg gacatttaga aggactggca 33840

gaatctgaag gttgctgctt gctgtgggtc ggcagaaaca aaggaggaga ttggtgttca 33900
caaaaaagag ggcccttaga gggaggggct ggggaaagga aggtcattcc cgccttagac 33960
aggggtgttt gagatgacgg tggaaattga ctcattgggc aatcggaaat tcagaagggg 34020
gtctagatga gaaaagcctg gatgtcgaag aggcagcagc aacaccatgg gagtaggtga 34080
gatcaactgc aaagacatga tgtgtttgca gggaagagaa gaaagtcgaa ggtgaggatc 34140
tgggggagtg aacagggtcg agaaggcttg tgggaggcct ggcctgggaa acgaagttca 34200
tgccaagggc ctggggccgc cttgaccaag acagccactg cgaatgagcg aatgagcagg 34260
ggcccagggg atacatggac acacagcaaa tgccccttgc cagccccgct acctggccag 34320
ggccgcctgc agctcctcct ccttcttggc cagctgcatc ttgagctctg cgatctgcgc 34380
ctggaggtca gcgatctgct cgtggaagtc gctggcatca ccctccagct tccgtttcag 34440
cttctccagc tcctgtcggc tcttctcttc cttctttagc cgcactgcaa aaaccaaggt 34500
gctcttcagg aaggggaggc cccagagaga tgcccggaaa tgagccttcc tgcccaggca 34560
ttttacccgg aggagatgaa cacatccccc aatcagtaac catcatagtc acggtctgga 34620
ccattatctc ccgatgaaca gcaccttata ttgtcataat attattatta gttgagatgg 34680
agttttgctc ttgttgccca ggctggagtg cagtggcgca atctcagctc actgcaacct 34740
ccgcctcccg ggttcaagca attctcctgc ctcagcctcc cgagtagctg ggattacagg 34800
cgcccgccac cacgcctggc taatttttgt attttgagac agggtttcac catgttggcc 34860
aggctggtct ccaactcttg acctcgtgat ccgcccacct cggcctccca aagtgctggg 34920
attacaggcg agagccaccg cacccagccc ttttatcata ttattagtgc atgtgatgac 34980
taagggtgat ttcgagaggt ggcatagagt tagttgtcac tcatgggatg atctgccatc 35040
ctcaaatttc ctttctgatt ttcctgcttt taaaaatacc cattcaagcc caggaatagt 35100
ggctcatgcc tataatccca gcactttggg aggccaaggt gggaggactg cttgggccca 35160
ggagtttgag actagactga gcatcatagt gaaaccccat ctctacaaaa taaaaaataa 35220
aaaaaaatta gctgggtgtg gtgatgcacg cctgtagtct cagctgctca ggaggctgag 35280
gcaggaggat cactggagct taggagtttc gggctgcagt gagccatgat cgcaccactg 35340
cactgcagcc tgggcaacag agcaagacct catctctaaa aaaaataata aaataaaata 35400
aaaataaatc tcttggtagc tggtttacct tccagttctg aaatcataga ttcatgcttg 35460
tttttcagct tggtaagatt cttggccttt tcttcctctt ctgcaagatt tgtcgttaag 35520
tcactaatcc tctcctcaag gagttttcgt tcctttttgg ggaaagagaa agagatagct 35580
ttaggatttt tcttttctct agaatctatg tttcactttt agtgatttcc atgtaaacag 35640

ttgaaagaaa aacccacatt ataacaaaat acaactagag ttttaaatgg taaagagaag 35700
tcccataaca atttccaaaa actggccatt tctcatctcg tgccagtttg tccctcatat 35760
tgtatggtca gctaaaggta ccctccttcc attcctgcca ctggccagct cttccctgcc 35820
ccagggcctt tgcacagtgg gccaaccagc tgagatgctc ttctactccc ctctaactgc 35880
tccttcatct ttaagtttct gcttaaatgt cacctcctcc aggaagtctt ctatgaatac 35940
ccttcctcta tcccccactg ctgtattctt tttttttttt tgagacagag tctcactctg 36000
tcgcccaggg tggagtgcag tgttgctatc tcagctcact gcaacctctg cctcccacgt 36060
tcaagtgatt ctcctgcctc agcctcccga gtagctggga ctacaggcgc ccgccaacac 36120
acccaactaa ttttttgtat ttttagtaga gatggggttt taccgtgttg gccaggctgg 36180
tctccaactc ctgacctcag gtaatccact tgcctcagcc tcccaaagtg ctgggattgc 36240
aggcatgagc caccgcaccc ggcctgccgc actcttaaaa tatggtgaac gtttccttca 36300
gagccctgat caccatggta aatgacgatt atttgccatc tcactacagt ataaacccca 36360
caagggaagg gtcctccttc accatgcata cctgcatcta acacagagcc tggctcacag 36420
taggggccaa gctgacatcc agctcaaaga actaaaagca ggtggggctg gttagtgact 36480
actgactacg gtgaccagct tgtcctggtt ttctaagggc ttttctgctt tgagaactga 36540
aagtcccaaa tccccaaaat ctccctgatc ctggtaagcc tgttgttttt aatgcagaaa 36600
cttgattcca cttttttttt tttttgaaac agagtcactc tgtcacccag gctgcagtgc 36660
agtggcacga tctcagctca ctgcaacctc tacctcccag gttcaagtga ttctcctgcc 36720
tcagcctccc aagtggctgg gattacagac actggccacc acacctggct aatctttgta 36780
tttttagtag agatggagtt ttaccctgtt ggccaggcta gtctcaaact cctggcctca 36840
agtgatccac ataccttggc ctcccaaagt gctcggatta taggcgtgag ccactgcacc 36900
cggcccctac tcacttttga tagtttattg ttctgatcat ccatgaccag gatctcatcc 36960
tccagtttct tgatcttggc ctcagccgtg accttctcaa gttgcagctt ctgcctggca 37020
gcttcctcct cctccagctg ttcttcaagg tcctttgttg tggagggaaa agagtaacag 37080
ctttggttat aacagattta ctctcgtggt gatctgggga ctaatgaata caggggctag 37140
gcctgaagat gcccagaact ctgtagcctc ctaaaaggaa gaaataaagg cctgagcgtg 37200
gtggctcaca cctgtaatcc cagcactttt ggaggccgag gtgggtggat cacttgaggt 37260
caggaattca agaccagcct ggccaacatg gtgaaacccc gtctctacta aaaatacaaa 37320
aaaaattagc caggcatggt ggtgggctcc tgtaatccca gctactcggg aggctgaggc 37380
aggagaatcg cttgaacccg agaggcagag gttgcagtga gctgagatcg caccatcgca 37440
ccccagccta ggcaagagag tgagactctg tctcaaaaaa aaaaaaaaaa taaagacctg 37500

ggtaagctgc tctgcttcat ggaacctcaa cctcctcatc tataaaatga gcacagtagg 37560

taccatggtg gttttaaaat ctatccacaa gttctttgcg actccaaaaa ggtggagcct 37620

aattctcctc cccttgagtg ggggttagat ttgcttctaa caacaacaac aaaaatcagg 37680

aagaagtgat ggaatgtaac ttcagagatt aggctataaa aggcattgcg gcttcctttc 37740

tcctctctca gatcactcct tctgggggaa ctctgctgcc atattggaag gacactcaag 37800

cagccctgag gggagaggcc ttcatgataa ggaactgagg cctcctccaa cagctgtgtg 37860

aataagccaa gtagaagtgg gtcctccagc cccagatgag ccttcagatg actgcagcct 37920

cagccaacac cttgactgca acctcaggcc ggaccctcag ccagaaccac tcacctaagc 37980

cactctcaaa ttcctgacta tgcaatactt tttattttta gctgctgagt ttggcatgtt 38040

ttgttatgta gcaatagcta acaaatacaa ttatcctcca ttcaggcttg ctgttaggag 38100

aaaaggggtt agcacatgca aggcagggca atagaacaga gtgggctcac tgtgttccag 38160

tcccaatccc agctgtgtgg ccttgatcag atgaccaacc ctctccaagc ccccgtctca 38220

tcatctgtcc cagcagggac acatatccct ggatgcacct ccacaggcct gtggtgaggg 38280

tcaagtgatt tgctacccac cacgggctgc ccctgtgaca ccttaccagc atctgctggg 38340

ccatcttctt cctttcagcc tgtagctgct ggcccctgtc ttcctcctcc tccaggcggg 38400

cctccatctc atgcagtatc tcctccagct cctgcttctt ggccgccagc cgcacccgca 38460

tctcctcagc ctctgcatac agctctgtct ctgcctgcag ctgttcctgt agcaggttct 38520

tctcctcggt cagctgcacg caggtggtgg ggaggaggcg ggtgagcccc acggggccaa 38580

gtcctgttcc ccagcaaccc cagccatgca tccatacagg tacctgcgag tgcttctgtt 38640

ccagctcctt aagctcattc tctgccttct gctgccgctc cttggtcttc tgcagttcat 38700

cctccttggc ctgcatctcc tcctcctgcc gtgtcacctg cagcagtggc ttcacctgca 38760

cacacacggt tagcccatca tttgtttttg tggcaaaggg atatgttgtc ataataaatt 38820

cgatcagtgg ttccgagcca gggacaatgt tgcccccacc gatccccact aggggatatt 38880

gtctggagac actgctgatt gtcacagctg gggtgggggg tgcctctggc atccagtggg 38940

tagaggccag ggatactgct aaacacccta cagtgcacag gacagccccc acaacagaat 39000

tagtcacccc aaaatatcaa cagtggcaag actgaggagc tctaaatcag agcaccagaa 39060

aaggtacaaa gtgaaaggaa aaagtctcat cttccccact ttagaaccat cccaacagag 39120

aactactgct gaaaagtgat atttccttcc aaggacaaaa gggagaaaaa aaatctttca 39180

tatcccaccc acctccctat caaagtcatt attaacattg tttacatttc cttcctggct 39240

ctttttctat gtttaaaaaa tagacttaag accatgctgc ctagttgtag aggctgcttt 39300

tattatttaa aattatatga tacagacagg aacagtgatg catgcctgta atcccagcat 39360
tttgggaggc cgaggcaagc gcttgagccc aggagtttgg gaccagcctg ggcaacatca 39420
tgagacccca tctctacaaa aacaagtaac caggcatggt ggtgtgtgcc tgtagtccca 39480
gctagtgagg ggtctgaggc aggaagatcg cttgaaccct gcaggctgag gctgcagtga 39540
gccacgattg tgccactgca ctccagcctg ggtgacacag tgagaccctg tctccacaaa 39600
taaaattttt ttaaaaatta taagatatgt attaccccat gctgttacat cattcttaca 39660
actttacata gccatctttt aagttaattt atttatttag cgatggtctt gttttgtcac 39720
ccaggctgga gtgcagtgac acaatcatag ctcactgcag ccttgaactc ctggcctcaa 39780
gcgatcctcc caccccagct ccagagtagc tgggactaca ggcacatgcc acagtgtcca 39840
actagacagc taggtagaag tctttttttt tttttttttt agcagaccta ttcagtagcc 39900
aaagccatca ttttaatagc tgaaaacatt ccattgatag atgaaccatt attgaactca 39960
tcattcagct aggactggac actgaaattc tgctattgta aataaggcca taatgaatgc 40020
ccttaactct tgattttctt ttctcttttt caggtggagt ctcgccctgt cacccaggct 40080
ggagtgcagt ggcacgatct cagctcactg aaacctctgc ctcctcggtt caagtgattc 40140
tcctgcctca atctcctgag tagctgggat tacaggtgcc caccaccaca cctggctaat 40200
ttttgtattt tcagtagaga tagggtttcg ccatgttggc caggctggtt tcgaactcgt 40260
gaacccaggt gatcctcctg ccttggccac ccaaagtgct gggattacag gcatgagcca 40320
ccacacctgg ctaagacgat tttcttagga tagtttttca gaagttggtt cagtggggac 40380
gaacatttcc aaaaagcagc atctgctgtc cttccctggg ctgcctccta actctttgtt 40440
ttcaagcccc tgcctggcac aaggaagccc ctgctatgta ctccagcagc ttgcttcatg 40500
cacttatcac agtttgccat tatgtatctg tctctgtgat tatctgttta ttgtttactc 40560
cccaccaatt cggctagaat attcatggag gtcagggacc atggtgtgtg ttcacagctg 40620
cacccccagc acctagcatg tgcctgggat acagcaggca ctcaatgtct gtttgcttgg 40680
tgaaaaagcc ttgcaccagt ggatacactc aagggcatat aaggagaagc ctagtctatt 40740
gcaacagagc atgtctttgt gccatgggga caatgcaacc agtgccacta caggaaccag 40800
ttgagtcctc tggaggccct gactctgcca gaaataaaaa ataatatgct atggccagat 40860
gaggtggctc atgcctgtaa tcccagcact ttgggagcct gaagcaggag ggttgcttga 40920
ggtcaggagt tcaagaccag cctgggtaac agagtgagac tccatttcaa aaaatgcaaa 40980
aagtaaaaaa aaaaaaacct gcagtattga tacatgcaac agtctggatg ctctctaatt 41040
ccatgtatag aatatttgtt ttattttttt ttgagacgga gtctcgctct gtcaccaggc 41100
tggagtgcag tacagtggcg cgatctcagc tctctgcaac ctctgcctcc caggttcaag 41160

tgattctctt gcctcagcct cctgagtagc tgtgactaca ggcgtttgcc accacgccca 41220

gctaattttt gtacatttag tagagacagg gtttcaccat cttggccagg atggtcttga 41280

tttcttgact ttgtgatcca cccgcctcgg cctcccaaag tgctgggatt acagccgtga 41340

gccaccgtgc ctggcctttt ttttaaataa aaaaaaaaag aaagaagaga cagagtctca 41400

atctttcacc caggctggag tgcaggcatg atcttggctc actgcaacct ctgtctcctg 41460

agttcaagag attctcctgc cttagcctcc tgagtatctg ggattacagg tgcctgccac 41520

catacccagc taatttttgt attttaagta gagacagggt tttgccatgt tggccaggct 41580

ggttttgaac tcctgaccgc aagtgatccg cctgcctcgg cctcccaaag tgctgggatt 41640

acaggcgtga gccgccgccc ccggcagcat tcttgaacca tcaaaattac agaactggag 41700

aacagagtgg tttccatggc tacggaggaa gtgggtgtgg ctataaatgg gccacacgag 41760

ggattccagt gcggatggaa atattaatac tttgtgttaa ggtcctggct atagcctatg 41820

tgccaaagga cagggcctcc tcgggccaga gaaccgccaa tgctcaaggt ctgagttcga 41880

gccctccatt ccacacccac ttgcgaccac ctcctgtccc aaatgtgaag gctccagagt 41940

gaagaagcag ggctgggggc ccctgggaag ggggctgggg cagagcactc actttggtga 42000

aaagcctcca ccactgccag ttccgcagct tgaggtaggc ggcgcagttc ctctgaatca 42060

ccttcatggc ggtcagctgc tgctgcctct tggcaaaagc cctagggagg ggggaagaga 42120

aggtgcgggg gtcatgaagc cacaccctgc tgtcaacaga gccctgccct gcagcatcct 42180

gaacctgcac agggacatgc cccaatctcc ccaactcagg ttcttccaga aaccctgtgt 42240

gacacatgtg ttgtctggct gcaaacaact taaatcatct acaggacaga gcctttttca 42300

gtggacaagg aagacccagg gctcacaaca tgccctggac ttagctgaga tccaaacacc 42360

actcagaaaa cacaactatg tgcatcattc ctaagggggc tcccgtactc cccagctgca 42420

ctagctgttt cttttttctt tctttctttt tttttttttt tttttttgga gatagagttt 42480

cgctcttgtt gcccaggctg gagtgcggtg gcacgatctc agctcactgc aaactccacc 42540

tccggggttc aagtgattct cctgcttcag cctcctgagt agcttggatt acaggtgccc 42600

acaaccatgc ctggctaatt tttgtatttt tagtagagat ggggtttccc atgttggcca 42660

ggtgggtctc aaactcctga cctcaggtga tccacccacc ttggcctccc aaagtgctgg 42720

gattacaggt aggagccacc atgcccagcc tacaccagct gtttctgact aacttattta 42780

tttatttgtt tattttcaga gacagggtct ggctctgcgg cccaggctgg agtgcagtgg 42840

tgtgatgatg gctcactgca gcctcaaatt cctgggctta aatgatcctc ctgcctcagc 42900

ctcccaagta gttgggacta cgggcacatg ctaccatgcc cagctaattt ttaaagtttt 42960

ttttttttgg tagagatagg ggtctcgcca tgttgcccag gctggtctca aactcctggg 43020
ctcaagcaat cctcctgcct cggcctccca aagtgcctgg gattacaggt gtcagctacc 43080
acacccagcc ctgtcaaacc tattctctcc tgggttgttt taggaatttc acactgtgac 43140
cctgaaccag cactcttccc gtttaagggc tatcgataga ttgagaaacg atagaacagt 43200
gaacgtaatt ctgtcttccc tgcttggctg ccatcttgga cacaggaaaa ctttaagaat 43260
aaaattgccc cagttatcca ggaaggacag caacagggaa cttagtaacg aaacttgaca 43320
agttaggtct gttgcgggag ggccaagagc tcccaaagct ggccttgacc atcttgggtc 43380
catgtgtcaa tgaatgaacc tgacacatca gggtcctact ggtgagggtg tggtctctgc 43440
ccaatgacaa cacacacccc agacagagag cgaccccacc tgtccctgca gtccctgggg 43500
tatgactttc attaaggaac ccgaaccctt ccccggctcc cagtcttcac agaacagctg 43560
ggacatcaca ccctccaaaa ccctgcctta taaatcccac tgggacacgg ggatctagac 43620
accgtcaagt cctctttcct caatggttta tccaattcta acatgtctag gctgggcgca 43680
gtgggtcatg cctataatcc cagcactttg ggaggccaag gcaggtggat cacttgagac 43740
caggaatttg agaccagcct ggccaacatg gtgaaacttt gtctctacta aaaatacaaa 43800
aattaggcca gtgtggtggt gggcgcctct aattccagcc actcgggagg ctcagacacg 43860
agaattgctt gagtccaaga ggcagaggtt gcagtgagtt gagactgcgc cactgcactc 43920
cagcctgggc gacagagaga gagagagaga ctctgtctca aaaaggaaga agaaaggaag 43980
gaaggaaggg agggagggaa ggaaggagtg agggagggag gtaaaaacaa caacaatcat 44040
ttctgacctg agaaccacgc actgttctaa gctctcgtca tgcgttatct tagtgacacc 44100
tcatgaacag ctctctgagg tcaaaatgat tatgctcagt ttccagctaa aaaactggaa 44160
gctcagagag gtagagtaac ttgcccaagg ttcatcagca aaaactgggg catccacgat 44220
gagaacccag gtctaaaact cgttcccttc ctgcactcgt aacttggtac atttatttaa 44280
agtagaagca tttcttcccc ttcaagaaaa ggctgtcatt cagtcaatga tgcatcttaa 44340
acaggtggcc tcttagaatc agggaatcag aacagaaagg cccctgtttg tgattcgcgt 44400
ttgaggtatt aggatgcagg aaagcatctt tactttctgg ccaagtagcc acgacacatc 44460
gcctggaagg ccatgatgac atcggtgatc ttcaaatctc gctcctcctc taggtgggcc 44520
aggacgccag ttcggaagaa gattttgctc tgccctatcc tgtataagtt ggggtcaagt 44580
tccagggctt tgatctgcaa aaggaaggaa aggaagagct cctgatttcc actgtgccat 44640
ttggccagtg atgacatggg taagaacggt cccaccaaga gcagcaggtg gcttgcggcc 44700
agaggttggg aggtctctgg tggacttctg ggctcaccat gagaatgcag gcctgcttcc 44760
cgtccatgaa gcctttgggg atggcattcg ccgccaggat ctcgtagctt gaaacacaga 44820

gcagaagtca ccccgggtac ctccagcatc cattcctcca cccagtcccg ctcaccccct 44880
gccctacctg ggccagacct tgggacttac cgttggcgga actcctggaa gacgatccgg 44940
ttggggaagc cctgccggca gatgcgaatg ccttccagca ccccattgca ccgcagctgc 45000
tccagcacca ggaacgcatc cagcttgccg gactgcaaag gtcaaagagg gcagtggatc 45060
cctggggcca gaaaccatgg cgcagcaaag ccccaaacct cctacctgga tgacccacct 45120
ggccaggcca tgagggtacc aacagaagca cccaaggagg gcttagctga gggccgaggg 45180
gcaaagcagt gcccccctct gggttttcat ctctttgctt cggcaatggc tgcctcatga 45240
tctctctggt tcttggtgac actagcatcg cctggctgga acctggggct tcctcctcat 45300
cacctcgtct ccctcacttc ctgtccatca gcagtttgat tgcttctagc tcccaaatat 45360
ctcccaaccc ccccacttct cttcaccacc attgtctcca ctatcttctc ctgcctggac 45420
aaccacagaa accacccacc tggtctctct ctctggtctt gcctctcccc tcaatccctt 45480
tgtccttttt attttttgag atagggtccc attctgtcag ccaggctgga gtccagtggt 45540
gcgatcatgg ctcactgcag cctcaaactc ctgggctcaa gtgatcctcc caaatcagcc 45600
tcccaagtat ttgggaccac aggtatgtgc taccatgcct ggaaaatttt taaaaatgtt 45660
tttgtagata tagggtcttg ctatgttgcc caggctagcc ttgaacacct ggtctcaagt 45720
gatcctccca cctcaacctc ccaaagtgtt gggattatag gcatgagcta gcatatgccc 45780
agccccctca ttcacttcct aaaatgtaaa tctgaccatg atatagccct gctttaacac 45840
ccccaatggc atgcctagag agagaaggcc aagctccaga aagaacacaa ggagcaccac 45900
gatctagccc ctgcctaccc attcagcctc aatcctacct cacaccatct gattcctacc 45960
tcatgccatc acacatttgc aacatgaaat gacctattta gcattttctt tccttttttt 46020
tttttttttt taagatggag tctcgctctg ctgcccaggc tggaatgctg tggtgcaatc 46080
tcagctcact gcaacctccg cctcctgggt tcaacagatt tttgtttctc agcctcctga 46140
gtagctggga ttacaggcgt gagcaacacg cctggacact atctagcatt ttcttaccta 46200
gttccttcac ttctcacctc cacactcttg cttagcctgg gccctctgct tatcctaggc 46260
cccctgctta tcctaggccc caccctgcct tccatgtcac caagttgatg gccacacatt 46320
cttaacgttc aagttcacca gaagctttct ctgagtcctg aggctgtggg aggagccctg 46380
gctctttggt gttctcctaa ctgaccacat tccatcacca tcatcagctg tatgtttacc 46440
tctccccgtg tctcccataa cctcagcact actggcatct tgtagggatt ctgtgctggg 46500
ggttgaggga ccatcctgtg cattgtacaa tgcttagggc atccctggcc cctacccact 46560
agatgctgac taacagcccc ctcctggttg tgacaaccaa aaatgtctgt agcatgtcca 46620

tatagaggac acaatcgccc caagctgaag atgcctgcac cagattatga gaaacccaca 46680

gggaagggct acaatttttc taacactgct ccccagcacc caggacaggg cagagcccag 46740

tgaatacttc ctgggataat gaatgaatga gtggctggat gacctgcaca gcaactccac 46800

aactgaaaag tctctggatg catcctccct tcctcctttt cctccaggga tggggaatgg 46860

gtctgagatt cagatagcct tccccacatg gaaaatgggg tcctcggggt aggtgggggc 46920

agagggcgcc ctggggacgc tgtgaccgct tgggacagcc ctggcttctg ggagccccag 46980

ggtctgggcg gcgggcctca ccctcttctc gtggttgggg atgatgcagc gcacgaagtt 47040

gggcgtggtg ttgcgtagcg tggtcatcag cttgcccagc tgctccttgt acagctgccc 47100

cactgtgcgg aacatgccct tcttggtctt ggaggcgctg ggcagcgagc tctccgtcat 47160

cttggccatc tggtccaggc ccacgatgcg gtccactatg gggcacagcc agggtggcat 47220

cagcctctgg cccacccacc cctaaatagg ccagaggctc agccccagcc ccacccacca 47280

acctgcccac tccaatcttt ccttccatca ccaacgcctc cttcggcagt cagggtttcc 47340

aagtattgtc tattggggaa atcctgagag cacctgagtg gcagaaagaa caaattccct 47400

gtgatggagt cacgctgacc taggagtcaa gcctgctctc gctgtccact cactgactag 47460

ccttggcttg gcactatcct gtgcctcagt ctccctttct acaaaaggga ggtaataata 47520

ccgtctacct tctagggcag gggtaaaaaa aaataaggca cagaaggcac ttattccctg 47580

ccagcctcca atctttcatc caccaacaaa tagttattga ggaccttctg tgctccaggc 47640

cctggggaca ctgctgtgaa caaaaaaaga cccggccccc tcaccccgct tatatcacag 47700

ggaaggagac agacagagat cacagatgtt aagtatggaa tatcttgagt agtgaggtgt 47760

gcgtagaaaa ataaaaatag ggtaaagcgg agagagagtg aagagggtgc tctcttagac 47820

aaggaggtgg tgggctctgc taagttgata tttgagcaga aaccagcaag aaatgagtgg 47880

gtaaccatgt aggtatccac ggaagaaata ttctgaggca gaagatacag cacacactcc 47940

gagatagcct ggcacatcag taagtgctca acaaaaagta ggtattatta ttattattat 48000

tattattatt attattatta ttatcattat tttattttat tgagacggag tttcactctt 48060

gttggccagg ctggagtgca atggcgtgat ctcggctcac cacaacgtcc gcctcccggg 48120

ttccagcaat tctcccgcct cagcctccca gtagctggga ttacaggcat gcgccaccac 48180

gcccggctca ttttttttat atttttagta gagacagagt ttctccatgt tggtcaggct 48240

ggtctcgaac tcctgacctc aggtgattcc cctgccttgg cctcccaatg tgctgggatt 48300

acaggcgtga gctaccgcgc ccggccatca tcatcatcat catcatcatc atcatcatca 48360

cctccaaggt tctttttcta ccagcccagc aggtcctgca catgccaagc ccaacccaca 48420

ggccatggat ggcaccattt cttcaccttc tactcagatt ctgtgtaaca atcttttttt 48480

tttttttttt tttttgagac aagctctcac tctgtcgccc agctggagag cagtggcacg 48540
atcttggctc actgcaacct tcaccaccca ggttcaggtg attctcctgc cttagcctcc 48600
tgagtagctg ggattacagg cacccaccag cacacccggc taattttgtt tttatttttt 48660
atttttagtt ttattttttc aagacagagt cttgctcttt tgcccaggct ggagtccaat 48720
ggcacaatct cagctcactg caacctgcgc ctcccaggtt caagcagttc tcctgcctca 48780
gcctcccgag tagctgggat taccaccacc acgcccagtt agtttttgta tttttagtag 48840
agatggggtt tcgccacgtt ggccagactg gtctcgaact cttgacctca actgatcctc 48900
ctgcctcagc ctcccaaagt gctgagatta caggtgtgag ccaccatgcg cagcctcatg 48960
cttgtctctc tgcttgtctt tgagagtctc atctctccca caagactgtg ggtcccgcag 49020
aggagggact acaggcttca tcatgtgtcc tgggatgaca cagacccagg gtgcgatgtt 49080
gttccctagg cccagagcct ggcatatggt tagggcacag ctggcccctc ctggagccct 49140
ttttttagca tgcttaaaac aaggggagac cagtcctgta agccccaggc ccatgggccc 49200
tgtgccaaga ataaaacctg ctgtggacac tgatggccag ggacaaacag caaaccccct 49260
cccctcatca tcactccagc agctcttcac agggatcatc tattcactgg ttctcaaagg 49320
gagccggagt gtggtcccta gaccagcagc atcacctggg atgaatgttt tcaggcccgc 49380
cccatccctg gccccagacc tgcagaatta gatcttaggg taggcccagc catctgggtt 49440
ttaaaaagcc ctccaaagag ggccaggtgc agtggctcac acctgtaatc ccagcacttt 49500
ttgaggctga ggtgggcgga tcacctgagg tcaggagttt gagaccagcc tggccaacat 49560
gggggaaccc cgtctctact aaaaatacaa aaattagcca ggtatggtgg tgtgtgcctg 49620
taatgctagc tacttgggag gctgaggcag gaaaatcact ggaacccaga aggtggaggc 49680
tgcagtgagc caagattgca ccaccgccct ccagcctggg tgacaaagca agactctgtc 49740
cctaaataaa taaacaaagc cttccagggg gtcatgatgc ctgcttgagt ttgagcatca 49800
ctggtctaat ttaaccctca taatacctct agtagagacg gcaactatta ttgcatctcc 49860
tgttttacta atgaggacaa tgaagcttaa tgacatcaag tgactcatcc aagaccacag 49920
agctaggagg tggcagagac aagatacagt gaagtccttc tgactctgga cagggcagtc 49980
taactccccg tgcctggcat gctgtttctc ctctattgcc tgagccctga cctcaggacc 50040
tcaatcccct cgggacatgt aggattgagg ggctcaggga cccccactca ggaagaaatt 50100
gtttcaacga gacatcacca gcccatgcca atgctcttcc ttcccctgcc cccatgtaaa 50160
ggcctcccct cctggggcag gtgtgagagt cgggggactt gggtgttcaa ttctccagct 50220
caaagcagaa catggacccg agcagagaag gccttaccgt ccttccacag gtcggccaca 50280

aacttgtcgg aggaggcatt gagcagggaa gtcacgttgt cattcagcgg gtccatattc 50340
ttggtcagcc aggcactcgc attatagtcc acctgccaag gacaccctgc tggtcagaac 50400
ccctgggaaa ctagaaactt agaaaccaag gccaggaccc cagccctccc attgtccttc 50460
cagatcttct gaggtcagag aggaaatgac gtttatgacc accatgatgg gactccacaa 50520
acaccagtga agagaccaac agcccaggga cctgtgccca cgtagtagga catcagccag 50580
tgatagccat tgctgggaaa gcctagtttc cattccatat tctcctacta acgctgctgt 50640
ggtagcctcc ctcctcctaa gccagcacag tacctgtgag ttatacacta ttaagagatt 50700
tcatgacggc agatgccctc ctgccgacat gcaaaactcc cccctcatca tcttcccaat 50760
catcctggtt tagaaattaa gcatggcagc caagcgtggt ggttcacact ggtaatccca 50820
gcactgtgga aggccaaggt gggtggatca tttgaggtca ggcgttcaag accaacctga 50880
ccaacatggt gaaaccccat gtctactaaa aatacaaaaa aaaaaaaaaa attagccagg 50940
catggtggtg cacacctgta atgccagcta ctcgggaggc tgaggcagga gaatcgcttc 51000
agcctgggag gcggagattg cagtgagctg agattgcgcc actgaactcc agcctgggtg 51060
acagagtgag actctgtctc aaaataatta aataaataaa ttaattaagc atggcatcct 51120
attttccact ctctaatgag cataatttgt aactcagaga tcctgatata tggatataca 51180
gaaaaaggac atataggctt tttacattcc tgactcaaac aatgacaatg cccaaagccg 51240
taatttttac tatggtgaat attaaatgaa aggaactgat aatacagctt tgctgggtta 51300
tcatgggatg tgggttatca ggataatagg taggcttgat ctgagagaag actgaaaata 51360
ataataaaaa aaatccactg atgaagtagg gaaaagaaaa gtgttgtctc tatatgctta 51420
aaggaacata tacccccttg aatataggct aggagttggg aaacttttgg tgaaggccca 51480
gatagtagat atttttggct tggcaagtca tacaatgttt tgtttgtttg tttgttttgg 51540
tttttgagac agagtggttc tctgttgccc agtctggagt gcagtggtgc agtctcagct 51600
cactgcaacc tccaacttcc tgggtcaagc agttctcttg cctcagcctc ctgagtagct 51660
gaggttacag ttgtgtgcct gtaatctcac agctggctaa tttttaaatt ttttttattt 51720
tttattttta gcagagacgg gctttcgcca tgttggccag gctggtctca aactgctgac 51780
ctcaagtaat ccacctgcct tggtgctggg gttacaggcg tgagccacca cacccagcca 51840
agtcatacag tctttgtcgc agctactcaa ctctagcact gtagtataca aaagcagcca 51900
gccatagaca ttatgcatac aaatgggcat ggctgtgttc caataaaact ttatttacaa 51960
taacaagctg tgggccagat cggactctgt tgccaggccc tggtagagag cattgtttgt 52020
gcccatgatg cagtggaggt actgaggagt caatagaata ctacaattct gcaagccaga 52080
ctaagctatg tcctttgtac attatccagg actcttgaga gccacagggc aagggcctat 52140

acaataacag ccaaggcatg ttgatgatga tgatgatgat gatgaataca actatcatag 52200

atttccctgg ccagagcagg ctggcaatag caaactcaag accgtgacat gggagatttt 52260

tgttgaagcc aggtgtggat gtattagtca ccccattcca gttggccccg cctgttgggt 52320

ataacttgta gcaggaagga tttagagtct atacaagaaa agcaggctga ggccaggtac 52380

agtggctcgt gctcataatc ccagcacttc aggaagccga ggcagggaga tctcttgagc 52440

ccaggaattc aagaccagcc tgggcaacac agcaagaccc catatctata cataatttaa 52500

aaatttagct gggcggccag gcacggtggc tcatgcctgt aatcctagca ccttgggagg 52560

ccgaggcggg tggatcacct gaggtcagga gtttgagacc agcctggcca acatggcgaa 52620

acccttctct actaaaaata caaaaattag ctgggtgtgg tggcaggtgc ctgtaatccc 52680

agctatttgg gaggctgagg caggagaatc tcttgaaccc ggagggagag gttgcagtta 52740

gcagagatca cgccattgca ctccagcctg gatgacaaga gcaaaattcc atctcaaaaa 52800

ggaaaaaaga ttttagctgg gcatggtggt gtacacctgt agtcccagct acttgggaag 52860

ctgaggcagg aggatcgtgt gagcccagga ggttgaggct gtagtgagct atgatcgcac 52920

cactgcactc cagcctgggc gacaaagcga gaccctgtct ccaacaaaaa cagaaagaaa 52980

taactgtctg gacgtcacgg ttggaaaaca tgagagtggc tacagctgat gccttacaga 53040

tgcgtagaca aattacgaat aggacttggt tgggattcgc ttagcagcag atggctttgc 53100

agtttccagg cagcccaagg ttctgccact ctcagactgt ctctgcgctg agcagccact 53160

gggggtcccc tgagacagag tcccctgggc cctgtggctg gtaccttccc agcataatgg 53220

atgatggaga actcagtctt gtccttgagc tgcttgggct tctggaactt ggggtggctg 53280

ccctgctccg tgcacagctt ctccacgaaa gacttgtccg tggctttggg gaaccagcat 53340

tcctcgtcca gcagggccag cacacctgga gggttgttct gtgggagaca agtagggctt 53400

gaatcagaga gaacacccaa cctcaggcat tccatgaaga gctggccaac tatacacaac 53460

gcatgggcat ccgccgagat ctgatactgt atttgcccac atataaagat gagtttatga 53520

cccccatgct tccaaatcta ccttcaaatt accctgtgcc caatggacca gtaacaggga 53580

tggaaggcag aacataatgt ttgctgccgc cccctagtgg gcaacacgca ggcaacatca 53640

tgaagagttc ataactcttt tttttttttt tttttttttg agacagagtt ttgccttgtc 53700

gcccaggcag acggtgcagt ggcgcgatct cgactcactg caagctccgc ttcctgggtt 53760

cacgccattc tcctgcctca gcctcccgag tagctgggac tacaggcgcc tgccacggcg 53820

cccggctgat tttttctatt tttagtagag gcggggtttc accgtgttag ccaggatggt 53880

ctccatctcc tgacctcgtg atccgcccgc ctcggcctcc caaagtgctg ggattacagg 53940

catgagccac cgcgcccggc cgagttcata attcttgcaa atgaatttgt tgcttgactt 54000

gaattcacct agggttcctg ttagaaaagt tttttttgga gacagggtct cactctgtta 54060

tccaggctgg aatgcagtgg cgcaatctcg gcttacagca acctctgcct ccagggctca 54120

agcaatcctc ccacctcagc ctcccaaata gctgggacca caggcgtgca ccaccaggcc 54180

cggctaatta gtagaaaccc tgtctctact gaaaatacaa aagttaggtg ggcgtggtgg 54240

tgcacgcctg tagtctcagc cacttgggag gctgaggcgg gaggatcact tgaggcaggg 54300

aggtggaggt tgcagtgagg caagattgtg ccactgcact ccagcctggg caacagagtc 54360

agaccatgtc ataaaaaaaa aaaaaaaaaa aaaaaaagca atggcagaga aggaaaggga 54420

agggaaagag ggtagggaag aaaaaaagga agaaaaaaga ggatggaagg aaggcagaaa 54480

ttaaagaaaa tacctttgat atgaaaatgt gggttaatgg aaattgtaag agttcacaag 54540

gattcagctt acagcctttc aggactgggt gatggattgg gtgggggaat gctatgtgca 54600

tggggagagt gtggggtcca cgtcggctcc acagaggcca cacacgtgta caaggtgtga 54660

cggagccccg cacgcccacg tgcccctcac cggtcgctcg atgagctcga tgcagggctg 54720

taggtccagc ccaaagtcga tgaagttcca ctcgatgccc tcgcgctggt actcctcctg 54780

ctccaggatg aacatggtgt ggttgaagag ctgctgcagc ttctcgttgg tgtagttgat 54840

gcacagctgc tcgaaggagt tcacctgagc acatggcgtg gggcggggcc gtgagcatct 54900

tggtatgaag tcagaagaca aggagcccca cagaagctcc acccgacagc gcccccatgg 54960

cccgcccaca tcctgttctg ccatcccagc acccagacag ggctgaggcc ccaaaacatg 55020

ctgcctagag tccaccaggg ctggtgaagt tgatatcaca tggccctcac acaccttaaa 55080

gatgcttatc tcctccatct gcacaaccat ctatggatgg acaaacctcg taaggcacgc 55140

tactgcatgt acagatgtgg aaacatttta cagatgtgaa aacagaggcg cagagagaaa 55200

gtgactcggt agtacacttc tttcgggaca tctatcaatc ttaaaacctc ctggctgggt 55260

gtagtgctca tgattgtaat cccagcactt agggaggcca aggcgggagg atcacttgag 55320

gtcaggagtt tgcgaccagc ctggcaagca tagcgaaacc tctctccact aaaaatacaa 55380

aaattggcca agcacagtgg ctcatacctg taatcccagc actttgggag gccgaggctg 55440

gtggatcatt tgagatcagg agtttgagac tagcctggcc aacagggtga aactctgtct 55500

ctaccaaaaa aaaaaaaaaa agtagccagg catggtggca ctaatcccag ctactcgaga 55560

ggctgaggca ggagaatcac ttgaacctgg gaggcggagg ttgcggtgag ccaagattgc 55620

actactgcac tccggcctgg gcaacagagc gagactctgt ctaaaaaaaa aaaaaaaatt 55680

agctgggcac agtggtacac tcctgtagtc ccagctactc aggaggctga gacaagagaa 55740

ttgcttgaac ccaggaggcg gaggcagcag tgagacaaaa ttgtgacact gcattccagt 55800

ctgggcgaca gagcaagact ctgtctcaaa aaaaaaaaaa caagacaaga caaaaaaaaa 55860

accttccctt tctctgagaa ctttcttcaa acccatgggt aaaggatcca atatccatgt 55920

ttctactaca tgaggtattg caagctcctg caaacagttc ttggcttgga ggtgaccatg 55980

tagcccaatt caggtgagta agagtcagtg agacttaatt ccaagatttt ggttggaact 56040

gttagaaaag agatatgtgc tttttttttt tttttttttt taccaggatt actgagaggc 56100

agtctggagc tgtggcagcc atcttgcccc cgtgagggga gagcctgctt gagaatggag 56160

ccaatatgga ggaaggcaga gagagagaga gaacgatggc gggagatcag accctgccta 56220

cgggatccag ctacatgtct ggccttctca gttccagaaa ccaatgcatt tcctgttgga 56280

tttctgtctc ttataccctc aatgatccac ccttaactag acagcctcct accctacagt 56340

agccatctac atgcctttct ccaaaatcat gactcctcca agaaaaagcc catctcagac 56400

aaccaagacc atggctctta ggatcccacc gagctgtacc tcaaagatct caaatccagc 56460

tatatccagg atccccagga aggaagcccc ttgccgatgg gtcttgtcca gggctttgtt 56520

cacgcgggtg agtatccagc ggaaaaggcg ctcatatgtt gccttggcca aagcctctac 56580

agcaaagtca gcctgcagag ggcaaccagg ggaacccggt tattctcaat gggctccatt 56640

ttcacataag ccaggctggt ggtgaagatt tttattcttg gccgggtgca gtgactcaca 56700

ctgtaatccc agcactttgg gaggccgagg caggtgggtc acttgaggtc gggagttcaa 56760

gaccagcctg accaacaagg caaaaaccct tctctactaa aaatacaaaa attagccagg 56820

cgttgtggtg catgcctgta gtcccagctg cttgggggac tgaggcagga gaatcgtttg 56880

aatctgggag gcagaggcta cagtgagctg cgatggcacc actgcacccc cccctgggca 56940

acagagcaag gctctggctc aaaaacaaaa aacaaacaaa caaacaaaac ctgacatggg 57000

actgtgaata ttctagtatt gaataaatga gggataaaga gatggatggg tggatgcatg 57060

gacagatgaa tgaatgaatg aatgaatgga atgacagctg gatgggtgat ggatggacgg 57120

acagatggac agatggctgg gtcatggatg gatggacaga tggatgagtg agggatggag 57180

agatgatggg tgagcagata gatgggcaga tgaatggatg gatggatgga tggatgaaca 57240

gacagatgaa tgagtgggta gaaagatgga tggatggaca gatgcagagg cagaccatgg 57300

gtgaatggat taatgaatac atgaatgagt gaacaggtgg atagatggat gaatggatgg 57360

atgggtgggt gcatggatgg ataagtgata agtacatcat tacctgttct tttgtctgag 57420

ctttctgtac cacatctcgc ccaaccttga tacgaggagt gaggatggat ctggtgaaat 57480

ctgtcacatt aattcccatg aggtggcaaa ctttctgagc agctggatgg agaaaagaaa 57540

catcgtgagt gcatcacaaa agaaatagct tggcaagaag acacatcgca tgggatattt 57600

gtgatctgtg aatacagcgg gttctcttgg ggaaatggga cccgctgacc atgaatatag 57660
cttgaagtgt cctaaaggct gctgtcatta gtggtcacac tccccagtat tttgctgaga 57720
ggcaatgatt ggggtgggct gaagcaggcc ccagttgggg gtttagggtc aggtagttct 57780
agtttcaagt ccctgtccct tcacacttcc aagtcatgtg actttggaaa agctctttac 57840
cctcccagca tcttgctttt ctcagcttta aaaagggtca gctcttcggt accttgttgg 57900
tacccaggac taaataaggt aaagtatgta aagtccctga gatagataga tttatttatt 57960
tatttatttg tttatttatt tatttatttt tggagacaga gtctcgctct gtcgcccagg 58020
ctggagtgca gtggcgcgat ctcagctcac tgcaacctcc gccttccggg ttcaagcaat 58080
tctcctgcct cagcctcccg agtagctggc attataggtg cctgccacca cacccagcta 58140
atttcttttg tattttagtt gagatggagt tacaccgtgt tgcccaggct ggtctcgaag 58200
tcctgaactc aggcaatccg cccacctcgg cctccctaag tgctgggatt acaggcgtga 58260
gccactggcc cttgagattt atttaacagt tacttagtaa gtattaaata gttattaata 58320
gtaacattta ttgaacagtt actgttcaat aaatatttct ttcacccttc taggatatgt 58380
gttgcagcat tgtttgcaat agcaaaatgc tagagtcaac ctaaatgctc ttcttttatt 58440
ttgagatgca gtcttcctgt cgcccgggct gaagtgcagt ggtgtgacca cgagctggtc 58500
ccttaacatg aaaatagcat tacatgctgc tttgcatgtg caaggcagag taaatagagt 58560
gattttagga tttttttttc cccatctctt ggcaagagca ctttttcatt tctctttccc 58620
tgaggtcttt ccttggcatc cggattccat taatttgcca gatttaggga ctctaataca 58680
taacaaacac ctgagaatga aactgccact gcaaaattat aactgagaca ctgaacaaga 58740
tctgacctaa cggactccac cttgcttcta acctccaagc tccactgaac catttgtggg 58800
tgtaagccga attaacttgg caggaactta gtttatagtt tattttttat gttttggaca 58860
cagagtctca ctctgttacc caggctggag tgcagtggca caatctcggc gcactgcaat 58920
ctccgcctcc tgcgttcaag cagttctcct gcctcagcct cctgagtagc tgggattaca 58980
ggcgcctgcc actacgcctg gctaattttt gtatttttag tagagatggg gtttcaccat 59040
gttggcgagg ctggtcttga actcctgact tcaagtgatc cacctgcctg ggcctcccaa 59100
aatcctagga ttacaggcat gagccaccac gcctagcctc ttagtttata gtttaaaaca 59160
caagaaccct ttcccaaaac aaaccccctt cttgcctgga gactagactg cctttgtagg 59220
aataacaaat tagcccaaag gtttgaaatt atggtttagg agtcctgtaa ctggaggcta 59280
caagagtctg accctcccta aattgctcct ggggataaca tcactattgt aaaacctaag 59340
accagtactt gagatatttt tgcggaccct ggcaccaggt ggatcagctg acaccaccca 59400
gatcgagaaa ctggctcatc tgatcttatg gcctctgtcc aggaactgac tcagggcaag 59460

aggacagctt cgactccctg taatttcatc tctgacccaa ccaatcagca cccccaactc 59520
actggcctcc ccctaaccac attatgctta aaaactctga tccacgaatg ctcaaggaga 59580
ttgatttgag taataataaa actctgttct cccatgcagc cggctctgcg tgagttactc 59640
ttactctatc gcaattcccc tgtcttgatt aattggctct gtctaggctg tgggcaaggt 59700
aaacccagta agcggttgca acaggtttga tcaaggtggg ctctagggag cgggaaaaag 59760
caacacaata tacagcctgg gctgggaaat aagaaaccta agttttgctc tcagctcagc 59820
caccaacaag catagtggta gccaggccag cttctcaaaa cacaaaatac tgtttatttg 59880
tgggtaggtt tatcagattt agggggaaaa ttacaggatg ctcagttaaa cctgaatttc 59940
agataaacaa gaatactctt ttggtataat cacggtccaa atgttacaag ggacataatt 60000
aaaatttaaa aaaaggtttg ttgtttacct aaaattgaaa tttatctgga catcctgtat 60060
tttatctggc acccctaatt ctcagggcaa ttgaggtctg tctccccaag acgaccacag 60120
ataccgtaag aagagctatt cttgtgtcat ttcctgctgt atcccagtgg catattgcag 60180
gggcttgaga aatatttctt taacaaaaca attaagtggg gtattttaga agagatggtc 60240
cctcgggatc ttgtctggtc actattaggg gtaggacgtg atgactcttg cctgtaatcc 60300
cagcattttg ggaggccaag gtgggaggat cacttgagct caggagttca agatcagctt 60360
gggcaacaca gcgagacctc gtctctatta aaacttgtaa aattttgtat ttaaaaaaaa 60420
gaacaaataa ataaaactat taggtttgtt tttttttaat ccctgcgctc tgattcaata 60480
actttccaca tagggtcttg agcactgaaa agtttgaaaa agtgagtgat aagaacctag 60540
tccaactgtt gttaaaatcc cagatacctt caccttgaaa aataaccagt ggttaaatgt 60600
cacctccccc acccccccaa ccccaaagtc attggtcatc aggaaaaagt ggcaagtacc 60660
tgtgttatct ggcatggacg cctggtctgt gtttctttcc ttcttgaaga cgatatttcc 60720
aagctgcagg accgatgata ccaccttcaa tatggctgag gtggggagag aagggcagag 60780
cagacacaaa ggtcaatgcc aaggtaccct ggagttttgg agcctaaagc cactggggac 60840
ccagagccag cttacatctt tcctctgaag aaactagatt ctgagctcaa tattttcata 60900
tggttgctta aaaccttgca ttacaatgga atgccacgca gcagggactg gcaaattaca 60960
gcccatggac caaatctggc ccactgcttg ctattataca taaagtttta gtggaaaaca 61020
gctatactca ttcatttaca tattgtctgt aactatttgc atgctacact ggcagagtta 61080
atgagttgca acaaagatcg tctggcctac aaaggctaaa gaatttacta tctgaccctt 61140
tacagaaaaa tgtgttagca acccctgcca cagaacagtg agaatgaatg agctacaact 61200
accacaaatc aaaactgatg tattggggcc agcatagtga ctcatgcctg cgaccccagc 61260

actttgggag gctgaggtgg gagaactgct tgaagccagg agcttgatac agcctgggca 61320
atgtagggag atcctgtctc taaaacaaaa aaaaaaccct gacatatctt ccagacacaa 61380
ataccgaaca aaagaggaca gatggaaaca agaacatact gtctaagtcc atatatagac 61440
cgtccattca agaagaggca aaactaatcg atgctgttag aagtcaggat aatgattatc 61500
cttaattgag ggaagttctt agaaggtgca tgtgggagct tccatggggc tggtggcatt 61560
gtcttattta aaatggattc tagttacaca gacgtgttca gtttataata attctttgca 61620
ctgcacgcta tggcatacac aattttctcc atgtaaatca tatgtcaata aaaggatttt 61680
tgaaataaaa accctgtgct tcttacatac tcttctgggt gtaacagatg cccatgttgg 61740
gatgaagaag atatacattt gtttccactt gttgtccagg tgtctccaaa caaaaacgct 61800
gtgttcccag aatacttgga acctttctga ctccatactc aggccatgtt actgttaaaa 61860
tagtttctat gtctgaaatc cccataagac catgagtttc tggagagtgg agggcctgcc 61920
ctaatcatct ttgaatttcc aaagcctatt ccaaggtcct gatacacatg ggagggaggg 61980
agagagatgg atggatggta ggtggaaggt ggatggatgg atgaatgaat aaatggatag 62040
aggatgggtt catggatgaa tggacggatg gatggatggg tgggtagatg aatgggtgaa 62100
tgatggacgg atgagtagag gatagatgga tgaatagagg atggattgat ggatgaataa 62160
ttcatggata gaggatagac ggatgatgga tggatggata gaggatagat ggatagatgg 62220
aatggacaga ggattgatgg atagacgatg gatagatgga tggagaattg atggaggaat 62280
ggatacagca ttgatggatg gatgatggat ggatgaatgg atggatggac agatgatgga 62340
tggatggtag atggatgaat ggatggatga tgattaatgc aaattcttgg cttagcttaa 62400
ctcaggtaca aatcacccag agttctggct tcaaatcagt cactatgttc caggaacaaa 62460
gcctagagat cccagacttc tctggttcct caggccaaac cagacattac aaggcccttc 62520
acaacagcta ttctcacgcc atggggtccc tggcccagct cagcaaaagg acccagctgc 62580
tgaccaccca tccatttgct cccacttact atggtagaaa gccttctccc caaaatgaat 62640
gaagctgctg ttgacagggc ttggaaacac acttcctttt aaccataaca tctatgaaag 62700
agtctttgta aactatgaag agttgcactc acagttttaa aagtgtcaat ttaaactaaa 62760
gattcctgaa atgcctgcac agcccaggcg agccggccaa ctcagggttt ctcaatcttg 62820
tcactgcaga aattgggggc cagataattc tctattgtgg ggagctgtcc tgggtatcat 62880
gggaagttta gtagcatccc tggcctctaa ccactagatg tcagtggtgg ttaccctgac 62940
agagttgtga caaccacaaa tgtcaccaga tactactcaa tgtcccctgg gaggcaaact 63000
cacctctggc agagaaccac tggtctaaat gaacaaagcc aactaatgag gactgtgggg 63060
acccaaggag gccacaaacc catctgaagg ggccagcagc tactccgtca ttgccacagg 63120

ggatataagc caagtgttgc tggaacttct gatttttcta gataatggaa acccagagtt 63180

tttatgtgaa gtgtacccat gttttttggt gtgtgtgtgt gtgtgtgtgt gtgtgtgtgt 63240

gtgtgtgtgt gtgagactga gtcttgctgt cacccaggtt ggagtgcatt ggtgcaatct 63300

cggctcactg caacctctgc gcttcccagg ttcaggggaa ttctcctgcc tcagcctcca 63360

gagtagctgg gattacaggc gtgtgccacc acacccggct aatttttcta tttttagtag 63420

agacggagtt tcaccatgtt ggccaggctg gtctcaaact cctgacttca agtgatccat 63480

gtgccttggc ctcccatagg gttgggatta taggccacag tgcccggcct acccatattt 63540

aaactttagg aaccaatttc taaaaatttt gaaggttgta gtggccgatc tctcctgtgg 63600

gctcaggaac cattggtttg cactttctgg tatagttaaa tgtaattcca gaaatgacaa 63660

aagaatgcca ccactcccag gccataagag atgaaccttg gggcagaatt agcagacagc 63720

aaagaagtaa accagtgagg agacacaaca cagcaagggt ggtgacggct ggcagcatcc 63780

tggtgtgctg gtgcaaagca cgcgctctgc agttagagag tcctgagttc aaatcccagc 63840

tttgcttctt ataaatctca ttatctctga atttccaaat cctagttccg ggtccagata 63900

cacatgggga gggagggaga tggatggatg gcagatggag ggtgaatgga tggatgaatg 63960

tataaatgta tagaggatgg gttcatggat ggatgggtgg gtagatggat ggacggatgg 64020

gtgatggatg gatgggtgat ggaaggatga gtagaggata gatggatgat agaggatgga 64080

ttgatggatg aatcatggat gaatagagga tagagactct acatgagtct caataagtcc 64140

catgagcctc agtttcccta tctgcaaaac ggagatggta acagtagtac ctgcttcgtg 64200

aagtttaggt cttgtaagat gctgagcaag ggcctggcct agtaggtgat aaagagtgga 64260

agccacctat attaggttga atgttggtct ttctaaaata tgtccacctg gaacctgtga 64320

atgtggccta atttgggggg ttacatttat ttttttttcc tttttgtgga taccggggtc 64380

tcactatatt gcccaggcag gtctcgaact cctgggctca agctatcctc ccgcctctgc 64440

ctccctgaga gctgggacta caggcgtgaa ccaccgcgcc cagcctgaac gtggcctatt 64500

ttggaaaaaa tcggtcttcg cagatggaat caagtacaag atctcaagat gagatcatct 64560

tggattaacc tggtgggccc tacatttaat gtcaagtgtc tttatgatgg acagaagaga 64620

agacccacag agtagaatgc cacatgaagg atgaggcaga gactggagtg agacaagcca 64680

aggaaaagcc aggaacagcc agagccacca gaagccagga cagaggcaag gaacagatct 64740

ccctcagtgc ctccaggagg aacaacccct gccaacatct tgtcttttaa aaaaaaaaaa 64800

attttttttt tttaaatttt tagtagagaa ggggtcttgc tatgttgctc aggctggtct 64860

tgaactcctg gcctcaaacg atcctcttgc cttggcctcc caaagtgctg ggattacagg 64920

catgagccat tatgcccggc tcctgccgac accctgattt cagaaccctg gcctccagaa 64980
ctgggagaga ataaattcct gttgttttaa ggcatcaagt ttgtgctaat tttttacagt 65040
ggccttacac ctctcttctt gctgaggaat aagaaacagg actttccaac atcagacatg 65100
tgtaccacca gggatacaca ggatggacaa tttaggtgat tttatttttt atcttcaaaa 65160
tacattagaa gcagtataac caggccaggc atggtggcgc atgaggatat cttgaggcca 65220
ggagttcaag gctgcagaac ccaatgtggt ggggaaaatc ctggtgtccc ctggtcactt 65280
ggagcctcat gaagctccag cctgaagcct gacccacccc cttctccata ggccccttga 65340
tggagggttt ggaagtagct cttggtttat aaatggggtt ggtgtacatt taggactaag 65400
ttggttccta tgagagttcg tcatttgcac gccatcgtct gaatgcttgc tctgtgtacc 65460
atctgctcca ttatttgctt aatcattttc tttaaattta cttccttctc tttccttaca 65520
tttgttttaa tagaaaactt tctctatctt cagtgacaaa tcatcttcac ttgccataaa 65580
tagatggcaa ttataaatat aaatacaagg aaaacagtat taccaaattc tagctagatg 65640
cttggggctg ccgaaggtgc tgggcctgga gtctggtctc tgtggttaaa ggggaaacaa 65700
gtgccaaagt acattgagaa cactggggac tgggcatggt ggctcatgcc tgtaatccca 65760
gcactttggg aggccaaggt gggctgattg tttgagccca ggagttcaag accagagaat 65820
actggataac atggtgaagc cttgtctcta cccacccctc caaaaatcaa accaaaacaa 65880
aacaaaaaac cccaggccag gcacggtggc tcatgcctgc aatcccagca ctttgggagg 65940
ccaagatggg tggatcatct gaggtcagga gtttaagacc agcctggcca acatggcgag 66000
acccagtatc tactaaaaat acaaaaaaaa ttagtggggc atggtggtgg gcgcctataa 66060
tcccagctac ccaggaggct cagccaagag aattgcttga acccaggagg cagaggttgc 66120
agtgagccga ggtcgtgcca ctgcactcca gcctgggtga caatagcaag cctccatctc 66180
aaacaaaaca aaacaaaaac cctaaaatca aaaattagcc aggcgtggta gcacatgtct 66240
gtagtctcaa cgacttgaga ggctgagctg ggaggactgc ttgagcctgc aaggcagaga 66300
ttgcagtgag ctgagactgc gccaccgtac tccagcctag gtgacagagt ggtgtgatct 66360
cggctcactg cagcttcgac ctcctgggct caagcaatct tcctgcctca gtctcccaag 66420
tagctgggac tacagtgtgc accaccacac ccagttaatt ttttgtatta tttgtagaga 66480
agggatcttg ccatgttgcc caggttagtc tcaaactcaa gctcaagtga tctgcctgcc 66540
ttggcttctc aaagtgctgg aattacaggc gtgagccacc atgcccagca aaacaattta 66600
aatggtctca ttgttttctt tcacaaggac agacaatgat ttaataaacc ctctattaat 66660
caacctattt tagtttgttc cagttttttt ctatttattt tttttgtaga gacagagtct 66720
cactgtgtta tccaggctgg tctcgaactc ctgggctcaa gcaatcctct tgcctcagtg 66780

tctccaaatg ctgggattac aggcatgagc cactgtgccc agcctgttcc agttttttat 66840

tatttaaata aaactatact gaacagtgta gtacatcaag aaaaaaggat ttggtaaatt 66900

cctggcacac agcaaatact taaccattgt tcattgttat ttaaaagcaa gattagaagc 66960

aaaactgacc cctgtcccaa atgaaatgaa tcattcttgg agggcctatg tcattcagac 67020

aaggaggcta tcagcactag attctgatgt gggaccctgg ccaagaccca catacaccat 67080

ccatttgtca actgacccat acagcctaat tttccagacc ccaagttatc ttcggaggag 67140

ctgcccctcc ccttatgcag catttcagca acctcagtgc tattctgggg ctccaaacgg 67200

gcgtaggcgt gggaaggaag ggaaactttc cacagttctg tcctggtgtt cctgaaagag 67260

attaaccttc aacactgagg cacatctaac gcagaaggaa aatcgtttct ggaaacacag 67320

tgtcccgacc aagaatttct ttctgagctg tcctgttctc aagacccagt gtgtttcttg 67380

gccattggtt ggatgatttt gatcaatatc aattgatcac cagagcaacc cctccaggaa 67440

aagcctgagt aaatccactg cctcgatcac tccccctccc ctaaaccctg cccacatgtt 67500

cctgatgagg ccaacatctc taccttcagc tccaagaatc cattcttagg gaccataatg 67560

aaaccaccct gcgctttcca caaccctgcc tatgctgcat agctcaggaa tgtgcatatc 67620

aacctaagcc aaccaatcag agtcaatgac actccattca ggaacttaag ccgggaatct 67680

actctgttct ttggacataa acccaaagaa tataagggct ggaacccagc tatgttgctg 67740

tcaacaaact gcatcctata gagaaacaat gtagtcgaga gagatagaag acaagaaatt 67800

aggttctaga aaaatctttt gaacacctag atctcactgt acctgaagct tgaattcctg 67860

aacttttttt ttttttgaga tggagtctag ctccatctcc caagctggag tggagtggtg 67920

taatcttggc tcactgcaac ctctgccttc cgggttcaag caatcctccc acctcagcct 67980

cctgagtagc taggactaca agtgtgcacc accagccctg gctaattttt tttttgtatt 68040

tttagtagag acagggtttc accaaattga ccaggttggt ctcgaactcc tgacctaaag 68100

tgatccgccc accttggccc cgcaaagcgc tgagattaca gacctgaacc actgtgccta 68160

acctccctga acttctcaac attctgagtt agtcactttc cctttggttt aagccagctc 68220

gggttacttg caaccctagt tatacagcca cttttctaca acacaaacaa accacctaga 68280

ataaaattca ttttcctcct tttttttttt tttttttaat ggaaggtggg gaattcagag 68340

agcagaaatc tgtcctgacc agagaagagt tcttaacagg ttccactggt atccaggcaa 68400

gctaccctcc agactcaagg tgtgaggctt acatagctgc tcctcctcgc tgaaacccat 68460

gattgccatg gcctccacgg tttcctggaa catctcatca tcctgggctg ctgggatggg 68520

cacaaagcca ttggagagga aggtgtagtt gttgaagccc tccaaaagca agtcacctag 68580

aaggagagga agacaggtca gggtcaataa gacatacttc taatttcaac atgagaccga 68640

gatctggtca agggtctggg ccatcataat tcctttccct ttatcaaggc ttgaaccgtt 68700

taaagccctc acgcatcgtc acgtagacag cctcaccaac cactgccagc cttacacagc 68760

ggtgcaggtt gtgcactgct ccagtgactg cagctgatat ccagccttgg ttctgcttgc 68820

taagccacaa tccctggcac gaagttacat ttacctggag gaagggatgc ctttacctca 68880

attgtctaga ggcaccctgc cttcagcctt cagagacaaa gctgtcagaa gagttagatc 68940

aacctttact cttttttttt tttttttttt tttgagacgg agtctcactc tgtcgtccag 69000

gcaggagtgc agtggcgcga tctcggctca ctgcaacctc cgcctcccag gttcaagcga 69060

ttctcctgcc tcaacctcct gagtagctgg gattacaggc gcccgccacc acgcccggct 69120

aaattttgta tttttagtag agatggggtt tcaccatgtt gaccacacta gacttgaact 69180

cctgacctca ggtgatccac ccaccttggc ctctcaaagt tctgggatta cagacgtgag 69240

ccaccgcacc tggccttatt catgtattta taaaattttt atcaaattca aacaggcaca 69300

tgagttttaa aaatcaatta ctaccaaagg tttataatga aaaatagcat tctcctgtca 69360

catcccttct ccaactctac cttactccat aatacctgct tttatctctt ttagctgctt 69420

tttatctggc agttaccact atctctctgt acaataggct tgctatatat actagcctat 69480

tataatgtat ataccattat gtattacata tcataggcca agggttcccc aacccatagg 69540

ccatagacca gtaccggtcc atgttaggaa ctgggccaca cagcaggagg tgagcagcag 69600

gtgagcaagt gaagctttat ccgctatttg cagctgctgc ccatcgcttg catcactgtc 69660

tgagctccgc ttcccataag atcagcagca gcattagatt ctcataggag cgcaaaccct 69720

atcgtgagct gcacatgtgc cctccttata acactctaat gcctgataat ctgtcactgt 69780

cccccatcac cccccgatgg gactgcagga aaacaagctc ccaatgattc tacattatgg 69840

tgactagtat aattatttca ttgtatatta caatgtaata ataaagtgca cagtaaatgt 69900

aatacacgtg aatcatcccc acaccatccc cccaaccctg cccgcccagt ctgtggaaaa 69960

cttttcttcc aggaaaccag tcctcagtgc caaaagtaac aggtgctttt ggcctattgg 70020

aagaccagag gcaaagcaca atcgctgtaa taggcgattt ctggatatgt caagtttaga 70080

tagtatctag tgacttcctg ctatggtagg caaaaatgca gctttcttat actacctttc 70140

ctttcctcca gctatttttt tttttttttt tgagagggag tttcaccttt gtccaggttg 70200

gagtgcagtg gtgcgatatt ggctgctcac tgcaacctct gcctcccgga ttcaagcaat 70260

tctcctgcct ctcgagtagc tgggattaca ggcacccacc accatgccca ggtaatttgt 70320

tgtattttta gtagagacgg ggtttcatca tgttggctag tctagtcttg aacccctgac 70380

atcaggtgat ccacccgcct cagcctccca aagtgctggg attacaggtg tgagccaccg 70440

tgcccagcca cccccagcta tttttagttg aatcaatata taaggcttat atcacaggtt 70500
ggcaaatctg gtcccctgtt gtttttgtaa ataaagtttt attgaaacac agctgctctg 70560
atgtccttgt ttatttctca tggctgcttt ctcactaggc aacagagttg agtagttgca 70620
acagaggcca tctggtcctt aaagtctaaa acgtttacta ctgacccttt agcaaaaaca 70680
gtttgccaac tcccttttta tgacattaat tagatattct gtgtcaaact gtatcctatg 70740
attacatttc ctttgttgtt gttcagcttc tcccccaccc ccaactttat aattgccttt 70800
ctttctcctg caccatttgc ttttattata ccctccaata catcaaggat cctcttccag 70860
atcaatcttt aattaggatg gattcggtta tacttttgga gagacgtcag caagatattt 70920
ttctttcaaa atcaaggtct tttatgggtc aagtcacttg gaagctgctt gataaactcg 70980
aggttaaatt gactgaaggc accagttaac tcaaactttt ggattaactt gaaatgcctg 71040
attcaagtca cgaggattga ggtctttgcc aagacttcat tatcccgagt ttggatgcat 71100
ttagcaatat ttggttcttt ctaaaagcct aacatctttc cagttctatc atctagaata 71160
caaacggccc ctgggaattt agttctgagc cctatacttg cccatttctg gaagaccaga 71220
ggcaaagcac aaaactggaa cccaaggtac caaacaacca aactgttttc ttgggtgcca 71280
aataaaagga aaaggaatgt caacattttt ccctggccca ttccacaggg tcctgcactg 71340
taactgctaa tataccatgt ttgctggtac ataaaaaatc cattcagcat tcccccttta 71400
ccaaaaattg ctccacctca tagcctatgt tcctgaaagc cagccattaa ggaaacttat 71460
ttttattgtt tgagacagag tctcactcta tcgcctaggc tggagtgcag tggtgcaatc 71520
ttagctcact gcaaactcca ccttctaggt tcaagtgatt ctcctgcctc agcctcccaa 71580
gtagctggga tgacaggcgc cagccaccac acctggctaa tttttgtatt tttggggaga 71640
cagggtttca ctatcttggc cgagctggtc tcaaactcct gacctcaggt aatccgcccg 71700
cctctgcctc cgaaagtgtg ggattacagg catgagccac tttgccccac cctggaaatt 71760
taaataatgg ataatgccac cccctagtag ctgaggtcta caatacactc cgacacagcg 71820
aagttctgag ctcacacaga tccttttggt aactgtctta accaaaataa ctaaccttgg 71880
gccacaaaag agatgggaag caaagtctgt attgttccca tagaatttca gactcaaaac 71940
ttattactaa catgtagact ctactatgta cagatagtca ctgatccatt ccttttttaa 72000
gctatgcgat ttgtatatat ttttaagagt tagtaaacag ctgcaggtcc agaactcata 72060
acttcctact ggagtttggt atacagtgta ttatatttac ataaaaaaac ttttccctac 72120
aacagaggtc agcaaacttc tgcacactgc ctgtttttgt aaataaagtt ttattgtaac 72180
acagtgatgc atctgcactc actgatttat gtgttgccta tgttcattgg tttatgtgct 72240

gcctgtgctc attggtttat gtactgcctg tgctcattgg tttatgtgtt gcctgagctc 72300

actggtttat gtgttgcctg agctcactgg tttatgtgtt gcctgcactc attggtttat 72360

atattgcctg tggctgctct tgtattacaa tggcagagtt gaatagctgt gccagaagcc 72420

acctggcctg caaagcctga agatttacta tctggccttt tacagaaaaa gtcttgaccc 72480

ctgttctaca aagtcactgg caaaacatgt ataatgaaaa atgcttagtc ctgctgcccc 72540

tgcaaattcc ttataaaacc agaatgcatc ttttatattt gcaagtttta taaattggca 72600

agtaaggcaa atgtctttct ccctttcctt gaaggcgtag attgtgtcta gttggacttg 72660

atgctctctg gtctaatgag acatgaatca ttaatgatct aatgtctaat tggagttgat 72720

ggctactatg tcacatagta ggtgctcagt aaatgagaat gactgaacac aagccacact 72780

cactcattgg gaggagatga agaactgttc atatgtcact cacagacatg gtcctttctg 72840

gcaggatctg agactgtttt aatagccaat cccttaaccc cggattctga tgaaagggaa 72900

ggctcaagcc atccaatcac atgtcattgc tagtcactta cttctcatct tctccttggc 72960

tccagcaatc atgtagtaaa agatgtggaa tgtcctctcg tctctggctt ggcgaattgc 73020

ccgtgatttt tctagcagat ctggtttgga gggagttagg gattctgggg atactgcggg 73080

tgttcctctg gtcttccacc tccatccctg tcacacccaa ttcacatggg atgactttcc 73140

agttctaccc ctgggatcgg taatgtctaa ctttgaccat tgcccctatg gacaaatgga 73200

accgctgggt gctgccagca gacttcagat gcaaaaaaaa gggaggggaa tagagtgaaa 73260

agaaaaagca taaaaaggct ccaggaagaa ggtaatggta acgagatgtt cccctaacag 73320

ccatcaccag caaatcttct ttaaaatgag ctgtcttcag acatgacaga atacaaataa 73380

tgacaccatc tatgatccta tgatttatga ttcattcctg tgtgaaatga ttgatataaa 73440

ttgtctcact gaagcctcac aaagtatgct aggaaataag tgcaactatt atcggtgttt 73500

tcaataaggg gaaactgagg ctcagagaag agaagtcctt tctcaagatg atacagctaa 73560

caagtggcaa agctgtcttc catgccctct gttccaagag tggggtctga cgggatggag 73620

gcgagagaga ccgtaaacac gccacactaa ttaatttcag gagagcttct ccgcgatgcg 73680

gggtggcagg agggagagca gaggggaggg caggtgaggg tggcatggag atcacagggt 73740

ccggggtgat gggagagggc agctgggagg ctgggctact gtactgggtg gatttccaca 73800

gcactaagca tgatggtgcg ggatgatggg gctagatcag cagaccttgt cttcttcaag 73860

tcctaaagcc agctagcagt gcccgggcta ccacagccca tgtccaatct ggaaagcagt 73920

gattctcaga ctttctcatg gtgtacatgg gtatacacac gcacacatac acacacacac 73980

acacacacac acgcacgtat gtttgagcca gagtctcgct ctgtcaccca ggctggaggg 74040

cagtgtgtga tcttggctca ctgcaacctc tgcctcccgg atccaagcaa ttctcctgcc 74100

tcagcctcct gagtagctgg gactacaggt gcatgccacc acgcctggat aatttttgta 74160

tttttagtag agacagggtt tcaccatatt ggccaggtgg tctcgaactc ctgacctcaa 74220

gtggtctgcc cgcctcggcc tcccaaagtg ctggaattac aggcgtgaac caccgtgcct 74280

ggctgagatg tgtgtttcta aagagctccc aagtgatgct gatgctgctg gtttggaaac 74340

atactttgag aaccactgat ctaaaaggaa ggctgaggcc agaaggacgg ggctatgcct 74400

ggggctcgct ctaaacacag gaaatgaatt gctgagaggt gtgtgcaggg ctgcaggaca 74460

agagggccct ggctctggac ttgaccgaag cctgacagcc ctcctagctg caggatggga 74520

atcctggctg gtttgggcag attaaagtag aaacgtagaa aacattgagt taacgcaata 74580

gccaccacct atgagatgct cactttgggc ccagtaccgt accaagggct gtgcatatta 74640

ttatgcatat tatttcattt aacccctgca ccagctccag aaactaggtg caatcacccc 74700

cattttaccg aggcaaaact gaggcccaga gagcttcagt gattttccca aatccccaca 74760

gcttagacaa gtacagtgga gaggaagcta ctccacaacc caccctctta tccagggcac 74820

ataacgtgga aaaatgggac cactaggtgc tgctagagga cctcaggtgc agaaagaagc 74880

taagtcaggc ccccaggtcc tatacattgc caagcacgtg ttccacagga agcctgagcc 74940

ctagggccgc ctgcaaagct cctgaggctg atcagtcact gaactctaga cctggttgtt 75000

cccccacgtc cagaaatttc taacttggct ccacttcacc gctcaggttc atgaaagaga 75060

tgctctactt gttccttgcg ataccagcat tcttctgggt aaactctgtt ttcatcaatt 75120

tcctacctaa gaagtggagc cgatcacaga ccaactctct aagaggacga caggtccaga 75180

atttggcctc ggacaatggc tgaataagga tgaagtgaag atcatacgat agacactacg 75240

tagccagccc ttactctgca cctcgcatgg tgcctagagc tttatatgag ttcattcact 75300

ctattctcac atttctatga ggcaggtacc attcattatc atccccattt tgctgatgag 75360

gcacagagag gtggagtaac cagcccaagg tcactcagct aagggacaga tcatttgagc 75420

atcacatgca gcctaggctg cttgtttagg gttcaaacca ggggtccggt ccacacatgt 75480

ataaccactg caccacaatg cctgctgtta aggggagatt tgttctggaa gaaacctaag 75540

aggctggaaa gatagaggtg gctcttggac tctcccagcc tctgggcagg agattggtag 75600

ggggcagggg tacccatttg gcccaggctc agggaaagga tacaggtctc aatgttggct 75660

cccacgatgt aacccgtgac gtcgaagttg atgcggatga atttgccctg ccaacaggaa 75720

aacacagttc aggctttgct gcccaacttc agccgttaac cccatgctgt gggcaagcag 75780

gaggcagatg gtacagagga tgggaggtgg ggcggggaga ttcttgcgaa cactcagaac 75840

cccacaggtc aagttgtcag gcggaaccaa catctgctga gctgaaacca ccccaggcct 75900

gacagcaaca gaacgtgtaa aacatgttca cagatcaatt tcattcggct tttgcaatga 75960

ccgtagtcca tcatggctat ccctatttaa aaaaacttat ttatttatta gagacaaggt 76020

tttgctctgt cagcgagggc tggagtgcag tggtgcaatc acagctcact gtaaccttga 76080

actcctgggc tcaagcaatc cccttttccc catgacctca ccctcccaag cagctaggac 76140

tacaggcatg caccaccata tctggctaat ttttaaattt ttttttgtag agataggatc 76200

tcactgtgtt gtccaggttg gcctcagact gctgcactca agcaatcctc ccacctcagc 76260

ctcccaaagt gctgggatta caggagtaaa ctaccacacc cagcccacta tccccatttt 76320

aaagctggga aaactaaggc taaaagagat caagtgactt gtccaacgtg acgttgctcc 76380

tgagaggtag ggccctaagc agaaagaagg tttttccata ttcaggggct agagggctcc 76440

ctccccagac cccaccctgc tccaaaacat ccacatcatc caactatgga ttaaaccttt 76500

tctgtgggac aggtgagtat gcattggcca cactgcccaa gcaggccctg tgtaggatgt 76560

cctttaggca ggaatggctc atgttcctgg ccctagaggc tcaacaagta tctccaaaga 76620

tggccctatc tacctgcctc agctaagcca tgaaaccaag gatggaaccc aatcctccat 76680

ctgttggcat gaaccctttt cctgtttcct caacaggtga gacaggattt catccttttc 76740

ccccatcatg acataacatg gtgggaagtg ttggcgtgtg tggacacaat cccctaggct 76800

tgctcagtcc ttgacaaaac cttcaaactt tccccattgg gaaacatgca gtagagtgat 76860

ttgggatgac catatcccag atggagccag ggagagccgg ctggtcacag gcaaggccca 76920

ggcaccttct tcactctcct tcccaagatg gcagggacgc cagtgagtgg aggatgtttt 76980

tagggatttg tcttgcacgt gcttccattt atgacaggct agaaacattg cacagaattg 77040

gaacatgcaa acgcagtgct tctcagtgga ggcggcacat ctgctatgga gattttggaa 77100

atcggtggaa gccattattt ggcttgtcac atgttggggg ggggccgctg ttgttagtga 77160

acagtggggc caaggatatt ttgggagtcc tgcaatgacc tggagagtct ctgatgccaa 77220

tccttgtcta tatcccttat gactcttcca atgtctcacc tgccactcat ggaggtaaaa 77280

atctgtttgt aattagccag gcttagaacc taacctttat gactttagat ttttacgctt 77340

tttttttttt tttttttttt ttgagacaag agcctcactc tgtcgcccag gttggagtgc 77400

agtggtgtga ctttggctca ctgcaacctc catctccccg ggttcaagga attctcccac 77460

ctcagcctcc cgagtagctg ggaatacagg catgtgtcac tgcgcccagc taatttttgt 77520

atttttagta gagacagggt ttcaccatgt tagccaggct ggtctcaaac tcctgacctc 77580

aggtgatctt cccaactcag cctcccaagg tgctgggttt acaggtgtga gccactctgt 77640

gcctggtcta gatttttatg cttttaaatg aaagcattaa caagtgttta aaatgttgtt 77700

ctaatacaag aatatcccat ctgtgtgact ctagagaaga ttgttctttg gttcagcgct 77760

tcactgagag tcagttgcca ttctggaaaa tcccatgcca acatcatcag tgcctgaact 77820
tgaccctttt ccttgtattt gtagctgtcg ccgtggatag acctaagtgt ctgtctctta 77880
ctgtgtcttc tgttggtgtg gcctggcccg agcattgata tgctgaaatg cactttattt 77940
atttattttt gtagagatgg ggtttcccca tgttgcccag gctggtctca aactcttgag 78000
ctcaagtgat ctgcccgcct cagtctccca aagtactggg actacgggca tgagccacct 78060
cgcctggcct gaaatgcaca ctttcattat gaattgtctt atgttatttc ttcttcatat 78120
tccagttagg gcattatgct gatgtgtatt catctaatac gtgtaggagg tgatactatc 78180
tatgattcta atttcgggcg aggaaaggag acattatgta attgtttgtt actaaaatgg 78240
gccttgggga tcacagggtc agggaccacc atgtgcattt gctcagcaga gcagagcttt 78300
ctgcaaagag tctttcacag ggccccatct cagcgagcaa cagttcagag acccccttct 78360
cctggaatgc agaggcaatt tttccttcat cttttctcac taaaatatct ttctgaccct 78420
cctacggaga caaccgcttc ttttgcacaa taaaaccatt gttgtctacc tatcatcaac 78480
catccacttt cagagttccc ctgccctgtt gggctcaggc aaaaactttt tgctttggag 78540
cctgggggag tgtgcatggg aaggtagtac cccctgcctc acttactctc tgttattcag 78600
ttggaagaac cagcttcatg aaggctggaa ccatatctga actattcatc gcaatcctgt 78660
gagactcgct gagtgcctgg tatacagtag gcacttaata aaaacagcaa atgagaaaaa 78720
aacaaagaga atggtggtac atgtaaaagt gcctaccaga gtaattgtac acagtaggta 78780
cttactaagt atttcttgga ggaggtgtgt gcctggtaca cagttggcac tcaattcatg 78840
ttttgttaca taaaatggta tcggatataa gggtctagcc cataccaggt gctcaatacc 78900
taatttttta aattttattt taataataga gatgggatct cgctttgttg cccaggctgg 78960
tctagaaccc ctgggctcaa gtgatcctcc cacctcagcc tcccaaagtg ctgggattac 79020
aggtgtgagc caccgcacca ggccaatcaa tatttgttga gtgaatagtg aataaattaa 79080
tgaatgaatg agagagtgag aggcaggagc ccttgcaaga ttgtgagata cagcccatct 79140
ctccagggtc agatgcccct cccacctctg cacgcaaaca ctctgcagcc ccaggcagga 79200
gatgacacca aagcttttct ggaaaatcca tgtggctttg ctacttacga atcgtgagga 79260
gttgtcgttc ttcactgttt tggcgttgcc gaaagcctcc agaatcgggt ttgcttgtag 79320
aagctgcttt tccagctctc cctaaaattc attcacatct agttattgga gaaagcaacc 79380
taggtcctga cagttctacc ttggatggat gttgtcacaa aactctgccc ttaacccaca 79440
ggttctctcc tatctcctac ctcctcttag accctgatgc taatgttcag atttacagga 79500
ggatttcaga agagaagcaa ctgtctgagc tctctaatag ctaggacatc tgctccctag 79560

gaaggtcact tttttcaaga ccagggcctg ctcatctgtg caacatcact gccactcaaa 79620
gccactgcct tgcacaatgt cggtgatgtc atttacatca tgatgaatgg tgccccccgg 79680
agttgtgcag tgcaccacct gtcccactgt acatggcagt cgtgctgctg ttcatgttta 79740
acaagcagcc ttcttcaagt taaaccacta caagcctcct tctaattatt ttagtgcaat 79800
tttttttaag ttgcaatttt ttctttctca ggactggttt tctgaagtct tctacagcag 79860
acaagtaagg aatgcacaaa acccaccagg aatcctctta gcggactgcg gaataaagct 79920
tcaaagcagg ttgcctgagc tccattcatt gggttaaagg aagctgagac tgtacacagc 79980
ccaccggccc cctctgccgg gatgcctttg cacaaacgcg gtcacgtgat taatcatggc 80040
aggaaagggc caatgtggta agttaagctg ggaaagcaaa cgagctgctc ttggcctagc 80100
cctaaaacat ggcggctgca gttgggcccc gaggcctcca ggccctggat tctgggagtt 80160
tttctccttg tcctcccctc ctgctttccc ttccttattt ttccaggcat cagggctggg 80220
ctggacagga gccgggaaag tgtctagagg ggacaaggat gggcatggct tcctcctcat 80280
tcctccgcat gctaggaagg catcagggta cctggtctac aggacaaagc aggaagctgc 80340
tatttgtttc tgttttgatt tgtccacaca tcctcctttt tgtagctccc agagaaagat 80400
gaagcaaaag cctcatccgc atacaaatct cagctacaag tggaatcacc acagctgaga 80460
ctggtggaaa tttacaagga gcaccacggg catttctttg ttggtgggaa gacgactaaa 80520
ggaatcactt ttgttcacat aaacttgggg tctccatgct ctctgaaaaa ggctcccttt 80580
ctagaatggg gattctggca ggtaaggttg aaaagtcttg gcaagagagc tttaaatcgg 80640
gggacctggt tcaaaccctc aattttatag atgatgaaac catagccccg aggagttaac 80700
tgactcggcc aaggtcacac aaaagcagat cccaaataag aactgtaatt ctccttctga 80760
tctgcaacac agtcacccag ggtctaacta agatggattg aatgatcttt tcgggagcca 80820
aggaagacac ctgtgggttt cttctaggct gggacccaca catcagcccc ccaagctctg 80880
tgttagagat gagtttacat atgcacaggt ccaagcagga ccaggttatg cgttgcgtag 80940
ctggggcctt accgcgccac caaagctagt gcatcgcgac ctccaagact gtttctggag 81000
gcttgagtca agcaaccagc atgcaagaga cagacacgca ctgcacacgc atgcacaggg 81060
acacactggt gcaccaggga cagggctgag ggaagacact tagggatgat ggcagggaga 81120
gaagagggag gcatgccaca gctgtgccat cgactgtcca tcgagccagg gaagctttgg 81180
ggcctgacag tcctaaaaca ctcagccaga tgggacagac atctctgtct aggttgttcc 81240
agtcaagggt accaggagcg gatgctttga agggtgcagg ggttcattct gaaccggaga 81300
aatccatttc aggctggagc gcaaccctgg ggactccaat aggcgtcccg tgggtggtaa 81360
atgcaccccc acacccttcc ctggcatcag acacccacag gatggtattt tggtgcattt 81420

actgatttaa gttgtgaaac tctagggcct accccatttc taccaccagc tacgggactc 81480
ggtgggtgca agaggatcat gcagatctaa gttcactccg tgcctcccct acacaccagg 81540
aaaacactct cagttactca cgtaggcaaa agatgggcct tgctgtggtt gaacaacaca 81600
gtataaaaca aatgtcagcg ttatttccat cacatggaca tcaggggctg gagaatatat 81660
gactttgatc cccccaaaca gcctggagtc tcccagacag actggtcagc tccttttcct 81720
tgatactgtt tcttttctct ctgtttagct gcaggaatac gatcacagct cactgcagtc 81780
tcaacctact gggctcaggc aatcctcctg cctcagcctc ccaagtagct aagactacag 81840
gcatgcacac caggcccagc taattctctt gatttttttt tttttttttt tttggtacag 81900
acagggtctc actatgtcac ccaggctggt cctgaactcc tgggctcaga ccatcctccc 81960
accttggcct cccaaaacac agattacagg catgagcccc catgctcagc cattccctcc 82020
tttttctaat ttcttagaga tccagacatt tcaaaggttt tcataattac tggggccaca 82080
ttaggctcct taaaacagta tccagtgaga caatgtctga gatgtgttta cgcatgcagc 82140
caaaacaagc agcacatagt aggcccttga taacgatgag ttgttagaaa gtattatctc 82200
ctgtgtttac tctttaggga aagggaaggc agtggaaact gtgtttaaac tgccacacag 82260
cctctggggc cattgttccc actcactatg gctggaaacc aacttgccag agacaattct 82320
aggatgctga atcttgagtt tcctgcattt gtttggagaa ggaaagttct ccaaagccac 82380
gaccagcgcc catcctagat ctccaagggc ctccagacaa gccggccatg gtggctgccc 82440
cagagtcccc tctgaggacg caaagtgaca tctggaatgg cgaacactgg agacacccca 82500
tgccgtggtc agatctccat cctaccatct tcccacccac aaacctgcag acggaggcac 82560
cggccagtcc tagcggccct accacgtgcc ccctcctgtg ggatagatgg ggcagggctg 82620
acacacgagg aggaagagaa ggatgggccc tagaggtgat gactctagaa acctgctctt 82680
cttttcctcc caagctctta gtcataccgt gtcatcatct gtttatttga gggattcttt 82740
gacaactaca tgctcttcca caggtctgga agctcagaga agagaataac agcatctctc 82800
agacttatcc ccaggtcccc accccaagtg gttcctggca tacagtaggt gctcaatcca 82860
cagttactga atggcattgc actgaagccc acgggccgag tgataccagc atctccatcc 82920
cagggacagg gggacacgtg gaggaatccg ggaacccaaa aggtacctta acccctggga 82980
cgttacagag attcatgaaa ggagtgcttc tcaatcaagg gcaactgtcc caccccaggg 83040
aacatctgac aatgtctaga gatatttttg gttgtcacaa ctggagggaa gggggctgtc 83100
ggcatctggc gggtcgaggc cagggatgtt gtgaaacatt ctacgagcca cagggaagcc 83160
cctccaagga agagttatct ggtccaaaat gacatcagtg ccaccactga gaagccccga 83220

cgtggaggaa gatctcgcta tggaaggaaa gggcttattc tcatttcaca aaacgggccc 83280

ccttctggag ggatgcttct agctgagcca agaaatcacc cacactcaac aggcctgctc 83340

gccaaaaaga cggtccctct ttgagtcttc aggggagggg tagtcagatg gggcctgagt 83400

tcttgcaatg atgttctgtt tgtatctcgg ttgggctgca agctggagac cggttggcta 83460

aatcatggcc tctgattggg aactgccact caccgtgata cttgtgtctt tcttgccctt 83520

gtgggaggag gccaccacgg ccaggtactg aatgaccttc ttggtgtttt cggttttccc 83580

ggctccagac tcgcctctga aagacacggg aacatcatct atgcacacgt tccatggtga 83640

cctttccgca gttccatgag ccaggcagga caataatgat catcaccacc atttcccaga 83700

gggtgaaaca gaggctccca gaggggaagt aacttgccca aggccacatc tctggtagtg 83760

agcggaactg ggtccccagc ctaggtccaa tgcccaatct attccttcca agaggacata 83820

gaaaagaatt cagatacaaa gagtgaaaag gtgggccgga tgtggtggct catgcctgtg 83880

atcccagcac ttggggaggc tgaggtggga ggatcatttg agcccaggag tgtgagacca 83940

gctcagcaac acagagagac actgcctcta gaaaaagatt taaaaattag ccaggcatgg 84000

tggcacatgc ctgtagtccc agctatttgg gaagttgagg caggaggatc actcgagtcc 84060

aggagttcaa gaccagcctg agcaacacag cacaaccctg actctataaa aaataagaaa 84120

aattaaccag gcttggtggt gcatgcctgt agtcccagct actcaggagg ctgaggtggg 84180

aggatggctt gagcccagga gtttgaggcc acagtgagcc atgatcacac cactgcactc 84240

cagcctgggt gacagagcaa gacctcatct ctaaaaaaat agagtgaaca agaaaacctt 84300

agttttctta cataggtcca aacaggttta tttggaatta tctacttttt tttttttttt 84360

tttttgagat ggagtctcac tctgtcccca ggctggagta cagtggcaca atttcagctc 84420

actgcaagct ccacctccct ggttctcctg cctcagcctc ccaagtagct gggactacag 84480

gcacccacca gcacactcgg ctaatttttt gtatttttag tagagacggg gtttcaccat 84540

gttagccagg atggtctcga tctcctgacc tcgtgatccg cctgcagaca tcattagcat 84600

tgctagaaat ggaaagacca ggcacaaggc agccttcaag aaaggatgta agtgcctctg 84660

gagtaccagg acctaaagtt taagaagctc ccttatggaa agaaaaattt accaaaaaac 84720

cggttatccc attggtcact gggcagatgc aagccccttt atcctctgag gaatcccacc 84780

ccatgtatca ggaagctggc tgatgatctc agccaagtat ctaagaataa acccagttga 84840

agtaattcac actaaagtgt aaatgactga tatgtttaga tcagcacgac atggtttgtc 84900

gtatcaaaaa agtatgtcct cctggaatat gaaggtagat cttttttttt ttttttttta 84960

ccaagatggc tttcgtgcac tagctgcctg ttttggccac cagaaatcat cacaatggaa 85020

ccgctccatc cagatactgc ctctatgttc caccactcct agtgaaagat aaggacagtg 85080

ccttgaccaa aacttcactt gtttatcgtc ttcttctatt tttcagttta cttttgtttt 85140
ttgtgtttcc tttctttgcc ttatctcttt tattttctga gctcacttaa tactctaaaa 85200
tataagagag tttttttttt aagttgaaaa aagaatttgt tcaccccgag gtcagcccaa 85260
ggttctctgt gctgaaccag tgccaccaaa gctagtacag tctctgctct tcttcctaat 85320
taggaaataa agaaattaat tttctctgcc aaaggcccat taataacttg gctcatggct 85380
taaaaaagaa atagaagaaa agaaaaaaga aagagaaaga aaaaaaaagt gccccaaatt 85440
gctgtgccta atcctacagg tcccactgtc cagaaatcaa gtgtctgatg gccaagaatg 85500
atgaaacatc ttgcggtgac tttgtgtggt cccttccctt cctagaggcc ttccttccca 85560
atggccacat tatttcacat ggcctcagag gcagtttatg aaatggggac catgaaggca 85620
ttaccccatt tccccagaag gggaaactaa gaccagaata tatgtgtgtg tgtgtgtgtg 85680
tgtgtgtgtg tgtgtgtgtg tgtgtaaaag aggagaactg gccaggtgca gcagagaact 85740
ggccacgttt ataatagcag ctactcagga ggctgagttg ggaggatgcc ttgaggccag 85800
gagtttgaga gaagcctggg caacatagca agatcccatc tcttaagaca gagaagaatt 85860
ggcgtttgtc attgggggga tataggggga agggtccctt tgctaaatgc tctacatgaa 85920
ttatttcctt tatcctcttc ctgcttccat gttattatag aaggaagagt cttgcccaga 85980
gtgggtaagc aattttccct aggtcacaca gcaaagaagg gatacagtca gaatggaaag 86040
tcaggtctct gattctaatg cagtcagaat tccttttact acatttcacc aagttgttcc 86100
atgacacagg cccaaccctc tagggacttg ctttctaagg ccagaagaag ggcaggggaa 86160
ggggaattga tatttgttga aggcctgcac agtactaggc tttgggctca cagatctttt 86220
catttactct acccagcaat ctaatgagag agttattaat tatccccatc acccatccgg 86280
ggaaactgag gcaaagagag gtttgaagta aattgctcca gcccacaatt gctcctgctt 86340
tcacacagat aggaaatgga ggagaagcag gatttgaacg taggtccact tgacaccagg 86400
gcccatgttc tgcctcctac ccttttattt taacaaatca gcaaagagaa aagacagagt 86460
gaagtcccag ccccactcca ctggaattct gccatatatt ccaggaggct ttacttgccc 86520
caatcaatga accaagccac tcagtcattc acacctaagt atgaattact tcaatcaggc 86580
taacgtcaga accttgcttg acttcattct aaagtgaagg ggttggattc tgattaaagc 86640
aagagaaagg agagaaattt ttggatgtag gaaaccactt gcgtaggtgg tcatgaacct 86700
attcagatcc aagacaagaa tcaggacagc agtgtgcaca aacaacatgt gtggatcaca 86760
ctcttcatgc tgacaaacca gtgctcctag actgctgctg ctcacttctc ctttcccaca 86820
atagttcctt cctttccacc ctggccttga acctctctca cctcctccct tttacaacaa 86880

agacatcacc gggtgtggtg gctcacgcct gtcatcccgg tactttggga ggctgaggcg 86940

ggtggatcac ttgaggtcag gagttcaaga ccagcctggc caacatggtg aaaccctgtc 87000

tctactaaaa atacaaaaag tagctgggca tggtggtggg tgcctgtagt cccagctact 87060

tggggagctg aggcaggaga atcccttgaa cccaggaggc ggagattaca gtgagctgag 87120

ctcgtgccac tgcactccag cctgggcaat agaatgagac tccatctcaa aaaacaaaca 87180

aacaaacaaa caaacagaca gacaaaaaaa aaaacaaaga catcaatgtg ccctatccgt 87240

ggctatggtc tcccactttg cctgggatgg ggagtgggac ataaactctg ggcatctacc 87300

ccttcccatg ttgctgctgg gctccactgt ttccaagttc tgttgagaaa aataaattgc 87360

cctgggaggt tctgccaaag aaaacatccc aagctccagg ctcgtgggcg tctgaaggtt 87420

gatgtgctta gagctggttc tagaaacatt catagcttgg cttttctgac ccctgacctc 87480

taggggtgaa gatgccttcc tggtctgagc accctaaact cctttcttat ttcttgggga 87540

atttggtact gagctccctc ttcccggaga aggaccagac tcatgaccac gttatggcct 87600

cctggccttg taactacccc agtgccaagt ccttttaatc agtccagccc cagcagctgg 87660

gaagaaccac ccaatggcca cttaatcccc cagccaggct ggcttcatga gtgtgacctg 87720

taccgtctca cagggaccac cctgtgcttc atactctaat gttgccatct ggaaattctt 87780

cctaattctt tataaacgct ctgcattttt tttcttttct tttttttttt ttttttttga 87840

gactgactct tgctctattg cccaggctgg agtgcagtgt gtgatggcgg ctcactgcaa 87900

cctctacctc ccaggttcaa gtgattctcc tgcctcaacc tcccaagtag ctggaactac 87960

aggtgcccgc cactatgcct gggtaatttt tatattttta gtagagatgg gatttcacca 88020

tgttggcgag gctggcctcg aactcctgac ctcatgtgat ctgcccgcct cagcctccca 88080

aagtgctggg attccaggtg tgagtcactg cactcagcca gcattttcat tttgcaaaat 88140

tctacaaatt ctgcagctgg tcctggctcc cagggtcgaa tattgccaag tccattgcag 88200

cctgtggttt gccactggta ggaaagccaa attgtctctg atgaacctca cagctattca 88260

gtccctggtc tttatgtgac tggcaaagca ccctggcatt ttgtactgta caactcctac 88320

acgtcctgca aaaccccagg cagaagcaac gcctctctcc cctatagcat caattggcag 88380

aatagcataa tgggctccag gaccaggttg ctcaagttca aatccctcct ctgccagtta 88440

cctgggtgac cttaggcaag tcatttagcc tctccatacc tcagtttctt taccccatta 88500

taaaatgggt ataataaaaa cacttacctt gtatgatttt tttttttttt tttttgagac 88560

aagatctcac tctgtggccc aggctggagt gcagtgatgc aatcatagct cactgcagct 88620

gagaattcct aggctcaagt gatactcctg ccccaatctc ccaggtagct gggactacag 88680

gtgcacacca ccacagctgg ctgattttta aattttctgt agagacaggg gtctcgctat 88740

gttgctcaag ctggtctcga actcctggtc tcaagtgatc tctagcctca gcctcccaaa 88800
gtgctgggac tacaggcatg agccaccatg cctgaccttt tatatgattc ttatgaggat 88860
taaatgagtg aacatttata taaaatgctt agaacaatgc ctggtatgcc aaaatgttag 88920
ttattattac ttttgttttt tgagacagag ttttgcttct tgctcaggcc ggagtgcagt 88980
ggcacaatct tgactcactg caatctcgaa ctcctgggtt caagtgaccc tcccacctca 89040
gcctcctgag tagctgggat gacaggcatg tgccactaca cctagctaat tttttgattt 89100
tgtggggtct cgctaagttg cccaggatag tcttgaactc caggcctcag gcaatcctcc 89160
tgcctcggcc ccctaaagtg ttgggattac aggtgtgagc cactgtgccc acctattatt 89220
acttttataa ccactgcttc cacagaagtt atatattgtt tgtataatga ttttcagcat 89280
tcatcctatc attacaatat aaataatatt cacattttcc cactgtttaa gctaggagtt 89340
agcaaactct ttttgtaaaa ggtcagacag taaatatttt agactttgag ggtcacgcat 89400
tctttgtcac atctgctcag ctctccactg cagcacaaaa gcaaatacgt aaacaagtgg 89460
gtgcggctgt gtttcaataa aactttatct ggaacagaca gacaagggcc tgatttgacc 89520
cactggttaa actttgtcct tccttggctt gagtccctta gcacgttctc atccatttca 89580
tttatttatt ttattgttat atatattttt tgagatgggg tctcactgtg tcacccaggc 89640
tgaagtgcag tggtgcagtc ttggctcagt gccaactttc tgcctcccag gctcagtgaa 89700
ccgagatcct cctgcctcat cctcctaagt agctgggact acaggcacat gccatcacgc 89760
tcagctaatt tttgtttttg tagagagggg gtttggccat gttggccagg ctggtcttga 89820
actcctgagc tcaagcgatc agcctgcctt ggcctcccga agtgctagga ttacaggcat 89880
gagccaccat gcccagcctg tcatccattt tagacgaaag tttaaactcc tccccagggc 89940
ctggaagcac ccccttacct ctctctaatt ttatgtgaaa ccaccctcct cctcttactt 90000
tagccccctg atcacacagc tctatctcca gccaggccca tggcctctaa ttaaactgat 90060
tatactggat ggattacatg ctagacaaaa tacaactcag gcctaaaaat tacatttatt 90120
cagctttcag tttccttcct ttcttgtctt ccttcccttc ctttccttcc ttcctgtctc 90180
tgggtcttgc tctgttgccc aggctgaggt gcagtggtgc catcatagct cacttagcct 90240
ctaactccca agctcaagtg atcctccctc cccaacctcc tgagtagctg ggactacagg 90300
catgcaccat cccacctagc taatttttaa attttttgta gtaatggggt cttactgtgt 90360
tcctcagttt ggtctcaaac ttctggcttc cagccaccct cccgcctcgg cctctcaagg 90420
ccctgagttt ccaggcataa gccaccacac ccagccaatt ttcagtttct tttcttttct 90480
tttttttttt tttttttttt tgagacagag tctcgctctg tcacccaggc tggagtgcac 90540

tggcgtgatc tcagctcact gcaagctccg ccccccaggt tcacgccatt ctcctgcctc 90600
agcctcctga atagctggga ctacaggcac ccgccaccat gcccggctaa tttttttgta 90660
ttttttttag tagagatggg gtttcaccgt gttagccagg atggtctcga actcctgacc 90720
tcgtgatcca cccgcctcgg cctcccaaag tgctgggatt acaggcgtga gccacggtgc 90780
gtggccaatt ttcagtttct tttcttttct tttttttttt tttttgagac agagtctcgc 90840
tctgtcaccc aggctggagt gcagtggtgc gatctctgct aactgcaagc cccgcccccc 90900
aggttcacac cattctcctg cctcagcctc ctgaatagct gggactacag gcacccgcca 90960
ccatgcccag ctaatttttt tgtatttttt tagtagagat ggggtttcac cgtgttagcc 91020
aggatggtct cgaactcctg acctcgtgat ccgcccgcct cggcctccca aagtgctggg 91080
attacaggcc tgagccacag tgcgcggcca attttcagtt tcttaactgc ataaccaaac 91140
agttggctcc agcctggctt ctcttaagca ccaactacgc gtaaaagctc agccctgctg 91200
tcaacttgta ggtccttcaa gtcactttgg ctgaatttca caacaagcaa aataaaattg 91260
aatacagaga ctgaagatgt tggtggttct gggctgactg gatgcatttg caaataaaag 91320
tctgatcgtt ttgggatttc cttcctaaca atggctaagg cctccttctc cagcactggc 91380
tgtgtgtggg cagtgtgcca agtccttgct aacgctgcat tccaggagat ggagatacac 91440
aatagccaag taaacaaata atcaagatag ttcgtggtgg agatatcatt gctacatctg 91500
ggtgatgtga ctgagaagat cgaggggaag ggaggagagg ggaggctgtg gatggggaag 91560
ccagggagtt gctgagcctt gaaagttaca agagagccct gtgggaagga tggagggaag 91620
ggtgggccag gcagggcaaa cagtatggac agaggtccaa gggagggaaa tgctgggccc 91680
aggtatgaga cagaatggct cccagcatgg ctggagggcc acaacagagg aggacagcag 91740
gaagtgaaga gggaagagca gatcacactc agtcttccaa gtcacagtaa ggaatctgag 91800
ttttattctc atgggtgcag gaaaccattt cacgagggga acgggacttt agaaccagtg 91860
tggggagaat cagaactgca ggatgggctg ggcgaagtgg ctcatgcctg taatcccagc 91920
actttgggag gctgactgag gtgggtggat cacctgagat caggagtttg agaccaggct 91980
ggccaacatg gagaaaaccc atctctatta acaatacaaa aattagctgg gcatgatggc 92040
ataatactcg ggaagctgag gcaggagaat tgcttgaacc caggaggcgg gggttgcagt 92100
gagccaagat cgcaccactg cactccagcc tgggcaacag agtgagactc tgtctcaaca 92160
aaaaaatcaa caacaacaac aaactgcagg atgggccagg ctcagcggct catgcctgta 92220
atcccagcac ttgggaggcc gaggctcccc gggtcacctg aggtcaggag ttcgagacca 92280
gcctgaccaa catgacaaaa ccctgtctct actaaaaata caaaattagc caggcatggt 92340
ggtacatgcc tgttatccca gctactcggg aggctgaggc aggagaatca cttgaacctg 92400

ggaggtggag gttgctgtga gccaagatca cgccattgca ctctagcctg ggcaatgaga 92460
gcaaaactcc atctcaaaaa caaacaaaca aacaaacaaa caaactgcaa gatggatact 92520
ggattgcagg aaggattttt aaaaaatagt gagcactttc actacaatag cagctgatcc 92580
ttacaaagtg ctcaccatgg ggcagtggcc tgtttctcag ttcttcacat gtagtgactc 92640
atttcatcct tccaacaacc caatggggtg cttattgttc ccattttaca gatggggaaa 92700
ctgaggctca gagaagtgaa ctgacttatt tcctggagca tacaactaac tagacgatga 92760
tggggccaga attcaaaccc aaggggttag gttctggaat ccacacttta tgacagcatg 92820
ctaatgaata tttattcaat gaataatgag tgaatattct tataatgtat atttattaaa 92880
ggaaggaagc aagcaagcaa tgggagcagg gagcttaaga gactgctaca gaggccctgg 92940
tgagaggtga cgggaccctg aactaggaag aggcggtgga gtcagaggaa tggacagaga 93000
tgagaaatat ttcagcatca taccgtccaa ggcaggaacc gctatctaaa ttaactaaaa 93060
tcttaaaaat tcagttctgc aatatacttg ccacttttta agagctcaat taagtgaact 93120
tttaagtgaa ctttgtgctc atcacaaagt tcttttggat agcaccgttt tagatgaaac 93180
agcacctgct gatggcatgg gtgctggaaa tgagagcagg gtggcctctt cttcatcttc 93240
acagcctctc cataagctca gctcagagag gtcaagtgac ccgtgcatag ctacatagct 93300
aggaagccat ctgaaagtcc tactctttcc tgcacctctc tgttaccaca cttaattcat 93360
tcactcaaca gagggcagtt gagctacact attttggttc ctggggagat gacatgtggc 93420
tcacacatgg gtttggctcc taggggatca aatgcctctg tggctagctg aaaaacagcc 93480
cccacaagaa tgtccatggc ctatcctcag aatctgtgaa tatgtttgct tgagatgagg 93540
agactgtctt ggatcatcta ggtggggcta atataaccac aaaggtactg ggccttataa 93600
gagagaggta gggggaccag agaaagagat ttgaagatac catgctgcag gatttaaaga 93660
tggaggaaga gaccatgagc caaggaatgc aggtgacccc tagaagccag aaaactagga 93720
aaccaagtcg gcaggttgtt ttagcattta tatcctcata gctaggtagc attatatctc 93780
tagatccaga ctattcattg tcaaacatca aatgtgtaca tcctaccatg gaatatgaga 93840
attcagttcc catctaatcc tcttcccttt ccccacagag ccaagtccat cacagggagt 93900
tattctggtt agataaatac tccaaacttg cacagctggg ctccttagtc caatgtgatt 93960
acttctcctt ccatcccaac ttttgttttt tcctggagtt tataattgcc tttgttttat 94020
tgtttccagg ttttctatct atatcaaccc caaacttatt aattgtttca atcttctctc 94080
aatacagttc attgccatcc taccatccat caccatctga aattctaatt actagttttt 94140
aattgtcctt ccagataatt tcatgcaaat aggaaacata tgcacgtcca tgcaatcctg 94200

ctctttttaa agacacacac ggttacatta tgcatatgct ggtttgcccc tgtgttttcc 94260
actcaacggt attatctata aagagcgttc tctctcagca taaatagatc agcctcactc 94320
tttgtagtgg ctacctggaa atgctatatt ttatttaaat aacctctgtt gattatagct 94380
ttagttgctt ccagaccttc aaatatatat atatataaat ataaatatat taaatataat 94440
acatattata tattatgtaa atatatatat gcacagtcta tcctcattat ttatatattc 94500
tatattttca aattcaccga cctgctgaaa gttatttgta accccaaaat caatattcat 94560
ggcacttttg tagtcatttg tgaacatgtg cagagtaagg aaaattcgat tcccccaatg 94620
cacacgttcc cagctcaggt tgaacagggg aatgctctac cttctagttt caactctcat 94680
actgtaaaca agtgtccttt ctgtagtgta tttagtgcca cctttttttg tatttttgtg 94740
gctttttgtt agttattttg cagtttaaaa tgtcccctca gatgtgctcc cttcggcagc 94800
acacatacaa aaatgtcccc agcatagtgc tcaagtgttg gctcatgttt ccaagcacaa 94860
taaagttgtg atacccatta tgtaataaat acacatcaga taaacttcat ccaggcatca 94920
gccacagagc tgctggctgt gagttcaatg tcaatgaatc aacaatatat attaatatat 94980
gttacttgca tataaataca atagtgaaaa ctctctcaat gattctaaaa ttgagttact 95040
atcttctgcc tatgtaccta ggtcttcaaa gaaacagtga taccaatcac caaatatggc 95100
actgatgtgt ttgtggcttg ataaatgtga aatactaaag tgacagttta acatcttccc 95160
tatcaacaca gatggctgtt atgcaattag ataagattct gcatataaaa tctttagttc 95220
aagtcctggc acagagtaca tgttcaataa atgctagttt tttagaagga ggagaggtat 95280
ggaaccaaat gatagcaggt gtcattaaga gtttaagttt gcctgcctcc gcagaagacc 95340
atatatcaca aatgtttagt gagcatctac atggtgccag gtataaagag aggcacttgg 95400
aaccatgaac aaatcaaaga tctctgcact catggatctt ttctttcata ggttggatct 95460
cgactacaga ttaaaaaaaa aaaaaaacaa aaaaaaaaca gaagaaaaag cagttccact 95520
tacgtgcata gaatggactg gtcctcccga tctgcaaaca gaaagaagaa aaaagagcca 95580
tgaattaaaa tgagctctga gctaagggtc tggacttggc tcctcccagg gccctcccat 95640
tctaaagctt ctccatgctg gcctcattgg aagtgacaac aggtcagctg ggctcattgc 95700
ccaggctaca ggggccacga ccaaagccaa aagggcagcc ccaaagtccc agaggcagcc 95760
ccagaaagat gctggagcct gactgatcca ggagtcatta gcatccagcc acatcagctc 95820
tggaggtgtc ccgggaggcc caccccaccc agattaggtt agggaccaaa cgcagatgtt 95880
ggatctatac agattggggt cccccttagc cacttctccg ctctgtggcc ttgggcaaat 95940
attcttaaac tccatgcctc agttccttca tctgtaatat agtattgtgc acatttgtgt 96000
ttttttgtaa atccagcatc catttcctct tcttccagaa gcatcctgtg gtccttgagg 96060

tttcccccat cacccctacc gtcatgtaca gtcttagtgg gactgcctca taagtacctt 96120
caacaaagag gtaagaatgt gttccaggct gcattggtca gactctctgg cctaaaactc 96180
caagctcagt caaggacagg gaagcacagc aggtggacag agccctgagg cagacttcct 96240
ttctgcccct gggcccaagc agcccagttt ctgtcattta cctagcctga ttcctagtgt 96300
tcccactgca actgtgagct cctccatact cccctacaaa tccctttcct ctactagatg 96360
acagagtcag tctctcttgt ttaccaccag agaaacccaa aagatacaga ggtcatcaaa 96420
gaactagatg tgattaagca gacattatcc atcaccttct actcttaaag aggcctctga 96480
ggtggagcca ggctctgaag acccatttcc ttcccaaaga gagatctgag aacagttacc 96540
aatacgttca atggtcacct aacaatgtag aaatcccagc atcatatctg ctggtagaca 96600
atgccacagt ggcaagcatt tcttaaatcc actccctggc ttcagtccta ctagttcagt 96660
gctcagctct cacaagcttc atttcatcaa aaatctccta ggcttggttt tacattgtat 96720
ctctagtact ggcacataga tggtaggtgc tgaattatgc ttgttagatg gatggattga 96780
gggatggatg agtagatgga tgggaagatg agtggacgga aggaaggaag aaaggaagga 96840
agggtagatg ggtgaatgga agggtgggta gatgggtagg tagatggatg ggtgggtggg 96900
tggatgaata gacagatgga cgggagggtg gataagcaga tgagtaggtg agtaggtggg 96960
tgggtggatg ggcagatggg tgggaaaatg gatggataga tgagtagatg gatgggagga 97020
tggaagaaga ggtggatggg caagtgagtg gaaggaagga agaaaggaaa gaaggaagga 97080
agaaaggatg ggtggatgga agaatggatg gataggtaaa tggacgggag gataaatgga 97140
tgggaggctg ggtgaatagg cagacagatg gctggatgga aggatggata gatggacagg 97200
tagagagaca ggaagacaaa tggatgagtg ggtgggtggg taggtggacg aatagatgga 97260
tggaaagaag gaaggatgga tggatagata ggaagaaaga aagaagaaag gagggaaggg 97320
tgtgtagatg ggtagatggg tgggtatata gctggttgga cggatggttg gacggaggag 97380
ggaaggatga gtgagtggat gagagagtgg atgggtgggt gaatgggagg gaggacagag 97440
tgtataggcc ggtaaatgag ttggatggat ggatggatgg atggatggat ggatggatgg 97500
atggatggga atatgaatgg gaggatggat gggactgaag aaggcaaggt gaaagagatg 97560
actctttctt ccatctgtaa tcttaagaga gaactgattg gatgtgggca cgttcctcag 97620
attctatgcc caactccaat cagttctctc tctcttccct cggattatag atggatgttt 97680
gggtggcctg gttgaacgaa atcctcaaga caaaaggcct aaagactagt cagctctggg 97740
gtttcaattc tggtgtgttt atataatggc acatttaaaa ataggcaggc cgggcgcagt 97800
ggctcatgcc tgtaatctca ccactttggg aggccaagga gggtggatca cttgagttca 97860

ggagttccag accagcctgg ctaacatggt gaaaccccat ctccactaaa aatacaaaaa 97920
ttagttgggt gtcatggcac acgcttgtaa tcccagctac tcagaaggct gaggcaggaa 97980
aattgcttga actcaggagg cggacgttgc agtgagccga gatcgtgcca ctgcatgcca 98040
ggctgggcaa cagagcaaga ctctgtttca aaaacaaata agatataaat acataaataa 98100
aataggcagc cattgaaaag aaccaggcag agctttgtgg atggacacag aaaaatatcc 98160
aaaatatatt aagctaaaaa taaaagatga ggtagcatag tgttatacct tttgtgtttt 98220
aaaaagacag atatataggt aggatgcagt ggttcatgcc tgtaatctca acactttggg 98280
aggctgagat gggaggatca cttgaggcca ggagttccag accagcctgg acaacatagc 98340
aagacccagt ctctacaaaa aaaaattttt ttttaattag ctatgtgtga tagacatacc 98400
ccttgtccca gctacttggg aggctgagac agtaggatcc cttgagccaa ggagttcaag 98460
gttgcagtga gctgtgatta caccactgca ctccagcctg ggcaatggag tgagacccta 98520
tctctaaaaa cagacctggg atgggtgcgg tggctcacac ttgtaatccc agcacttcgg 98580
aaggctgagg cgggcagatc acttgaggtc aggagttcaa gaccagcctg gccaacatgg 98640
tgaaacgctg tctctactaa aaatacaaaa attagctggg catggtagca tgcacctgta 98700
atccaaggta ctccggaggc tgaggcagga gaatcacttc aacccaggag gtggaggttg 98760
cagtgaggtg agatcatgcc actacactcc agcctgggca acagagcgag actccaaaaa 98820
caaaacaaaa caaacaaaaa aaaccaacaa acaggcctga gcctgaagta ttgagaaaag 98880
ggacaggagg aagacctact ttcacccatt ctacttctat cctggttatg tttgtgctat 98940
gaacacatac aatcttttcc tcatcccacc aaaaatggtc aatagaaaaa gagtcggtat 99000
tgttctttat gaaaccatca tgaccacaaa gctaaaaaaa gacagaaaag aaagagccac 99060
cgcattagga tcccactaac agctccgatc tcccgagcac tcactgcact gcaggcgtat 99120
gctgaacttc tgtgttgaag tgacccactg aattctctca gcagctcctg gggtgggcac 99180
tggcattgcc tccattttac aaataaggag agtgaggttc agaagtgagg caacttgccc 99240
aaggtcacta gggtgggttg aactgtgact cccaaaagct atgttcactc agaacctcag 99300
actgcgatgt tattgactaa ccaattgatt gagatggggt ctcactttgt cgcccaggtg 99360
ggtgtacagt ggcgtgatct cagctcactg caacctcaac ctcccaggct caagcaatcc 99420
tccctcttcg gcctcccctg tagctgggac tataggcaca tgccagcatg cccggctaat 99480
ttttgtattt tttgtagaga ttgggtcttg ctatgttgcc caggctggtt tcacactctt 99540
gggctcaagt gacccacctg cctcagcctc ccaaagtgct gggattacag gcgtgagcca 99600
ctgtatccca cctgtgattt tatttagaat aagggtcttg cagatgtaat taagggaagg 99660
atgttgagat gaggtcatcc tggcttggag tgagacctaa atcaatggtg tccctattta 99720

taagagaaag gaggccgggc gtggtggctc acacctgtaa tcccagcacg tgggtggatc 99780
acctgaggtc aggagttcga gaccagcctg ggcaacatgg tgaaacccat ctctactaaa 99840
aatacaaaaa attagccagg catggcggca ggcacctgta gtcccagcta ctcgggaggc 99900
taaggcagga gaatcacttg aacccaggag gtggaggttg cagtgagttg agattttgcc 99960
actgcactcc agcctgggca acagagcaag accccatctc aaagaaaaaa aaagcaggaa 100020
aaaagcaatc aaactttaat ctctccatta tactctggag acagacagat acccgtgcct 100080
ttagcttcca cccaggggga gaaattcact cgaggacaga gcaaaatggg aaaccagttt 100140
aatttttttt tttttctgga aatggagtct cgctctgtca cccaggctgg agtgcagtgg 100200
ctcgatcttg gctcactgca acctctgcct cctggtttca agcaattctt ccacctcggc 100260
cccccgagta gctgggacta caggtgcgtg ccaccacgcc tggctaattt ttctattttt 100320
tgtagagacg gggtttcacc atgttggcca ggctggtctc gaactcctga cctcaggtga 100380
tccgcccgcc tcagcctccc aaagtactaa aaagtgattt tttaaaagag ggtgaaggat 100440
agaagaacca ggtaccctgt gattagaacc cagacagaga gaacctcctg ggaccggccc 100500
agacaacctc cactatctta aggaaagatt ttgagatgga cagacagatg ggctgtgttg 100560
atccactact tcagggagag aggcaggctt tcctggcagc cttgtgaatc ctaggcccag 100620
agcaggagag atttataggc tcagatgctc acagggcccc caggagaggg agcgggctcc 100680
aggagaaggc ctgggagctg ccagcgcggc tccgagctgg gggctgctgg gagacctctg 100740
gcctgacgtt cccatcaaaa acacaccact tccaggcgtg ggaagcttgc agggtgctgg 100800
ttacgagcag aaggactcca tgtgggtgct gtctgccaac ccgtgtggag cacagggaac 100860
tctgagatcc tggaaatttt tatctgattc cttccccaag cctgagcttg tgttggactc 100920
aaaagcagtg ttctccaccg tacaaagcag tttcactccc tccttgcttt gcacaagtga 100980
tttcttctgc ccagtgcacc caccaccact tcttctctgg cctaatccat tctctctctc 101040
tctctctttt ttccttccat tgaggtaaaa tttacactac ataaaaagaa ccattttaaa 101100
gtgaataatt caagccaggt gcggtggctt atgcctgtaa tcccagcact ttgggaggcc 101160
gacacagctg gatctcctaa agtcgggagt ttgagaccaa cacggcaaaa ccccgtctct 101220
accaaaaata caaaaattag ctgggcgcgg tggtgcacac ctgtaatccc agctactcag 101280
gaggctgagg caggagaatc gcgtgaacca gggaggtgga gtttgcagtg agctgagatc 101340
gcgccactgc actccagcct gggtgacaga gggagactcc atctcaaaaa atagtaataa 101400
agataaaaaa ataactaaag tgcataattc aggagcattt agtacattca caatgttacg 101460
taactaccac ctttatctag ttctaaaata ttctgtcacc cccaaatgaa accctctacc 101520

cgttaagcag ccactccccc ataagccctt gcaaccgcca atttgctttc tgtctctatg 101580
gtcttaccta ttctagatat ttttgcataa atggaattat aacatgtgcg gcctttcatg 101640
tctgacttct ttcactcagc atgtggctga agttcatcca tgttgtagca ggtcccagac 101700
tttatgccta tttgtggctg agtcatatca cattgtacgg atggatcata ttttgtttat 101760
ccattcaaca gttgacggac atttgggtta tttccacctt ttggctattc tgcatagtgc 101820
tgctatgaac attcctgtac aagttttagt ttgagtacct tttcaattct tcgggtacat 101880
accctaatct gtatctgaaa catcgtcttg ggagtcaact cttctatgat accttctcta 101940
actctgcact tttctagagc tgagttggag acctttccac ttgacctcct acctgcagga 102000
tggccatgga ccgtggtgca tgttgtacac tgcataactc tatggagaaa ctatttgcat 102060
tggtcattta ttgtattgca ttgtgtattt attgtaataa tctttcagca gatggaggaa 102120
aagtgctttg agaaaacggt gcctttctct aatataaaca tgagtgtcac atggcctagt 102180
ggttcccatg gtccacaggt tctctccatt ttagtaatta tcatcattgt gatccttact 102240
taataaatgt ctcaaaacat tctggcggaa aaaggtacag atgatatgga atgataatag 102300
gccatctatc catgatatgg cattcagagt ttttcttgtt tttcttttcc atattggtaa 102360
aaattatgga ctttattcat aatatccaaa aaatagaaac aaccaaatgt tcatcaatgg 102420
gtagatggat aaacaaaatg tggtctaccc agacagtggg atagtattca gccatgaaaa 102480
gaaaggatgt tatggccggc ggccatggct catgcctgaa attccagcac tttgggaagc 102540
tgaggcaggt ggattgcttg agcccgggag tccgaaacca gcctgggcaa catggtgaaa 102600
ccctatctct acaaaaaaaa atgcaaacat tagctgggca tagtggcaca atccctgtgg 102660
tctcagctac tcaagaggct gaggtgggac aatggcttga gcccaggagg tggaggttgt 102720
agtaagccac gactgcacca cggtactcca gctggggtga cagagacaga ccctgtctta 102780
aaaaatatat aaatagtatg aatccatgat acaacgtgga tgaaccttga caacattata 102840
ctaagtgaaa gaagctagcc ggctgggcac ggtggctcac acctgtaatc ccagcacttt 102900
gggaggctga ggtgggcaga tcacgaggtc aggagttcaa gaccagcctg gccaatacag 102960
tgaaaccccg tctctactaa aaatacaaaa attagccagg tgtggtggca cgtgcctata 103020
gtcccagcta ctcgggaggc tgaggcagga gaatcacttg aacccaggag gcagagtttg 103080
tggtgagccg agaccacatc actgcactcc agcccgggcg agtgcagtga gacactctgt 103140
ctcaaaaaaa aaaaaaaaaa aaaaaaaaaa aaaaaaaaag aagccagcca caaaaggtca 103200
catatgattc catttatatg aaatgtccag aataggcaaa tccatagaga cagaaagtag 103260
atttgtgaat tcgtggctgc caggagctgc gggtaggaat atggagtgac aaaaaaatgt 103320
gcacaagatt tctttggggg atgatagaaa tataaaatta tattgcagca atggctgcat 103380

aaccatgtaa attatgagtg aatttagaca gttatgcaga catagctgca attttaggtg 103440
tgaactgtat ccttaaaatg aatttattta aattataccg cataaacctt ttttaaaaat 103500
gatgttcatt ttataccacc tctgaatgat tataaatctg acttcttcca cttcatgcag 103560
ctctattagg tttccaacaa ttacaaaatt gttcattttc aatgcttttc ccctgaacca 103620
atgagctcct gaaggtacaa ggaacaatgc ctgacacaca gtaggtgctc aggaaatgct 103680
tgttattttg aattgctctc tggctcagag aggcaaccaa ggtagaagca atggaaaatg 103740
catattcacg tgttcacata ttcattcaac aaacgagtcc tgagccaggt accgaaagca 103800
caaaagtcaa taagatctgc tttttttttt cttttctttt ttaagagatg aggtgtcact 103860
ctgatgccca ggctgaagtg cagtagcacg atcatagcta actgcagtct ggaactcctg 103920
ggctaacaga tccttccatc tcagcctcct gagcagctgg gactacaggt gcatgccact 103980
acgcctggct aatggttttt ttgttttttg ttttcatttt ttttgtagag acagggccta 104040
gtaaggtaac ttagtaatat tacccaggct ggtcccaaac tcctggcctc aagtgatcct 104100
cccgccttgg cctcccaagt aactgaaatc acaggctcaa gccaatgcac cgagcttcat 104160
gacttgctta tccttgcttt tgagaagttc atgacccagg gtgggagtca gatgggcaaa 104220
gaaatgaccg taagagatga gcagtgctac gatggatgtg aacccaagtg ctatagacgt 104280
caggtttttt gtttgtttgt tttttcacac tgacctgagc aagacaggaa gccttcacgg 104340
cagaggtgac cactgaacag cattaagaaa aatgtgaaag aatctcctgg ggagaggtcg 104400
aggcagagca tcccaagcag agggcctggg acagaagctc caaagtgggc ggggacacag 104460
taccgagcct ccgtggagtc tgggaaggct ggagctgggg gcggccggcc atcatcagga 104520
cccgcctggg gctgagcttc ccagcccaga tccctggggc tcatgtcacc tcacacccca 104580
tccttaaaca tgcctctctc ccttcctcct ccccagactc cccacccagc ctcggatgtg 104640
taaggcccca ggcacgtttc ctgagaagat aaagggcttc agcggttcct cccccgctgt 104700
gggcctgagg actcccagga ggtacccagc tccccacaga ccagctattt cggcccaagc 104760
ccaaacacaa gctggtggat tctgtctggg aagccagagc actgtaggct gaccataaaa 104820
ggagaaaacc cagcggggac tgagcacaga cccaggctcc ctgcgtctgc ccttctttct 104880
ggtaacaggc tgctggggga tccagtgaga ggtaatcaaa acccgtctct gcagggccat 104940
caatacccct gggctagtaa agaagaaagg tctcagcctc cattgcctgc agaaaagtgg 105000
ctctctacct aatgccccgc acggaggcag atgccagagg ttctggatct tctgtggtgt 105060
gactgtgaag gtggccactg catggccctt gacggtgggt cctcggcagc tgactgctaa 105120
tgcctgttga acttgtcctg tccatgcacc gtgtccctac acactgaccg aatcaccctt 105180

gtaacaactg gagtaagcac agtcatcatc ctcacgggac aagtgaggaa actgaggcac 105240
aaagtaatag ccggggcaga gctgggaggt gaacccagct ccagagccca tgacctaagc 105300
cattctgtaa gtgagggccc ttctctttct ccagccttca tctccccact ctactctccc 105360
ggcagagttg gttaggatga catgctgagt aagaagagat actttgagag cctttgctgc 105420
aacccaacag gtgttttcca aacctacagg aagccctggt attgtaaaag cagaactggg 105480
ctgggcatag gagctcatgc ctgtaatccc agtgctttgg gaggctgagg cagaaggatt 105540
gcttgaggcg aggagtttga gactcaaagc gagaccccta tctctatgaa aaaaaaattt 105600
tttttaatta gctgtggaca gaggtgtgtg cctgtagccc cagctatttg gaaggctgag 105660
gcaggaaaat tgcttgagcc taggagttcg aggttacagg gagctattat tgtgccactg 105720
cgctctagct tgggtgaaag agtgaaactc tgtctcaaaa aatatataat aaataaaaac 105780
atgaaagcag aactggccag tgaaggggtg taggagacag gaaaaccaac gctctccctc 105840
ttgggcctcc actcctacct cctacattct cccccttctc tctgctccag ccccagcagc 105900
ctcatagaac ctatatcaag ttccctagcc aagcctacac ttcaccttcc aaggaagcct 105960
cccctgtcct cccaaggtcc ccagggctac acttccccac tctctggacc tcctctactt 106020
agcagctact tgtgggatga ttcaattcgt atttacacat acgcacagta ggctgtgtct 106080
agggcaacat ctaataatca atattgttag aatgaccggg cgtggtggat cacatctgta 106140
attccagcac tttgggagac cgaggcggga ggattgcttg aggccaggag ttcgagacca 106200
gcctgggcaa cataatgaga cctcatctct acaaaaaatc aaaaaattag ccaggcgtga 106260
tgatctgtgc ctgtggtccc agctacttgg gaggctgaga ggggaggatc acttgagccc 106320
aggagttgga ggctggagtg agctatgagt gtgccactcc actccagcct gggcaacagg 106380
gcaaggcctt gtctcacaaa aaaatgctgt tggataaaga aaggaatggg gagccagact 106440
gcccagaggc tgggaggaca ccgagggagg tggctgacac atgcaattta acaaggaagg 106500
tgctggccag gcactgtggg tcacgcctat aatcccagca ctttgggagg ccgaggtagg 106560
tggattgctt gggctcagga gttcaaggcc agcctgaaca acatagcaaa acctcatctc 106620
tacaaaaaat acaaaaatta gccagttgta ctggtgcatg cctgtatgca gctactctgg 106680
aggctgaggt gggaggatca cctgagcctg gagaggctga ggctcaggtg agccgagatc 106740
acaccactac actccagcct gggcaacaga gtgagactct gtctcaaaaa agcccaaaac 106800
aaacaaggaa ggtgttgcca agtgtcagct ggggcttttc aggttcactc ccttaagtat 106860
gtcccttttt caatttaata tttacggttt ttgtttttgt tttgtttttt ttttgagacg 106920
gagttttgct cttgttgccc aggctggagt gcagtggggc aatctcggct cactgcaacc 106980
tctacctccc aggttcaagc gattctcctg cctcagcctc ccgagtagct gggattatag 107040

tcgcccacca ccatgcctgg ctaatttttt gtatttttag tagagatggg gtttcactac 107100

attggccagg ctggtctcga actcctgacc tcagatgatc tgcctgcctc tgcctcccaa 107160

agtgctgaga ttacaggtgt gaaccactgt gcctggccaa tatttactat tttataacaa 107220

ataattctcc tttgctgtct cccttccctg taatgcacca tgggtaccaa aaataaaaat 107280

aaaataaaga taaaaacgtg aacttttcat gattagtcag agcaccaaac agacactcct 107340

agcacctctg taaagactga gccagccaga cagagccatt cattcccaca cacagtcccc 107400

catcattccc aaccgggcca gacacatagc tgacagccac tgcaaaaccc acaagagaca 107460

gagaagcatt tggctgtacc ccttcctgga gatgtgtttg tagcagcact gcctctgcag 107520

agaaacagct gggaattact tcttggctcc accctatcac ctctgccccc agggcccgtt 107580

tcctcaccag ggaaatcgac aaaaatccag acctttcaag ggcatgaaga tgattgaaag 107640

agactggatt cagaaagagc ttaaaattgt tttacccaca gcacatttaa gagcttggta 107700

attgccgtta atatcatctt tatcatcatc atctaaaaca gtgatctagg gaagaggtta 107760

tagacagcag tgattctgaa tcggcaggac aggatattca accaaagata tttaacccct 107820

ttctgattac aaggtgtcat ggtctgaatg ctcatgtctc cctcaaattc atatgctgga 107880

atccttaccc ctaaagtgat gatattagga ggtggggcct ttgggaggtg atgagatcat 107940

gggggttcaa tcctcattgg gggagagacc cagagagcta gcaggtccct tccaccatgt 108000

gagacacagc gagaaggcag cactaggccc tcaccagata ccaagcttga tcttggactt 108060

cccagcctcc agaaccacga gaaacaaata tctgtttaaa agccacccag tctatggtat 108120

tttgatatag cagccttaac aggctaagac acaaggccta tgctagtccc ttagccttct 108180

gggggcctca gtttcctctt ccgttatatg gggacaagaa tactacacta tctataccac 108240

gctacccagg agggaagagt aacattctga gggcagcgaa gctgggtccc caggtttgac 108300

tctcagttct ggggccaagt aggtgtgctg agttttcccc attgatcaaa tggggaggct 108360

gccacccacc ctgtgtggtg ctagtgaggg ccgagcaatg tgggattgat ggagcagtga 108420

acccaggtcc tgacctattg caggggccct gaggacatca gtgcccttcc tctctctgcc 108480

tctactgggg agcagcctca ggggagttgg gtgagcagga accatcccca gggggctgcc 108540

aatctcaagc agaaggatac agccgagtgt gggaacttgc aaagtggaga aagtcgtttg 108600

gatcactcca cccgcagagt tgtcttaagc ttaaagagga attagccaag agccccaagc 108660

tgataaaggc tggctgttat ttcttttttt aatcccctga gtgtggccat ttgttgctaa 108720

tgaggtgagg acaaaacatt acttgtggct atcttgcata gggcaagtgc agaggctcag 108780

aatggaatct tatctgcttt cctgcccctc ctacacccca gggacccatg gaggggctcc 108840

aaagtggttc ttgtctggag ggctttaatg gcgcttttct cctattcatc acgttcattt 108900
attgaactta gctccagcct cctaagcttt gagctgtcaa cactctgaga tgaagacagt 108960
agacagcctt ccgtgggatt gagggggatc cctgtggttg cccggtgtca ccgactgacc 109020
agcagggctc cggctttgga gtcaggataa tccaagtgta tatttggctc tgacacttaa 109080
caaatctgca gcaagttctc cactcctctg agtctgtttt cccagctgta aaagggagct 109140
aataatagct cttgcggcta gcgttggatc acccaataaa ccttgtaagc atatgctgag 109200
ggcacaccca cacaggcatg cacgagaagg aagaatgcca tagttgccat ttcataagca 109260
ccacgaagat aaacatcatg ggaaaaatag caactttagg ctgggcacgg tggctcatac 109320
ccataatccc agcactttgg gaggtcaagg caggaggatc acttgaggcc aggaatgaga 109380
gaccagcctg ggcaacacag taagatctta tctctaaaaa aaaaaaaaaa aaaaaaaaaa 109440
aaaaaagttt tttcactagg cacagtgggc tcacacctgt aatcccagca ctttgagaag 109500
cctaggcagg tggatcactt gagctcagga gttcaagacc agcctgggca acatggtgaa 109560
atcccgtctc cacaaaaaat acaaaaatta gccgggtgtg gtggtgcatg gctgcagtcc 109620
cagctacttg gggagctgag gtgagaagat cactggagcc taggaggtca agcctgcagt 109680
gagccgtgtt tgtgccactg cactctagcc tgggtgacaa agtgagaacc tgtctcaaaa 109740
ataaaaaaga ctgggcacgg tggctcatgc ctgtaatccc agcactttgg aaggccaagg 109800
tgggtggatc acctgaggtc aggagttcaa gaccagtctg gtcaacacgg tgaaacacca 109860
tctctactaa aagtacaaaa attagctggg catggtggca tgtgcctgta atcccagcta 109920
ctcaggaggc tgaggcagga gaatcacttg aacccaggag gtggaggttg cagtgagctg 109980
agatcgtgcc attgcactcc agcctgggtg acaggggtga aactctgtct caaaacaaac 110040
aaacaaaaaa cagttgtttt tttttaatta gctgagcatg gtggtacatc tctgtagtct 110100
cagctacttg ggaagctgag gcaggattgc tgaacccagg agtttgaggc tgcagtgagt 110160
tacaatcgca ccattacact ccagcctggg caacagagca agaccctgtc tctaaaaaaa 110220
gcaaaaacaa aaacaatttt gtcagccttc tttactttat aatttaattt ttttttatga 110280
catatgcctg caccttaata gtttcagggc ttaattttaa aattcctaat ctggtcctgt 110340
ccaccttgta ggtttgtggt aaagattaac tgagaatatt tctgtaaagc cctgagcttt 110400
ataggaagca tataataaaa atccatgatt attattatca cttctttgta aaagaaggca 110460
tgagagaatt cccatgagaa gatggtaggc tgagtgcatg catttagctc tcttccttcc 110520
taaaccttac taaaataact gtaaaagact ttccaaaaat acatgaacca gtttagccgg 110580
gcatggtggc tcatgcctgg aatcccagct cttaggaagg cacaggcatg aggaaagctg 110640
gagcccagga gttcaagacc tgcctggaaa atataacaag accccattct ccacacaaaa 110700

gaggggggaa aaggcaaaaa aaaaaaagtg taaaaaggcc gggcgtggtg gttcacgcct 110760
gtaatcccag cactttggga ggctgaggtg agtggatcac ctgaggtcag gagctcgaga 110820
cgagcctggc caacccgtct ctactaaaaa tacagaaatt agctgggcat ggtggcatgc 110880
acctgtaatc ccagctactc aggaggctga ggctgcagaa tcgcttgatc ctgggaggtg 110940
gaggctgcag tgagcagaga tcgtgcactc cagcttgcgc gacaagaggg aaactccatc 111000
ccccaaaaaa aaacaaacaa accaaaaagt gtaaaaatac atgaggcagt ttaaaaagaa 111060
ctagagaggg gagaacagca aagccatctt ggaagacaga aaatgggata agcgttgaaa 111120
agtaccaaga aaaagtccag acaccgtggc tcacgcctgt aatcccagca ctttgggagg 111180
ccgagttggg tggatcacct aaggtcggga gttcaagacc agcctgacca acatggttga 111240
accccatctc tatttaaaat acaaaaatta gccgggtgtg gtggcggatg cctgtaatcc 111300
cagctacttg ggaggctgag gcaggagaat cacttgaacc cgggaggtgg aggttgcagt 111360
gagccaagat catgccattg cactccagcc tggacaacga gagtgaaact ccatctcaaa 111420
aaaaaaaaaa aaaaaaaaaa aaaaaaaagg taccaagaaa aaagtctagg tgagcagtga 111480
ggaaaggtgg gaaacgacct aatttcaaca ccgaattccc catttctgcc ccaaacctca 111540
gaaactggca atgccagggg cctctggaag aaaagatcaa ggaggagaca aaaacataag 111600
gactgggtca aatataaggc actgttagac ccctcccata tcttctacac tccctgaagc 111660
caagtgactg cccttctcac accccacaga aggtggtggg tttagtctct ggtgagggtt 111720
aaacagaggg tctccgtcct ggggggacac caggaaccat actgaaagaa ggaaggtata 111780
atgtacaaat ttcactgcac attttgaaac cctcagcttc ttttcaacta gaccctcaaa 111840
atgatgctag acaaatcttt aacctccagg cagaagattt ttctacaaac cttctctgaa 111900
gaatcagatt agcccatgaa caaaagactg aaagacgaaa cagaagtgtc ctaatggggt 111960
agcccagtgc tttcatatta tagccaagca catcagcaag cccatcctat aagcactgag 112020
cttccatcaa caatttggta tcctgtgtac taaagcatcc aagatgaatg gatagccaag 112080
aatcccaaga catctgaaga aatccactaa tatgacagac agaagttaaa acaattagga 112140
aaatatgtat cttggaggaa atagactctg cattaaatat cctctgagaa acaggatatt 112200
gcaaccacag aaaaagaatg tcatatgaaa gaaacattta aaaataaaat agaatttttg 112260
gaatttaata aaatatatac cagaaattta aaacgcaatg gaagggttag ggaaaaaagt 112320
tgaggtaagt gcccagaaaa taccattaaa aacattaaag tgatgaaaat gacaaaagaa 112380
agagatttaa attataggat tggtctagga attccattat ggaaataaaa agacttctaa 112440
gaaaaagaaa cagagaacac agggagaaag acagcatcaa tgaaataatt tgcaacattt 112500

cccagaattt gtggacacac gtttatagat taattgggcc actaagtgtc caggataaca 112560
aataaaaatg gactcatagc aagtcccatc atcctgaata ggggtacaaa gacagtctaa 112620
aagcttctag agaaggagga aaaacaaaaa ctggtcatgt acaaaggatc aagaatattc 112680
ccagccagga aggtgaatat aatcacacac acacacactc acaaacatac acacatacga 112740
atcagattgg gtttcatctt ctcaaaactg aaactagaag gcaacaacac aaatgcctta 112800
aaaagctcag gcaaaaaaag ttatttataa ctttctgaaa acaactttct tgcctgaaat 112860
tttaaaccca actaaactat cctttaagtg ttagagaagc atcaagcctc tcagatatgg 112920
aagttctcaa tgcctcaaga tcacgtacat tctttcttgg gaagctactg gacaaagtgc 112980
ttgatttaag caggagagta aagcaagaaa gaggaagaca tgaaaactgg gaaacaagga 113040
atccactatg aaagcactaa ggggaatttc aaggatggtg aaaaagatcc tatatgacag 113100
ttactagcta tagagaaagc cagggcagat cagaaggcta tagtagagat cacattcaag 113160
gagatgatat caatatgata tccaatacat gtctggacat attaaaagga gattaacaca 113220
tttagaaaga gattttgggg cagaattggt ggttaaatac atggaaaaca agattgggga 113280
aaaaaaaggc aattgctaac tccaggaaaa agaaaagtca tacagaaaga agaaaaatag 113340
tcacaaagca gtgcaaagct cagctttgaa taacatctac ataatcataa taatgtaaac 113400
tctagatagc attacatagt cataataatg gaaactctga acactgagtt tgtcaaaact 113460
aagataaaat tatactggga ctctgatatg aggcatgtgt gttggaacga ggaaataggg 113520
gcaaatccag agggtgaatg agctaaattt catctttcat ggaggaaagt caatagatga 113580
tacctaaaat tgaaaacaaa attaagatag cagcacaatg atcaagtgtt gttttcagag 113640
atatggtggt aaataccaaa aaaaaaaaaa atcaattaaa ggagttgaga agtagcagag 113700
gatggctatt tttcttaata agccttcaaa aattacttga ctctaagccg tctgcatgaa 113760
tacttttaac aaaaataaaa accaattttt taaaaaaggg aacaaggaat tcctacatgc 113820
actgatatgt aacatcctct ggcaaaatat aggactgtat atatatggaa tgctagtatt 113880
tgtagggaaa tcttagggaa gaaaatagat acctatttgc ttatacttgc aaaaatatct 113940
ctaggagaaa acctcataca ctagaaacca taattgtctc ttgggaggga agctaagggt 114000
ctaatgcaca agggttgaag ggagaagttc accttttgaa tttgtatgta acatctttcc 114060
aaaaacaaac cacggccggg cgcagtggct catgcctgta atctcagcac tttgggaggt 114120
tgaggcgggc agatcacctg aggtcaggag tttgaaacca gcctggccaa catggcaaaa 114180
ccccatctct actaaaaata caaaaattag ccaggcatgg tggtgtgtgc ctgtagtccc 114240
agctactcag gaggctgagg cacaagaatt gcttgaaccc aggagacaga ggttgcagtg 114300
agcagaaatc ataccactga gctgagatcg tgctgctgca ctccagcctg ggtggcaggg 114360

cgagactctg cctcaaaaaa aaaggggggg agaatttaaa aaataaacat gacataattt 114420
atgcatatgc agatataaga tacatatttt aaatatcagc ttctcttccc ataccctcaa 114480
ggggcttggc ttccacttgg gagggtgcca tgttgtgaag tatctcttag aaagtccctc 114540
ccattttccc aactcctgtg aggcagcaaa gggctgagct ctggctctga gctccctgcc 114600
acagtatact agtcaacccc tctaaaccag ctcctgccac actcatcata cccattctca 114660
cccctacatt tatgctttta ttattcttgg ctagaaagcc atccctgccc ccttctctca 114720
ccttagcaaa ccattcttcg aggcttataa agccccacct ctctcaaata acccacccca 114780
tccatcctga ccctggtagg ttcttttcta taaataactc taataattca ttgattcaac 114840
aaatactgag cagctactat gtgccaggca ccagaaaatg tgctggagat acaatgatgc 114900
acatgcaaga aggatggagt ttataggtcc tcaggaactt actgtctatg tgacacaact 114960
gcaatgcttc atcttatatt cagtgcatgt gtgttaccca tccctcctca atcccgcctt 115020
ttcacacccc atcttttcca tgaagaactc aaggaagaag ccacatgtgc tagctttctc 115080
tctccacctt catggtaagc ttagtgtgga tcgcagccca gtgacttaac agaatgagat 115140
cgtgccattt atcaatggag tcaacacact gggcctacgt gtttcttcac aaaaaccaat 115200
ttcctacttg ggaatggaga aaaaattaag caggtgaacg gaattcttcc caagcttaaa 115260
tttttctcca ttatttttga cattcttttt attttttatt tatttattta ttttgagatg 115320
gagtctcgct ttgttgccca ggctagagtg cagtggcgtg atctcggctc actgcaagct 115380
ccgcctcttg ggttcacacc attctcctgc ctcagcaccc cctgagtagc tgggactaca 115440
ggcgcctgcc actgtgcctg gctagttttt tttgtatttt tagtagagac ggagattcac 115500
cacgttagcc aggatggtct cgatctcctg atctcatgat ccccccacct tggcctccca 115560
aagtgctggg attacaggcg ttagccaccg tgcccgattc tttttatttt tggagtccgc 115620
catcaatgat ttgccagaaa gcaccaggtc atgccaaaat agacgagggc atagcaggtg 115680
ttggttaaca tttaaattaa gtaaagagtt tatcttttat ggatgtgcat attcacggtt 115740
ttcattttct ttctttcttt cgtgacaggg tcttactctg ttgccaaggc tggagtacca 115800
tagcacgatc atagcacact gcattcttga agtcctgggc tcaagccatc ctcctgcctc 115860
agcctccaga gtagctagga ctacagactc acgcactgtg gccagctaat tatttttttg 115920
tagagacaag agctcactat gttggccagg ctggtctcta actcctggcc tcaagtgatc 115980
ctcctgcctt ggcctcccaa agtgctagga ctgaagacat gctgctgtgc tcaatcatga 116040
tagtcatttc ctattggagc ttactatgtg tcaagtgagc atttctaagt agtttcaaga 116100
gagctgacta tgcaattttg tgttaaaacc tagagaccat gaggaatcaa atatatgaac 116160

acagggtgtc cttgtcaatt acaacattgc ctcacagtta agaggtggtc tagcgcaggc 116220
atctcaaacc accgactggt actgcacagc aggaggtgag tagtgggctt catctgtatt 116280
tacagccact ccctatcact ccaattactg cctgagctct gcttcctctc agatcagagg 116340
cggcattaga ctcttataga accaccaacc ctactgtgaa ctgcacataa gagggatcca 116400
ggctgtgtgc tccttatgag aatctaatgc ctgatgatct gtcactgtct cccatcaccc 116460
ccagatggga ccgtctagtt gcaggaaaac aagctcaggg ctcccactga ttctacatga 116520
tggtgagttg tataatgatt tcattatata tgacaatgta atcatagaaa taaagtgcac 116580
aataaatgta atgcgctcga atcatcccga accatctctc ccctgcggtc catggaaaaa 116640
ctgacttcta tgaaactggt ccctggtgtc aaaaaggttg gggactgctg gtctaggaaa 116700
gcaaaagtcc ccacaacaca aaagtattct tcaatgacta caagagcgat caaactccac 116760
cgaaacctcc ttctccgacg attcagtggc ttcaccttgc caattattgg gtatcaatag 116820
ccaccatcat gatatgaaca caaaacaaag tggcagttct ggaaaacaag gtgcagtgac 116880
tggcaaagtc agtggccttg gaaaggccaa aaaaaagatt caggggccaa gtgtggtggt 116940
gcacacctgt aatcccacca ctttgggagg ccgaggtggg aggatcacct gaggccagga 117000
gttcaagacc agtctgacca acatggtaaa gccccatctc tactaaaaat acaaaattag 117060
cctggtgtga tggtgcatgc ctgtaatccc agcactatgg gaggctgagg cgggcagatc 117120
acaatgtcag gagatcgaga ccatcctggg taacatggtg aaaccccatc tctactaaaa 117180
atacaaaatt agccgggtgt ggtggcacat gcctataatc ccagttactt gggaggctga 117240
ggcagtagaa tcgcttgaac ccaggaggtg gaggttgcag tgagctgaga tcacaccatt 117300
gcactccaac ctgggcacca agagggaaac tccatccgga aaaaaaaaaa aaaaaagaaa 117360
gaaagaaaga caaaagaaag aaaaagattc agggcaattc acagctgaat gaacgctaag 117420
accacagggg ctttgagaga cttaaacact aaagaagtaa aagaagccct taggaaaact 117480
ggtgacccat caaataaatc tgtaagaatg atccttttta gctcgggctg cagaagtcaa 117540
gcatgacatg aagatgtcgt tttatgctca catgatttca tccctcagcc cagaaaaagt 117600
tacagaaaaa aaccccacac ccactcttaa ttcattttat actgaatcta caaattagca 117660
cctacttgta attaaaataa aattcttatt acatgtaagc tgaaattaac ctatttttat 117720
aatttgcaat gttggttttc ttttcttttc ttttgagaca aggtctggct ctctcaccca 117780
ggctggaatg cagtggcgtg atctcggctc actgcaacct ccacttcctg gtctcaagcg 117840
atcctcccac ctcagcctcc caagaagtgg gactacaggt atgtgctacc acaccaaagt 117900
aatttttgta ttttttgtag agacagggtt tcgccatatt gcccaggctg gtctcgaact 117960
catgacctcg agcaatccac ccatctgggc ttcccaaagt gccaggatta caggcgtgag 118020

ccactgtgcc tggcctgcaa tgtcattttt caagctcagt caaatatttt gcccactctc 118080
tttccctcct ccacacacat actatctaca gtgaaacttt ggtctctgtt ttaagtgatc 118140
tttttctgga acgaattatc ttcagatagt aaaaatttcc ttcatttcat gccatttagt 118200
tcagttagtg taatgaaagg tcacgtaaca aaatgttaat ggtgatgagt tttggatgat 118260
agaatcatag gtgccagcaa gttctctttt acttaaatat atgtttaagt ttttctagaa 118320
tgaatataca atattaaaga ctttttcaag ttacaaataa attttttcag agcactgtag 118380
aaatgcttac ccatctgtcg ctccccctcc ttctcctttg ctaacagaat tttctttctt 118440
tttggtattt agattaaaat gtgcttagtg ggacctcagc tccagaaaag aaccaggaat 118500
tgctgggtat ggtggcttat gcctgtaatc ccagcacttt gggaggccga ggcgggtgga 118560
tcatgaggtc aggagatcga gaccatcctg gctaacaagg tgaaaccccg tctctactaa 118620
aaatacaaaa aattagccgg gcgcggtggc gggcgcctgt agtcccagct actcgggagg 118680
ctgaggcagg agaatggcgt gaacccggga agcggagctt gcagtgagcc gagattgcgc 118740
cactgcagtc cgcagtccgg cctgggcgac agagcgagac tccgtctcaa aaaaaaaaaa 118800
aagacagata cctgactcca ggagccatca ctttacaacc tgagattaca gctccaagat 118860
tgcctttgca aggctccacc tgctggtctt tggaggtaat ggcaggccct gcctagaagg 118920
gccaagattc tggtcctacc tgtggagtct ggctcactgt cagaaagttg gggcacttat 118980
acctgagtcc ctctatcttg ggggcggggt cgtggcatct ctcaggcaca cctggattcc 119040
ggcctcaaga acttccagaa ttgggctcct ctcaactccc aactcagatc cctgggttcc 119100
cacctgttgg tgccctttca gagggggtgt gagttttcga gtttcttgta ctcttcgcct 119160
gacctggtac acttgtttgc attaactgcc tagtccctct gtttgacttg ggggtggcta 119220
tttcaggggc tccaaagtgt tctaaataga aggtgagaag gcctgggtgc agtggctcat 119280
gcctataatc ccagaacttt gggaggccaa agtgggagga tcgcctgagg ccaagagttt 119340
gagaccaacc agtatagcaa gatcctgtca cttccaaaaa caaaacaaaa caaaacaaaa 119400
acaaaacaaa aaaacccacc tttttaaaat tagcttgaca tgacagtcca cacctatagt 119460
cccagctact tgggaggctg aggcaggaga attgcttgag cccaggaggc ggaggttgca 119520
gggagccgag atggcaccac tgcacttcag cctgggcgag aaagtgagac cctgtctcaa 119580
aaataataaa acaatagtaa aaataataac gtataaacat gaaagtaata ggcacaagtc 119640
ttaagtttgg aaaaaccagc attaacaatc ttcacaagaa gctacaagat agagactatc 119700
accccttttg acaaatgagg aaactgaggc tgaagaggaa aagatattta cccacagtcc 119760
cacaggggtt gagggacagg taggtgtcaa cacagaccac aatggctcag ccccagggct 119820

gttccccacc ctctttccat cctttgcaga cagctggccc gacggggacc ttgggcagcg 119880
agccccggag ctggagctgg agcgggccca tgcccgtatt tgcccaagct cttggctctc 119940
cctcaccctc ccaggctgca aacacagatg ttccttttgc tttttccctt tttcagccac 120000
agtaactcct ggtccctcgc agtgcctgcc aaactccaca ttcctggcaa gaactgcaga 120060
caagcaggac aggaggggct ccctggagct ggccccgtgc agccctgagt tcactcacct 120120
tgaagcatgc tccggtaggc cgtgtctgcg atggcgtaga tgtgaggcgg catctcgtgc 120180
ctcttcttgc ccttgtacat gtcgacgatc ttctccgagt agatgggcag gtgtttatag 120240
gggttgacca ccacgcagaa gaggccagag tacgtctgca gacagagaac ccagcttact 120300
tccagacctc ctccagggta gacagattgc acagaagcac tcaatattct catgttagag 120360
atggggaaac tggagcttgg agtcttagtg gaaaccctgg gcctcactga tattccaggt 120420
acgtgggaag aggagaaagg gcgagagtgg gtttccaagc tgcctatgtt ttattttgca 120480
actgtggggg gttttgtgtt tgttttgaga tggggtctca ctctgtcacc taggctggag 120540
tgcggtggtg tgaacgtgac tcactgaaac cttgaccttt ctggactcag gtgatcctcc 120600
tacctcagcc tcctgagtag ccaggactac aggcgggcgc caccaggcct ggctgatgtt 120660
tatattttct gtagagacat gggtttcact gtgttgctca ggctggtctc aaattcctgg 120720
gctcaagcaa ttctctcatt tcagcctccc aaagcactgg gattatagtt gtgagccatt 120780
gcacctggcc aaactgtgga gttttgaaga gctctgggtt caaatctcag ctccactcct 120840
ccgacctgcc actttacctc cccggacctc actggcttaa ataacagcca ccgttcattg 120900
agcacttact atgtgttggg caccgaacta agcactacgc aggatctttt ttggatgctg 120960
acaatcatat taaatgctca tcatttaaca ggtggcacag aaaatgaaca gtgtgcttaa 121020
gatattaact caggaacatt taataaagaa agtgtttaca gatatgtgtg cagaattaaa 121080
gaaacccaac cagtgacggg agcagttaag tctgaagagc aagaacagaa gagaatgttg 121140
tttacttttt tttttttttg agacagggtc ttgctctgtc gcccaggctg cagtgtagtg 121200
gggtgatctt ggctcactac aaccttgagt ttctgggctt aagtgatcct ctcgcctcag 121260
cctcccaagt aggtggtacc acaggtgtgc acaaccatgc ccaggtaatt tttattttgt 121320
tttttgtaga gacagggttt caccatattg cccaggctgg tctcgaactc ctgagctgag 121380
gtgatgcgcc agtctcagcc tcccaaagtg ctgggattac aggtgggaac caccgcactc 121440
agccagagag ggaatgtcgt taactgaaca ttggaggcat agaaagccac acacccctaa 121500
aaaggggctg tccagaggga tccgcagtca tacagaaaca cagctgccaa aactgtggtc 121560
caggagggag ggaggaaaag gtaataatcc tccaagctct ctctcctccc accaccctct 121620
gatctctttc tgggacctcc caatgtccat atctaagaag aaaccccagt gactcagtct 121680

ctagaagatg gcagaggcat ctcctcgtgg catcttcaca actactcagt aacagtgatt 121740
tccccaccgc ctgtttgaca ggagggcaaa gtgaggcatg gtgaggttgt caccaggtta 121800
cacagctgga gggaagttgg gaactagaac tcctgtttac cccacagcgc ctgctcctga 121860
tcattaatac cttccccatc tgcaaaacag agaccaacac tgacatgagc tctccaatgt 121920
ggatgaagct tgaaaatatg cctagtgaaa gaagccaggc acgaaaagtc acgtactcta 121980
tcatctcatt tatgtgaagt gtctagagta ggcaaagcca tggagacaga aaacagactt 122040
gtggttccca gaggctggaa agaggtggga aatgaggagg aactgctcat gggtataagg 122100
tttccttttg aggctataaa aatgctctgg ggccaagtgt agtggctcat gcctgtaatc 122160
ccagcacttt gggaggccaa ggtgggagga ttgcttcagg ctaagtgttt gagaccagct 122220
caggcaacaa cagccggacc ccctctctac aaaaaaaaaa aaaaaaaaaa aatagctggc 122280
catggtggtg atcacctgta gtcccagcta cttgggaggc tgagtcagga ggattgcttg 122340
atcccaggag ctcaaggtaa tagtgagcta tgattgtacc attgcactct agcccctggg 122400
tgacagagca agaccctgtc ttgaaattga tagctgtaca acattgtgaa tgcacttaat 122460
ggcactgttt tctttaaaat gtttaaaatg gtaaatttta tgtcatatgc actttaccat 122520
ttaaaaaata gcagtcctct cctactgggt gaataggagc atctgaaaat aatcagatca 122580
gggacggagg agtcaaggca caaggcgagt cccgccatgc tgtacgtggt aaggcactat 122640
tgtcatgatt tttccttact ggatactaga ttttcccatg gttgaggtca cagttcagga 122700
gacaggctcc aacccgtctc acaggaactg ctttccccaa acgataaaca tcagtactgc 122760
ccaagactct gatggccata aagcgtatcc tcttccctgc aagtgactta gggagaacca 122820
cagatccatc tctcagttac agctagggtg aaccaagcaa cagagggtta agtgacttga 122880
ccgaggtcac caaagaagat cctggcagca ccaagcccag cctggcccgc ccccaggtat 122940
gagcatggtc cattatgcta tccagcctag gcccatccaa cagaaacaga aactactgat 123000
cgttcgttca ttcattcatt cattcattca ttcattcatt cattctgtac gtcccagatg 123060
ccatgcaaaa ggtgctggga ctacattaga acattaaaaa aacacaggat cccagccctc 123120
aagggccttt tagtcaaggg ctggagccaa ttagagagtg ggaaagtctg gctgggtgtg 123180
gtggctcaca actgtaatcc cagcactttg ggaggtcaag gcaggaagat tgcttgagcc 123240
cagaagtctg agaccagctt gggcagcaca gtgagacccc catctctata agaaatttaa 123300
aaattagctg ggcatggtgg cattcacctg tagtcccagc cacttgggag gctgaggtgg 123360
gaggtttgct taagcctggg aggttgaggt tgcagtaagc cgtgattgtg ccatgcactc 123420
cagcctgtgt gacacggtga gaccttgtct caaaaaagaa aaagaaagaa aaagagagag 123480

tgggaaggtc cttccagggc tcagtccact cagggacaat ggtatgtaat gtctgggggt 123540
gctgcagagg aatattcttt gaaggtacag gattgttggg cgcagtggtt cacacctatg 123600
atcccagtac tttgggagac caaggagggc agattgctca cactcaggag ttcaagacca 123660
acctaggcaa cacagtgaga ccctgtttct accaaaaata caaaaaatta gcagggcatg 123720
gtggcacatg cctgtagtcc cagctactcg gcaggctgag gcaggagaat cacttgaacc 123780
tgggaggtgg agggaggttg tagtgagcca aggtggtgcc attgcactcc agcctgggtg 123840
acagagggag aacccatctc aaaaaaaaaa aaaaaaaaaa aaaaaaggaa acaggattga 123900
tggagacgga ggttctcacc atcccctaaa aggagaatga atgagtcaac atatgcctgt 123960
ctaaatagcc atcaaccatt tactgagcac ctactatata ccacatctgt gtcagaggct 124020
ggagataaag aaatcaatga tagctcctgc ccttaaagaa cttaaatcag agggggagac 124080
aagtacatta aaacacgtcg taagaacaga gaatgatagg aacaaggaag ggaaggccct 124140
aacccacatt gggggtggtc agggaaacct ccaggtgggg aggtattccc tgcactgagt 124200
cttaaaggac aacaaggtgt taaccagctc aagacaaagc agggacatgg gcagaaaaac 124260
aggacacggg gtccgtgggg gtgaactata agcagcttgg tataagcggc tccatcggac 124320
cgaaatggag aagcaaagca agggttagag gtaagcgggg cccaggagca acatccagcc 124380
tggtggggct gggagtgggg ggctgcctgc ctggacaccc ctcttcccca ttccatcacc 124440
caggaccctc ccacccatcc tgcaaacacc agctgcaagt gccttgagtc cggggaaccc 124500
cctttctcct cttcagtccc agcacaggcc ttggtataga ataggtgcta actaaatgct 124560
tgctaaatgc atgccagggg aataaattaa tgatacagct tcctgggagc agggacagcc 124620
tgacttcacc tccgtgacct gggggccttg cacggcggca gtaggtactc agtggactac 124680
actgagccag gccaggggct atttcctgtt caatcagtgt cccctgtccc caggacaagc 124740
ctcccaaaga gaagagccca gtctgtggtc acctgaacaa aggtcaatca agccccaccc 124800
tccttcaggc ctcgactcca tctccagctc ctctggggcc atgtccactg ccacgcccct 124860
tttgaatgta ggtatcattt acagcccaac ctgtcctcac tcaagccctt gacctggagg 124920
cagtaaaggg cagtggctaa gggctcagaa ctgggtatgg ggtacttgaa tttgaacctt 124980
cactttgcta cttcttcgct gtgtgaccct gggcaagtcc cttaacctct ctgagtctca 125040
cttatcctca tctacaaaac agggaggcat acgaacaagc ccttcataga tgtgctgcga 125100
gataatgctc gaaaagcatt tagcatggcg gctggtatgt agtaggtgct caatcaatgt 125160
tatttagtga ctcttactat gctcccttca tttcacgaga gggaagtctt gccaccttag 125220
agccttcaat atgctgttct ctccacttgg aacacttttc ccctcatctt cccacagctg 125280
gttccctctc ctcttttaga gctctgccta aatgttacct cctggactgt gacccacaca 125340

ctactgtatc tcctgcaccc cgcacagtgc cagggacagg gtagacactc agtcagctgt 125400
aatcccagca cttcgggtgg ccaaggaggt cagatcactt gaggtcagga gttcgagacc 125460
agcctggcca acatggcaaa accccgtctc tactaaaaat acaaaaatta gccaggcatg 125520
atggcaggcg cctgtaatcc cagctagtcg ggaggccgag gtaggagaat cacttgaacc 125580
caggaggtgg aggttatagt gagctgagat catggtgctg cactccagcc tgggcaacag 125640
agtgagactc taaaaaaaaa aaaaaaagaa ggaaggaagg aaggaaagta agaaagaaag 125700
gaagaaagag agatgagaga tagagaaaag aaaagaataa aaggaatttt taaaaagaag 125760
gaagaggccg ggcatggtgg ctcacacctt taatgccagc actttgggaa atcgaggagg 125820
tcagatcact tgaggtcagg agtttgagaa cagcctggct aacatggcaa aaccccgtct 125880
ttactaaaaa tacaaaaatt agctgggtgt ggtggcgcat gcctgtaatc ccagctactc 125940
aggagtctga ggcaggagag tcacctgaac caaggaggca gaggttatgt gagccgagat 126000
catgccacca cactccagcc tgggtgacaa agtgagactg tctcaaagaa aaaaaaaaaa 126060
agaataaaat gaatttttaa aaagaaggaa ggaccacact gccttttcaa ctccatcact 126120
agcttctgaa agaggaatat gacttccatc ctctttgttt gggacatctc tcccagtgcc 126180
gtgcacacaa caagcactta atccatactt ccaggtagat ctgaatctct gctgagttct 126240
gtaaaagctt tctaagcaag ccagggtatg acgatggagt gaaggtaaga ctggtgcagg 126300
cagccgcaca tctcacggca gggtcgtggg gcttcagcat cgggctcttc ctcccacatt 126360
attcagccac ttctccttca cctcgaccca gcccaaccat agttggctgt gtttctgcct 126420
ctctggcccc acgagcccct ctccagctgg ttaaattcag aggtttcaac atcccagcct 126480
cctgtgtccc agccggcatc tcatgggccc tgtgccacca gcaagtccca gctgcaggcc 126540
caggatttcc ctcttgtgac cgtattagga agcagctcca gtttttctct gacgctttat 126600
atggtcagcc acagagccct gagcaggggg tgctcccggg gcctgcagca gaaatgagga 126660
acagggaggg aagggaggga agtggaagga acactggctt tcagacacac agggatggcc 126720
gggcgcagtg gctcacacct gtaatcccgg cactttggga ggccaaggtg ggtggatcac 126780
ctgaggtcaa gagttcgaga ccagcccggc caacacagtg aaacctcatc tctactaaaa 126840
atacaaaaat tagcaaggtg tggtggcgcg tgcctgtaat cccagctact cgggaagctg 126900
aggcaggaga actgcttgaa ctcaggtggc agaggttgca gtgagctgag attgtgccac 126960
tccactccag cctgggtgat acagcgagac ttggtctcaa aaaaaaaaag acacacaggc 127020
ctgggcagag tgttggcttt gccttatcac ctgcctgact tgtgcataca gacctacccc 127080
tgagcttcct cagtaaagtg atggataatc acggcttctt tatgaagctg cagtgcaata 127140

tcacggcttt ctaaataccc aacctagaga cagacgtggt ccaccaagca cgtgcttttg 127200
ctgcagcctt gcttttaaga gcaaaaagac tagaaacaac ctaaatacct gtcaatagca 127260
gacaggctta atcattatgg ttcatcccta tagcagaaga ctgtgcagtg gccctctctc 127320
tactaccatg gatgaatggc caaaatacac agttagatgc aaagattgag acgcacaaca 127380
gccggggcct gtgctgccat tttggagggg tgcaggaggg gctctctatg tggctgcatg 127440
tgtataaaac atcactggag gctggatgga gcagctcaca cctgtaattg tagcactttg 127500
ggaggctgag gtgggtggat cacttgaggt caggagttca agaccagcct ggctaacatg 127560
gcaaaaaccc atctttacaa aaaatacaaa aattatctgg gtgtggtgat gcacacctgt 127620
aatcccagct actcaggagg ctggggcggg atgatcgctt gaatccagaa ggcataggtt 127680
gcaggagccg agatcgcacc attgcactcc agcctgggtg acagagtgag actttatcac 127740
aaaaaaagca ataggctggg cacagtggct taagcctgta atcccagcac cttggcaggc 127800
cgaggcaggt ggatcacctg aggtcaggag atcgagacca gcctggccaa catggcaaaa 127860
ccccctctct actaaaaata caaaaatcag ctgggtgtgg tagtacacgc ctgtagtctc 127920
agctacttgg gaggctgagg gaggagaatt gcttgaacct gggagggggc tgttgcagtg 127980
agccaagatc atgccacttc actccagtct gggtgagaga gtgagactcc atctcgaaaa 128040
ataatataaa taaataaata acatgactgg agggctacat atgtaaccag caaccacagt 128100
gtctctaaga aggggaactg gaagggcgag aaataggaac gaagggggaa gttctatatt 128160
cccttataca ctattttatt tcatatttgc atgtcttacc tattcaaaaa catatttgaa 128220
gtctgactag atagttatga acagacagaa aaaactataa ggtagggtta agtggaaaaa 128280
aaatcatact gcaaaacata caaggagtga ctttttctta tgtttggggt gtgtgtgtgt 128340
gtgtgtgtgt gtgtgtgtgt gtacatacat gaacctgtga atcatgtttg cctccaggga 128400
agagggctgc tttttgtgat acatacttat ttttaaatat tctcctgagg tctttgcata 128460
gattatcctt gtattactgg aggaggttct ttgaagcaat aatgacttga aagatcagaa 128520
ggctgggtgc agtggctcat gcctgtaatc ccagcacgtt gggaggcgga ggtgggagga 128580
tcatctgagt tcaggagttc aagaccaacc tggccaacat ggtgaaacca cttctctact 128640
aaaaatacaa aaattagctg ggcgtggtgg catgtgcctg taatcccagc tactgggagg 128700
ctgaggcaca agaatcgctt gaactcagga aatggaggtt gcagtgagcc aggatcacac 128760
cactgctctc cagcctgcgt gacagagcga gactccatgt taaaaaaaaa aaaagatcag 128820
aagggctaat gcttggggct tgacaaaggg cagcttccag ttacagtgtc tttctaccct 128880
ctagggacca gtgcagtggc tggcacccag gaggcctctg ttcacaatct gcttgttcct 128940
tgtgtccccg ggtggacact gcttccagag taggctaaga ttgctgggcc aggtctctaa 129000

ggcggaaaaa ttgtcacctg ctaaagtttg gaaggagggc ttctcctgac cccctgggca 129060
ttccagaagc agaaccagaa ctggaaaaat gtcctttggg gttcatggtt cagggacttg 129120
ggcttggagt tccaatgagc aggggggccc atccaccttg gctctggatc atgattttcc 129180
caaccccaga ggtttggtat agggtggagg gtcccactgt ctacagagac aaagcaggta 129240
aggcatgagt gaagcagggc gtgtgtgagt gaacaaaaga ggtgccacgg tcaatggtta 129300
ccatggcctg ggagacgaca gggagtggtg aggtctgcgg aaaagcagag caaaaagccc 129360
atctcacagg gactgctact cgcactctgc attctgcagt gtcaggtcct ctgctttttc 129420
gagcaatgcc agaaacagat ttcttttttt gatatattaa gttaactcaa tttaaaaaca 129480
aaaatcactc ctctgtgtta gcaggttgta atcattagtc tagcacctag ttaggtgctc 129540
aaggaatcat aataaaaggt tcaattcctt cttgctctgc acttaaagct tgtctacctc 129600
aaagctaaca aggcctctgg cactgtcccc tcctgctcca gcccacgacc atcacctcct 129660
gctctaagcc tattacccca agagcccttt gctactaatc ataggtagtc accctggccc 129720
tggactcaca cagaccttgt ctgaatccca gctctgccca ccttccagtg gtgggactaa 129780
gcacatctga gcttcagcaa cctcatcttt tttttttttt tttttttttt tttttttttt 129840
ttttgagatg tagtctcact ctgtcaccca ggctggagtg cagtggcaca atatcatgcc 129900
tgtaatccta acactttggg aggccgaagt ggtgaagatt gcttgagctc aggagttcga 129960
gatctgtttg ggcaatacgg tgtgacccca cctctaccaa aaacacaaaa aaattagctg 130020
ggcatcgtgc tacatgcctg tagtcccagc tactaaggag actgagatag gatcccttga 130080
gttccaggag gcagaggttg cagtgagctg agatcatgcc actgtactcc agcctgggca 130140
acagagtaag accccatctc aaaagaaaga aaaagaaaaa agaagaaaga aaaaagaaga 130200
cagaaagaca gagaaagaaa ggaagaaaga gagagggagg gagggaggga ggaaggaagg 130260
aaggaaggaa ggaagggagg aacgaactaa ctaactcgag gcagacgaac tcagccaaaa 130320
gacagcattc agaagaccaa gggaccctta ggaccaggcg tcaaagccat gctccccgcc 130380
aggtatgcca caaacggtct tcagcagttt aggtagactg ctggacattg ctgagcccag 130440
aacctgcatc tctaagatta ggaggaaagc tcctctggct tctatagact cttctagatc 130500
caagagcaca agaggtctgc cagaaccttc tcaagggcac atcgtttgag gcgagccttg 130560
cagaacggag gagaaagttt atcttcatca cagagggcaa agtcatgcat gccagaccac 130620
ggacctggta tcacctcact acccgcctat taacaagact catgcatgct gctattatgg 130680
gctgggagag taagttactg tgctccccca ccccaacccc tttccttctt tatcttctcc 130740
gtcatggacg acggtttcta ttaagcaatt aaactgacca gaaaggaaca agagaagagc 130800

gggaccatca tcacaaggct gaaaggattt taatctaatc tgtccagaga agcatagcag 130860

gaatttgaag ctttgattca tttattttta atctgctgtc agaagatatt ttgggtctca 130920

ggaccttcat atgccctttt tttttctgat ttctatttga ggacagcagg tgagatgaat 130980

acacaggcct gtcctgaaac tccctagatg gtgatgatac gtcttagatg aaagacctgg 131040

gccccaaaga gtccaatttt ccctctagct ccctacagac gggcctcttt agaaatatcc 131100

aacatactgg ctgggcgtgg tggttcaggc ctgtgatcct agcactttgg gaggccgagg 131160

caagtggatc acctgaggtc aggagttgga gaccagccta gccaacatgg tgaaacccgt 131220

ctctactaaa aatacaaaaa tcagccaggc atggtggcac atccctgcaa tcccagctac 131280

tcgggaggct gaagcaggag aatcacttca acccgggagg tggaggttgc aatgagccaa 131340

gatcgctcca cttcactcca gcctgagcaa caaaagtgaa attccatctc aaaaaaaaaa 131400

aaaagaagaa agaaagaaaa agaaaatgtc caccatacaa acagccccag atcaataatt 131460

tttccataca aacagtgcca cagacaagag acttccccta cttaagaaag taaagataca 131520

ttttgcaaca ctgcacctca gcttccttcc ctaaaagggg caaacatttt taactcaggg 131580

gatgctgtgt taaataaaat acaatgtgtc aatgtacagc acttggattg gaggacatat 131640

ttaatagtta cggccaggca cagtggctca cacccgtaat cccagcgccg tgaaaagtca 131700

aggcaggaga tggcttgagg ccaggagttt gagaccagcc tgggcaatat agtgagatcc 131760

cagtctctac agaagttttt tttttttttt ttaattagcc aggtgtggtg gtgcacacct 131820

gtggtccaca gctactcagg aggctgaggt gagaggatcg tttgaatcca ggacttggaa 131880

gctgtactga gctacgatag aaccactgca ctccagcctc aaagacagag taagccccca 131940

tctcaaaaaa aaaaaaaaaa gttatgagtt gctaagttgc tcactcttac catttggctc 132000

tttaaaaagc cactctaact ccttctgaaa tatctataag aacagtggaa aaatggccta 132060

tccccatgac agattctata ctacccctta caaggggtat ctttggggct tcctgagata 132120

tattacacaa gtcaacactg agcatttcca aatggaaaaa agagcaagtc actaaactgt 132180

gtgcccaata ggcttttaaa tatgtaaaca gataccatgc tcacaaagag acaaagaatg 132240

caccgcatct ctggttaagg actaattttt ctgttttgtg ttcactatgc tttctgaact 132300

ctccaagtgt tccatagtga acgtgtctta gtttcctaat taggaaaaaa atgtgcagga 132360

catttttaga gatgacaagg ggctttttgt aattttcatt tggcaaaatg tacagaccag 132420

aaccaaagag acacaaggat gccaagtgat ggaagaagaa catcttacta gtaaaatcaa 132480

aagtgatcat gaccacagca aaaaccaatc tagtaacact taacattttc agtgcctgca 132540

cttaacatgt agcaggaagt gtattaagtg ctttctattt agagtaaccc actccatgaa 132600

gctggtacta tttttttttt tttttttttt tgaaacagac aaggtcttgc tctgtcaccc 132660

aggctagagt gcagtggcac aatcatagct cactgcagcc ttgaactcct gggctcaagt 132720

gatcctcctg cctcagcctc cctgagtagc tgggattatg agtacgcacc actgcacctg 132780

gctaatttat ttttgcagag atggggtctc gttgtgttgc ccaggctggt ctcaaactcc 132840

tgggctcaag tgagccacct gcctcagttt ctcaaagtgc tgggattaca ggcatgagcc 132900

actatgcccg gctcccaaga ggctggcact attcttgcat ccattttaca gaaaaggaaa 132960

caaagcttca gaggcatgag gtaacctgct cgagggtgag caaggccttt cagagagaag 133020

acatagacat aggccgggtt gggtgacctc tgcacatcag tctggcgacc cgtgccccac 133080

aacatcacac tccaagcccc aatcatctag tccaagcagt ccaacagaac tttctatgat 133140

aatgggaaca ttctgcactt atgctaccca gtacaccagc cactagccac aggtggctat 133200

tgactacttg acctagggcc aacacaacaa aagaaataaa tttgttattt tttattgtct 133260

tttacttatg tatttattta gagatgaagt tttgctctta ttgcccaagc tggagtacaa 133320

taacgcgatt ttggctcact gcaacctcca tctcccaggt tcaagcgatt ctcctgcctc 133380

ggcctcccaa gtagctggga ttacaggcgc acaccatcat gcccagctaa tttttgtatt 133440

tttagtagag acggggtttt accatgttgg ccaggcttgt ctagaactcc tgacctcagg 133500

tgatccaccc accttggcct cccaaagtgc tgggattaca ggtgtgagcc cacgcaccca 133560

gctaataaat tttttaatgt ttcttttttt tttttttttt tttttgagat ggagtttcgc 133620

tcttattgcc caggctggag tgcaacggca ccatctcggc tcactgcaac ctctgcctcc 133680

cgggttcaag caattctcct gcctcagcct ctcaagtagc tgggattaca gatgtgcact 133740

accatgcctg gctaattttg gcatttttag tagagacagg gtttcatcac gttggccagg 133800

ctggtctcaa actcctgacc tcaagtgatc tacccgcctt ggcctcccag cgtgctggga 133860

ttacaggcat gagtcgccaa acctggtcta atgtttcttt taattaattt aaatctaaat 133920

ttagttagcc acgagtggcc agtggctacc atattgtgcc atctctagtc cactctgttc 133980

tgcaagtcta cctaaaatag tcctacgacc aacctgaaca ttcccctctg tgacaaggaa 134040

ggcctcccta aaacctagcc acatcctcat gtctcctacc cacctggctg ccaagacaaa 134100

ccagcctttc caaagtggtt atgagctctt ctttactttc aagaggaagg aaggacagag 134160

aacgatgcca atgaaatctg ttgagagttt atcatacgtc ttggcacttg gcacatgtga 134220

tgacatggga ttgtcacacc tcctgtctgt aatgagggtg attattccca ttttacggat 134280

aggaaaacca aggcttcgag agaggaaata acctcttcaa gggctgagat gtgaacccac 134340

cttggaatga ttccacctca tcttgcaatc tccctcgatc aaaccttcag cctcccattt 134400

cctttttttt tttttttgag acagagtctt attctgtcac ccaggctgga gtgcagtggt 134460

acagtcttgg ctcactgcac ctccacctcc tggattcaag caattcctct gccccagcct 134520

ccccagtagc tgggattaca gatgcgcacc accacccttg gctacttttt gtatttttag 134580

tagagacggc atttcatcat gttggccagc gtggtctcga acttctgacc tcaggtgatc 134640

tgcccacctc ggcctcccaa agtgctggga gtacaggcat gagccactgt gcccagccct 134700

cccaacacat ttctaatgcc tactttcctt atgaggccag gagtaggtac ctggctgcct 134760

gcaatactca catatattag ccctgagaag taccgctccc tcaggttgtg tagcacggag 134820

gcttcgttga ggcacgtcag ctccgccatg tcctccacct tggagaactt gggtgggttc 134880

atcttctgga tgtcatcttt cccaaccgtg accttcttgc cattctccac cagctccaca 134940

accacctcat cccccttctc ctccttaatg ctggctgcct cgaagccctg cttctccgag 135000

gggacccaga cgagtctctt ggcggcccag tcagcctggg ccactgggct gttgatgaag 135060

tttttgtcca caaagaggaa cttctcatcg tcactgagtt ggcccttctg cgccatggtg 135120

ccttgttggt cccctgtgga ataaggtaac agggtcagaa tcagaccaca accaagcccg 135180

gaagacagac caaccactca ccttgccctg tctaggaact cggtctgcag agacatccca 135240

ccaagtacaa agacctgcag tattgtgtat gtggtcaaag aacagagacc acccaaaggc 135300

ccttcagaaa gggatgtgtg tcatcgccta aaataccaaa agtcagacaa cccaaatgtc 135360

caccaacata cagctggata cataagccat gctatttcca cactgtgaaa tactacccag 135420

tgctcaggag gcaggaagta gggctggaca aggtggctca cacctgtaaa cccaacactg 135480

tgggaggccg aggtgggcaa atcacttgag gccaggagtt caagaccagc ctggccaaca 135540

tggtgaaacc ccgtctctac tgaaaataca aaaattagcc aggtgtgatg gtgtgcacct 135600

gtaatcccag ccacttggga ggctcaggca gaattgctca aacccaggag gcagaggttg 135660

cagtgagcca agatagtgcc actgcactcc agcctgggtg acagagccag actccatctc 135720

aaaaaaaaaa aaaaaaaaag aagaagaagc cggaagtaag tgtacacgtg ctaatatggg 135780

aagatggtca agtctaactg tgcgctgcaa aataagagtc ctcatacgat taaacataga 135840

attacccaga ttccttgagc ccaggagttg gagaccagcc taagcaacat ggcaaaacct 135900

tgtctctaca aaaaatacaa aaaattagcc aggcatggta gcgcacacct gtagtctcag 135960

ctacttggga ggctgaggtg ggagaatcac ctgagcctgg gaggtcaaga ttgcagtgag 136020

ccaagatcaa gccactgcac tccagcctgg gtgacagagt gagactctgc cttaaaaaca 136080

aacaaacaac ccaaggtata taactggaaa ctcagtaaaa ggaaagaaat tatgcaataa 136140

aataaatgag tctcaaaagc atcatgctca gtgaaacaaa tcagaaaggc cacacgctct 136200

attactcctt caatacgaaa aactattagg ctggtgcaaa cgtaagtgtg gttattgaga 136260

tagaaggtaa tggcggccgg gcgcggtggc tcacgcctgt aatctcagca ccttgggagg 136320

ctgaggtgag tggatcacct gaggtcagga gttcaagacc agcctgggca acatggtgag 136380
acccctgtct ctactaaaaa tacaaatatt aatggagtgt ggtggcacac acctgtagtc 136440
ccagctactc aggaggctga ggcaagagga ttgcttgaac ccaggaggcg gaggttgcag 136500
tgagctgaga tcgcgccact gcactccagc ctcggcgaca gagcgagact ctgtctcgaa 136560
aaaaaaaaaa agtaatggca aaatagaaaa ggcatatcta tggtgacagg caatcaagag 136620
ttgccagagg aggccatgca cggtggctca cacctgtaat cccagcactt tgggaggcca 136680
aggcaggggg gatcacttga ggtcaggagt ttgaaaccag cctgcccaat gtggtaaaac 136740
cccatctcta ctaaaaatac aaaaattagc cgggcatggt ggtgcacacc tgtaatccca 136800
gttactcaaa aagctgaggc aggagaatca cttgaacctg ggaggcagag gctgcagtga 136860
gccgagactg caccattgca ctccagcctg agtgacagag tgagacgctg tctcaaaaaa 136920
attaaaaaaa agagttgcca gtggcaagga aaggaatgga ttagtaagag gcacagaaga 136980
ggtacataag aggtaagagg caccaccttt cggggtgata aaagtattcc ctgtggtcat 137040
ggtggtgctt acaagactgt atacatttgt caaaactcat taagaatgtt ctatccacta 137100
taggatgagt aaagtgaaca ataaaatatt acatcgtttc caagagctag aagaaggaca 137160
ttgaatgttc ccaacacaaa gagatgataa atgtcattat ataggaagta gcaaaataaa 137220
agtaatggca aatgcttgag atgatggata tgctaattat cctgatctta tctccccaca 137280
cattatatgt attaatatca aaacattact aagagctgga ctcagtggct cacacctgta 137340
atcccagcac tgtgggaagc tgagacagga ggatcccttg aggccaggag ttcaagacca 137400
acctgggcaa cataagaagc ccctgtctct gtaaaaaaat taaaaattag cctggcacgg 137460
tggtgcatgc ctacggttcc agctactcag gaggctaaga cgggaagatg gctagagccc 137520
aggaggtcaa ggctgcagtg agttgtaatc atgccactgc actccagcct gggccacaga 137580
gcgagaccct gtctcaaaac catccaacca aacaactaaa aaaatcatta tggaccccat 137640
gaacatgtat gattatttgt caattaagaa ataaattttt taagttgaaa aaactcactc 137700
aagctgtaca cttaaaaaaa cttgaatttg ttttaaatcg gtgagtttta ctttatatag 137760
attatacctc aataaagctg attaaaatat ttgtcataaa gtatttgtct tcattttata 137820
tgtttgtgtg tacatttcta aaagtctaga aaactgcata ccaaaatatt atactagggt 137880
tctatctggt caaatacgag acagtgtgaa tatcaataag aaaaataatt atatgtccat 137940
caccctaaca ccccaaaaag ggtggaaaga caaatatatt taaatgcatt tgttcaaaat 138000
gatttttgtt gttgttgttt gagatggagt cttgctctgt tgtctatgct ggagggcagt 138060
ggtgcaatct tggctcactg caacctctgc ctctgggttc aagcgattct cctgccttag 138120

cttcccgagt aactaggatt acaggcgtgc accatcacac ccagctaatt tttgtatttt 138180
tagtagagac ggggcttcgc caagttggcc aggctggtct caaactcctg acctcaagtg 138240
atccaccagc ctcgggctct caaagtgaag ggattacagg catgagccac tgtgcccagc 138300
cccaaatgca ttatttttta aatgggtaca gaaagtgggg tggaaatgtg aaaatcttga 138360
ttctgcgttt tctatacaaa ttgttcctta gggtaactaa attaagaagg ggaagtctct 138420
cttgatggaa gattaggtca ttaaatgtcc tgtctacatg ggaagcccat ggatggtaga 138480
ttcacgttat ctgtcgttat ccagatgtgt ttatgaggag agggacagag acaacacact 138540
ctataggcct gtccggcccc ccgttcctga ctctctgacc atagctgcag gggctgtcct 138600
ggaaacactg gcttacagcc ccttctacct cttgctgctg ggctccaaag aactgagttg 138660
ctggctaagt gaggacatat ggccaccagg atctatgcgt ggttgacatc cctccttcag 138720
cagggaattc acaactcctt tgaattcact gataatcccc acgcttgggc cccaaatccc 138780
acagcgacag aataaagacc catgtcccac tcctggatcc ctagagctga gcagccaact 138840
cctgaaaccc aacctttggg gtgatgcaga ttttgcaaga cagaatggga gtggcagata 138900
ttccaatacc tatttttttc ctttcagggc tgaaaataat aattaaaaaa ctaattttaa 138960
gaacctgctg ctgggtgcag tggctcatgc ctgtaatccc agcactttgg gaggccaagg 139020
ccggtggaac acctgaggtc aggagttcga gaccagcctg gccaacatgt gaaaccccat 139080
ctctactaaa aatacgaaaa gttgctgggc gtggtagcat gcgcctacaa tcccagctac 139140
tcgggaggct gaggcaggag aatcacttga acccgggagg tggaggttgc agtgagccaa 139200
gattgtgcct ctcaccgcac tccagcctgg gcagtaagag cgaaactcag tctcaaaaaa 139260
acgaaaaaca aacctggaag ttgatttgct cagagatttt ctgctgggag acctgatatc 139320
gtagctgatg aaaaaaaagt ggcggctagg tgcggtggct cacacctgta atcctagcac 139380
tttgttgggg ctgaggtagg aggattgctt gaggtccgga gttcaagacc agctggccaa 139440
cgtggcgaaa cctgatctct actaaaaaca caaaaatatt agctgggctt ggtggcaggc 139500
acctgtaatc ctagctactc gggaggctga ggcacgagaa ttgcttgaac ccaggaagca 139560
gaggttgcag tgagctgaga tggtgccact gcactctggc ccaggcaaca gagcaagact 139620
tcatccaaaa aaaaaaaaaa aaaaaaaaaa aaaagggcag agaaatgagg aaatcccatc 139680
taaatctgaa taatcacttc agatatccag aaagagaaat aaagatctgg tagacaacat 139740
gtattgtcct aagtttaaca atcctccttt ctgcatttta aaattgttat tgaatttggc 139800
ttagctcata gtaagaccaa cagtgtcctg cccctgactc cagttaccct atcttaaaaa 139860
agactccctt ctacatggaa gaattccagc gaatacatgc agaacaataa aactaggatg 139920
taactgttta taaagctaat agtgggtctc aagctgggca cggtggttca cacctgtaat 139980

cccagcactt tgagaggccc aggtgggcgg accacctgag gtcaagagtt tgaggccagc 140040
ctggtcaaca tgggaaaacc ctgtctctac taaaaaatac aaaaattagc tgggcatagt 140100
ggcatgtgcc tgtaattcca gctactcagg aggctgaggc aggagaatta cttgaactcc 140160
aaaggtggag gttgcaatga gccgagatca tgccactgta ctccagcctg gacaacagag 140220
caagactttg ccaaggaagg aaggaaggaa agaaagaaga aaggaaggaa gaggccgggc 140280
acagtggctc acacctgtaa tcccagcact ttgggaggct gaggcgggca gatcacgaag 140340
tcaagagatc gagaccatcc tggctaacac ggcaaaaccc tgtctctact aaaaaaaaaa 140400
aaaaaaatta gccgggcgtg gtggcggccg cgtgtagtcc cagctactct ggaggctgag 140460
gcaggagaat ggtgtgaacc tgggaggcgg agcttgcagt gagtgggtga gtggagatgc 140520
gccgctgcac tccagcctgg acgacagagt gagactccgt ctcaaaaaaa aaaaaagaaa 140580
gaaaggaaga aagaaagggt cttggcaagt tggcaagttc taccagtaat tgctaaaacc 140640
attaggccaa tggggctcac atttcctggt gcattagctt ctgagtcagt aggactggga 140700
tggggcccaa gaatatgcat tcctaaccag cttccgggtg atgatgatga tgctggtcta 140760
gggtccccac cacaagactg actatatcag atgaagagct cttgtggaat ttttaaggga 140820
tgggtccgcc tgccatcacc taaatctact aattaaccct aacatcacaa aaagagagag 140880
ctgactagac attataaaca ttacatgcct gcagatgggc cactacagga agtgccgtta 140940
ccactaatga catattcttg ctcaaaagaa aaggggatgg agatggggga tgaaggtgat 141000
gaagcctcca gatctgacgc ctgtctatgg aaaatgcaag gcacaggaga gcatgctaaa 141060
caatgcagta aactcccaaa ggtgagagac tatgctctgt cgcccagcta gagtgcaatg 141120
gcgggatctt ggctcactgc aacctccacc tcctaggttc aagtgattct cctgcctcag 141180
cctcccgagg agctaggatt acaggtgcat gccaccacgc ctggctgatt tttgtatttt 141240
tagtagagac ggggtttcac catattgtcc aggctggtct caaactcctg acctcaggtg 141300
atctaccggc cttggcctct caaagtgctg acattacagg tgtgagccac cacgctcggc 141360
cagtactctc tttctttaac aaataaaata caaacaaaaa ggaggggaac ccatagatta 141420
aaaaataatt gaaagaaaag aggctggcag tagcccacat gtataatccc atattttggg 141480
aggccaaggc aggaggattg cttgagccca ggagttcgag accagactgg gcattatagc 141540
aagaccccat cccttaaaaa aaatatcagc caggcatgat gatgcgtgct tgtagtccca 141600
gctactagga ggctgaggca ggagggttgc ttgagcccag gagtttgagg ctgcagtgag 141660
ctatgattac atcactgcac tccagcctgg gtgtcagggt gagaccctgt ctcaaaaaag 141720
agaaagccac agagaacgag aacatttcat aaatataggg cattttcatt agcctaaaag 141780

atctttctga gcctaaataa agtacaagtt cattggtgct ggcaacattt gattggttgc 141840

ctcctcttga gaggtaagat aacctgtagt catgtaaaga cacccagact ttgcaatcag 141900

atgctaattc aaacgtgact ctgccactgc agaatgcgtg cttctgggtg acttacgtag 141960

atctgagact cagatttact gtatattaat actggggacc taacgggtgc cctgcagagc 142020

tgccatgaag tttagatgaa actctctaca ccaagcttgt ctaacctgca gcccacaggc 142080

tgcatggggc ccaggacggc tttgaatgtg gcccgataca aattcataaa ctttcttaaa 142140

acagtatgat attttttcgc gatttttttt ttttttttta gctcatcagc tatcattagt 142200

gctagtgtat tttacgtgtg gcccaagaca attcttcttt ttctggggtg gcccagggaa 142260

gccaaaagat aggacacccc tgatctacac caagtacctt ctgtttctat accctttctc 142320

acttccggtg actctaagcc acttcgctca ttcctaacca atagcattta ctactgttgg 142380

atgttttaat tatgtttatt tctggacagc catagtcaca gaagtggctt gatacgacgc 142440

gtcttctttt aatagtataa ccaaggttag tttaaacagg cagcaaaata ggaaatgcgg 142500

caaaatattc aatcattccc tgataatctc ttgtttctta aggtgtcgca gcagatcgtg 142560

tgtgtgtgtg tgagagagag agagagaaaa agaaggagag agagagagag aaactggagg 142620

atgcgtaaga gaaatgggag ggtgtgaaga tgaagtaaga ttgatcacaa gtaggccgag 142680

tgtgatggct catgcctata atcccagaac tttgggaggc tgaggtggga ggataacttg 142740

aggtcaggag tttgagacca gcttggccaa cattgtgaaa ccctgtctct actaaaaata 142800

caaaaattaa aaattagcca ggattggtgg tacgtgcctg tagccccagc tacttgggag 142860

gctgagtcag gagaatcact tgaacccggg aggtggaggt tgccgtaagc cgacatcata 142920

ccactacact ccagccttgg cgacagagtc agcctccatc tcaaaaaaaa agaagtgtca 142980

gagctgggac ccaaacctgg gtctcattct ggagtctatt tggttctgcc tttcacataa 143040

ggtgggtggg cttgtcagat gcccataaag gaatcaatcc tgtaaggcac ctggcacagt 143100

acctgacaca ttacaaacac ccaaaatagg actcgaatca ggtgaagtaa actacagctc 143160

cacaccacaa aaaaggacat ctcacaacac atccttgagc aaaagaaggt ggtcacaaaa 143220

agcacaggtt ctgtgactat tgaactgagg tccaaaacca ggcagaatta atctacggtg 143280

atagaactca gataaggaat gaatgagtct ccggtgtgca atgattgcaa ggacagaacg 143340

tgaggggggc ttgtgagggt tgacctctag atcttcatct gaatgccagc cctatgagtg 143400

ttcactttct gaaaattcat caaactgttg ggccaggtga catggctcac gcctgtaatc 143460

ccagcacttt gagaagccaa gatgggagga ccacttgagc ccaggagttt gagaccagcc 143520

tggccaacac agcaagaccc atctctaaga aaaaatgttt tttaattacc tgtgtggaag 143580

actgaggtag gaggataacc tgagcccagg aggctgagac cggcctggtc aacatggtga 143640

aaccttgtct ctagcaaaca tacaaaaatt aaccggttgt gatagtgtgc acctgcagtc 143700
ccagctactc aggaggctga ggtgggagga tcacttaagc tcaggaggtc ggggatgcag 143760
tgagccatga tcatgccact gcactgcagc ctgggtgaca gagtgagacc cccatctata 143820
aaaaatatat cctcttgaaa attcatcaag ctgcacactt ttttgcacat caagcgtact 143880
tttttggttg taaagtacac ttcattttcc ttccctctta tctttaattg tggtaaaata 143940
cagctaacat tcaccacctc ggccacttaa acacagttca gtagcattaa gtatattgat 144000
attcttgtgc aataatgaag agtttgtgca gatggtagcc aattacatcc caagagggct 144060
aggagagtgt ttcatttgtt ctaagcacta tgacaagtaa agtggacttc tttttttttt 144120
tttttttttt ctgagacagg gtcttgctct gtcgcccagg ctggagtgca gtggcatgac 144180
ctcagctcac ggcaacctcc gcctcccagg ctcaagtgat cctcccaccc cagcctccca 144240
aatagttggg accacaggca cctgccacca tgcccagcta ttttcctgta ttctttgtag 144300
agacggggtt tcaccatgtt gtccaggctg aggtcttgaa ctcctgacct caagtgatct 144360
gcctgcctcg gtctcccaaa gtgctgggat tacaggcatg agccgctgtg cccagcccta 144420
agtgggctct tcctaattgc catgtggatg agttgcctta aaagtcagaa aaaatagcaa 144480
aagctacaac agaaaaataa aaggacacgc acgcctccat tgcctgggac cctgtctaac 144540
ctcagactta aaaacacaaa caagatcaag tgcaaagatt cagtctctgt acaaaaaagg 144600
agagcttgga gcaggctgga ttccctctgg ctttgatgtc tctgacagtt acgacgtgca 144660
aaatctgctt tggtcaaaag acagcaagat gggggatgag aagcttcgac tcatgctaga 144720
atggtgtttt ccatagcatg ccccttggaa ccctaattcc ctgaaatatc tgggctgggg 144780
tggagctgcg gaggttgaga agctcttcaa acagtggcgc agtcaaggtc aaatccatct 144840
ggaaggtgct acatattccc ctttccctca ttgagacaca aaacacacaa caggacactt 144900
gagtctctgg cagatccgga aacctgaaag ctgttttgct cctacgagcc ttgggtcccc 144960
aaaacaacat ttagccacag atttgccaaa tgttttgaga atgttttcct aaaaatttgc 145020
caaatttttg agaaaattca gtgagcctga caaattaata atactaatac tagtaataat 145080
agttgctaag tctttttttt tttttttttt ttttgacaca gagtctcgct ctgttaccca 145140
ggctggagtg cagtgacgca atctcagctc actgcaacct ccacgtttct gggttcaagt 145200
gattctcgtg cctcagcctc ccaagtagct gggactacag gcatgcgcta ccatgcctgg 145260
ctaatttttg tatttttagt agagatgagg tttcaccata ttggccaggc tggtcttgaa 145320
ctccaggcct caagcaatcc tcccaccttg gccccccaaa gtgctgggat tacaggcgtg 145380
agccaccgca gctggccaca aactctgcac tgtgactctt gggctgcgtc attctgtgtc 145440

gtgaggggag ctcttccgtg cattgtggga tgttccgcag catccctggt ctctgcccac 145500

ctgatgccag tagcacccct tcctccactc atgacaatca aaaatgtctc cagacactgc 145560

tagatgtccc ctggggtggc aaaactgctt ccagttgaga accacagctt tagaataaaa 145620

acaagggctg tcttgggaaa ggcagttaaa gctgagagca gctctcactt agtcaaactc 145680

aactcggaat accaactgca gaagatcaga cgggggcaag gtgaacacac tggaccagag 145740

acggaccgcc agttggccca accccaggga cactcttcat ctaccttgat gcaccttccc 145800

aaccctccct cctccaccat cgctcatgtt taaggtcctg catcagctcc aagggctgaa 145860

aggacatctc tgaaattact acccagaact gctgaccacg aagtacagtt cactcatcaa 145920

gagctgctct caatgttttt ggttggggga agggtctcac tatatgccca gggtggagtg 145980

cagtggtgca atcatagctt actgcaggct caaactcatg ggctcaaggg atcctcccac 146040

ctcaggtgtg ggccactatg cccggcttat tttttgtaga gatgggggtc ttgctatttt 146100

gcctaggctg gtctcaaact cctgatctca aatgatcctc ctgccttggc ttcccaaagc 146160

tctaggatga caggcgtgag tcacctcgtc caactttctc gaatgtttgg atgaaaacaa 146220

atgactctgt cacctttggc attagcgatg tttgacacat ctgagtgtgt cagccttgtt 146280

tacagtcctg tcctgaccaa caactgcttc attctttttt tttgagacag ggtctggctc 146340

tgttgcccag gctagagtga agtggcgcga tctctgcttt ctgcaaactc tgcctcccag 146400

gctcaagcca ttctcccacc tcagcctccc aagtagctgg gatcacaggc acgcaccaga 146460

acacccagct aattttttaa ttcttgcatt tttttgtaga gacaggggtc tcactttgtg 146520

gcccaggctg gtctcaaact cctgagctca agcgatctgc ctgcctcagc ctcccaaagt 146580

gctaggatta caagcatgag ccaccgcgcc caggctgctt cattttttga gcacaaacaa 146640

gctaatcatt agcctctgga agaccttagg atgtcaagta acgagtctca gctttgcccc 146700

aaacctgctg ggtgactctg gacaagtcac ttgccctctc tgggccttgt atttttcatt 146760

tagggaaaat aaatcaatgg taggctggcc cagctctacc attttatggg acccgtctgt 146820

gactcccagt caaagggctg aaactggacc aactgagaat gtcaggttgc tttagtttgt 146880

tttaccatca gtttcatttt tcccagaaga actcaggggc aaaagatctt gccctctctg 146940

cttcagcatt aataaaaagt aaattaggcc gggcatggtg gctgatgcct gtaatcccag 147000

tacttcggga ggccgaggca ggcggataac ctgaggtcag tagttcaaga ccagcctggc 147060

caacatggtg aaaccccgtc tctactaaaa atacaaaaaa tagccaggtg tggtggtggg 147120

cgcctgtaat accagctact caggaggctg aggaaggaga atcgcttgaa cccaggaggt 147180

ggagggtgca gtgagctgag atcccatcac tgcactccag cctgggcgac aagagcaaaa 147240

ctctgtcttg aaaataaata aataaatagt aaattaaata tcccagagcc cagagagtat 147300

aggatttgca catcacctca cctggtcaac agcttccctc aggattttac tgccaagcca 147360
ttggctaaag attttggacc agagacaaaa acccaaatgt ttttggagat caggtaagta 147420
atacagagtg acccagacca gatgttagag tattgcttat attctaaata ccatgcaggg 147480
ttcagcactg tggctcacgt ctgtaatccc aacactggga ggccaaggca ggcagttcgc 147540
ttgagcttag gagtttgaca ccagcctgcg caacatggtg aaaccccatc tctacagaaa 147600
atacaaaaat tagccaggca tggcagcaca tgccggtagt cccagctact cgggaggctg 147660
aggtgggaag atcgcttgag cctgggaggt agaggttgca gtgagccaag atcatgccac 147720
tgcactctag cctgggtgac agagtgagac tccatctcaa aaagaaaaaa aagaaaaaag 147780
aaaaattgct catattctaa atgtcataaa gggctggata cagtggttca cacctgtaat 147840
ccaagaacta tgggaggcca agccaagagg attgcttgaa ttaggagctc tagactagcc 147900
tgggcagcac agtgagatcc catctctatt tcaaataaac gaatgaatga atgccacaca 147960
ggcaaaacag aagttctctg aacctgatct gcaacacaat tcgccagctg gaaggacctc 148020
tgcagtggaa atcctcgctc aaggcctggt ccaggtgtag ggaggcatca aatgtgtcgc 148080
agggagaggc actgggcact ctaaatgcac ctgcttggca ggtccccagt agtaccaaaa 148140
aggcagttta gatctattat tcataacagc cactgtattt attgggcccc ttgtattcat 148200
caggtatcag gccagacagc ttcctatata gaccatctca ctgtctccta agcgctctgt 148260
aaggtggcat cacctgctat tgctattttc ctgctaagat gaccgaggtc ctggaaggta 148320
aatgcaagac ccccacttgt aaaatttgga gtcaggagta caatccagaa tctaatccta 148380
gttttctgct aagagtaaat ggacacccga ttaattctta gcagaaagtc cctttcttgc 148440
ccctgaccct actccaccat ttctaccctc ccctcaccat tcacatcccc ccacctccca 148500
agggcttgtt cccaagaaag ccctacttca cttttacagg aaaaaaagtg aatggtgaac 148560
ctgtcacatt cttattgcct tgtgacatct gcctaatcat ctgaatacca atggtttcaa 148620
gtaactccag aaagaagttc tcttaatgca taacactatt ttttcctccc aaccaagaga 148680
acttggttgg caagagttac ggtagcccct gtgaaggatg cttctggaag cgagcaagaa 148740
ctgattattt ctggccctaa aagccccact gtgttttata ttttacaatt ctggcatacc 148800
acagcttaaa ataaatgtat agtctcctat gtcaggggtc cccaaccctt gtgctgcagt 148860
atcagtcaat ggcctgttag gaaccaggcc acatagcagg aggggacggt gggtgagtga 148920
ccatcactgc ctaagctccg cctcctgtca gataagcagc attagatttt cacagaagca 148980
caaaccctat tgccaactcc acctgcaagg gatctaggtt gtacgttcct tatgagaatc 149040
taaccaatgc ctgatgatga ggtgggacag tttcatccca aaaccatcct ccccactcca 149100

ccctggaaaa tattgtcttc cacgaaacca gtctctggtg ccaagaggat ggggactgct 149160
gtcatatgtg actgcaacaa gttgcaattt gcaatgcctt tttttttttt tttttgagac 149220
cgagtctcac tctttgccca ggctggagtg cagtggtgtg atctcggctc aatgcaacct 149280
ctgcctctga ggctcaagcg attctcgtgc ctcagcctcc cgagtagctg ggattacagg 149340
tgcccacgac catgcccaga taatttttgt atttttagta gagatagggt ttagcagaga 149400
ccatgttggc caggctggtc ttgaactcct gacctcaggt gacccacctg ctgcggcctc 149460
ccaaagtgct gggattacag gcgtgagcca ctgcacctgg tctgcaatgt ctttagccag 149520
tcatttgcaa tatctattaa ttttagatac gcatgccctt taactcagca agcgcatttc 149580
tgggaattta tcctacagat aaattcatat aattagacaa aaagtccaag gatgaaagag 149640
tggcattatc tattattccc attttcctgc taagcctatc ggggtcttga aaggtaaatg 149700
caaggccctc ccttgcaaaa gttggggaac caggattaga atcccgatct gtttgaagcc 149760
taatttactc agagatggca ttagcgatgt ttgacacatc tgaatatgtc agccctattc 149820
acagtcctgt cctaacaact cactgcttca tcccttgagc acaaacaagc tattcattag 149880
cctgtggaag atccttaggt cttcgagtta caagtctcag ctctgcccaa aactcgctgg 149940
gtgatgctag gcaagtcact tgccctctct gggccttgta tttttcagcc tggtacagta 150000
aagttctatg cccctcacct tttttttgag acagagtctc actctgttgc ccaggctaga 150060
gaacagtgat gcaatcaggg cccacggcag ccttgaactc ctgggctcaa gcaatcctcc 150120
tgcctcactc aacctcctga ataactagga ctacagatgc atgccaccat gcccagctca 150180
ttttaaaaat ttttttgtag agatggtgcc tcgctatgtt gcccactctg gtctcaaact 150240
cctgacttca agccatcccc ccacctcagc ctcccaaatt gctgggatta caggtatgac 150300
ctgccatacc tggcctctat ggccctttcc tagcaacttt gatgaggttg ttcctcctta 150360
gcccctgaaa ccctgataca agtcacagat gtccgacctc ccctttcagc cactgtgagc 150420
agcttcttcc tctgtccttg gatcacatac aaggtttctt tcaaattctt gcctgccttt 150480
ctctaaattc agtctttcct ccattgtgtg gccagggtta aaagttgaaa ggtcaggagt 150540
ggaaggagaa gacctagaga gcaaaaaggc catcccttaa agtatgccat caagacccta 150600
aataagccag gcgcggtggc tcacgcctgt aatctgagca ctttgggaga ccaagccagg 150660
cgaatcactt gaggtcagcg gttcgagacc agcctggcca atatggtgaa accccatctc 150720
tactaaaaat acaaaaaatt agccaggcat ggtggtgggc gcctataaac ccagctactc 150780
tctactcagg aggctgaggc tggagaagtg cctgaaccta ggaggcggag gttgcagtga 150840
gccaagatcg caccactgca ctccagcctg ggcgacagag caagactctg tctcaaaaac 150900
aaacaaacaa aaccctaaac aaatgtaatt ggaatttgta acattcccaa tccaggtaag 150960

gaatggtagc ttaatatatt aaagattcta atcaccactt tcagcccaaa tagaaacgag 151020
gtttatgaga tcaaagggaa gaaaattaag agaagcttct cctttaacgg ccacatagtt 151080
taggcttccc ggttctgcca aagatgagcc atgccatact gagcaaacat cttgaactct 151140
ctggttgtgt ttgctataaa taaccatcac tgaagcttac caggggccag gcactgtgct 151200
aaacccttca tacacattag ctcatcaaat cctcccatga gggtaatctg tgaaactctg 151260
gagttagaaa gacctagatt tgtagaatct tcttttcctt actggttagc tgagctatct 151320
taagcaagtt attcagactc catatgtgta aaataaagcc agttagaata acagtatcta 151380
ctctaaaggg ttgttatgaa gacgtaagat aatgtccaca aagcgtttag catctcgcca 151440
ggcaaaagtg cccaaaaaac atgagttatt ctcattagcc ccatttcccg gatgagaaag 151500
tgaggtttgg ggaggtgcgg gagatgattt ttaaatttgc ttatggcctt ctatgattct 151560
actggattga atattgaact tggttttcaa gaaaaatcta ctatatatct gctcttggaa 151620
ttacacgctt gtgcctaacg tggaagaccc acctgtttgg attcaagacc tgtggaaagg 151680
agacaattcc ctcccacccc ccacccaaaa aaatcaaacc tctgaataag agggggtagg 151740
aaaaaagaat cccactttcc tcccaaatct cttctcctta gggtttctcc actagttctt 151800
caatgggtga gggacatggt agacaaatcc ccagggccag gaccaaccgg gctgcggtgc 151860
aaggctgggt aactgacgag ggggccgggg atggcctcat ggaaaacgct tccataaatg 151920
ggcatgtttg cctcaatctc tgccctccca cggaaaagta atgataacaa atccataaca 151980
gataccttgt acggaataac acatctattt cggacatgat aataaaaaaa catggtgttt 152040
acaaggcaag cctggcggct tctcggaaaa gacccagcta gttcacgttc tggactctcg 152100
gcatggcagc ccacctaggg tcactggttt cagctaggtc accccctcaa tcaggggaca 152160
aacagtgcct tttattttaa atgtgcaaaa agtcagttta tgttttcttc aaaggacacg 152220
gtgataaaca tttactttgt tggaagcatt tagtcccacc agaaacctga ccccaacaag 152280
tttgtgtcca tggtaaagaa gaaagatagt ggggtcaagg gtatgaactt ggactcaaac 152340
tgacccggga atgggttcta ccacttgctg gctgtgtgac actttacctc tctgagcctc 152400
agctgccttg tatactaaac aggcatcata aagcctttct tatgaagttg ttgcaaagat 152460
taaatgagat aaattaagtt aaatgttgca acagcatgtg gcactgaagt gttccatcaa 152520
cggtagctat ctcaaaggga aaacatcaat ttccttcaaa caaaattctc tatcttattt 152580
cacaccctgc cacctaatga ctttcagttc agcgtcaagt tccgtctctg aacaattcta 152640
aaatagtatc gttgagtaaa tcaaagaaac aaaaaactat gcaaagcagt ttattaatag 152700
cctgcattct ttttgccaaa ccccggacgg acagttcacc atttaatctc tcagttcttc 152760

atactgcaac taaacactcc caccttgggt ttctaaatat tttcttttac ttcttgacaa 152820

gcatggcttt ggatggtacc ttttctcaag tggcaaaatc cctaccagac cctgtgctgg 152880

aagaaaactt acaacaggca gtagccacac tttctctcgt tttacccatt tggttgggca 152940

cgagagtggc attgacacag gtggctctgc gggaaattct agtgtaatct tacctaacgc 153000

acctgcagat cgtggaagca aatggagact ttggagccca cctgggtatc tgggggccct 153060

ttggggtcaa cctgggtgag ttgttttttt tttttttaat ttggagaaga ggaaaaagtg 153120

ggctgtaaca aactagatca caaccagcct tatctcccag gggttctcaa catagaagaa 153180

cctcccctaa actgattaaa aaaaaaaaac aacaacactc ctttctgcta agcctatgcc 153240

catgtgtttt taactgattt aatgcattat caacacaact gtgcaatacg gtcaaatcca 153300

caaggagcct cactttgccc ttctgtgtga tagcaagtga cttttcagtg aagttcagct 153360

gtaaattttt tggtgcctag aataaaagtg agatgccttg attgtctgca ccggacctct 153420

gagatttcca agggagcata tctcctggat tagcctctta acaagaatca ctcgggacta 153480

attttttttt ttttaaggca gtttttctgg ggattctaga gttgggaaat ggagttgggt 153540

ccaggcattg atatcgttaa taagcacccc caaaatggta cttctcactg gcaatcttaa 153600

gaaatgctgt ttggggaaag tctaggataa tctaagacga taaggcagag agagatccaa 153660

atcacaactc tgcccccacc atggccctta cgaggttgac aacagggacc cctaagtgca 153720

ccccagcccc tatagaatac cctataggac ttttaacacc cccctgccct gcaatctgcc 153780

cccagaatga caactactac ttacagatct ccaactgggg ggactagtgg agaggaaact 153840

ggatggcgag ctcggatctg acgcacctcc caggccggag cgtccaaatc tc 153892

<210> 2
<211> 6861
<212> DNA
<213> Homo sapiens

<220>
<221> misc_feature
<222> (1)..(6861)
<223> myosin heavy polypeptide 11, smooth muscle (MYH11), transcript va
riant SM1 mRNA

<220>
<221> misc_feature
<222> (4454)..(4666)
<223> exon 32

<220>
<221> variation
<222> (505)..(505)
<223> T replace C

<220>
<221>  variation
<222>  (1075)..(1075)
<223>  G replace A


<220>
<221>  variation
<222>  (2560)..(2560)
<223>  T replace C


<220>
<221>  variation
<222>  (2678)..(2678)
<223>  G replace A


<220>
<221>  variation
<222>  (4016)..(4016)
<223>  T replace C


<220>
<221>  variation
<222>  (4055)..(4055)
<223>  T replace C


<220>
<221>  variation
<222>  (4489)..(4489)
<223>  G replace A


<220>
<221>  variation
<222>  (5527)..(5527)
<223>  G replace A


<220>
<221>  variation
<222>  (5566)..(5566)
<223>  G replace A


<220>
<221>  misc_feature
<222>  (5874)..(5875)
<223>  missing exon 41


<220>
<221>  variation
<222>  (6012)..(6012)
<223>  G replace C

<220>
<221>  variation
<222>  (6064)..(6065)
<223>  replace T
       replace -

<220>
<221>  variation
<222>  (6750)..(6750)
<223>  Treplace C

<400>  2
gcctgggagg tgcgtcagat ccgagctcgc catccagttt cctctccact agtcccccca      60

gttggagatc tgggaccaac aaggcaccat ggcgcagaag ggccaactca gtgacgatga     120

gaagttcctc tttgtggaca aaaacttcat caacagccca gtggcccagg ctgactgggc     180

cgccaagaga ctcgtctggg tcccctcgga gaagcagggc ttcgaggcag ccagcattaa     240

ggaggagaag ggggatgagg tggttgtgga gctggtggag aatggcaaga aggtcacggt     300

tgggaaagat gacatccaga agatgaaccc acccaagttc tccaaggtgg aggacatggc     360

ggagctgacg tgcctcaacg aagcctccgt gctacacaac ctgagggagc ggtacttctc     420

agggctaata tatacgtact ctggcctctt ctgcgtggtg gtcaacccct ataaacacct     480

gcccatctac tcggagaaga tcgtcgacat gtacaagggc aagaagaggc acgagatgcc     540

gcctcacatc tacgccatcg cagacacggc ctaccggagc atgcttcaag atcgggagga     600

ccagtccatt ctatgcacag gcgagtctgg agccgggaaa accgaaaaca ccaagaaggt     660

cattcagtac ctggccgtgg tggcctcctc ccacaagggc aagaaagaca caagtatcac     720

gggagagctg gaaaagcagc ttctacaagc aaacccgatt ctggaggctt tcggcaacgc     780

caaaacagtg aagaacgaca actcctcacg attcggcaaa ttcatccgca tcaacttcga     840

cgtcacgggt tacatcgtgg gagccaacat tgagacctat ctgctagaaa aatcacgggc     900

aattcgccaa gccagagacg agaggacatt ccacatcttt tactacatga ttgctggagc     960

caaggagaag atgagaagtg acttgctttt ggagggcttc aacaactaca ccttcctctc    1020

caatggcttt gtgcccatcc cagcagccca ggatgatgag atgttccagg aaaccgtgga    1080

ggccatggca atcatgggtt tcagcgagga ggagcagcta tccatattga aggtggtatc    1140

atcggtcctg cagcttggaa atatcgtctt caagaaggaa agaaacacag accaggcgtc    1200

catgccagat aacacagctg ctcagaaagt ttgccacctc atgggaatta atgtgacaga    1260

tttcaccaga tccatcctca ctcctcgtat caaggttggg cgagatgtgg tacagaaagc    1320

tcagacaaaa gaacaggctg actttgctgt agaggctttg gccaaggcaa catatgagcg    1380

ccttttccgc tggatactca cccgcgtgaa caaagccctg gacaagaccc atcggcaagg 1440
ggcttccttc ctggggatcc tggatatagc tggatttgag atctttgagg tgaactcctt 1500
cgagcagctg tgcatcaact acaccaacga gaagctgcag cagctcttca accacaccat 1560
gttcatcctg gagcaggagg agtaccagcg cgagggcatc gagtggaact tcatcgactt 1620
tgggctggac ctacagccct gcatcgagct catcgagcga ccgaacaacc ctccaggtgt 1680
gctggccctg ctggacgagg aatgctggtt ccccaaagcc acggacaagt ctttcgtgga 1740
gaagctgtgc acggagcagg gcagccaccc caagttccag aagcccaagc agctcaagga 1800
caagactgag ttctccatca tccattatgc tgggaaggtg gactataatg cgagtgcctg 1860
gctgaccaag aatatggacc cgctgaatga caacgtgact tccctgctca atgcctcctc 1920
cgacaagttt gtggccgacc tgtggaagga cgtggaccgc atcgtgggcc tggaccagat 1980
ggccaagatg acggagagct cgctgcccag cgcctccaag accaagaagg gcatgttccg 2040
cacagtgggg cagctgtaca aggagcagct gggcaagctg atgaccacgc tacgcaacac 2100
cacgcccaac ttcgtgcgct gcatcatccc caaccacgag aagaggtccg gcaagctgga 2160
tgcgttcctg gtgctggagc agctgcggtg caatggggtg ctggaaggca ttcgcatctg 2220
ccggcagggc ttccccaacc ggatcgtctt ccaggagttc cgccaacgct acgagatcct 2280
ggcggcgaat gccatcccca aaggcttcat ggacgggaag caggcctgca ttctcatgat 2340
caaagccctg gaacttgacc ccaacttata caggataggg cagagcaaaa tcttcttccg 2400
aactggcgtc ctggcccacc tagaggagga gcgagatttg aagatcaccg atgtcatcat 2460
ggccttccag gcgatgtgtc gtggctactt ggccagaaag gcttttgcca agaggcagca 2520
gcagctgacc gccatgaagg tgattcagag gaactgcgcc gcctacctca agctgcggaa 2580
ctggcagtgg tggaggcttt tcaccaaagt gaagccactg ctgcaggtga cacggcagga 2640
ggaggagatg caggccaagg aggatgaact gcagaagacc aaggagcggc agcagaaggc 2700
agagaatgag cttaaggagc tggaacagaa gcactcgcag ctgaccgagg agaagaacct 2760
gctacaggaa cagctgcagg cagagacaga gctgtatgca gaggctgagg agatgcgggt 2820
gcggctggcg gccaagaagc aggagctgga ggagatactg catgagatgg aggcccgcct 2880
ggaggaggag gaagacaggg gccagcagct acaggctgaa aggaagaaga tggcccagca 2940
gatgctggac cttgaagaac agctggagga ggaggaagct gccaggcaga agctgcaact 3000
tgagaaggtc acggctgagg ccaagatcaa gaaactggag gatgagatcc tggtcatgga 3060
tgatcagaac aataaactat caaaagaacg aaaactcctt gaggagagga ttagtgactt 3120
aacgacaaat cttgcagaag aggaagaaaa ggccaagaat cttaccaagc tgaaaaacaa 3180
gcatgaatct atgatttcag aactggaagt gcggctaaag aaggaagaga agagccgaca 3240

ggagctggag aagctgaaac ggaagctgga gggtgatgcc agcgacttcc acgagcagat 3300
cgctgacctc caggcgcaga tcgcagagct caagatgcag ctggccaaga aggaggagga 3360
gctgcaggcg gccctggcca ggcttgacga tgaaatcgct cagaagaaca atgccctgaa 3420
gaagatccgg gagctggagg gccacatctc agacctccag gaggacctgg actcagagcg 3480
ggccgccagg aacaaggctg aaaagcagaa gcgagacctc ggcgaggagc tggaggccct 3540
aaagacagag ctggaagaca cactggacag cacagccact cagcaggagc tcagggccaa 3600
gagggagcag gaggtgacgg tgctgaagaa ggccctggat gaagagacgc ggtcccatga 3660
ggctcaggtc caggagatga ggcagaaaca cgcacaggcg gtggaggagc tcacagagca 3720
gcttgagcag ttcaagaggg ccaaggcgaa cctagacaag aataagcaga cgctggagaa 3780
agagaacgca gacctggccg gggagctgcg ggtcctgggc caggccaagc aggaggtgga 3840
acataagaag aagaagctgg aggcgcaggt gcaggagctg cagtccaagt gcagcgatgg 3900
ggagcgggcc cgggcggagc tcaatgacaa agtccacaag ctgcagaatg aagttgagag 3960
cgtcacaggg atgcttaacg aggccgaggg gaaggccatt aagctggcca aggacgtggc 4020
gtccctcagt tcccagctcc aggacaccca ggagctgctt caagaagaaa cccggcagaa 4080
gctcaacgtg tctacgaagc tgcgccagct ggaggaggag cggaacagcc tgcaagacca 4140
gctggacgag gagatggagg ccaagcagaa cctggagcgc cacatctcca ctctcaacat 4200
ccagctctcc gactcgaaga agaagctgca ggactttgcc agcaccgtgg aagctctgga 4260
agaggggaag aagaggttcc agaaggagat cgagaacctc acccagcagt acgaggagaa 4320
ggcggccgct tatgataaac tggaaaagac caagaacagg cttcagcagg agctggacga 4380
cctggttgtt gatttggaca accagcggca actcgtgtcc aacctggaaa agaagcagag 4440
gaaatttgat cagttgttag ccgaggagaa aaacatctct tccaaatacg cggatgagag 4500
ggacagagct gaggcagaag ccagggagaa ggaaaccaag gccctgtccc tggctcgggc 4560
ccttgaagag gccttggaag ccaaagagga actcgagcgg accaacaaaa tgctcaaagc 4620
cgaaatggaa gacctggtca gctccaagga tgacgtgggc aagaacgtcc atgagctgga 4680
gaagtccaag cgggccctgg agacccagat ggaggagatg aagacgcagc tggaagagct 4740
ggaggacgag ctgcaagcca cggaggacgc caaactgcgg ctggaagtca acatgcaggc 4800
gctcaagggc cagttcgaaa gggatctcca agcccgggac gagcagaatg aggagaagag 4860
gaggcaactg cagagacagc ttcacgagta tgagacggaa ctggaagacg agcgaaagca 4920
acgtgccctg gcagctgcag caaagaagaa gctggaaggg gacctgaaag acctggagct 4980
tcaggccgac tctgccatca aggggaggga ggaagccatc aagcagctac gcaaactgca 5040

ggctcagatg aaggactttc aaagagagct ggaagatgcc cgtgcctcca gagatgagat 5100
ctttgccaca gccaaagaga atgagaagaa agccaagagc ttggaagcag acctcatgca 5160
gctacaagag gacctcgccg ccgctgagag ggctcgcaaa caagcggacc tcgagaagga 5220
ggaactggca gaggagctgg ccagtagcct gtcgggaagg aacgcactcc aggacgagaa 5280
gcgccgcctg gaggcccgga tcgcccagct ggaggaggag ctggaggagg agcagggcaa 5340
catggaggcc atgagcgacc gggtccgcaa agccacacag caggccgagc agctcagcaa 5400
cgagctggcc acagagcgca gcacggccca gaagaatgag agtgcccggc agcagctcga 5460
gcggcagaac aaggagctcc ggagcaagct ccacgagatg gagggggccg tcaagtccaa 5520
gttcaagtcc accatcgcgg cgctggaggc caagattgca cagctggagg agcaggtcga 5580
gcaggaggcc agagagaaac aggcggccac caagtcgctg aagcagaaag acaagaagct 5640
gaaggaaatc ttgctgcagg tggaggacga gcgcaagatg gccgagcagt acaaggagca 5700
ggcagagaaa ggcaatgcca gggtcaagca gctcaagagg cagctggagg aggcagagga 5760
ggagtcccag cgcatcaacg ccaaccgcag gaagctgcag cgggagctgg atgaggccac 5820
ggagagcaac gaggccatgg gccgcgaggt gaacgcactc aagagcaagc tcaggcgagg 5880
aaacgagacc tctttcgttc cttctagaag gtctggagga cgtagagtta ttgaaaatgc 5940
agatggttct gaggaggaaa cggacactcg agacgcagac ttcaatggaa ccaaggccag 6000
tgaataagca actttctaca gttttgcacc acggcaagaa aaccaaaaac caaaacaaac 6060
aaacaaaaaa aacccaacaa caacccagaa caaagcaaaa cccagcagac tgtacttagc 6120
attgtctaaa tccattctca aattccaaat atcacagaca cccctcacac aaggaatata 6180
aaaaccacca ccctccagcc tgggcaacgt agtaaaacct catctataca agaatttaaa 6240
aataagctgg gcgtggtggt acacacctgt ggtcccagct actagggagg ctgagccagg 6300
aagaacgctc cagcccagga cttcgaggct gcaatgagct ataattgcat cattgcactc 6360
cagcctgggc aacagagacc ctgtctcaac caccaccacc accaccaccc ctactacccc 6420
tgtattcaag gtaaaaattg aagtttgtat gatgtaagag atgagaaaaa cccaacagga 6480
aacacagaca catcctccag ttctatcaat ggattgtgca gacactgagt ttttagaaaa 6540
acatatccac ggtaaccggt ccctggcaat tctgtttaca tgaaatgggg agaaagtcac 6600
cgaaatgggt gccgccggcc cccactccca attcattccc taacctgcaa acctttccaa 6660
cttctcacgt caggcctttg agaattcttt ccccctctcc tggtttccac acctcagaca 6720
cgcacagttc accaagtgcc ttctgtagtc acatgaattg aaaaggagac gctgctccca 6780
cggaggggag caggaatgct gcactgttta caccctgact gtgcttaaaa acactttcac 6840
taataaatgg ttataaatca c 6861

<210>  3
<211>  1954
<212>  PRT
<213>  Homo sapiens

<220>
<221>  MISC_FEATURE
<223>  MYH11 SM1 isoform

<400>  3

Met Ala Gln Lys Gly Gln Leu Ser Asp Asp Glu Lys Phe Leu Phe Val
1               5                   10                  15

Asp Lys Asn Phe Ile Asn Ser Pro Val Ala Gln Ala Asp Trp Ala Ala
            20                  25                  30

Lys Arg Leu Val Trp Val Pro Ser Glu Lys Gln Gly Phe Glu Ala Ala
        35                  40                  45

Ser Ile Lys Glu Glu Lys Gly Asp Glu Val Val Val Glu Leu Val Glu
    50                  55                  60

Asn Gly Lys Lys Val Thr Val Gly Lys Asp Asp Ile Gln Lys Met Asn
65                  70                  75                  80

Pro Pro Lys Phe Ser Lys Val Glu Asp Met Ala Glu Leu Thr Cys Leu
                85                  90                  95

Asn Glu Ala Ser Val Leu His Asn Leu Arg Glu Arg Tyr Phe Ser Gly
            100                 105                 110

Leu Ile Tyr Thr Tyr Ser Gly Leu Phe Cys Val Val Val Asn Pro Tyr
        115                 120                 125

Lys His Leu Pro Ile Tyr Ser Glu Lys Ile Val Asp Met Tyr Lys Gly
    130                 135                 140

Lys Lys Arg His Glu Met Pro Pro His Ile Tyr Ala Ile Ala Asp Thr
145                 150                 155                 160

Ala Tyr Arg Ser Met Leu Gln Asp Arg Glu Asp Gln Ser Ile Leu Cys
                165                 170                 175

Thr Gly Glu Ser Gly Ala Gly Lys Thr Glu Asn Thr Lys Lys Val Ile
            180                 185                 190

Gln Tyr Leu Ala Val Val Ala Ser Ser His Lys Gly Lys Lys Asp Thr
        195                 200                 205

Ser Ile Thr Gly Glu Leu Glu Lys Gln Leu Leu Gln Ala Asn Pro Ile
    210                 215                 220

Ala Phe Gly Asn Ala Lys Thr Val Lys Asn Asp Asn Ser Ser Arg Phe
225                 230                 235                 240

Gly Lys Phe Ile Arg Ile Asn Phe Asp Val Thr Gly Tyr Ile Val Gly
                245                 250                 255

Ala Asn Ile Glu Thr Tyr Leu Leu Glu Lys Ser Arg Ala Ile Arg Gln
            260                 265                 270

Ala Arg Asp Glu Arg Thr Phe His Ile Phe Tyr Tyr Met Ile Ala Gly
        275                 280                 285

Ala Lys Glu Lys Met Arg Ser Asp Leu Leu Leu Glu Gly Phe Asn Asn
    290                 295                 300

Tyr Thr Phe Leu Ser Asn Gly Phe Val Pro Ile Pro Ala Ala Gln Asp
305                 310                 315                 320

Asp Glu Met Phe Gln Glu Thr Val Glu Ala Met Ala Ile Met Gly Phe
                325                 330                 335

Ser Glu Glu Glu Gln Leu Ser Ile Leu Lys Val Val Ser Ser Val Leu
            340                 345                 350

Gln Leu Gly Asn Ile Val Phe Lys Lys Glu Arg Asn Thr Asp Gln Ala
        355                 360                 365

Ser Met Pro Asp Asn Thr Ala Ala Gln Lys Val Cys His Leu Met Gly
    370                 375                 380

Ile Asn Val Thr Asp Phe Thr Arg Ser Ile Leu Thr Pro Arg Ile Lys
385                 390                 395                 400

Val Gly Arg Asp Val Val Gln Lys Ala Gln Thr Lys Glu Gln Ala Asp
                405                 410                 415

Phe Ala Val Glu Ala Lys Ala Thr Tyr Glu Arg Leu Phe Arg Trp Ile
            420                 425                 430

Leu Thr Arg Val Asn Lys Ala Leu Asp Lys Thr Gln Gly Ala Ser Phe
        435                 440                 445

Leu Gly Ile Leu Asp Ile Ala Gly Phe Glu Ile Phe Glu Val Asn Ser
    450                 455                 460

Phe Glu Gln Leu Cys Ile Asn Tyr Thr Asn Glu Lys Leu Gln Gln Leu
465                 470                 475                 480

Phe Asn His Thr Met Phe Ile Gln Glu Glu Tyr Gln Arg Glu Gly Ile
                485                 490                 495

Glu Trp Asn Phe Ile Asp Phe Gly Leu Asp Leu Gln Pro Cys Ile Glu
            500                 505                 510

Leu Ile Glu Arg Pro Asn Asn Pro Pro Gly Val Leu Ala Leu Leu Asp
        515                 520                 525

Glu Glu Cys Trp Phe Pro Lys Ala Thr Asp Lys Ser Phe Val Glu Lys
    530                 535                 540

Leu Cys Thr Glu Gln Gly Ser His Pro Lys Phe Gln Lys Pro Lys Gln
545                 550                 555                 560

Leu Lys Asp Lys Thr Glu Phe Ser Ile Ile His Tyr Ala Gly Lys Val
                565                 570                 575

Asp Tyr Asn Ala Ser Ala Trp Leu Thr Lys Asn Met Asp Pro Leu Asn
            580                 585                 590

Asp Asn Val Thr Ser Leu Leu Asn Ala Ser Ser Asp Lys Phe Val Ala
        595                 600                 605

Asp Leu Trp Lys Asp Val Asp Arg Ile Val Gly Leu Asp Gln Met Ala
    610                 615                 620

Lys Met Thr Glu Ser Ser Leu Pro Ser Ala Ser Lys Thr Lys Lys Gly
625                 630                 635                 640

Met Phe Arg Thr Val Gly Gln Leu Tyr Lys Glu Gln Leu Gly Lys Leu
                645                 650                 655

Met Thr Thr Leu Arg Asn Thr Thr Pro Asn Phe Val Arg Cys Ile Ile
            660                 665                 670

Pro Asn His Glu Lys Arg Ser Gly Lys Leu Asp Ala Phe Leu Val Leu

        675                 680                 685

Glu Gln Leu Arg Cys Asn Gly Val Leu Glu Gly Ile Arg Ile Cys Arg
    690                 695                 700

Gln Gly Phe Pro Asn Arg Ile Val Phe Gln Glu Phe Arg Gln Arg Tyr
705                 710                 715                 720

Glu Ile Leu Ala Ala Asn Ala Ile Pro Lys Gly Phe Met Asp Gly Lys
                725                 730                 735

Gln Ala Cys Ile Leu Met Ile Lys Ala Leu Glu Leu Asp Pro Asn Leu
            740                 745                 750

Tyr Arg Ile Gly Gln Ser Lys Ile Phe Phe Arg Thr Gly Val Leu Ala
        755                 760                 765

His Leu Glu Glu Glu Arg Asp Leu Lys Ile Thr Asp Val Ile Met Ala
    770                 775                 780

Phe Gln Ala Met Cys Arg Gly Tyr Leu Ala Arg Lys Ala Phe Ala Lys
785                 790                 795                 800

Arg Gln Gln Gln Leu Thr Ala Met Lys Val Ile Gln Arg Asn Cys Ala
                805                 810                 815

Ala Tyr Leu Lys Leu Arg Asn Trp Gln Trp Trp Arg Leu Phe Thr Lys
            820                 825                 830

Val Lys Pro Leu Leu Gln Val Thr Arg Gln Glu Glu Glu Met Gln Ala
        835                 840                 845

Lys Glu Asp Glu Leu Gln Lys Thr Lys Glu Arg Gln Gln Lys Ala Glu
    850                 855                 860

Asn Glu Leu Lys Glu Leu Glu Gln Lys His Ser Gln Leu Thr Glu Glu
865                 870                 875                 880

Lys Asn Leu Leu Gln Glu Gln Leu Gln Ala Glu Thr Glu Leu Tyr Ala
                885                 890                 895

Glu Ala Glu Glu Met Arg Val Arg Leu Ala Ala Lys Lys Gln Glu Leu
            900                 905                 910

Glu Glu Ile Leu His Glu Met Glu Ala Arg Leu Glu Glu Glu Glu Asp
        915                 920                 925

Arg Gly Gln Gln Leu Gln Ala Glu Arg Lys Lys Met Ala Gln Gln Met
    930                 935                 940

Leu Asp Leu Glu Glu Gln Leu Glu Glu Glu Glu Ala Ala Arg Gln Lys
945                 950                 955                 960

Leu Gln Leu Glu Lys Val Thr Ala Glu Ala Lys Ile Lys Lys Leu Glu
                965                 970                 975

Asp Glu Ile Leu Val Met Asp Asp Gln Asn Asn Lys Leu Ser Lys Glu
            980                 985                 990

Arg Lys Leu Leu Glu Glu Arg Ile  Ser Asp Leu Thr Thr  Asn Leu Ala
        995                 1000                1005

Glu Glu  Glu Glu Lys Ala Lys  Asn Leu Thr Lys Leu  Lys Asn Lys
    1010                 1015                 1020

His Glu  Ser Met Ile Ser Glu  Leu Glu Val Arg Leu  Lys Lys Glu
    1025                 1030                 1035

Glu Lys  Ser Arg Gln Glu Leu  Glu Lys Leu Lys Arg  Lys Leu Glu
    1040                 1045                 1050

Gly Asp  Ala Ser Asp Phe His  Glu Gln Ile Ala Asp  Leu Gln Ala
    1055                 1060                 1065

Gln Ile  Ala Glu Leu Lys Met  Gln Leu Ala Lys Lys  Glu Glu Glu
    1070                 1075                 1080

Leu Gln  Ala Ala Arg Leu Asp  Asp Glu Ile Ala Gln  Lys Asn Asn
    1085                 1090                 1095

Ala Leu  Lys Lys Ile Arg Glu  Leu Glu Gly His Asp  Leu Gln Glu
    1100                 1105                 1110

Asp Leu  Asp Ser Ala Ala Arg  Asn Lys Ala Glu Lys  Gln Lys Arg
    1115                 1120                 1125

Asp Leu  Gly Glu Glu Leu Glu  Ala Leu Lys Thr Glu  Leu Glu Asp
    1130                 1135                 1140

Thr Leu  Asp Ser Thr Ala Thr  Gln Gln Glu Leu Arg  Ala Lys Arg
    1145                 1150                 1155

Glu Gln Glu Val Thr Val Leu Lys Lys Ala Leu Asp Glu Glu Thr
    1160                1165                1170

Arg Ser His Glu Ala Gln Val Gln Glu Met Arg Gln Lys His Ala
    1175                1180                1185

Gln Ala Val Glu Glu Leu Thr Glu Gln Leu Glu Gln Phe Lys Arg
    1190                1195                1200

Ala Lys Ala Asn Leu Asp Lys Asn Lys Gln Thr Leu Glu Lys Glu
    1205                1210                1215

Asn Ala Asp Leu Ala Gly Glu Leu Arg Val Leu Gly Gln Ala Lys
    1220                1225                1230

Gln Glu Val Glu His Lys Lys Lys Lys Leu Glu Ala Gln Val Gln
    1235                1240                1245

Glu Leu Gln Ser Lys Cys Ser Asp Gly Glu Arg Ala Arg Ala Glu
    1250                1255                1260

Leu Asn Asp Lys Val His Lys Leu Gln Asn Glu Val Glu Ser Val
    1265                1270                1275

Thr Gly Met Leu Asn Glu Ala Glu Gly Lys Ala Ile Lys Leu Ala
    1280                1285                1290

Lys Asp Val Ala Ser Leu Ser Ser Gln Leu Gln Asp Thr Gln Glu
    1295                1300                1305

Leu Leu Gln Glu Glu Thr Arg Gln Lys Leu Asn Val Ser Thr Lys
    1310                1315                1320

Leu Arg Gln Leu Glu Glu Glu Arg Asn Ser Leu Gln Asp Gln Leu
    1325                1330                1335

Asp Glu Glu Met Glu Ala Lys Gln Asn Leu Glu Arg His Thr Leu
    1340                1345                1350

Asn Ile Gln Leu Ser Asp Ser Lys Lys Lys Leu Gln Asp Phe Ala
    1355                1360                1365

Ser Thr Val Glu Ala Leu Glu Glu Gly Lys Lys Arg Phe Gln Lys
    1370                1375                1380

Glu Ile Glu Asn Leu Thr Gln Gln Tyr Glu Glu Lys Ala Ala Ala
    1385                1390                1395

Tyr Asp Lys Leu Glu Lys Thr Lys Asn Arg Leu Gln Gln Glu Leu
    1400                1405                1410

Asp Asp Leu Val Val Asp Leu Asp Asn Gln Arg Gln Leu Val Ser
    1415                1420                1425

Asn Leu Glu Lys Lys Gln Arg Lys Phe Asp Gln Leu Leu Ala Glu
    1430                1435                1440

Glu Lys Asn Ile Ser Ser Lys Tyr Ala Asp Glu Arg Asp Arg Ala
    1445                1450                1455

Glu Ala Glu Ala Arg Glu Lys Glu Thr Lys Ala Leu Ser Leu Ala
    1460                1465                1470

Arg Ala Leu Glu Glu Ala Leu Glu Ala Lys Glu Glu Leu Glu Arg
    1475                1480                1485

Thr Asn Lys Met Leu Lys Ala Glu Met Glu Asp Leu Val Ser Ser
    1490                1495                1500

Lys Asp Asp Val Gly Lys Asn Val His Glu Leu Glu Lys Ser Lys
    1505                1510                1515

Arg Ala Leu Glu Thr Gln Met Glu Glu Met Lys Thr Gln Leu Glu
    1520                1525                1530

Glu Leu Glu Asp Glu Leu Gln Ala Thr Glu Asp Ala Lys Leu Arg
    1535                1540                1545

Leu Glu Val Asn Met Gln Ala Leu Lys Gly Gln Phe Glu Arg Asp
    1550                1555                1560

Leu Gln Ala Arg Asp Glu Gln Asn Glu Glu Lys Arg Arg Gln Leu
    1565                1570                1575

Gln Arg Gln Leu His Glu Tyr Glu Thr Glu Leu Glu Asp Glu Arg
    1580                1585                1590

Lys Gln Arg Ala Ala Ala Ala Lys Lys Lys Leu Glu Gly Asp Leu
    1595                1600                1605

Lys Asp Leu Glu Leu Gln Ala Asp Ser Ala Ile Lys Gly Arg Glu

1610                 1615                 1620

Glu Ala Ile Lys Gln Leu Arg Lys Leu Gln Ala Gln Met Lys Asp
    1625                 1630                 1635

Phe Gln Arg Glu Leu Glu Asp Ala Arg Ala Ser Arg Asp Glu Ile
    1640                 1645                 1650

Phe Ala Thr Ala Lys Glu Asn Glu Lys Lys Ala Lys Ser Leu Glu
    1655                 1660                 1665

Ala Asp Leu Met Gln Leu Gln Glu Asp Leu Ala Ala Ala Glu Arg
    1670                 1675                 1680

Ala Arg Lys Gln Ala Asp Leu Glu Lys Glu Glu Leu Ala Glu Glu
    1685                 1690                 1695

Leu Ala Ser Ser Leu Ser Gly Arg Asn Ala Leu Gln Asp Glu Lys
    1700                 1705                 1710

Arg Arg Leu Glu Ala Arg Ile Ala Gln Leu Glu Glu Glu Leu Glu
    1715                 1720                 1725

Glu Glu Gln Gly Asn Met Glu Ala Met Ser Asp Arg Val Arg Lys
    1730                 1735                 1740

Ala Thr Gln Gln Ala Glu Gln Leu Ser Asn Glu Leu Ala Thr Glu
    1745                 1750                 1755

Arg Ser Thr Ala Gln Lys Asn Glu Ser Ala Arg Gln Gln Leu Glu
    1760                 1765                 1770

Arg Gln Asn Lys Glu Leu Arg Ser Lys Leu His Glu Met Glu Gly
    1775                 1780                 1785

Ala Val Lys Ser Lys Phe Lys Ser Thr Ile Ala Ala Leu Glu Ala
    1790                 1795                 1800

Lys Ile Ala Gln Leu Glu Glu Gln Val Glu Gln Glu Ala Arg Glu
    1805                 1810                 1815

Lys Gln Ala Ala Thr Lys Ser Leu Lys Gln Lys Asp Lys Lys Leu
    1820                 1825                 1830

Lys Glu Ile Leu Leu Gln Val Glu Asp Glu Arg Lys Met Ala Glu
    1835                 1840                 1845

Gln Tyr Lys Glu Gln Ala Glu Lys Gly Asn Ala Arg Val Lys Gln
    1850                1855                1860

Leu Lys Arg Gln Leu Glu Glu Ala Glu Glu Glu Ser Gln Arg Ile
    1865                1870                1875

Asn Ala Asn Arg Arg Lys Leu Gln Arg Glu Leu Asp Glu Ala Thr
    1880                1885                1890

Glu Ser Asn Glu Ala Met Gly Arg Glu Val Asn Ala Leu Lys Ser
    1895                1900                1905

Lys Leu Arg Arg Gly Asn Glu Thr Ser Phe Val Pro Ser Arg Arg
    1910                1915                1920

Ser Gly Gly Arg Arg Val Ile Glu Asn Ala Asp Gly Ser Glu Glu
    1925                1930                1935

Glu Thr Asp Thr Arg Asp Ala Asp Phe Asn Gly Thr Lys Ala Ser
    1940                1945                1950

Glu

<210>  4
<211>  6900
<212>  DNA
<213>  Homo sapiens

<220>
<221>  misc_feature
<222>  (1)..(6900)
<223>  myosin heavy polypeptide 11, smooth muscle (MYH11), transcript va
       riant SM2 mRNA.

<220>
<221>  misc_feature
<222>  (4454)..(4666)
<223>  exon 32

<220>
<221>  variation
<222>  (505)..(505)
<223>  T replace C

<220>
<221>  variation
<222>  (1075)..(1075)

<223>  G replace A


<220>
<221>  variation
<222>  (1831)..(1831)
<223>  T replace C


<220>
<221>  variation
<222>  (2149)..(2149)
<223>  G replace A


<220>
<221>  variation
<222>  (2560)..(2560)
<223>  T replace C


<220>
<221>  variation
<222>  (2678)..(2678)
<223>  G replace A


<220>
<221>  variation
<222>  (4016)..(4016)
<223>  T replace C


<220>
<221>  variation
<222>  (4055)..(4055)
<223>  T replace C


<220>
<221>  variation
<222>  (4330)..(4330)
<223>  T replace G


<220>
<221>  variation
<222>  (4489)..(4489)
<223>  G replace A


<220>
<221>  variation
<222>  (5527)..(5527)
<223>  G replace A


<220>
<221>  variation
<222>  (5566)..(5566)
<223>  G replace A

<220>
<221>  misc_feature
<222>  (5875)..(5913)
<223>  exon 41


<220>
<221>  variation
<222>  (6051)..(6051)
<223>  G replace C


<220>
<221>  variation
<222>  (6103)..(6104)
<223>  replace T
       replace -


<220>
<221>  variation
<222>  (6789)..(6789)
<223>  T replace C


<400>  4
   gcctgggagg tgcgtcagat ccgagctcgc catccagttt cctctccact agtcccccca      60
   gttggagatc tgggaccaac aaggcaccat ggcgcagaag ggccaactca gtgacgatga     120
   gaagttcctc tttgtggaca aaaacttcat caacagccca gtggcccagg ctgactgggc     180
   cgccaagaga ctcgtctggg tcccctcgga gaagcagggc ttcgaggcag ccagcattaa     240
   ggaggagaag ggggatgagg tggttgtgga gctggtggag aatggcaaga aggtcacggt     300
   tgggaaagat gacatccaga agatgaaccc acccaagttc tccaaggtgg aggacatggc     360
   ggagctgacg tgcctcaacg aagcctccgt gctacacaac ctgagggagc ggtacttctc     420
   agggctaata tatacgtact ctggcctctt ctgcgtggtg gtcaacccct ataaacacct     480
   gcccatctac tcggagaaga tcgtcgacat gtacaagggc aagaagaggc acgagatgcc     540
   gcctcacatc tacgccatcg cagacacggc ctaccggagc atgcttcaag atcgggagga     600
   ccagtccatt ctatgcacag gcgagtctgg agccgggaaa accgaaaaca ccaagaaggt     660
   cattcagtac ctggccgtgg tggcctcctc ccacaagggc aagaaagaca caagtatcac     720
   gggagagctg gaaaagcagc ttctacaagc aaacccgatt ctggaggctt tcggcaacgc     780
   caaaacagtg aagaacgaca actcctcacg attcggcaaa ttcatccgca tcaacttcga     840
   cgtcacgggt tacatcgtgg gagccaacat tgagacctat ctgctagaaa aatcacgggc     900
   aattcgccaa gccagagacg agaggacatt ccacatcttt tactacatga ttgctggagc     960
   caaggagaag atgagaagtg acttgctttt ggagggcttc aacaactaca ccttcctctc    1020
   caatggcttt gtgcccatcc cagcagccca ggatgatgag atgttccagg aaaccgtgga 1080
   ggccatggca atcatgggtt tcagcgagga ggagcagcta tccatattga aggtggtatc 1140
   atcggtcctg cagcttggaa atatcgtctt caagaaggaa agaaacacag accaggcgtc 1200
   catgccagat aacacagctg ctcagaaagt ttgccacctc atgggaatta atgtgacaga 1260
   tttcaccaga tccatcctca ctcctcgtat caaggttggg cgagatgtgg tacagaaagc 1320
   tcagacaaaa gaacaggctg actttgctgt agaggctttg gccaaggcaa catatgagcg 1380
   ccttttccgc tggatactca cccgcgtgaa caaagccctg gacaagaccc atcggcaagg 1440
   ggcttccttc ctggggatcc tggatatagc tggatttgag atctttgagg tgaactcctt 1500
   cgagcagctg tgcatcaact acaccaacga gaagctgcag cagctcttca accacaccat 1560
   gttcatcctg gagcaggagg agtaccagcg cgagggcatc gagtggaact tcatcgactt 1620
   tgggctggac ctacagccct gcatcgagct catcgagcga ccgaacaacc ctccaggtgt 1680
   gctggccctg ctggacgagg aatgctggtt ccccaaagcc acggacaagt ctttcgtgga 1740
   gaagctgtgc acggagcagg gcagccaccc caagttccag aagcccaagc agctcaagga 1800
   caagactgag ttctccatca tccattatgc tgggaaggtg gactataatg cgagtgcctg 1860
   gctgaccaag aatatggacc cgctgaatga caacgtgact tccctgctca atgcctcctc 1920
   cgacaagttt gtggccgacc tgtggaagga cgtggaccgc atcgtgggcc tggaccagat 1980
   ggccaagatg acggagagct cgctgcccag cgcctccaag accaagaagg gcatgttccg 2040
   cacagtgggg cagctgtaca aggagcagct gggcaagctg atgaccacgc tacgcaacac 2100
   cacgcccaac ttcgtgcgct gcatcatccc caaccacgag aagaggtccg gcaagctgga 2160
   tgcgttcctg gtgctggagc agctgcggtg caatggggtg ctggaaggca ttcgcatctg 2220
   ccggcagggc ttccccaacc ggatcgtctt ccaggagttc cgccaacgct acgagatcct 2280
   ggcggcgaat gccatcccca aaggcttcat ggacgggaag caggcctgca ttctcatgat 2340
   caaagccctg gaacttgacc ccaacttata caggataggg cagagcaaaa tcttcttccg 2400
   aactggcgtc ctggcccacc tagaggagga gcgagatttg aagatcaccg atgtcatcat 2460
   ggccttccag gcgatgtgtc gtggctactt ggccagaaag gcttttgcca agaggcagca 2520
   gcagctgacc gccatgaagg tgattcagag gaactgcgcc gcctacctca agctgcggaa 2580
   ctggcagtgg tggaggcttt tcaccaaagt gaagccactg ctgcaggtga cacggcagga 2640
   ggaggagatg caggccaagg aggatgaact gcagaagacc aaggagcggc agcagaaggc 2700
   agagaatgag cttaaggagc tggaacagaa gcactcgcag ctgaccgagg agaagaacct 2760
   gctacaggaa cagctgcagg cagagacaga gctgtatgca gaggctgagg agatgcgggt 2820
   gcggctggcg gccaagaagc aggagctgga ggagatactg catgagatgg aggcccgcct 2880
   ggaggaggag gaagacaggg gccagcagct acaggctgaa aggaagaaga tggcccagca 2940
   gatgctggac cttgaagaac agctggagga ggaggaagct gccaggcaga agctgcaact 3000
   tgagaaggtc acggctgagg ccaagatcaa gaaactggag gatgagatcc tggtcatgga 3060
   tgatcagaac aataaactat caaaagaacg aaaactcctt gaggagagga ttagtgactt 3120
   aacgacaaat cttgcagaag aggaagaaaa ggccaagaat cttaccaagc tgaaaaacaa 3180
   gcatgaatct atgatttcag aactggaagt gcggctaaag aaggaagaga agagccgaca 3240
   ggagctggag aagctgaaac ggaagctgga gggtgatgcc agcgacttcc acgagcagat 3300
   cgctgacctc caggcgcaga tcgcagagct caagatgcag ctggccaaga aggaggagga 3360
   gctgcaggcg gccctggcca ggcttgacga tgaaatcgct cagaagaaca atgccctgaa 3420
   gaagatccgg gagctggagg gccacatctc agacctccag gaggacctgg actcagagcg 3480
   ggccgccagg aacaaggctg aaaagcagaa gcgagacctc ggcgaggagc tggaggccct 3540
   aaagacagag ctggaagaca cactggacag cacagccact cagcaggagc tcagggccaa 3600
   gagggagcag gaggtgacgg tgctgaagaa ggccctggat gaagagacgc ggtcccatga 3660
   ggctcaggtc caggagatga ggcagaaaca cgcacaggcg gtggaggagc tcacagagca 3720
   gcttgagcag ttcaagaggg ccaaggcgaa cctagacaag aataagcaga cgctggagaa 3780
   agagaacgca gacctggccg gggagctgcg gtcctgggcc aggccaagca ggaggtggaa 3840
   cataagaaga agaagctgga ggcgcaggtg caggagctgc agtccaagtg cagcgatggg 3900
   ggagcgggcc cgggcggagc tcaatgacaa agtccacaag ctgcagaatg aagttgagag 3960
   cgtcacaggg atgcttaacg aggccgaggg gaaggccatt aagctggcca aggacgtggc 4020
   gtccctcagt tcccagctcc aggacaccca ggagctgctt caagaagaaa cccggcagaa 4080
   gctcaacgtg tctacgaagc tgcgccagct ggaggaggag cggaacagcc tgcaagacca 4140
   gctggacgag gagatggagg ccaagcagaa cctggagcgc cacatctcca ctctcaacat 4200
   ccagctctcc gactcgaaga agaagctgca ggactttgcc agcaccgtgg aagctctgga 4260
   agaggggaag aagaggttcc agaaggagat cgagaacctc acccagcagt acgaggagaa 4320
   ggcggccgct tatgataaac tggaaaagac caagaacagg cttcagcagg agctggacga 4380
   cctggttgtt gatttggaca accagcggca actcgtgtcc aacctggaaa agaagcagag 4440
   gaaatttgat cagttgttag ccgaggagaa aaacatctct tccaaatacg cggatgagag 4500
   ggacagagct gaggcagaag ccagggagaa ggaaaccaag gccctgtccc tggctcgggc 4560
   ccttgaagag gccttggaag ccaaagagga actcgagcgg accaacaaaa tgctcaaagc 4620
   cgaaatggaa gacctggtca gctccaagga tgacgtgggc aagaacgtcc atgagctgga 4680
   gaagtccaag cgggccctgg agacccagat ggaggagatg aagacgcagc tggaagagct 4740
   ggaggacgag ctgcaagcca cggaggacgc caaactgcgg ctggaagtca acatgcaggc 4800
   gctcaagggc cagttcgaaa gggatctcca agcccgggac gagcagaatg aggagaagag 4860
   gaggcaactg cagagacagc ttcacgagta tgagacggaa ctggaagacg agcgaaagca 4920
   acgtgccctg gcagctgcag caaagaagaa gctggaaggg gacctgaaag acctggagct 4980
   tcaggccgac tctgccatca aggggaggga ggaagccatc aagcagctac gcaaactgca 5040
   ggctcagatg aaggactttc aaagagagct ggaagatgcc cgtgcctcca gagatgagat 5100
   ctttgccaca gccaaagaga atgagaagaa agccaagagc ttggaagcag acctcatgca 5160
   gctacaagag gacctcgccg ccgctgagag ggctcgcaaa caagcggacc tcgagaagga 5220
   ggaactggca gaggagctgg ccagtagcct gtcgggaagg aacgcactcc aggacgagaa 5280
   gcgccgcctg gaggcccgga tcgcccagct ggaggaggag ctggaggagg agcagggcaa 5340
   catggaggcc atgagcgacc gggtccgcaa agccacacag caggccgagc agctcagcaa 5400
   cgagctggcc acagagcgca gcacggccca gaagaatgag agtgcccggc agcagctcga 5460
   gcggcagaac aaggagctcc ggagcaagct ccacgagatg gagggggccg tcaagtccaa 5520
   gttcaagtcc accatcgcgg cgctggaggc caagattgca cagctggagg agcaggtcga 5580
   gcaggaggcc agagagaaac aggcggccac caagtcgctg aagcagaaag acaagaagct 5640
   gaaggaaatc ttgctgcagg tggaggacga gcgcaagatg gccgagcagt acaaggagca 5700
   ggcagagaaa ggcaatgcca gggtcaagca gctcaagagg cagctggagg aggcagagga 5760
   ggagtcccag cgcatcaacg ccaaccgcag gaagctgcag cgggagctgg atgaggccac 5820
   ggagagcaac gaggccatgg gccgcgaggt gaacgcactc aagagcaagc tcagagggcc 5880
   ccccccacag gaaacttcgc agtgatgcac caggcgagga aacgagacct ctttcgttcc 5940
   ttctagaagg tctggaggac gtagagttat tgaaaatgca gatggttctg aggaggaaac 6000
   ggacactcga gacgcagact tcaatggaac caaggccagt gaataagcaa ctttctacag 6060
   ttttgcacca cggcaagaaa accaaaaacc aaaacaaaca aacaaaaaaa acccaacaac 6120
   aacccagaac aaagcaaaac ccagcagact gtacttagca ttgtctaaat ccattctcaa 6180
   attccaaata tcacagacac ccctcacaca aggaatataa aaaccaccac cctccagcct 6240
   gggcaacgta gtaaaacctc atctatacaa gaatttaaaa ataagctggg cgtggtggta 6300
   cacacctgtg gtcccagcta ctagggaggc tgagccagga agaacgctcc agcccaggac 6360
   ttcgaggctg caatgagcta taattgcatc attgcactcc agcctgggca acagagaccc 6420
   tgtctcaacc accaccacca ccaccacccc tactacccct gtattcaagg taaaaattga 6480
   agtttgtatg atgtaagaga tgagaaaaac ccaacaggaa acacagacac atcctccagt 6540
   tctatcaatg gattgtgcag acactgagtt tttagaaaaa catatccacg gtaaccggtc 6600
   cctggcaatt ctgtttacat gaaatgggga gaaagtcacc gaaatgggtg ccgccggccc 6660
   ccactcccaa ttcattccct aacctgcaaa cctttccaac ttctcacgtc aggcctttga 6720
   gaattctttc cccctctcct ggtttccaca cctcagacac gcacagttca ccaagtgcct 6780
   tctgtagtca catgaattga aaaggagacg ctgctcccac ggaggggagc aggaatgctg 6840
   cactgtttac accctgactg tgcttaaaaa cactttcact aataaatggt tataaatcac 6900

<210> 5
<211> 1920
<212> PRT
<213> Homo sapiens

<220>
<221> MISC_FEATURE
<223> MYH11 SM2 isoform

<400> 5

Met Ala Gln Lys Gly Gln Leu Ser Asp Asp Glu Lys Phe Leu Phe Val
1               5                   10                  15

Asp Lys Asn Phe Ile Asn Ser Pro Val Ala Gln Ala Asp Trp Ala Ala
            20                  25                  30

Lys Arg Leu Val Trp Val Pro Ser Glu Lys Gln Gly Phe Glu Ala Ala
        35                  40                  45

Ser Ile Lys Glu Glu Lys Gly Asp Glu Val Val Val Glu Leu Val Glu
    50                  55                  60

Asn Gly Lys Lys Val Thr Val Gly Lys Asp Asp Ile Gln Lys Met Asn
65                  70                  75                  80

Pro Pro Lys Phe Ser Lys Val Glu Asp Met Ala Glu Leu Thr Cys Leu
                85                  90                  95

Asn Glu Ala Ser Val Leu His Asn Leu Arg Glu Arg Tyr Phe Ser Gly
            100                 105                 110

Leu Ile Tyr Thr Tyr Ser Gly Leu Phe Cys Val Val Val Asn Pro Tyr
        115                 120                 125

Lys His Leu Pro Ile Tyr Ser Glu Lys Ile Val Asp Met Tyr Lys Gly
    130                 135                 140

Lys Lys Arg His Glu Met Pro Pro His Ile Tyr Ala Ile Ala Asp Thr
145                 150                 155                 160

Ala Tyr Arg Ser Met Leu Gln Asp Arg Glu Asp Gln Ser Ile Leu Cys
                165                 170                 175

Thr Gly Glu Ser Gly Ala Gly Lys Thr Glu Asn Thr Lys Lys Val Ile
            180                 185                 190

Gln Tyr Leu Ala Val Val Ala Ser Ser His Lys Gly Lys Lys Asp Thr
        195                 200                 205

Ser Ile Thr Gly Glu Leu Glu Lys Gln Leu Leu Gln Ala Asn Pro Ile
    210                 215                 220

Ala Phe Gly Asn Ala Lys Thr Val Lys Asn Asp Asn Ser Ser Arg Phe
225                 230                 235                 240

Gly Lys Phe Ile Arg Ile Asn Phe Asp Val Thr Gly Tyr Ile Val Gly
                245                 250                 255

Ala Asn Ile Glu Thr Tyr Leu Leu Glu Lys Ser Arg Ala Ile Arg Gln
            260                 265                 270

Ala Arg Asp Glu Arg Thr Phe His Ile Phe Tyr Tyr Met Ile Ala Gly
        275                 280                 285

Ala Lys Glu Lys Met Arg Ser Asp Leu Leu Leu Glu Gly Phe Asn Asn
    290                 295                 300

Tyr Thr Phe Leu Ser Asn Gly Phe Val Pro Ile Pro Ala Ala Gln Asp
305                 310                 315                 320

Asp Glu Met Phe Gln Glu Thr Val Glu Ala Met Ala Ile Met Gly Phe
                325                 330                 335

Ser Glu Glu Glu Gln Leu Ser Ile Leu Lys Val Val Ser Ser Val Leu
            340                 345                 350

Gln Leu Gly Asn Ile Val Phe Lys Lys Glu Arg Asn Thr Asp Gln Ala
        355                 360                 365

Ser Met Pro Asp Asn Thr Ala Ala Gln Lys Val Cys His Leu Met Gly
    370                 375                 380

Ile Asn Val Thr Asp Phe Thr Arg Ser Ile Leu Thr Pro Arg Ile Lys
385                 390                 395                 400

Val Gly Arg Asp Val Val Gln Lys Ala Gln Thr Lys Glu Gln Ala Asp
                405                 410                 415

Phe Ala Val Glu Ala Lys Ala Thr Tyr Glu Arg Leu Phe Arg Trp Ile
            420                 425                 430

Leu Thr Arg Val Asn Lys Ala Leu Asp Lys Thr Gln Gly Ala Ser Phe
        435                 440                 445

Leu Gly Ile Leu Asp Ile Ala Gly Phe Glu Ile Phe Glu Val Asn Ser
    450                 455                 460

Phe Glu Gln Leu Cys Ile Asn Tyr Thr Asn Glu Lys Leu Gln Gln Leu
465                 470                 475                 480

Phe Asn His Thr Met Phe Ile Gln Glu Glu Tyr Gln Arg Glu Gly Ile
                485                 490                 495

Glu Trp Asn Phe Ile Asp Phe Gly Leu Asp Leu Gln Pro Cys Ile Glu
            500                 505                 510

Leu Ile Glu Arg Pro Asn Asn Pro Pro Gly Val Leu Ala Leu Leu Asp
        515                 520                 525

Glu Glu Cys Trp Phe Pro Lys Ala Thr Asp Lys Ser Phe Val Glu Lys
    530                 535                 540

Leu Cys Thr Glu Gln Gly Ser His Pro Lys Phe Gln Lys Pro Lys Gln
545                 550                 555                 560

Leu Lys Asp Lys Thr Glu Phe Ser Ile Ile His Tyr Ala Gly Lys Val
                565                 570                 575

Asp Tyr Asn Ala Ser Ala Trp Leu Thr Lys Asn Met Asp Pro Leu Asn
            580                 585                 590

Asp Asn Val Thr Ser Leu Leu Asn Ala Ser Ser Asp Lys Phe Val Ala
        595                 600                 605

Asp Leu Trp Lys Asp Val Asp Arg Ile Val Gly Leu Asp Gln Met Ala
    610                 615                 620

Lys Met Thr Glu Ser Ser Leu Pro Ser Ala Ser Lys Thr Lys Lys Gly
625                 630                 635                 640

Met Phe Arg Thr Val Gly Gln Leu Tyr Lys Glu Gln Leu Gly Lys Leu
                645                 650                 655

Met Thr Thr Leu Arg Asn Thr Thr Pro Asn Phe Val Arg Cys Ile Ile
            660                 665                 670

Pro Asn His Glu Lys Arg Ser Gly Lys Leu Asp Ala Phe Leu Val Leu
        675                 680                 685

Glu Gln Leu Arg Cys Asn Gly Val Leu Glu Gly Ile Arg Ile Cys Arg
    690                 695                 700

Gln Gly Phe Pro Asn Arg Ile Val Phe Gln Glu Phe Arg Gln Arg Tyr
705                 710                 715                 720

Glu Ile Leu Ala Ala Asn Ala Ile Pro Lys Gly Phe Met Asp Gly Lys
                725                 730                 735

Gln Ala Cys Ile Leu Met Ile Lys Ala Leu Glu Leu Asp Pro Asn Leu
            740                 745                 750

Tyr Arg Ile Gly Gln Ser Lys Ile Phe Phe Arg Thr Gly Val Leu Ala
        755                 760                 765

His Leu Glu Glu Glu Arg Asp Leu Lys Ile Thr Asp Val Ile Met Ala
    770                 775                 780

Phe Gln Ala Met Cys Arg Gly Tyr Leu Ala Arg Lys Ala Phe Ala Lys
785                 790                 795                 800

Arg Gln Gln Gln Leu Thr Ala Met Lys Val Ile Gln Arg Asn Cys Ala
                805                 810                 815

Ala Tyr Leu Lys Leu Arg Asn Trp Gln Trp Trp Arg Leu Phe Thr Lys
            820                 825                 830

Val Lys Pro Leu Leu Gln Val Thr Arg Gln Glu Glu Glu Met Gln Ala
        835                 840                 845

Lys Glu Asp Glu Leu Gln Lys Thr Lys Glu Arg Gln Gln Lys Ala Glu
    850                 855                 860

Asn Glu Leu Lys Glu Leu Glu Gln Lys His Ser Gln Leu Thr Glu Glu

865                 870                 875                 880

Lys Asn Leu Leu Gln Glu Gln Leu Gln Ala Glu Thr Glu Leu Tyr Ala
                885                 890                 895

Glu Ala Glu Glu Met Arg Val Arg Leu Ala Ala Lys Lys Gln Glu Leu
            900                 905                 910

Glu Glu Ile Leu His Glu Met Glu Ala Arg Leu Glu Glu Glu Glu Asp
        915                 920                 925

Arg Gly Gln Gln Leu Gln Ala Glu Arg Lys Lys Met Ala Gln Gln Met
    930                 935                 940

Leu Asp Leu Glu Glu Gln Leu Glu Glu Glu Glu Ala Ala Arg Gln Lys
945                 950                 955                 960

Leu Gln Leu Glu Lys Val Thr Ala Glu Ala Lys Ile Lys Lys Leu Glu
                965                 970                 975

Asp Glu Ile Leu Val Met Asp Asp Gln Asn Asn Lys Leu Ser Lys Glu
            980                 985                 990

Arg Lys Leu Leu Glu Glu Arg Ile  Ser Asp Leu Thr Thr  Asn Leu Ala
        995                 1000                1005

Glu Glu  Glu Glu Lys Ala Lys  Asn Leu Thr Lys Leu  Lys Asn Lys
    1010                1015                1020

His Glu  Ser Met Ile Ser Glu  Leu Glu Val Arg Leu  Lys Lys Glu
    1025                1030                1035

Glu Lys  Ser Arg Gln Glu Leu  Glu Lys Leu Lys Arg  Lys Leu Glu
    1040                1045                1050

Gly Asp  Ala Ser Asp Phe His  Glu Gln Ile Ala Asp  Leu Gln Ala
    1055                1060                1065

Gln Ile  Ala Glu Leu Lys Met  Gln Leu Ala Lys Lys  Glu Glu Glu
    1070                1075                1080

Leu Gln  Ala Ala Arg Leu Asp  Asp Glu Ile Ala Gln  Lys Asn Asn
    1085                1090                1095

Ala Leu  Lys Lys Ile Arg Glu  Leu Glu Gly His Asp  Leu Gln Glu
    1100                1105                1110

Asp Leu Asp Ser Ala Ala Arg Asn Lys Ala Glu Lys Gln Lys Arg
    1115                1120                1125

Asp Leu Gly Glu Glu Leu Glu Ala Leu Lys Thr Glu Leu Glu Asp
    1130                1135                1140

Thr Leu Asp Ser Thr Ala Thr Gln Gln Glu Leu Arg Ala Lys Arg
    1145                1150                1155

Glu Gln Glu Val Thr Val Leu Lys Lys Ala Leu Asp Glu Glu Thr
    1160                1165                1170

Arg Ser His Glu Ala Gln Val Gln Glu Met Arg Gln Lys His Ala
    1175                1180                1185

Gln Ala Val Glu Glu Leu Thr Glu Gln Leu Glu Gln Phe Lys Arg
    1190                1195                1200

Ala Lys Ala Asn Leu Asp Lys Asn Lys Gln Thr Leu Glu Lys Glu
    1205                1210                1215

Asn Ala Asp Leu Ala Gly Glu Leu Arg Val Leu Gly Gln Ala Lys
    1220                1225                1230

Gln Glu Val Glu His Lys Lys Lys Lys Leu Glu Ala Gln Val Gln
    1235                1240                1245

Glu Leu Gln Ser Lys Cys Ser Asp Gly Glu Arg Ala Arg Ala Glu
    1250                1255                1260

Leu Asn Asp Lys Val His Lys Leu Gln Asn Glu Val Glu Ser Val
    1265                1270                1275

Thr Gly Met Leu Asn Glu Ala Glu Gly Lys Ala Ile Lys Leu Ala
    1280                1285                1290

Lys Asp Val Ala Ser Leu Ser Ser Gln Leu Gln Asp Thr Gln Glu
    1295                1300                1305

Leu Leu Gln Glu Glu Thr Arg Gln Lys Leu Asn Val Ser Thr Lys
    1310                1315                1320

Leu Arg Gln Leu Glu Glu Glu Arg Asn Ser Leu Gln Asp Gln Leu
    1325                1330                1335

Asp Glu Glu Met Glu Ala Lys Gln Asn Leu Glu Arg His Thr Leu
    1340                1345                1350

Asn Ile Gln Leu Ser Asp Ser Lys Lys Lys Leu Gln Asp Phe Ala
    1355                1360                1365

Ser Thr Val Glu Ala Leu Glu Glu Gly Lys Lys Arg Phe Gln Lys
    1370                1375                1380

Glu Ile Glu Asn Leu Thr Gln Gln Tyr Glu Glu Lys Ala Ala Ala
    1385                1390                1395

Tyr Asp Lys Leu Glu Lys Thr Lys Asn Arg Leu Gln Gln Glu Leu
    1400                1405                1410

Asp Asp Leu Val Val Asp Leu Asp Asn Gln Arg Gln Leu Val Ser
    1415                1420                1425

Asn Leu Glu Lys Lys Gln Arg Lys Phe Asp Gln Leu Leu Ala Glu
    1430                1435                1440

Glu Lys Asn Ile Ser Ser Lys Tyr Ala Asp Glu Arg Asp Arg Ala
    1445                1450                1455

Glu Ala Glu Ala Arg Glu Lys Glu Thr Lys Ala Leu Ser Leu Ala
    1460                1465                1470

Arg Ala Leu Glu Glu Ala Leu Glu Ala Lys Glu Glu Leu Glu Arg
    1475                1480                1485

Thr Asn Lys Met Leu Lys Ala Glu Met Glu Asp Leu Val Ser Ser
    1490                1495                1500

Lys Asp Asp Val Gly Lys Asn Val His Glu Leu Glu Lys Ser Lys
    1505                1510                1515

Arg Ala Leu Glu Thr Gln Met Glu Glu Met Lys Thr Gln Leu Glu
    1520                1525                1530

Glu Leu Glu Asp Glu Leu Gln Ala Thr Glu Asp Ala Lys Leu Arg
    1535                1540                1545

Leu Glu Val Asn Met Gln Ala Leu Lys Gly Gln Phe Glu Arg Asp
    1550                1555                1560

Leu Gln Ala Arg Asp Glu Gln Asn Glu Glu Lys Arg Arg Gln Leu
    1565                1570                1575

Gln Arg Gln Leu His Glu Tyr Glu Thr Glu Leu Glu Asp Glu Arg
    1580                1585                1590

Lys Gln Arg Ala Ala Ala Ala Lys Lys Lys Leu Glu Gly Asp Leu
    1595                1600                1605

Lys Asp Leu Glu Leu Gln Ala Asp Ser Ala Ile Lys Gly Arg Glu
    1610                1615                1620

Glu Ala Ile Lys Gln Leu Arg Lys Leu Gln Ala Gln Met Lys Asp
    1625                1630                1635

Phe Gln Arg Glu Leu Glu Asp Ala Arg Ala Ser Arg Asp Glu Ile
    1640                1645                1650

Phe Ala Thr Ala Lys Glu Asn Glu Lys Lys Ala Lys Ser Leu Glu
    1655                1660                1665

Ala Asp Leu Met Gln Leu Gln Glu Asp Leu Ala Ala Ala Glu Arg
    1670                1675                1680

Ala Arg Lys Gln Ala Asp Leu Glu Lys Glu Glu Leu Ala Glu Glu
    1685                1690                1695

Leu Ala Ser Ser Leu Ser Gly Arg Asn Ala Leu Gln Asp Glu Lys
    1700                1705                1710

Arg Arg Leu Glu Ala Arg Ile Ala Gln Leu Glu Glu Glu Leu Glu
    1715                1720                1725

Glu Glu Gln Gly Asn Met Glu Ala Met Ser Asp Arg Val Arg Lys
    1730                1735                1740

Ala Thr Gln Gln Ala Glu Gln Leu Ser Asn Glu Leu Ala Thr Glu
    1745                1750                1755

Arg Ser Thr Ala Gln Lys Asn Glu Ser Ala Arg Gln Gln Leu Glu
    1760                1765                1770

Arg Gln Asn Lys Glu Leu Arg Ser Lys Leu His Glu Met Glu Gly
    1775                1780                1785

Ala Val Lys Ser Lys Phe Lys Ser Thr Ile Ala Ala Leu Glu Ala

1790                    1795                    1800

Lys Ile  Ala Gln Leu Glu Glu  Gln Val Glu Gln Glu  Ala Arg Glu
    1805                    1810                    1815

Lys Gln  Ala Ala Thr Lys Ser  Leu Lys Gln Lys Asp  Lys Lys Leu
    1820                    1825                    1830

Lys Glu  Ile Leu Leu Gln Val  Glu Asp Glu Arg Lys  Met Ala Glu
    1835                    1840                    1845

Gln Tyr  Lys Glu Gln Ala Glu  Lys Gly Asn Ala Arg  Val Lys Gln
    1850                    1855                    1860

Leu Lys  Arg Gln Leu Glu Glu  Ala Glu Glu Glu Ser  Gln Arg Ile
    1865                    1870                    1875

Asn Ala  Asn Arg Arg Lys Leu  Gln Arg Glu Leu Asp  Glu Ala Thr
    1880                    1885                    1890

Glu Ser  Asn Glu Ala Met Gly  Arg Glu Val Asn Ala  Leu Lys Ser
    1895                    1900                    1905

Lys Leu  Arg Gly Pro Pro Pro  Gln Glu Thr Ser Gln
    1910                    1915                    1920

<210>  6
<211>  1958
<212>  PRT
<213>  Homo sapiens

<400>  6

Met Ala Gln Lys Gly Gln Leu Ser Asp Asp Glu Lys Phe Leu Phe Val
1               5                   10                  15

Asp Lys Asn Phe Ile Asn Ser Pro Val Ala Gln Ala Asp Trp Ala Ala
            20                  25                  30

Lys Arg Leu Val Trp Val Pro Ser Glu Lys Gln Gly Phe Glu Ala Ala
        35                  40                  45

Ser Ile Lys Glu Glu Lys Gly Asp Glu Val Val Val Glu Leu Val Glu
    50                  55                  60

Asn Gly Lys Lys Val Thr Val Gly Lys Asp Asp Ile Gln Lys Met Asn
65                  70                  75                  80

Pro Pro Lys Phe Ser Lys Val Glu Asp Met Ala Glu Leu Thr Cys Leu
                85                  90                  95

Asn Glu Ala Ser Val Leu His Asn Leu Arg Glu Arg Tyr Phe Ser Gly
            100                 105                 110

Leu Ile Tyr Thr Tyr Ser Gly Leu Phe Cys Val Val Val Asn Pro Tyr
        115                 120                 125

Lys His Leu Pro Ile Tyr Ser Glu Lys Ile Val Asp Met Tyr Lys Gly
    130                 135                 140

Lys Lys Arg His Glu Met Pro Pro His Ile Tyr Ala Ile Ala Asp Thr
145                 150                 155                 160

Ala Tyr Arg Ser Met Leu Gln Asp Arg Glu Asp Gln Ser Ile Leu Cys
                165                 170                 175

Thr Gly Glu Ser Gly Ala Gly Lys Thr Glu Asn Thr Lys Lys Val Ile
            180                 185                 190

Gln Tyr Leu Ala Val Val Ala Ser Ser His Lys Gly Lys Lys Asp Thr
        195                 200                 205

Ser Ile Thr Gly Glu Leu Glu Lys Gln Leu Leu Gln Ala Asn Pro Ile
    210                 215                 220

Ala Phe Gly Asn Ala Lys Thr Val Lys Asn Asp Asn Ser Ser Arg Phe
225                 230                 235                 240

Gly Lys Phe Ile Arg Ile Asn Phe Asp Val Thr Gly Tyr Ile Val Gly
                245                 250                 255

Ala Asn Ile Glu Thr Tyr Leu Leu Glu Lys Ser Arg Ala Ile Arg Gln
            260                 265                 270

Ala Arg Asp Glu Arg Thr Phe His Ile Phe Tyr Tyr Met Ile Ala Gly
        275                 280                 285

Ala Lys Glu Lys Met Arg Ser Asp Leu Leu Leu Glu Gly Phe Asn Asn
    290                 295                 300

Tyr Thr Phe Leu Ser Asn Gly Phe Val Pro Ile Pro Ala Ala Gln Asp
305                 310                 315                 320

Asp Glu Met Phe Gln Glu Thr Val Glu Ala Met Ala Ile Met Gly Phe
                325                 330                 335

Ser Glu Glu Glu Gln Leu Ser Ile Leu Lys Val Val Ser Ser Val Leu
            340                 345                 350

Gln Leu Gly Asn Ile Val Phe Lys Lys Glu Arg Asn Thr Asp Gln Ala
        355                 360                 365

Ser Met Pro Asp Asn Thr Ala Ala Gln Lys Val Cys His Leu Met Gly
    370                 375                 380

Ile Asn Val Thr Asp Phe Thr Arg Ser Ile Leu Thr Pro Arg Ile Lys
385                 390                 395                 400

Val Gly Arg Asp Val Val Gln Lys Ala Gln Thr Lys Glu Gln Ala Asp
                405                 410                 415

Phe Ala Val Glu Ala Lys Ala Thr Tyr Glu Arg Leu Phe Arg Trp Ile
            420                 425                 430

Leu Thr Arg Val Asn Lys Ala Leu Asp Lys Thr Gln Gly Ala Ser Phe
        435                 440                 445

Leu Gly Ile Leu Asp Ile Ala Gly Phe Glu Ile Phe Glu Val Asn Ser
    450                 455                 460

Phe Glu Gln Leu Cys Ile Asn Tyr Thr Asn Glu Lys Leu Gln Gln Leu
465                 470                 475                 480

Phe Asn His Thr Met Phe Ile Gln Glu Glu Tyr Gln Arg Glu Gly Ile
                485                 490                 495

Glu Trp Asn Phe Ile Asp Phe Gly Leu Asp Leu Gln Pro Cys Ile Glu
            500                 505                 510

Leu Ile Glu Arg Pro Asn Asn Pro Pro Gly Val Leu Ala Leu Leu Asp
        515                 520                 525

Glu Glu Cys Trp Phe Pro Lys Ala Thr Asp Lys Ser Phe Val Glu Lys
    530                 535                 540

Leu Cys Thr Glu Gln Gly Ser His Pro Lys Phe Gln Lys Pro Lys Gln
545                 550                 555                 560

Leu Lys Asp Lys Thr Glu Phe Ser Ile Ile His Tyr Ala Gly Lys Val

                565                 570                 575

Asp Tyr Asn Ala Ser Ala Trp Leu Thr Lys Asn Met Asp Pro Leu Asn
            580                 585                 590

Asp Asn Val Thr Ser Leu Leu Asn Ala Ser Ser Asp Lys Phe Val Ala
        595                 600                 605

Asp Leu Trp Lys Asp Val Asp Arg Ile Val Gly Leu Asp Gln Met Ala
    610                 615                 620

Lys Met Thr Glu Ser Ser Leu Pro Ser Ala Ser Lys Thr Lys Lys Gly
625                 630                 635                 640

Met Phe Arg Thr Val Gly Gln Leu Tyr Lys Glu Gln Leu Gly Lys Leu
                645                 650                 655

Met Thr Thr Leu Arg Asn Thr Thr Pro Asn Phe Val Arg Cys Ile Ile
            660                 665                 670

Pro Asn His Glu Lys Arg Ser Gly Lys Leu Asp Ala Phe Leu Val Leu
        675                 680                 685

Glu Gln Leu Arg Cys Asn Gly Val Leu Glu Gly Ile Arg Ile Cys Arg
    690                 695                 700

Gln Gly Phe Pro Asn Arg Ile Val Phe Gln Glu Phe Arg Gln Arg Tyr
705                 710                 715                 720

Glu Ile Leu Ala Ala Asn Ala Ile Pro Lys Gly Phe Met Asp Gly Lys
                725                 730                 735

Gln Ala Cys Ile Leu Met Ile Lys Ala Leu Glu Leu Asp Pro Asn Leu
            740                 745                 750

Tyr Arg Ile Gly Gln Ser Lys Ile Phe Phe Arg Thr Gly Val Leu Ala
        755                 760                 765

His Leu Glu Glu Glu Arg Asp Leu Lys Ile Thr Asp Val Ile Met Ala
    770                 775                 780

Phe Gln Ala Met Cys Arg Gly Tyr Leu Ala Arg Lys Ala Phe Ala Lys
785                 790                 795                 800

Arg Gln Gln Gln Leu Thr Ala Met Lys Val Ile Gln Arg Asn Cys Ala
                805                 810                 815

Ala Tyr Leu Lys Leu Arg Asn Trp Gln Trp Trp Arg Leu Phe Thr Lys
            820                 825                 830

Val Lys Pro Leu Leu Gln Val Thr Arg Gln Glu Glu Glu Met Gln Ala
        835                 840                 845

Lys Glu Asp Glu Leu Gln Lys Thr Lys Glu Arg Gln Gln Lys Ala Glu
    850                 855                 860

Asn Glu Leu Lys Glu Leu Glu Gln Lys His Ser Gln Leu Thr Glu Glu
865                 870                 875                 880

Lys Asn Leu Leu Gln Glu Gln Leu Gln Ala Glu Thr Glu Leu Tyr Ala
                885                 890                 895

Glu Ala Glu Glu Met Arg Val Arg Leu Ala Ala Lys Lys Gln Glu Leu
            900                 905                 910

Glu Glu Ile Leu His Glu Met Glu Ala Arg Leu Glu Glu Glu Glu Asp
        915                 920                 925

Arg Gly Gln Gln Leu Gln Ala Glu Arg Lys Lys Met Ala Gln Gln Met
    930                 935                 940

Leu Asp Leu Glu Glu Gln Leu Glu Glu Glu Glu Ala Ala Arg Gln Lys
945                 950                 955                 960

Leu Gln Leu Glu Lys Val Thr Ala Glu Ala Lys Ile Lys Lys Leu Glu
                965                 970                 975

Asp Glu Ile Leu Val Met Asp Asp Gln Asn Asn Lys Leu Ser Lys Glu
            980                 985                 990

Arg Lys Leu Leu Glu Glu Arg Ile  Ser Asp Leu Thr Thr  Asn Leu Ala
        995                 1000                1005

Glu Glu  Glu Glu Lys Ala Lys  Asn Leu Thr Lys Leu  Lys Asn Lys
    1010                1015                1020

His Glu  Ser Met Ile Ser Glu  Leu Glu Val Arg Leu  Lys Lys Glu
    1025                1030                1035

Glu Lys  Ser Arg Gln Glu Leu  Glu Lys Leu Lys Arg  Lys Leu Glu
    1040                1045                1050

Gly Asp Ala Ser Asp Phe His Glu Gln Ile Ala Asp Leu Gln Ala
    1055                1060                1065

Gln Ile Ala Glu Leu Lys Met Gln Leu Ala Lys Lys Glu Glu Glu
    1070                1075                1080

Leu Gln Ala Ala Arg Leu Asp Asp Glu Ile Ala Gln Lys Asn Asn
    1085                1090                1095

Ala Leu Lys Lys Ile Arg Glu Leu Glu Gly His Ile Ser Asp Leu
    1100                1105                1110

Gln Glu Asp Leu Asp Ser Glu Arg Ala Ala Arg Asn Lys Ala Glu
    1115                1120                1125

Lys Gln Lys Arg Asp Leu Gly Glu Glu Leu Glu Ala Leu Lys Thr
    1130                1135                1140

Glu Leu Glu Asp Thr Leu Asp Ser Thr Ala Thr Gln Gln Glu Leu
    1145                1150                1155

Arg Ala Lys Arg Glu Gln Glu Val Thr Val Leu Lys Lys Ala Leu
    1160                1165                1170

Asp Glu Glu Thr Arg Ser His Glu Ala Gln Val Gln Glu Met Arg
    1175                1180                1185

Gln Lys His Ala Gln Ala Val Glu Glu Leu Thr Glu Gln Leu Glu
    1190                1195                1200

Gln Phe Lys Arg Ala Lys Ala Asn Leu Asp Lys Asn Lys Gln Thr
    1205                1210                1215

Leu Glu Lys Glu Asn Ala Asp Leu Ala Gly Glu Leu Arg Val Leu
    1220                1225                1230

Gly Gln Ala Lys Gln Glu Val Glu His Lys Lys Lys Lys Leu Glu
    1235                1240                1245

Ala Gln Val Gln Glu Leu Gln Ser Lys Cys Ser Asp Gly Glu Arg
    1250                1255                1260

Ala Arg Ala Glu Leu Asn Asp Lys Val His Lys Leu Gln Asn Glu
    1265                1270                1275

Val Glu Ser Val Thr Gly Met Leu Asn Glu Ala Glu Gly Lys Ala
    1280                1285                1290

Ile Lys Leu Ala Lys Asp Val Ala Ser Leu Ser Ser Gln Leu Gln
    1295                1300                1305

Asp Thr Gln Glu Leu Leu Gln Glu Glu Thr Arg Gln Lys Leu Asn
    1310                1315                1320

Val Ser Thr Lys Leu Arg Gln Leu Glu Glu Glu Arg Asn Ser Leu
    1325                1330                1335

Gln Asp Gln Leu Asp Glu Glu Met Glu Ala Lys Gln Asn Leu Glu
    1340                1345                1350

Arg His Thr Leu Asn Ile Gln Leu Ser Asp Ser Lys Lys Lys Leu
    1355                1360                1365

Gln Asp Phe Ala Ser Thr Val Glu Ala Leu Glu Glu Gly Lys Lys
    1370                1375                1380

Arg Phe Gln Lys Glu Ile Glu Asn Leu Thr Gln Gln Tyr Glu Glu
    1385                1390                1395

Lys Ala Ala Ala Tyr Asp Lys Leu Glu Lys Thr Lys Asn Arg Leu
    1400                1405                1410

Gln Gln Glu Leu Asp Asp Leu Val Val Asp Leu Asp Asn Gln Arg
    1415                1420                1425

Gln Leu Val Ser Asn Leu Glu Lys Lys Gln Arg Lys Phe Asp Gln
    1430                1435                1440

Leu Leu Ala Glu Glu Lys Asn Ile Ser Ser Lys Tyr Ala Asp Glu
    1445                1450                1455

Arg Asp Arg Ala Glu Ala Glu Ala Arg Glu Lys Glu Thr Lys Ala
    1460                1465                1470

Leu Ser Leu Ala Arg Ala Leu Glu Glu Ala Leu Glu Ala Lys Glu
    1475                1480                1485

Glu Leu Glu Arg Thr Asn Lys Met Leu Lys Ala Glu Met Glu Asp
    1490                1495                1500

Leu Val Ser Ser Lys Asp Asp Val Gly Lys Asn Val His Glu Leu

1505                 1510                 1515

Glu Lys  Ser Lys Arg Ala Leu  Glu Thr Gln Met Glu  Glu Met Lys
    1520                 1525                 1530

Thr Gln  Leu Glu Glu Leu Glu  Asp Glu Leu Gln Ala  Thr Glu Asp
    1535                 1540                 1545

Ala Lys  Leu Arg Leu Glu Val  Asn Met Gln Ala Leu  Lys Gly Gln
    1550                 1555                 1560

Phe Glu  Arg Asp Leu Gln Ala  Arg Asp Glu Gln Asn  Glu Glu Lys
    1565                 1570                 1575

Arg Arg  Gln Leu Gln Arg Gln  Leu His Glu Tyr Glu  Thr Glu Leu
    1580                 1585                 1590

Glu Asp  Glu Arg Lys Gln Arg  Ala Ala Ala Ala Lys  Lys Lys Leu
    1595                 1600                 1605

Glu Gly  Asp Leu Lys Asp Leu  Glu Leu Gln Ala Asp  Ser Ala Ile
    1610                 1615                 1620

Lys Gly  Arg Glu Glu Ala Ile  Lys Gln Leu Arg Lys  Leu Gln Ala
    1625                 1630                 1635

Gln Met  Lys Asp Phe Gln Arg  Glu Leu Glu Asp Ala  Arg Ala Ser
    1640                 1645                 1650

Arg Asp  Glu Ile Phe Ala Thr  Ala Lys Glu Asn Glu  Lys Lys Ala
    1655                 1660                 1665

Lys Ser  Leu Glu Ala Asp Leu  Met Gln Leu Gln Glu  Asp Leu Ala
    1670                 1675                 1680

Ala Ala  Glu Arg Ala Arg Lys  Gln Ala Asp Leu Glu  Lys Glu Glu
    1685                 1690                 1695

Leu Ala  Glu Glu Leu Ala Ser  Ser Leu Ser Gly Arg  Asn Ala Leu
    1700                 1705                 1710

Gln Asp  Glu Lys Arg Arg Leu  Glu Ala Arg Ile Ala  Gln Leu Glu
    1715                 1720                 1725

Glu Glu  Leu Glu Glu Glu Gln  Gly Asn Met Glu Ala  Met Ser Asp
    1730                 1735                 1740

Arg Val Arg Lys Ala Thr Gln Gln Ala Glu Gln Leu Ser Asn Glu
    1745                1750                1755

Leu Ala Thr Glu Arg Ser Thr Ala Gln Lys Asn Glu Ser Ala Arg
    1760                1765                1770

Gln Gln Leu Glu Arg Gln Asn Lys Glu Leu Arg Ser Lys Leu His
    1775                1780                1785

Glu Met Glu Gly Ala Val Lys Ser Lys Phe Lys Ser Thr Ile Ala
    1790                1795                1800

Ala Leu Glu Ala Lys Ile Ala Gln Leu Glu Glu Gln Val Glu Gln
    1805                1810                1815

Glu Ala Arg Glu Lys Gln Ala Ala Thr Lys Ser Leu Lys Gln Lys
    1820                1825                1830

Asp Lys Lys Leu Lys Glu Ile Leu Leu Gln Val Glu Asp Glu Arg
    1835                1840                1845

Lys Met Ala Glu Gln Tyr Lys Glu Gln Ala Glu Lys Gly Asn Ala
    1850                1855                1860

Arg Val Lys Gln Leu Lys Arg Gln Leu Glu Glu Ala Glu Glu Glu
    1865                1870                1875

Ser Gln Arg Ile Asn Ala Asn Arg Arg Lys Leu Gln Arg Glu Leu
    1880                1885                1890

Asp Glu Ala Thr Glu Ser Asn Glu Ala Met Gly Arg Glu Val Asn
    1895                1900                1905

Ala Leu Lys Ser Lys Leu Arg Arg Gly Asn Glu Thr Ser Phe Val
    1910                1915                1920

Pro Ser Arg Arg Ser Gly Gly Arg Arg Val Ile Glu Asn Ala Asp
    1925                1930                1935

Gly Ser Glu Glu Glu Thr Asp Thr Arg Asp Ala Asp Phe Asn Gly
    1940                1945                1950

Thr Lys Ala Ser Glu
    1955

<210>  7
<211>  19
<212>  DNA
<213>  Homo sapiens

<220>
<221>  misc_feature
<222>  (1)..(9)
<223>  3' end of exon 32


<220>
<221>  mutation
<222>  (10)..(10)
<223>  G>T


<220>
<221>  misc_feature
<222>  (10)..(19)
<223>  5' end of intron 32


<400>  7
ggcaagaacg taagtggct                                                    19


<210>  8
<211>  27
<212>  DNA
<213>  Homo sapiens

<220>
<221>  misc_feature
<222>  (1)..(27)
<223>  junction of exons 31 and 33


<220>
<221>  misc_feature
<222>  (1)..(14)
<223>  3' end of exon 31


<220>
<221>  misc_feature
<222>  (15)..(27)
<223>  5' end of exon 33


<400>  8
ggaaatttga tcaggtccat gagctgg                                           27


<210>  9
<211>  213
<212>  DNA
<213>  Homo sapiens

<220>

<221>  misc_feature
<223>  exon 32


<400>  9
ttgttagccg aggagaaaaa catctcttcc aaatacgcgg atgagaggga cagagctgag      60

gcagaagcca gggagaagga aaccaaggcc ctgtccctgg ctcgggccct tgaagaggcc     120

ttggaagcca aagaggaact cgagcggacc aacaaaatgc tcaaagccga aatggaagac     180

ctggtcagct ccaaggatga cgtgggcaag aac                                  213


<210>  10
<211>  25
<212>  DNA
<213>  Homo sapiens

<220>
<221>  misc_feature
<222>  (1)..(12)
<223>  nucleotides 3798 to 3809 of MYH11 coding sequence


<220>
<221>  misc_feature
<222>  (13)..(25)
<223>  nucleotides 3882 to 3894 of MYH11 coding sequence


<400>  10
ccggggagct gcagtccaag tgcag                                            25


<210>  11
<211>  72
<212>  DNA
<213>  Homo sapiens

<220>
<221>  misc_feature
<223>  nucleotides 3810 to 3881 of MYH11 coding sequence


<400>  11
gggtcctggg ccaggccaag caggaggtgg aacataagaa gaagaagctg gaggcgcagg      60

tgcaggagct gc                                                          72


<210>  12
<211>  71
<212>  PRT
<213>  Homo sapiens

<220>
<221>  MISC_FEATURE
<222>  (1)..(71)
<223>  amino acids 1456 to 1526 of MYH11 protein

<400>  12

Leu Leu Ala Glu Glu Lys Asn Ile Ser Ser Lys Tyr Ala Asp Glu Arg
1               5                   10                  15

Asp Arg Ala Glu Ala Glu Ala Arg Glu Lys Glu Thr Lys Ala Leu Ser
            20                  25                  30

Leu Ala Arg Ala Leu Glu Glu Ala Leu Glu Ala Lys Glu Glu Leu Glu
        35                  40                  45

Arg Thr Asn Lys Met Leu Lys Ala Glu Met Glu Asp Leu Val Ser Ser
    50                  55                  60

Lys Asp Asp Val Gly Lys Asn
65                  70

<210>  13
<211>  23
<212>  PRT
<213>  Homo sapiens

<220>
<221>  MISC_FEATURE
<222>  (1)..(23)
<223>  amino acids 1242 to 1264 of MYH11

<400>  13

Val Leu Gly Gln Ala Lys Gln Glu Val Glu His Lys Lys Lys Lys Leu
1               5                   10                  15

Glu Ala Gln Val Gln Glu Leu
            20

<210>  14
<211>  18
<212>  DNA
<213>  Artificial Sequence

<220>
<223>  primer

<220>
<221>  misc_feature
<223>  primer

<400>  14
acaaccagcg gcaactcg                                                    18

<210>  15
<211>  19
<212>  DNA
<213>  Artificial Sequence

<220>
<223>  primer

<220>
<221>  misc_feature
<223>  primer


<400>  15
cgtcttcatc tcctccatc                                                    19




112

## Description

The invention relates to the identification of mutations in the MYH11 gene or protein, associated with an arterial disease, and to diagnostic and therapeutic applications that benefit from this identification.

Thoracic aortic aneurysm and/or aortic dissection (TAA/AD) is one of the most severe cardiovascular conditions occurring in adulthood. It can be part of inherited connective tissue disorders such as Marfan (MIM 154700) and Ehlers-Danlos (MIM 130050) syndromes. Familial clustering of common TAA/AD is however more complex and heterogeneous (Coady et al., 1999), and three loci have been mapped to chromosomes 11q23.2-q24 (*FAA1*) (Vaughan et al., 2001), 5q13-14 *(TAAD1)* (Guo et al. 2001), and 3p24-25 *(TAAD2)* (Hasham et al., 2003).

The inventors recently reported one American (Glancy et al., 2001) and one French (Khau et al., 2004) kindreds in which TAA/AD was unexpectedly associated to patent *ductus arteriosus* (PDA), apparently with transmission in an autosomal dominant manner (Glancy et al., 2001 ; Khau et al. 2004).

PDA is one of the most common congenital cardiovascular malformation, affecting approximately 1 in 2000 live births (Mitchell et al., 1971). It is generally regarded as a sporadic disease, but its recurrence among 5% of siblings of PDA cases suggests a genetic component (Polani et al., 1960). No specific gene defect has yet been reported, other than mutations in the transcription factor TFA2B in the rare Char syndrome (Satoda et al., 2000) and a recessive locus at *12q24* in Iranian pedigrees (Mani et al., 2002). Further clinical characterisation and genetic analysis of the large French "Bourgogne" kindred with TAA/AD and PDA excluded the previously known candidate genes and loci (Khau et al., 2004).

The inventors have now identified the TAA/AD and PDA disease locus at chromosome 16p13-p12. They have further demonstrated that the disease is caused by mutations in the *MYH11* gene, affecting the C-terminal coiled-coil tail of the smooth muscle myosin heavy chain (SM-MHC), a major contractile protein expressed in vascular smooth muscle cells (VSMC). Patients bearing the mutations are either affected or display a strongly reduced aortic compliance. Immuno-histological analyses of pathological tissues show large areas of disruption of elastic fibers, strong reduction in VSMC content and no SM-MHC expression.

Up to now, inherited aortic diseases have been associated with defects in proteins of the extracellular matrix (Nienaber et al., 2003). Human MYH11 gene mutations identified herein are the first examples of a direct alteration of VSMC leading to inherited arterial disease.

Based on these results, the inventors associate mutations in the *MYH11* gene or protein with a higher risk to develop an arterial disease.

Determining mutations in the *MYH11* gene or protein is thus useful to assess the risk of developing arterial disease, in particular inherited arterial disease, in a subject, as well as to diagnose or predict an arterial disease.

### Definitions

A *"coding sequence"* or a sequence *"encoding"* an expression product, such as a RNA, polypeptide, protein, or enzyme, is a nucleotide sequence that, when expressed, results in the production of that RNA, polypeptide, protein, or enzyme, i.e., the nucleotide sequence encodes an amino acid sequence for that polypeptide, protein or enzyme. A coding sequence for a protein may include a start codon (usually ATG) and a stop codon.

The term "gene" means a DNA sequence that codes for or corresponds to a particular sequence of amino acids which comprise all or part of one or more proteins or enzymes, and may or may not include regulatory DNA sequences, such as promoter sequences, which determine for example the conditions under which the gene is expressed. Some genes, which are not structural genes, may be transcribed from DNA to RNA, but are not translated into an amino acid sequence. Other genes may function as regulators of structural genes or as regulators of DNA transcription. In particular, the term gene may be intended for the genomic sequence encoding a protein, i.e. a sequence comprising regulator, promoter, intron and exon sequences.

As used herein, the term *"oligonucleotide"* refers to a nucleic acid, generally of at least 10, preferably at least 15, and more preferably at least 20 nucleotides, preferably no more than 100 nucleotides, still preferably no more than 70 nucleotides, and which is hybridizable to a MYH11 genomic DNA, cDNA, or mRNA. Oligonucleotides can be labelled according to any technique known in the art, such as with radiolabels, fluorescent labels, enzymatic labels, sequence tags, etc. A labelled oligonucleotide may be used as a probe to detect the presence of a mutated MYH11 nucleic acid. Alternatively, oligonucleotides (one or both of which may be labelled) can be used for amplifying a MYH11 nucleic acid, for instance by PCR (Saiki et al., 1988), to detect the presence of a mutation. Generally, oligonucleotides are prepared synthetically, preferably on a nucleic acid synthesizer. Accordingly, oligonucleotides can be prepared with non-naturally occurring phosphoester analog bonds, such as thioester bonds, etc.

A nucleic acid molecule is *"hybridizable"* to another nucleic acid molecule, such as a cDNA, genomic DNA, or RNA, when a single stranded form of the nucleic acid molecule can anneal to the other nucleic acid molecule under the appropriate conditions of temperature and solution ionic strength (see Sambrook et al., 1989).

The conditions of temperature and ionic strength determine the *"stringency"* of the hybridization. For preliminary screening for homologous nucleic acids, low stringency hybridization conditions, corresponding to a Tm (melting temperature) of 55°C, can be used, e.g., 5x SSC, 0.1 % SDS, 0.25 % milk, and no formamide ; or 30 % formamide, 5x SSC, 0.5 % SDS). Moderate stringency hybridization conditions correspond to a higher Tm, e.g., 40 % formamide, with 5x or 6x SCC. High stringency hybridization conditions correspond to the highest Tm, e.g., 50 % formamide, 5x or 6x SCC. SCC is a 0.15 M NaCl, 0.015 M Na-citrate. Hybridization requires that the two nucleic acids contain complementary sequences, although depending on the stringency of the hybridization, mismatches between bases are possible. The appropriate stringency for hybridizing nucleic acids depends on the length of the nucleic acids and the degree of complementation, variables well known in the art. The greater the degree of similarity or homology between two nucleotide sequences, the greater the value of Tm for hybrids of nucleic acids having those sequences. The relative stability (corresponding to higher Tm) of nucleic acid hybridizations decreases in the following order: RNA:RNA, DNA:RNA, DNA:DNA. For hybrids of greater than 100 nucleotides in length, equations for calculating Tm have been derived (see Sambrook et al., 1989, 9.50-9.51). For hybridization with shorter nucleic acids, i.e., oligonucleotides, the position of mismatches becomes more important, and the length of the oligonucleotide determines its specificity (see Sambrook et al., 1989 II.7-11.8). A minimum length for a hybridizable nucleic acid is at least about 10 nucleotides, preferably at least about 15 nucleotides, and more preferably the length is at least about 20 nucleotides.

In a specific embodiment, the term *"standard hybridization conditions"* refers to a Tm of 55°C, and utilizes conditions as set forth above. In a preferred embodiment, the Tm is 60°C. In a more preferred embodiment, the Tm is 65°C. In a specific embodiment, *"high stringency"* refers to hybridization and/or washing conditions at 68°C in 0.2 X SSC, at 42°C in 50 % formamide, 4 X SSC, or under conditions that afford levels of hybridization equivalent to those observed under either of these two conditions.

As used herein, an amplification primer is an oligonucleotide for amplification of a target sequence by extension of the oligonucleotide after hybridization to the target sequence or by ligation of multiple oligonucleotides which are adjacent when hybridized to the target sequence. At least a portion of the amplification primer hybridizes to the target. This portion is referred to as the target binding sequence and it determines the target-specificity of the primer. In addition to the target binding sequence, certain amplification methods require specialized non-target binding sequences in the amplification primer. These specialized sequences are necessary for the amplification reaction to proceed and typically serve to append the specialized sequence to the target. For example, the amplification primers used in Strand Displacement Amplification (SDA) include a restriction endonuclease recognition site 5' to the target binding sequence (US Patent No. 5,455,166 and US Patent No. 5,270,184). Nucleic Acid Based Amplification (NASBA), self-sustaining sequence replication (3SR) and transcription based amplification primers require an RNA polymerase promoter linked to the target binding sequence of the primer. Linking such specialized sequences to a target binding sequence for use in a selected amplification reaction is routine in the art. In contrast, amplification methods such as PCR which do not require specialized sequences at the ends of the target, generally employ amplification primers consisting of only target binding sequence.

As used herein, the terms *"primer"* and *"probe"* refer to the function of the oligonucleotide. A primer is typically extended by polymerase or ligation following hybridization to the target but a probe typically is not. A hybridized oligonucleotide may function as a probe if it is used to capture or detect a target sequence, and the same oligonucleotide may function as a primer when it is employed as a target binding sequence in an amplification primer. It will therefore be appreciated that any of the target binding sequences disclosed herein for amplification, detection or quantisation of MYH11 may be used either as hybridization probes or as target binding sequences in primers for detection or amplification, optionally linked to a specialized sequence required by the selected amplification reaction or to facilitate detection. As used herein, the term *"MYH1 gene"* denotes the smooth muscle myosin heavy polypeptide 11 gene of any species, especially human, but also other mammals or vertebrates to which the methods of the invention can apply. The MYH11 gene encodes the smooth muscle myosin heavy chain (SM-MHC). Unless otherwise indicated, the term "MYH11" is used indifferently to designate the MYH11 gene or the encoded protein SM-MHC throughout the text. Homo sapiens MYH11 gene is localized on chromosome 16, the sequence of which is deposited in Genebank under accession number AF001548 (Loftus et al., 1999). Homo sapiens MYH11 gene (5'-3' forward strand) together with its intron-exon structure are shown in the sequence SEQ ID No.1. The intron-exon structure of the complementary strand is further indicated in Table 1 below.

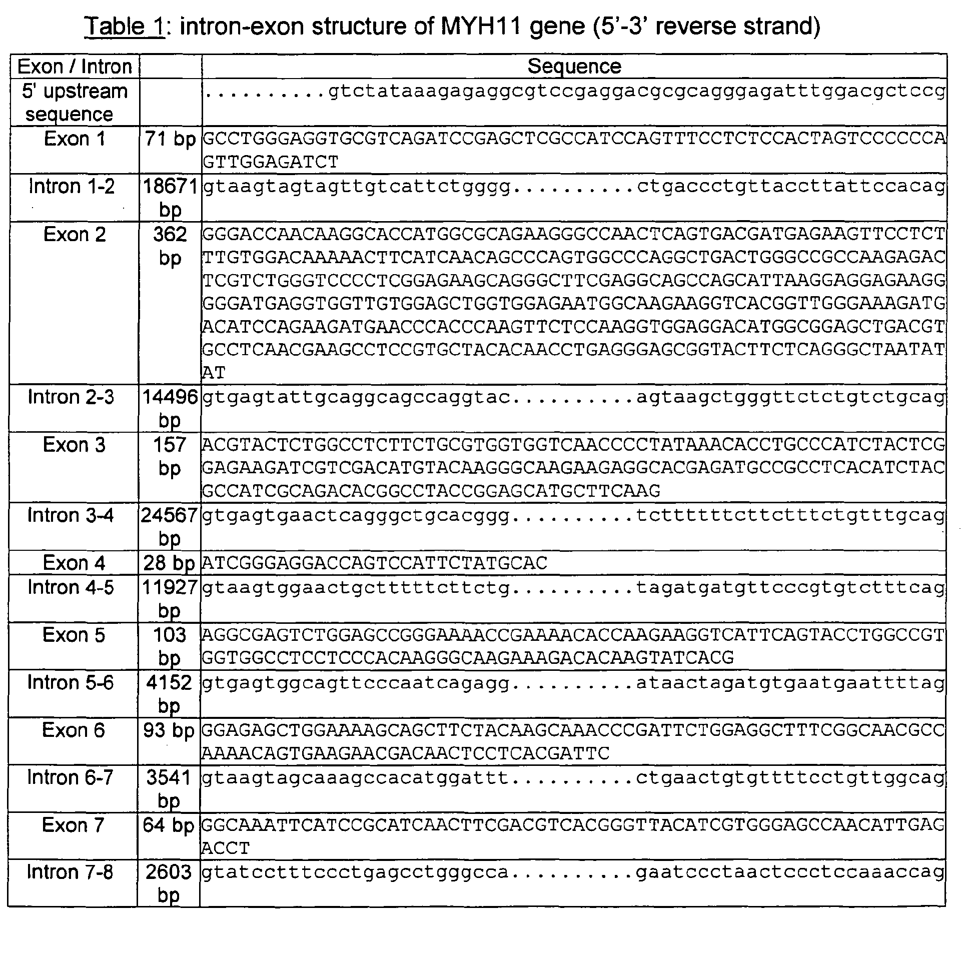

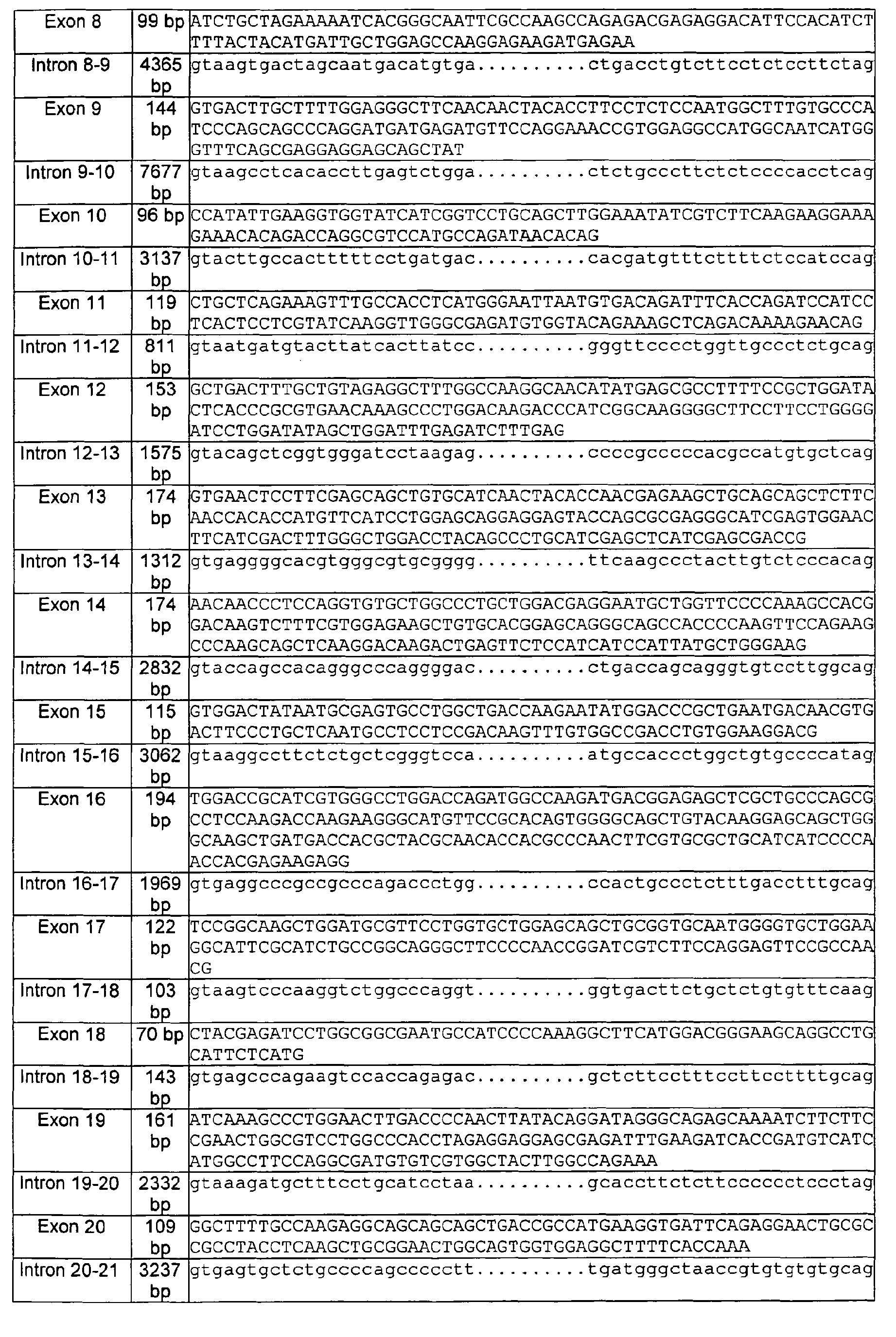

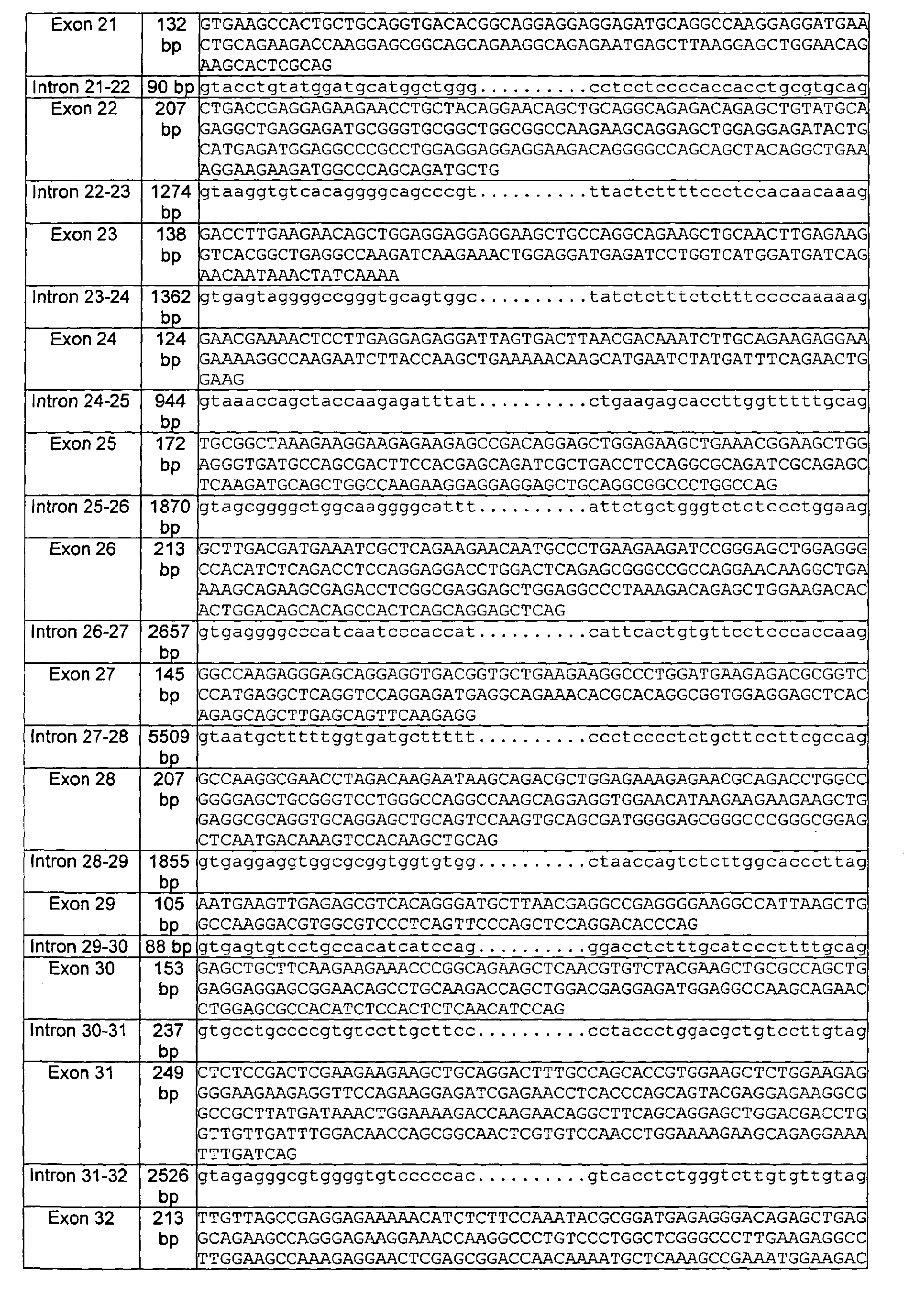

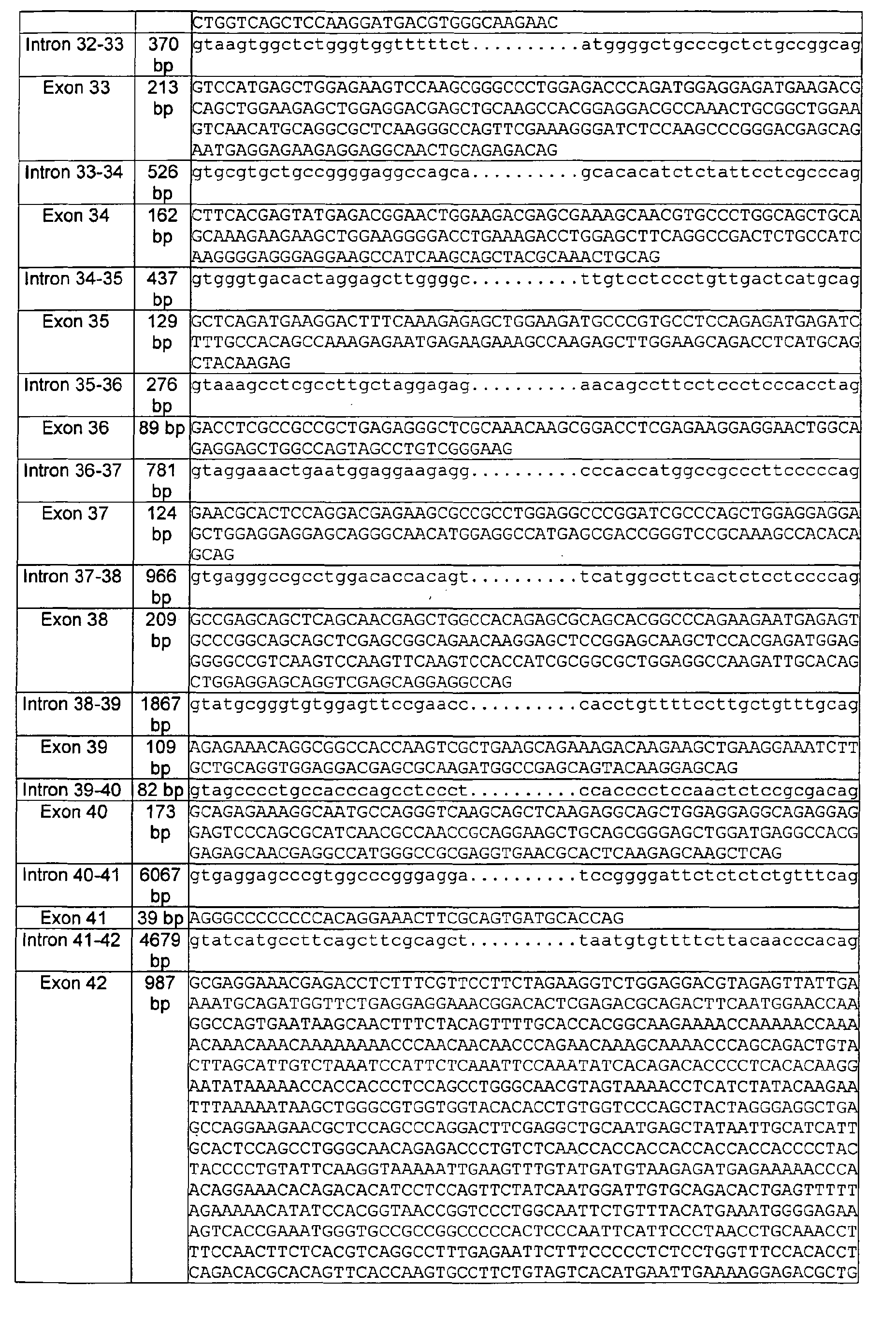

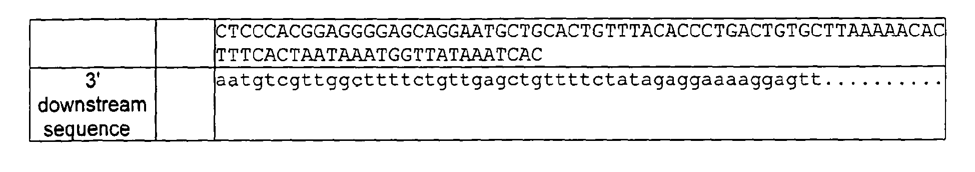

Two MYH11 transcript variants have been described. SM1 mRNA encodes the longer isoform of MYH11 and is deposited in GeneBank under accession numbers NM_002474 (SEQ ID No.2). The corresponding amino acid sequence is shown in SEQ ID No. 3). SM2 variant (NM_022844, SEQ ID No.4) includes an additional exon in the 3' coding region, resulting in the use of an alternate stop codon, compared to variant SM1. The encoded isoform (SM2, SEQ ID No.5) is shorter and varies in the carboxyl terminus compared to isoform SM1.

The terms *"mutant"* and *"mutation"* mean any detectable change in genetic material, e.g. DNA, RNA, cDNA, or any process, mechanism, or result of such a change. This includes gene mutations, in which the structure (e.g. DNA sequence) of a gene is altered, any gene or DNA arising from any mutation process, and any expression product (e.g. protein or enzyme) expressed by a modified gene or DNA sequence. Generally a mutation is identified in a subject by comparing the sequence of a nucleic acid or polypeptide expressed by said subject with the corresponding nucleic acid or polypeptide expressed in a control population. A mutation in the genetic material may also be "silent", i.e. the mutation does not result in an alteration of the amino acid sequence of the expression product.

In the context of the instant application, mutations identified in MYH11 gene or protein are designated pursuant to the nomenclature of Dunnen and Antonarakis (2000). As defined by Dunnen and Antonarakis at the nucleic acid level, substitutions are designated by ">", e.g. "G>T" denotes that at nucleotide 1997 of the reference sequence a G is changed to a T. Deletions are designated by "del" after the deleted interval (followed by the deleted nucleotides). For instance 1997-1999del (alternatively 1997-1999-deITTC) denotes a TTC deletion from nucleotides 1997 to 1999. A TG deletion in the sequence ACTGTGTGCC (A is nucleotide 1991) is designated as 1997-1998del (or 1997-1998delTG). Insertions are designated by "ins," followed by the inserted nucleotides. For example, 1997-1998insT denotes that a T was inserted between nucleotides 1997 and 1998. A TG insertion in the TG-tandem repeat sequence of ACTGTGTGCC (A is nucleotide 1991) is described as 1998-1999insTG (where 1998 is the last G of the TG-repeat). Intron mutations are designated by the intron number (preceded by "IVS") or cDNA position; positive numbers starting from the G of the GT splice donor site, whereas negative numbers starting from the G of the AG splice acceptor site. For instance IVS4-2A>C (1998-2A>C) denotes the A to C substitution at nt -2 of intron 4, at the cDNA level positioned between nucleotides 1997 and 1998. IVS4+1G>T (1997+1G>T) denotes the G to T substitution at nucleotide+1 of intron 4. When the full-length genomic sequence is known, the mutation is best designated by the nucleotide number of the genomic references.

The term *"arterial disease"* denotes a condition associated with an altered structure and/or function of an arteria, such as abnormalities of aortic media, reduction of aortic compliance. Said arterial disease is preferably an inherited arterial disease Examples of arterial diseases according to the invention include thoracic aortic aneurysm and/or aortic dissection (TAA/AD), patent *ductus arteriosus* (PAD), intracerebral aneurysm, fibromuscular dysplasia, cerebral arterial dissection, and carotid arterial dissection.

As used herein, the term *"subject"* denotes a mammal, such as a rodent, a feline, a canine, and a primate. Preferably a subject according to the invention is a human.

### Mutations in MYH11 gene and protein

The inventors identified various mutations in the MYH11 gene.

To make it simple, mutations located in exons are herein numbered according to a MYH11 coding sequence as shown in SEQ ID No.2, starting from nucleotide 1. Mutations located in introns are herein numbered according to a MYH11 genomic sequence as shown in SEQ ID No.1.

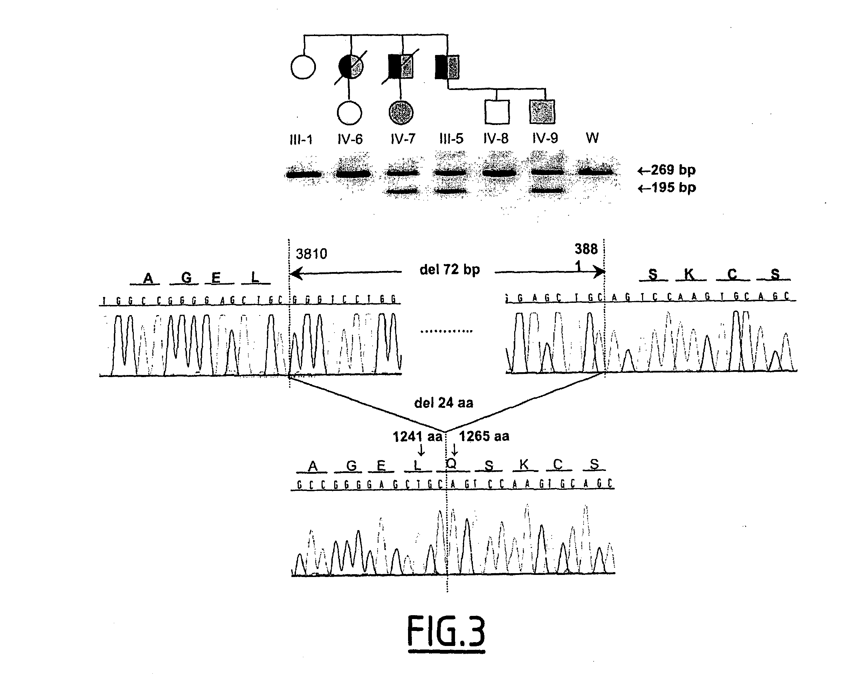

As shown in Figure 3, the inventors detected the presence of a new restriction site that is created by the mutation at the splice-donor site of intron 32 (IVS32+1G>T) (position 18285 of SEQ ID No.1). This mutation was further found to be associated with a missense mutation at exon 37 (5361 G>A) in the examined subjects (position 15201 of SEQ ID No.1).

The IVS32+1G>T mutation is localized in the splice-donor site of intron 32, as figured in the nucleotide reverse stretch 5' GGCAAGAAC**G**TAAGTGGCT 3' (SEQ ID No.6), wherein the underlined characters correspond to the 3' end of exon 32 and the character in bold designates the mutated nucleotide. This mutation results in skipping of exon 32 and thereby creates a junction between exon 31 and exon 33 such as shown in the reverse sequence 5' GGAAATTTGATCAGGTCCATGAGCTGG 3' (SEQ ID No.7) wherein the underlined characters correspond to the 3' end of exon 31 and the normal character correspond to the 5' end of exon 33.

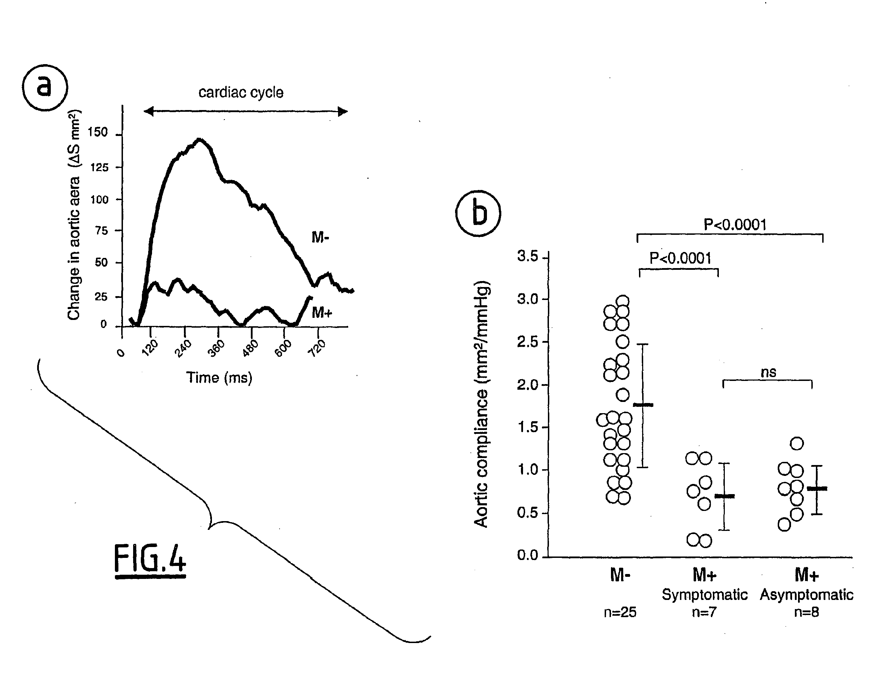

As shown in Figure 4, another mutation was identified by the inventors in MYH 11 gene. The latter consists in a deletion of nucleotides 3810-3881 from MYH 11 coding sequence (SEQ ID No.2 or 3) (or deletion of nucleotides 23777 to 23848 of SEQ ID No.1) which results in the creation of a junction contained in a polynucleotide stretch such as shown in the sequence 5' CCGGGGAGCTGC**A**GTCC**AA**GTGC**A**G 3' (SEQ ID No.10) wherein the underlined characters correspond to nucleotides between positions 3798 and 3809 and characters in bold correspond to nucleotides between positions 3882 and 3894, as shown in SEQ ID No.2.

A nucleic acid comprising a MYH11 nucleotide sequence, or a fragment thereof, carrying a mutation such as defined above is part of the invention.

Accordingly, the invention relates to an isolated nucleic acid encoding the smooth muscle myosin heavy chain, which nucleic acid comprises or consists in a MYH11 gene sequence that contains a mutation selected from the group consisting of IVS32+1G>T, 5361 G>A and 3810-3881 del. Said nucleic acid may contains one or more mutations. Preferably said nucleic acid comprises a MYH11 gene sequence carrying the mutation IVS32+1G>T and the 5361 G>A substitution.

It will be readily understood that a nucleic acid comprising a MYH11 gene sequence that comprises the IVS32+1G>T denotes either a MYH11 genomic sequence with a nucleotide T at the splice-donor site of intron 32, or a MYH11 mRNA or cDNA wherein the nucleotide sequence corresponding to exon 32 (SEQ ID No.8) is deleted.

The invention further relates to the polypeptide encoded by said nucleic acid. Mutations in MYH11 protein are herein numbered according to the MYH11 polypeptide sequence shown in SEQ ID No.5.

More specifically, the IVS32+1G>T mutation leads to an in-frame loss of 71 amino acids (L1456_N1526del); the in-frame deletion of nucleotides 3810-3881 causes the loss of 23 amino acids, i.e. a deletion of amino acids 1242 to 1264; and 5361G>A substitution results in an amino acid substitution R1758Q.

Accordingly, the invention further provides an isolated polypeptide which comprises or consists in the polypeptide sequence of MYH11 containing a mutation selected from the group consisting of a L1456_N1526 deletion, a deletion V1242_L1264, and a substitution R1758Q. Said polypeptide may contain one or more of the above mutations.

### Diagnostic method

The inventors have further shown that the mutant MYH11 haplotype, which was found identified in patients with thoracic aortic aneurysm and/or aortic dissection, is also associated with vascular abnormalities in asymptomatic subjects from the same kindreds. In particular, individuals harbouring a mutation in MYH11 gene, either symptomatic or asymptomatic, display a marked reduction of aortic compliance compared with a control population. Histological analyses further showed that MYH11 mutation correlates with an alteration of aortic wall structure as a dramatic decrease in vascular smooth muscle cell (VSMC) number and an alteration in extracellular matrix composition were observed. No expression of SM-MHC could be observed in the remaining VSMCs.

Mutations in MYH11 gene reported herein appears to be dominant negative mutations, i.e. that mutation in one allele leads to a structural change in the MYH11 protein that interferes with the function of the wild-type protein encoded by the other allele.

Therefore, the invention provides an *ex vivo* method of diagnosing or predicting an arterial disease, or a risk of arterial disease, in a subject, which method comprises detecting a mutation in MYH11 (myosin heavy polypeptide 11 of smooth muscle cell) gene or protein, as compared to a control population, wherein the presence of a mutation is indicative of an arterial disease or of a risk of arterial disease.

### Nucleic acid assays

According to a first embodiment, said mutation may be detected by analyzing a MYH11 nucleic acid molecule. In the context of the invention, MYH11 nucleic acid molecules include mRNA, genomic DNA and cDNA derived from mRNA. DNA or RNA can be single stranded or double stranded. These may be utilized for detection by amplification and/or hybridization with a probe, for instance.

Thus the invention provides an *ex vivo* method of diagnosing or predicting an arterial disease, or a risk of arterial disease, in a subject, which method may comprise the steps consisting of :
(a) obtaining a nucleic acid sample from the subject; and
(b) detecting a mutation of MYH11 gene in said nucleic acid sample;
wherein the presence of a mutation is indicative of an arterial disease or of a risk of arterial disease.

The nucleic acid sample may be obtained from any cell source or tissue biopsy. Non-limiting examples of cell sources available include without limitation blood cells, buccal cells, epithelial cells, fibroblasts, or any cells present in a tissue obtained by biopsy. Cells may also be obtained from body fluids, such as blood, plasma, serum, lymph, etc. DNA may be extracted using any methods known in the art, such as described in Sambrook et al., 1989. RNA may also be isolated, for instance from tissue biopsy, using standard methods well known to the one skilled in the art such as guanidium thiocyanate-phenol-chloroform extraction (Chomocyznski et al., 1987).

A MYH 11 mutation according to the invention may be found in a regulating region of MYH11 gene (e.g. a promoter sequence, or a binding site for transcription factor), in introns of MYH11 gene or in exons that encode MYH11 protein.

Preferably, said mutation is localised in the region of MYH11 gene that encodes the coiled-coil domain of MYH11 protein, i.e. the domain defined by amino acid residues 844 to 1934 of MYH11 polypeptide sequence shown in SEQ ID No.5. Still preferably, a mutation of MYH11 gene according to the invention is selected from the group consisting of a substitution IVS32+1G>T, a substitution 5361 G>A, and a deletion 3810-3881del. Mutations 5361G>A and 3810-3881del are positioned according to MYH11 coding sequence shown in SEQ ID No.2 or 3.

In particular, said IVS32+1G>T mutation may be identified either by detecting the IVS32+1G>T substitution in the genomic sequence of MYH11 (SEQ ID No.1), or by detecting the absence of exon 32 (SEQ ID No.9) in a MYH11 mRNA or cDNA, or the presence of an exon 31 and 33 junction (such as shown in SEQ ID No.8) in a MYH11 mRNA or cDNA.

MYH11 mutations may be detected in a RNA or DNA sample, preferably after amplification. For instance, the isolated RNA may be subjected to coupled reverse transcription and amplification, such as reverse transcription and amplification by polymerase chain reaction (RT-PCR), using specific oligonucleotide primers that are specific for a mutated site or that enable amplification of a region containing the mutated site. According to a first alternative, conditions for primer annealing may be chosen to ensure specific reverse transcription (where appropriate) and amplification; so that the appearance of an amplification product be a diagnostic of the presence of a particular MYH11 mutation. Otherwise, RNA may be reverse-transcribed and amplified, or DNA may be amplified, after which a mutated site may be detected in the amplified sequence by hybridization with a suitable probe or by direct sequencing, or any other appropriate method known in the art. For instance, a cDNA obtained from RNA may be cloned and sequenced to identify a mutation in MYH11 sequence.

Actually numerous strategies for genotype analysis are available (Antonarakis et al., 1989 ; Cooper et al., 1991 ; Grompe, 1993). Briefly, the nucleic acid molecule may be tested for the presence or absence of a restriction site. When a base substitution mutation creates or abolishes the recognition site of a restriction enzyme, this allows a simple direct PCR test for the mutation. Further strategies include, but are not limited to, direct sequencing, restriction fragment length polymorphism (RFLP) analysis; hybridization with allele-specific oligonucleotides (ASO) that are short synthetic probes which hybridize only to a perfectly matched sequence under suitably stringent hybridization conditions; allele-specific PCR; PCR using mutagenic primers; ligase-PCR, HOT cleavage; denaturing gradient gel electrophoresis (DGGE), temperature denaturing gradient gel electrophoresis (TGGE), single-stranded conformational polymorphism (SSCP) and denaturing high performance liquid chromatography (Kuklin et al., 1997). Direct sequencing may be accomplished by any method, including without limitation chemical sequencing, using the Maxam-Gilbert method ; by enzymatic sequencing, using the Sanger method ; mass spectrometry sequencing ; sequencing using a chip-based technology (see e.g. Little et al., 1996); and real-time quantitative PCR. Preferably, DNA from a subject is first subjected to amplification by polymerase chain reaction (PCR) using specific amplification primers. However several other methods are available, allowing DNA to be studied independently of PCR, such as the rolling circle amplification (RCA), the Invader™ assay, or oligonucleotide ligation assay (OLA). OLA may be used for revealing base substitution mutations. According to this method, two oligonucleotides are constructed that hybridize to adjacent sequences in the target nucleic acid, with the join sited at the position of the mutation. DNA ligase will covalently join the two oligonucleotides only if they are perfectly hybridized (Nickerson et al., 1990).

Therefore, useful nucleic acid molecules, in particular oligonucleotide probes or primers, according to the present invention include those which specifically hybridize :
a) to MYH11 sequences in the region of the splice-donor site of intron 32 (SEQ ID No.7), or of the junction of exons 31 and 33 as shown in SEQ ID No.8, or to exon 32 (SEQ ID No.9) or fragments thereof; or
b) to a region of MYH11 nucleic acid (e.g. genomic DNA, cDNA or mRNA) that contains the nucleotide in position 5361 according to a sequence such as shown in SEQ ID No. 2 or 3; or
c) to a region of MYH11 nucleic acid consisting of or comprising nucleotides 3810-3881 (SEQ ID No.11), or to the junction created by deletion of nucleotides 3810-3881 from MYH11 as shown in SEQ ID No. 10.

Thus, according to the method of the invention, the nucleic acid sample may be contacted with an oligonucleotide which specifically hybridizes to a junction of exons 31 and 33 of MYH11 as shown in SEQ ID No.8, wherein hybridization of the oligonucleotide to said nucleic acid sample is indicative of the presence of a substitution IVS32+1G>T and therefore of an arterial disease or of a risk of arterial disease.

Alternatively, said method may comprise contacting the nucleic acid sample with an oligonucleotide which specifically hybridizes to exon 32 of MYH11 as shown in SEQ ID No.9, wherein non hybridization of the oligonucleotide to said nucleic acid sample is indicative of the presence of a substitution IVS32+1G>T and therefore of an arterial disease or of a risk of arterial disease.

The method of the invention may further comprise contacting the nucleic acid sample with an oligonucleotide which specifically hybridizes to the junction created by deletion of nucleotides 3810-3881 from MYH11 as shown in SEQ ID No.9, wherein hybridization of the oligonucleotide to said nucleic acid sample is indicative of the presence of a deletion 3810-3881 del and therefore of an arterial disease or of a risk of arterial disease.

### Protein assays

According to a second embodiment said mutation may be detected in MYH11 protein.

In particular, said mutation may be localised in the coiled-coil domain of MYH11 protein. This domain corresponds to amino acid residues 844 to 1934 of the MYH11 polypeptide sequence shown in SEQ ID No.5.

The IVS32+1G>T mutation, which results in skipping of exon 32, leads to an in-frame loss of 71 amino acids (L1456_N1526del). Therefore, a MYH11 mutation may be identified by detecting the absence of amino acid residues 1456 to 1526 (SEQ ID No.12) from the amino acid sequence of MYH11 (SEQ ID No.5).

Furthermore, the in-frame deletion of nucleotides 3810-3881 that has been detected in exon 28 causes the loss of 23 amino acids (V1242_L1264del, the sequence defined by amino acids 1242 to 1264 being shown in SEQ ID No.13) in the C-terminus of the MYH11 protein.

As to substitution 5361 G>A, it is a missense mutation that leads to the amino acid substitution R1758Q.

Accordingly, a mutation of MYH11 protein according to the invention is preferably selected from the group consisting of L1456_N1526del, V1242_L1264del, and R1758Q.

Said mutation may be detected according to any appropriate method known in the art. In particular a sample, such as a tissue biopsy, obtained from a subject may be contacted with antibodies specific of the mutated form of MYH11 protein, i.e. antibodies that are capable of distinguishing between a mutated form of MYH11 and the wild-type protein (or any other protein), to determine the presence or absence of a MYH11 specified by the antibody.

Antibodies that specifically recognize a mutated MYH11 protein also make part of the invention. The antibodies are specific of mutated MYH11 protein, that is to say they do not cross-react with the wild-type MYH11 protein.

The antibodies of the present invention may be monoclonal or polyclonal antibodies, single chain or double chain, chimeric antibodies, humanized antibodies, or portions of an immunoglobulin molecule, including those portions known in the art as antigen binding fragments Fab, Fab', F(ab')₂ and F(v). They can also be immunoconjugated, e.g. with a toxin, or labelled antibodies.

Whereas polyclonal antibodies may be used, monoclonal antibodies are preferred for they are more reproducible in the long run.

Procedures for raising *"polyclonal antibodies"* are also well known. Polyclonal antibodies can be obtained from serum of an animal immunized against the IgA receptor or the IgA/gliadin peptide complex, which may be produced by genetic engineering for example according to standard methods well-known by one skilled in the art. Typically, such antibodies can be raised by administering mutated MYH11 protein subcutaneously to New Zealand white rabbits which have first been bled to obtain pre-immune serum. The antigens can be injected at a total volume of 100 µl per site at six different sites. Each injected material may contain adjuvants with or without pulverized acrylamide gel containing the protein or polypeptide after SDS-polyacrylamide gel electrophoresis. The rabbits are then bled two weeks after the first injection and periodically boosted with the same antigen three times every six weeks. A sample of serum is then collected 10 days after each boost. Polyclonal antibodies are then recovered from the serum by affinity chromatography using the corresponding antigen to capture the antibody. This and other procedures for raising polyclonal antibodies are disclosed by Harlow et al. (1988) which is hereby incorporated in the references.

A *"monoclonal antibody'* in its various grammatical forms refers to a population of antibody molecules that contains only one species of antibody combining site capable of immunoreacting with a particular epitope. A monoclonal antibody thus typically displays a single binding affinity for any epitope with which it immunoreacts. A monoclonal antibody may therefore contain an antibody molecule having a plurality of antibody combining sites, each immunospecific for a different epitope, e.g. a bispecific monoclonal antibody. Although historically a monoclonal antibody was produced by immortalization of a clonally pure immunoglobulin secreting cell line, a monoclonally pure population of antibody molecules can also be prepared by the methods of the present invention.

Laboratory methods for preparing monoclonal antibodies are well known in the art (see, for example, Harlow et al., 1988). Monoclonal antibodies (mAbs) may be prepared by immunizing purified mutated MYH11 protein into a mammal, e.g. a mouse, rat, human and the like mammals. The antibody-producing cells in the immunized mammal are isolated and fused with myeloma or heteromyeloma cells to produce hybrid cells (hybridoma). The hybridoma cells producing the monoclonal antibodies are utilized as a source of the desired monoclonal antibody. This standard method of hybridoma culture is described in Kohler and Milstein (1975).

While mAbs can be produced by hybridoma culture the invention is not to be so limited. Also contemplated is the use of mAbs produced by an expressing nucleic acid cloned from a hybridoma of this invention. That is, the nucleic acid expressing the molecules secreted by a hybridoma of this invention can be transferred into another cell line to produce a transformant. The transformant is genotypically distinct from the original hybridoma but is also capable of producing antibody molecules of this invention, including immunologically active fragments of whole antibody molecules, corresponding to those secreted by the hybridoma. See, for example, U.S. Pat. No. 4,642,334 to Reading; PCT Publication No.; European Patent Publications No. 0239400 to Winter et al. and No. 0125023 to Cabilly et al.

Antibody generation techniques not involving immunisation are also contemplated such as for example using phage display technology to examine naive libraries (from non-immunised animals); see Barbas et al. (1992), and Waterhouse et al. (1993).

Antibodies raised against mutated MYH11 protein may be cross reactive with wild-type MYH11 protein. Accordingly a selection of antibodies specific for mutated MYH11 protein is required. This may be achieved by depleting the pool of antibodies from those that are reactive with the wild-type MYH11 protein, for instance by submitting the raised antibodies to an affinity chromatography against wild-type MYH 11 protein.

Alternatively, binding agents other than antibodies may be used for the purpose of the invention. These may be for instance aptamers, which are a class of molecule that represents an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by EXponential enrichment (SELEX) of a random sequence library, as described in Tuerk C. and Gold L., 1990. The random sequence library is obtainable by combinatorial chemical synthesis of DNA. In this library, each member is a linear oligomer, eventually chemically modified, of a unique sequence. Possible modifications, uses and advantages of this class of molecules have been reviewed in Jayasena S.D., 1999. Peptide aptamers consists of a conformationally constrained antibody variable region displayed by a platform protein, such as E. coli Thioredoxin A that are selected from combinatorial libraries by two hybrid methods (Colas et al., 1996).

### Kits

According to another aspect of the invention, the MYH11 mutation is detected by contacting the DNA of the subject with a nucleic acid probe, which is optionally labeled.

Primers may also be useful to amplify or sequence the portion of the MYH11 gene containing the mutated positions of interest.

Such probes or primers are nucleic acids that are capable of specifically hybridizing with a portion of the MYH11 gene sequence containing the mutated positions of interest. That means that they are sequences that hybridize with the nucleic acid sequence to which it refers under conditions of high stringency.

The present invention further provides kits suitable for determining at least one of the mutations of the MYH11 gene.

The kits may include the following components :
(i) a probe, usually made of DNA, and that may be pre-labelled. Alternatively, the probe may be unlabelled and the ingredients for labelling may be included in the kit in separate containers ; and
(ii) hybridization reagents : the kit may also contain other suitably packaged reagents and materials needed for the particular hybridization protocol, including solid-phase matrices, if applicable, and standards.

In another embodiment, the kits may include :
(i) sequence determination or amplification primers : sequencing primers may be pre-labelled or may contain an affinity purification or attachment moiety ; and
(ii) sequence determination or amplification reagents : the kit may also contain other suitably packaged reagents and materials needed for the particular sequencing amplification protocol. In one preferred embodiment, the kit comprises a panel of sequencing or amplification primers, whose sequences correspond to sequences adjacent to at least one of the polymorphic positions, as well as a means for detecting the presence of each polymorphic sequence.

In a particular embodiment, it is provided a kit which comprises a pair of nucleotide primers specific for amplifying all or part of the MYH11 gene comprising at least one of mutated that are identified herein, especially positions 5361 and 3810 to 3881 of SEQ ID No.2 and position IVS32+1G>T.

### Therapeutic methods

The inventors have demonstrated that the mutations identified in the MYH11 gene directly affect vascular smooth muscle cell function, thereby altering vascular wall structure and leading to the development of an arterial disease. These results identify mutated MYH11 gene, as well as vascular smooth muscle cells, as targets for the preventive or curative treatment of an arterial disease.

Thus the invention further relates to a method of treatment of an arterial disease which comprises the step of administering a subject in need thereof with a MYH11 polynucleotide, i.e. a nucleic acid sequence that encodes a wild-type MYH11 protein, so that MYH11 protein is expressed in vivo by the cells of the subject that have been transfected with said polynucleotide. Accordingly, said method leads to an overexpression of wild-type MYH11 which compensates expression of defective mutated MYH11 protein.

The invention also relates to the use of a MYH11 polynucleotide for the manufacture of medicament intended for the treatment of an arterial disease.

In the context of the invention, the term "treating" or "treatment", as used herein, means reversing, alleviating, inhibiting the progress of, or preventing the disorder or condition to which such term applies, or one or more symptoms of such disorder or condition.

Preferably said MYH11 polynucleotide is administered in a therapeutically effective amount. A "therapeutically effective amount" is intended for a minimal amount of active agent (e.g., MYH11 polynucleotide) which is necessary to impart therapeutic benefit to a subject. For example, a "therapeutically effective amount" to a mammal is such an amount which induces, ameliorates or otherwise causes an improvement in the pathological symptoms, disease progression or physiological conditions associated with or resistance to succumbing to a disorder.

The administered polynucleotide comprises the nucleotide sequence SEQ ID No.2 or SEQ ID No.4, or any homologous or similar sequence as defined below:
a) a sequence showing at least 70%, preferably at least 75% or 80% or 85% or 90% or 95% or 99%, sequence similarity with SEQ ID No.2 or SEQ ID No.4;
b) a sequence hybridizing with SEQ ID No.2 or SEQ ID No.4, or its complementary sequence, under stringent conditions;
c) a sequence encoding a protein of sequence SEQ ID No.3 or SEQ ID No.5, or any sequence substantially similar with SEQ ID No.3 or SEQ ID No.5.

The term "sequence similarity" in all its grammatical forms refers to the degree of identity or correspondence between nucleic acid or amino acid sequences of proteins that may or may not share a common evolutionary origin. Preferably the degree of sequence identity is calculated compared with the totality of a reference sequence.

In a specific embodiment, two DNA sequences are "substantially homologous" or "substantially similar" when at least 70%, preferably at least 75% or 80% or 85% or 90% or 95% or 99%, of the nucleotides match over the defined length of the DNA sequences, as determined by sequence comparison algorithms, such as BLAST, FASTA, DNA Strider, etc. An example of such a sequence is an allelic or species variant of MYH11 gene. Sequences that are substantially homologous can be identified by comparing the sequences using standard software available in sequence data banks, or in a Southern hybridization experiment under, for example, stringent conditions as defined for that particular system.

Similarly, in a particular embodiment, two amino acid sequences are "'substantially similar" when greater than 80%, preferably than 85% or 90% or 95% or 99%, of the amino acids are similar (functionally identical). "Functionally identical" polypeptides are those in which a given amino acid residue has been changed without altering the overall conformation and function of the polypeptide, including, but not limited to, replacement of an amino acid with one having similar properties (such as, for example, polarity, hydrogen bonding potential, acidic, basic, hydrophobic, aromatic, and the like). Amino acids with similar properties are well known in the art. For example, arginine, histidine and lysine are hydrophilic-basic amino acids and may be interchangeable. Similarly, isoleucine, a hydrophobic amino acid, may be replaced with leucine, methionine or valine. Such changes are expected to have little or no effect on the apparent molecular weight or isoelectric point of the protein or polypeptide. Preferably, the similar sequences are identified by alignment using, for example, the GCG (Genetics Computer Group, Program Manual for the GCG Package, Version 7, Madison, Wisconsin) pileup program, or any of the programs described above (BLAST, FASTA, etc.).

Preferably the MYH11 polynucleotide sequence according to the invention is associated with elements that enable for regulation of its expression, such as a promoter sequence.

Such a nucleic acid may be in the form of a DNA vector. The terms "vector" means the vehicle by which a DNA or RNA sequence (e.g. a foreign gene) can be introduced into a host cell, so as to transform the host and promote expression (e.g. transcription and translation) of the introduced sequence. A common type of vector is a "plasmid", which generally is a self-contained molecule of double-stranded DNA, usually of bacterial origin, that can readily accept additional (foreign) DNA and which can readily introduced into a suitable host cell. A plasmid vector often contains coding DNA and promoter DNA and has one or more restriction sites suitable for inserting foreign DNA.

The MYH11 polynucleotide may be introduced into a target cell by means of any procedure known for the delivery of nucleic acids to the nucleus of cells, *ex vivo,* on cells in culture or removed from an animal or a patient, or *in vivo.*

*Ex vivo* introduction may be performed by any standard method well known by one skilled in the art, e.g. transfection, electroporation, microinjection, transduction, cell fusion, DEAE dextran, calcium phosphate precipitation, or use of a gene gun.

The MYH11 polynucleotide can also be introduced *ex vivo* or *in vivo* by lipofection. In certain embodiments, the use of liposomes and/or nanoparticles is contemplated for the introduction of the donor nucleic acid targeting system into host cells.

Nanocapsules can generally entrap compounds in a stable and reproducible way. To avoid side effects due to intracellular polymeric overloading, such ultrafine particles (sized around 0.1 µm) should be designed using polymers able to be degraded *in vivo.* Biodegradable polyalkyl-cyanoacrylate nanoparticles that meet these requirements are contemplated for use in the present invention, and such particles may be are easily made.

Liposomes are formed from phospholipids that are dispersed in an aqueous medium and spontaneously form multilamellar concentric bilayer vesicles (also termed multilamellar vesicles (MLVs)). MLVs generally have diameters of from 25 nm to 4 µm. Sonication of MLVs results in the formation of small unilamellar vesicles (SUVs) with diameters in the range of 200 to 500 A, containing an aqueous solution in the core.

Synthetic cationic lipids designed to limit the difficulties and dangers encountered with liposome mediated transfection can be used to prepare liposomes for *in vivo* transfection of a gene encoding a marker (Mackey, et al., 1988). The use of cationic lipids may promote encapsulation of negatively charged nucleic acids, and also promote fusion with negatively charged cell membranes (Felgner et al., 1989).

Alternatively, one of the simplest and the safest way to deliver MYH11 polynucleotide across cell membranes *in vivo* may involve the direct application of high concentration free or naked polynucleotides (typically mRNA or DNA). By "naked DNA (or RNA)" is meant a DNA (RNA) molecule which has not been previously complexed with other chemical moieties. Naked DNA uptake by animal cells may be increased by administering the cells simultaneously with excipients and the nucleic acid. Such excipients are reagents that enhance or increase penetration of the DNA across cellular membranes and thus delivery to the cells delivery of the therapeutic agent. Various excipients have been described in the art, such as surfactants, e.g. a surfactant selected form the group consisting of Triton X-100, sodium dodecyl sulfate, Tween 20, and Tween 80; bacterial toxins, for instance streptolysin O, cholera toxin, and recombinant modified labile toxin of E coli; and polysaccharides, such as glucose, sucrose, fructose, or maltose, for instance, which act by disrupting the osmotic pressure in the vicinity of the cell membrane. Other methods have been described to enhance delivery of free polynucleotides, such as blocking of polynucleotide inactivation via endo- or exonucleolytic cleavage by both extra- and intracellular nucleases.

The above methods do not limit the scope of the invention and it is to be understood that the one skilled in the art may readily make use of any other known appropriate methods for delivering a nucleic acid to a cell *in vivo* or *in vitro.*

The invention will be further illustrated by the following figures and examples.

### FIGURES

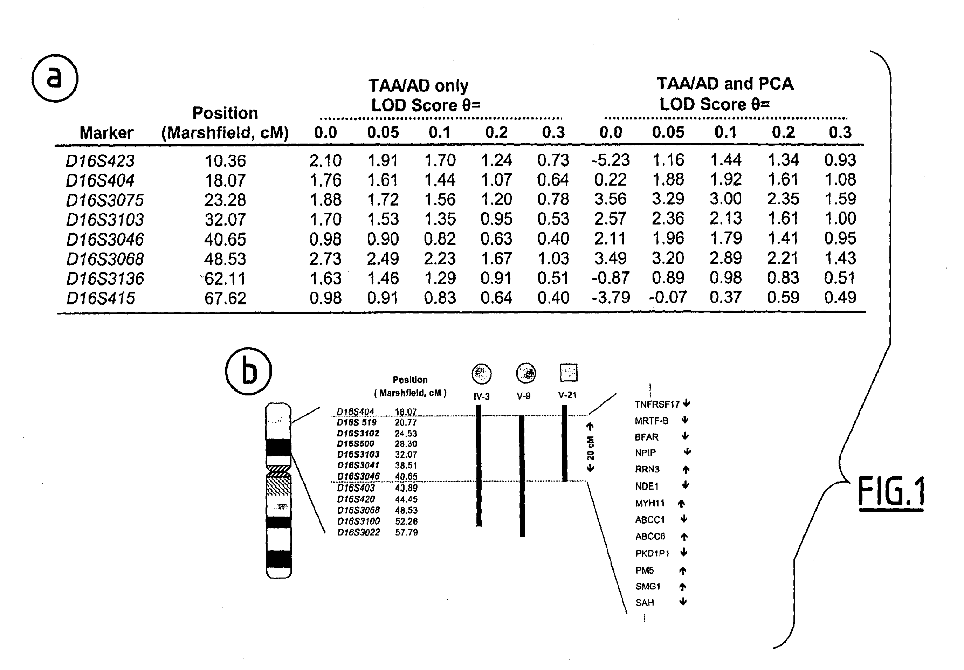

Figure 1 shows (a) the results of a two-point linkage analysis at chromosome 16p obtained including only TAA/AD (left part) or both TAA/AD and PDA (right part); (b) identification of the disease locus at chromosome 16p in the French kindred. Affected individuals with the most informative recombinant haplotypes locate the defective locus to *16 p13.13-p12.3,* between markers D16s519 and D16S3046. The major candidate genes within this 20cM interval are indicated. The arrows indicate the direction of transcription.

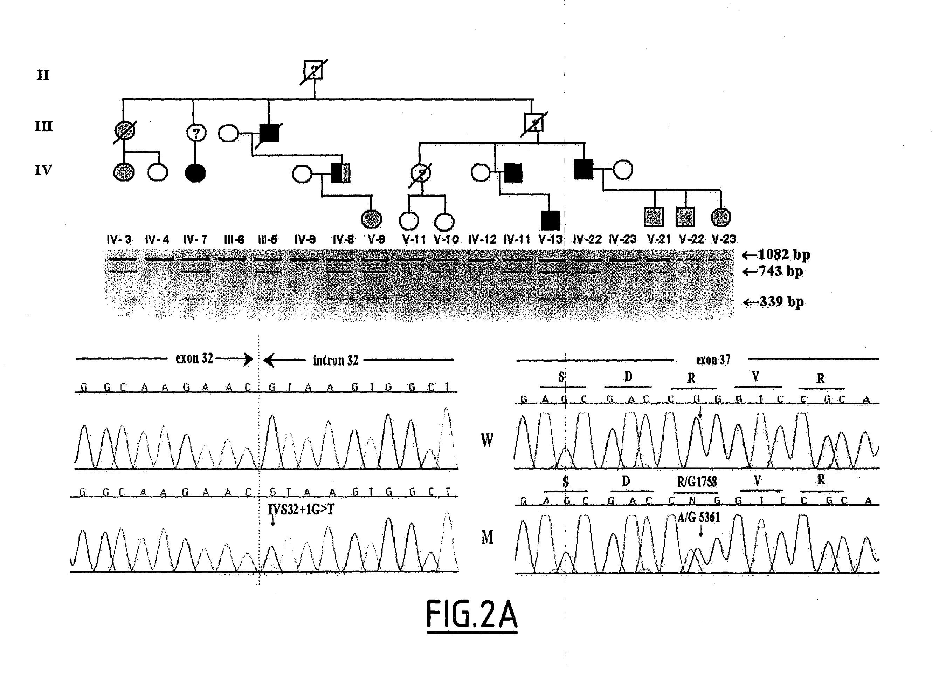

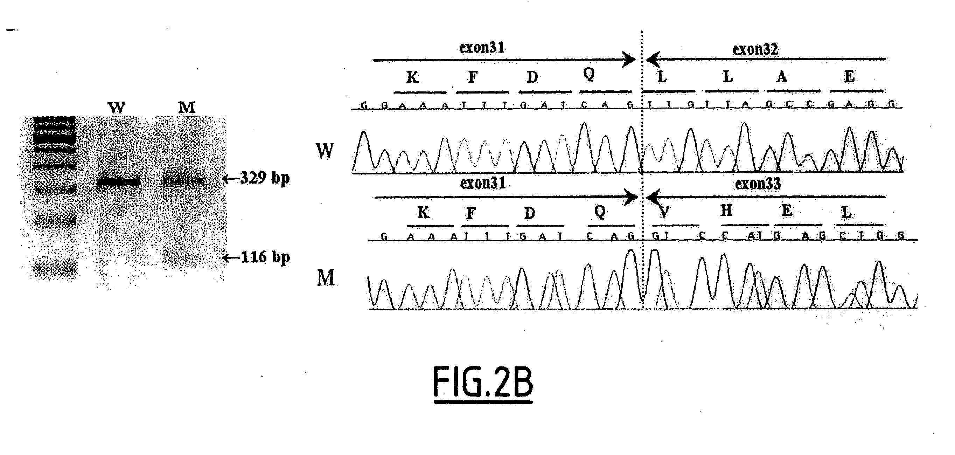

Figure 2 depicts the partial structure of the French kindred and identification of *MYH11* mutations in the French kindred.
a. Unaffected and affected individuals are shown as unfilled, and filled symbols depending on their status : TAA/AD (black), PDA (grey). Unknown phenotype is indicated by "?".
   The gel electrophoresis shows the absence (1082 bp amplification product) or the presence (743 bp + 339 bp) of a new A*f*lll restriction site that is created by the mutation at the splice-donor site of intron 32 (IVS32+1G>T). Subject V-10 is mutated but asymptomatic. *Left* : electrophoregrams of a wild-type (W) and a mutated (M) subject for the heterozygous splice-donor site of intron 32 (IVS32+1 G>T) mutation. *Right :* electrophoregrams of a wild-type (W) and a mutated (M) subject for the heterozygous missense mutation at exon 37 (R1758Q). This additional mutation was present in all individuals bearing the first mutation.
b. Consequence of the splice-donor (IVS32+1 G>T) mutation : skipping of exon 32. Partial *MYH11* cDNA amplification was performed using total RNA extracted from cultured fibroblasts of one affected individual (IV-11, M) and one control (W). The expected 329-bp wild-type (W) fragment was obtained in the control, but an additional 116 fragment in the mutated (M) subject. Direct sequencing the W and M fragments demonstrates the skipping of exon 32 (213-bp) leading to an in-frame loss of 71 aa (L 1456_N1526del).

Figure 3 illustrates identification of *MYH11* mutation in the American kindred. The symbols used for the structure of the American kindred are the same as those used in Figure 1. An in-frame deletion was detected at exon 28. Amplification products show a 72 bp deletion at the heterozygous state. Direct sequencing of the wild-type (above) and mutant (below) alleles show a 72-bp deletion (3810-3881 bp), causing the loss of 24 aminoacids (1242-1264aa) in the C-terminus of the protein.

Figure 4 illustrates the decreased aortic compliance in affected subjects.
a. Changes of the cross-sectional area (AS) of the ascending aorta during the cardiac cycle. The grey line corresponds to a typical result obtained in a wild-type subject (M-; subject V-15), the black line corresponds to that obtained in a mutated asymptomatic subject (M+; subject V-17).
   Despite similar diastolic aortic diameter, the asymptomatic M+ subject had a markedly reduced systolic dilatation of the thoracic aorta ΔS = 29 mm² compared to 148 mm² for subject M-) and strong alteration of the aortic compliance (0.45 vs 2.96 mm²/mmHg, respectively). These two subjects were similar for variables known to influence aortic compliance : age (25 vs 24 years), body surface area (1.72 vs 1.74 m²), blood pressure (123/59 vs 116/66 mmHg), absence of tobacco exposure, dyslipidemia and diabetes *mellitus.*
b. Ascending aortic compliance values in non-mutated subjects (M-), mutated subjects with TAA/AD or PDA (M+, symptomatic), and asymptomatic mutated subjects (M+, asymptomatic).

For each group, mean values and standard deviation are indicated on the right of the individual values. Groups did not differ for blood pressure. M+ symptomatic were slightly older (38 +/-12 years) than M- (33 +/-12 years) and M+ asymptomatic subjects (30+/-12 years). M- and M+ asymptomatic groups did not differ for sex ratio (0.68 vs 0.625, NS), age (33+/-12 vs 30+/-12 years, NS), systolic blood pressure (118+/-10 vs 122+/-12 mmHg, NS), diastolic blood pressure (68+/-10 *vs* 67+/-7 mmHg, NS), and aortic diastolic diameter at the level of the ascending aorta (28.0+/-3.2 vs 28.1+/-4.1 mm, NS).

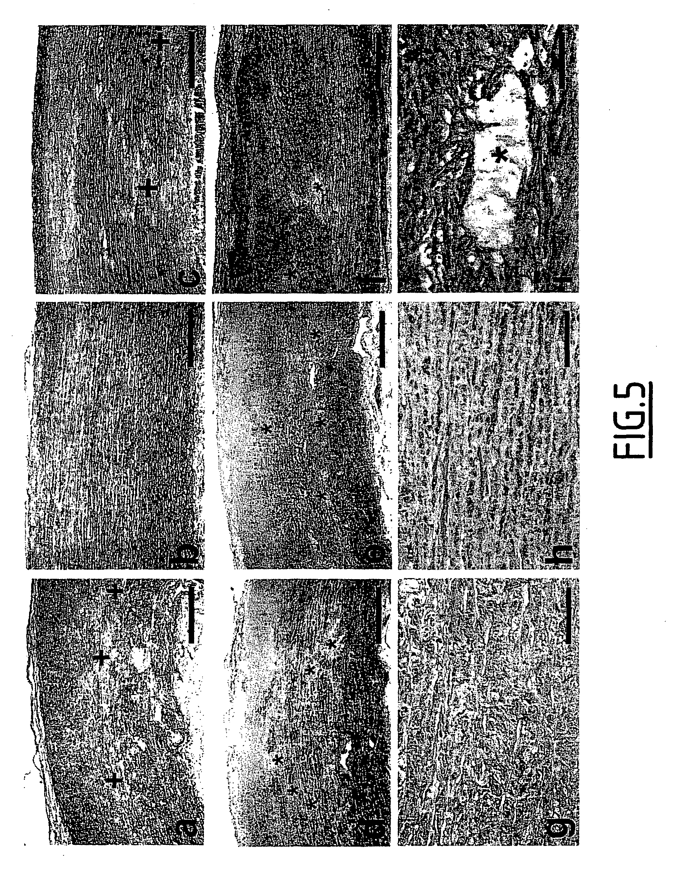

Figure 5 illustrates the structural abnormalities observed in the aortic tissue
a: Major decrease in elastic fibres with a patchy pattern (+) in the media of one affected subject with TAA (Elastic stain; bar = 900 µm).
b: Dense and concentric pattern of elastic fibres in the media of a normal control aorta. (Elastic stain; bar = 900 µm).
c: α-smooth muscle actin labelling emphasizes the loss of vascular smooth muscle cells within the media resulting in patchy decellularized areas (*).(Bar = 900 µm).
d : Cellular loss in the media is confirmed by vimentin labelling showing areas (*) where the vascular smooth muscle cells are not replaced by any other interstitial cell type. (*).(Bar = 900 µm).
e : In other areas, the loss of vascular smooth muscle cells exhibits a pattern of laminar necrosis appearing as a band with no α-smooth muscle actin labelling (*) in the media of the second tissue from patient with TAA/AD. (Bar = 450 µm).
f: : In addition to a decreased number, the elastic fibres are disrupted (+). In areas with a laminar necrosis pattern, the elastic fibres are squeezed together. This is resulting from the collapse of lamellar units (arrows). (Elastic stain; bar = 450 µm).
g: The vascular smooth muscle cells exhibit an abnormal phenotype characterized by the absence of immunohistochemical labelling with anti-smooth muscle myosin heavy chain antibody (case of TAA patient). (Bar = 225 µm).
h: Normal pattern of brown labelling with smooth muscle myosin antibody in the media of a normal control aorta. (Bar = 225 µm).
i: Normal smooth muscle myosin heavy chain expression (brown labelling) in the vascular smooth muscle cells of the media in a Marfan's syndrome control patient aorta. Note the typical pattern of medial cystic necrosis (*). (Bar = 225 µm).

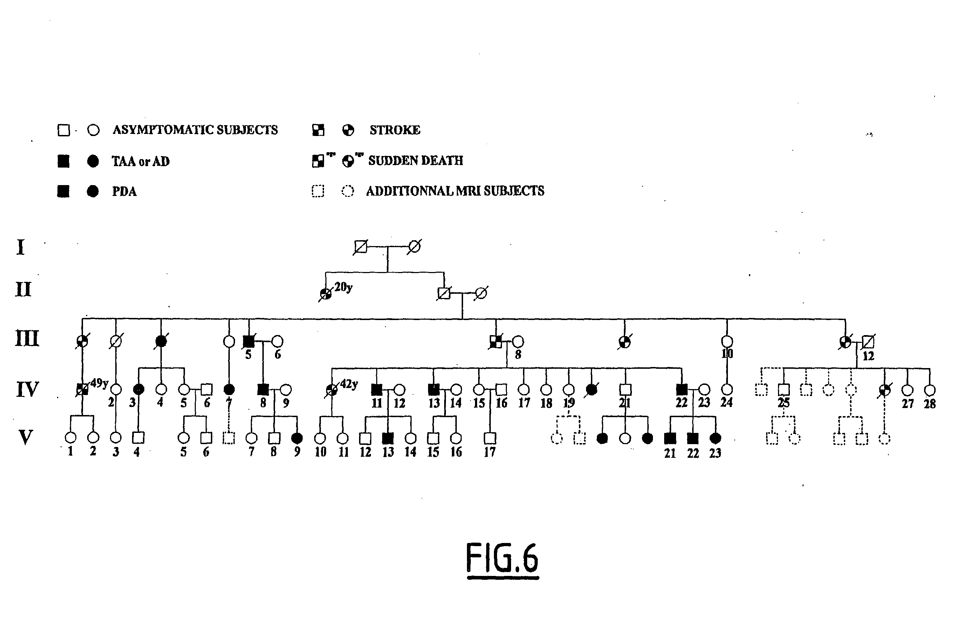

Figure 6 depicts the French "Bourgogne" pedigree

Details on the phenotype of affected subjects are available in Khau van Kien, P. et al (2004).
° indicates individuals enrolled in the genome wide scan.
* indicates individuals enrolled in the MRI assessment of aortic compliance.
+/+ indicates the wild type genotype at the MYH11 gene
+/- indicates subjects heterozygous for the MYH11 mutation

### EXAMPLES

### Example 1: Methods

### Subjects

40 members of the French "Bourgogne" family and 6 members of the American family were enrolled in this study as described before Glancy et al., (2001) and Khau et al. (2004). In addition to classical cardiological work-up, subjects were evaluated at least one time for the aortic root measurements with trans-thoracic echocardiography as described by Roman et al. (1989). Written informed consent from each participant or from the parents of younger children was obtained and this study was approved by local ethical committees.

### Genome-wide screen and linkage analysis

Genomic DNA was isolated from peripheral blood. Two phenotypes, TAA/AD only or TAA/AD and PDA were investigated. The genome wide scan was performed at the Centre National de Genotypage with 382 microsatellite markers distributed with an average spacing of 10 cM (ABI Prism Linkage Mapping Set 2; Applied Biosystems, Foster City, CA, USA). MLINK from LINKAGE software package (version 5.2) was used for two-point linkage analysis (Lathrop et al., 1984). Classical affected-only linkage analysis was performed under a dominant model of inheritance, as described previously (Khau et al. 2004). For this analysis, 8 TAA/AD, 5 PDA and 2 subjects with TAA/AD and PDA on 3 generations were considered (Supplementary Figure 1). Multipoint analysis was performed using the GENEHUNTER package (Kruglyak et al., 1996). Markers within the *16p13.13-p12.1* region that were used for refine mapping are indicated (Fig. 1). Eight of them belong to ABI PRISM® Linkage Mapping Set V2.5 (Applied Biosystems), the remaining 3 markers (D16S519, D16S420, D16S3022) being selected to be evenly spaced within the critical region (http://research.marshfieldclinic.org/genetics/). GeneScan version 3.7 (Applied Biosystems) and Genotyper version3.7 (Applied Biosystems) were used for gel analysis and genotype assignment, respectively.

### Gene sequencing

Four positional genes confined to the disease interval between markers D16S519-D16S3100 (namely PM5 encoding the PM5 protein (NM_014287), ABCC6 encoding the ATP-binding Cassette Subfamily C Member 6 (MIM 603234), *BFAR* encoding the bifunctional apoptosis regulator (NM_016561) and *MYH11* encoding smooth muscle myosin heavy chain 11 isoform (MIM 160745) were screened for mutations in two affected individuals of the French Bourgogne family (IV-11, V-21) and two controls using the ABI BigDye terminator cycle sequencing kit (Applied Biosystem). Primers used are available upon request. The Sequencher® 4.0.5 program (Gene codes Corporation) was used to identify variation between the sequence of putative mutations and control sequences. The same mutational screening protocol was applied for the MYH11 gene in the American kindred.

### Aortic compliance

Aortic compliance was analysed in 40 subjects of the French "Bourgogne" kindred (28 subjects among the 40 genotyped for genomewide linkage analysis and 12 additional relatives). It was measured by magnetic resonance imaging (MRI) using an automatic detection of the contour of the aorta over the whole cardiac cycle, as previously described¹⁸. Ascending aorta was imaged in the axial plane using a cine sequence with a repetition time of 15 milliseconds. Forty to sixty images of a single slice were acquired, thereby covering the whole cardiac cycle. Area of the aorta was computed using an automatic program and a curve was obtained for each patient. Compliance of the thoracic aorta was defined by: ΔS/ΔP (S: aortic surface, P: blood pressure).

### Cell culture

Dermal fibroblasts were obtained by skin biopsy from one French family member IV-11 and one normal volunteer. Cells were maintained in the medium of RPMI1640 with 10% fetal bovine serum (Life Technology), penicillin, streptomycin, and fungizone. They were incubated at 37° C with 5% CO₂. Cells were harvested when they reached 65-70 % confluence.

### cDNA analysis

RNA were extracted from cultured skin fibroblasts by Qiagen RNeasy mini Kit. The first-strand cDNA was synthesised by using 1 µg of total RNA. Primers were designed to bind to exon 31 and exon 33 : ex31 Forward 5'-ACA ACC AGC GGC AAC TCG-3' (SEQ ID No.14); ex33 Reverse 5'- CGT CTT CAT CTC CTC CAT C-3' (SEQ ID No.15). The genomic DNA was amplified using classical PCR conditions with an initial denaturation step 5 min at 95°C, followed by 30 cycles of DNA amplification : denaturation step 95°C, 45 seconds, hybridation step 56°C, 45 seconds, extension step 72°C, 1 min, and a final extension step at 72°C for 10 min.

### Histology and immunohistochemistry

Ascending aortic replacement was required in two affected subjects of the French kindred (IV-11 with TAA, IV-22 with TAA/AD). Aortic tissue samples obtained during surgery were immediately frozen in dry ice. Part of the specimens was fixed in 10% formalin and embedded in paraffin. For histology, hematoxylin and eosin staining for cells and elastic stain, alcian blue, and Sirius red for the extracellular matrix were performed, using standard techniques. For immunohistochemistry, anti-vimentin, α-smooth muscle actin, heavy caldesmon, and SM-MHC antibodies (all from Dako, Trappes, France) were used at the recommended dilution and revealed with a standard 3-step technique.

### Statistics

Quantitative values are expressed as mean +/- 1 standard deviation. Comparison between groups was performed by non-parametric analysis, using the Statview 5.0 software (Abacus Concept, Berkeley, CA, USA).

### Example 2: a unique genetic locus is responsible for both TAA/AD and PDA

A genome-wide linkage scan was conducted in 40 subjects belonging to 3 generations of the "Bourgogne" kindred using only affected subjects (8 TAA/AD, 5 PDA) and 382 microsatellite markers that span chromosomes 1 to 22 (see Figure 6 for complete description of the family and linkage results). Using only the TAA/AD cases, positive two-point LOD scores were observed on adjacent markers at chromosome 16p, with a maximum LOD score value of 2.73 at θ=0 that increased to 3.56 when the 5 PDA cases were included in the analysis (Fig.1 a). Multipoint linkage analysis yielded a maximum multipoint LOD score of 4.10 for marker D16S3103 (odds ratio greater than 10⁴:1 in favor of linkage). Fine mapping allowed the observation of recombinant haplotypes that delimited the critical interval to a 20 cM region (Fig.1 b). Genetic analysis of the candidate region in 3 affected and 3 unaffected individuals of the American kindred showed complete cosegregation with the disease with the markers of the region, but haplotype analysis did not modify the recombinant interval. Thus, the genetic analysis of these two families identified a unique genetic locus responsible for both TAA/AD and PDA.

### Example 3: identification of MYH11 mutations in the French and American kindreds

The candidate region contained 15 known, 29 provisional and 17 predicted genes. Sequence of several candidates, including PM5, ABBC6 and BFAR, did not show any mutation. Among the known genes, MYH11, which encodes the SM-MHC, a major contractile protein expressed in VSMC, was located ~1.5 Mb to the peak of maximum LOD score. A systematic mutational screening showed two heterozygous mutations in the French kindred, which were belonging to the same allele (Fig. 2a). The first was a substitution located at the splice-donor site of intron 32 (IVS32+1G>T). The second was a missense mutation located at exon 37 (c.G5361 >A), that resulted in a charged aminoacid Arginine substituted by a noncharged aminoacid Glutamine (R1758Q). Both mutations were confirmed in all subjects carrying the disease haplotype, but were not found in 340 normal chromosomes. The transcriptional consequences of the splice mutation were confirmed by the analysis of mRNA extracted from cultured fibroblasts of two affected subjects. Amplification and direct sequencing of the corresponding cDNA demonstrated the skipping of exon 32 (Fig. 2b), resulting in an in-frame deletion of 71 amino acids (L1456_N1526del) in the C-terminal tail of the SM-MHC. A similar search for mutation in the American kindred allowed the detection of a 72 nucleotides deletion within exon 28 of the MYH 11 gene (c.3810_3881del) (Fig. 3) that was not detected in 340 normal chromosomes. This in-frame deletion corresponds to the loss of 24 amino acids (R1241_L1264del), in the same C-terminal region of SM-MHC as that observed in the French kindred.

The smooth muscle (SM) myosin molecule is composed of two heavy chains (SM-MHC), two essential light chains and two regulatory light chains. Alternative splicing at the 3' and 5' ends of the primary *MYH11* transcript generates several isoforms differentially expressed in a tissue-specific and developmental manner (White et al., 1998). One SM-MHC dimer has two N-terminal globular heads where ATP and actin-binding sites are located, and one coiled-coil rod assembled by two SM-MHC α-helical C-terminal tails. The rod region is composed of a series of heptad repeats resulting in a 28-residue periodicity with alternate bands of positive and negative charges that are essential for the rod assembly (Babu et al., 2000). The use of specific monoclonal antibodies and of mutant constructs has shown the importance of the C-terminal end for myosin filament formation (Ikebe et al., 2001). Altered stability of the α-helical coiled-coil structure has been demonstrated for mutations in α-tropomyosin (Golitsina et al., 1997) and desmin (Sjoberg et al. 1999), acting through a dominant-negative effect on filament formation. It is therefore likely that the *MYH11* mutations observed in the French and American kindreds that locate within this segment, affect the coiled-coil structure through a similar dominant-negative mechanism and consequently the formation of thick myosin filament.

### Example 4: mutated MYH11 is associated with altered aortic compliance in either symptomatic or asymptomatic patients

Clinical investigation of 22 additional members of the French kindred showed that the mutant allele was also present in 8 apparently asymptomatic subjects (Figure 6), suggesting either an incomplete penetrance, as already observed in familial TAA/AD (Milewicz etal., 1998), or the possibility of subclinical vascular abnormalities. According to the clinical research protocol applied in that family, aortic compliance was measured in 40 individuals (27 asymptomatic, 7 PDA, 6 TAA) using magnetic resonance imaging (MRI), a very precise and non-invasive technique (Lalande et al., 2002). Analysis of asymptomatic mutated individuals showed no dilatation of the aortic root but strong abnormalities in the amplitude of variation of the aortic cross-sectional area during a cardiac cycle (Fig. 4A). When all individuals were compared according to their clinical and mutational status, a marked reduction of the aortic compliance was observed in both in symptomatic and asymptomatic individuals bearing one mutant allele (Fig. 4B). There was a difference between the values of ascending aortic compliance for all wild-type subjects (n=26, range: 0.68 to 2.96, mean 1.76 mm²/mmHg) and subjects with mutations (n=14, range: 0.15 to 1.28, mean 0.73 mm²/mmHg, p<0.001). A significant negative correlation was observed between aortic compliance and age in the wild-type group (Spearman test, p=0.002) but not in the mutant group (Spearman test, p=0.12), showing that ascending aortic compliance was abnormally low and independent of age in case of *MYH11* mutation. In subjects less than 30 years of age, the decrease in aortic compliance was so marked that values did not overlap between the wild-type (n=11, range:1.32 to 2.96, mean 2.24 mm²/mmHg) and the mutated subjects (n=7, range: 0.43 to 1.28, mean 0.83 mm²/mmHg, p<0.001). Thus, altered aortic compliance is an early hallmark of the disease which is in accordance with the role of VSMC on vascular tone.

### Example 5: mutated MYH11 is associated with altered aortic media structure and reduced vascular smooth muscle cell counts

The structural consequences of the mutation on the aortic wall were also investigated by histology and immunohistochemistry (Figure 5A-H) in two aortic tissue samples obtained from surgery performed in two affected subjects (one with TAA, the other with TAA/AD). Both tissues revealed similar severe abnormalities of the elastic fibers that were disrupted and lost. This was associated with a dramatic reduction in VSMC number that were replaced by loose myxoid alcian blue-positive or fibrous Sirius red-positive extracellular matrix. In the remaining VSMC, there was no labelling for SM-MHC (Fig. 5G-H) whereas α-smooth muscle actin, heavy caldesmone, and vimentin were normally expressed. This absence of immunoreactive protein, whereas expression of its mRNA is detected, suggests that the *MYH11* mutation induces a change in the conformation of the protein, consistent with an altered stability of the α-helical coiled-coil structure. Thus, *MYH11* mutations were associated with a major alteration of the basic structure of the aortic media, possibly the consequence of an altered VSMC ability to synthesize essential components of the extracellular matrix, (Patel et al., 1996) and to VSMC apoptosis (Rowe et al., 2000), thus accounting for the observed predisposition to aortic aneurysm and possible rupture.

Strong changes in the structure and composition of the *ductus arteriosus* occur prior and after delivery in which the capabilities of VSMC to migrate, proliferate, differentiate and contract are essential (Slomp. et al., (1997) ; Imamura et al., (2000)). Mice deficient in SM-MHC have been created (Morano et al., 2000). Homozygous *MYH11* ^{-/-} mice were characterized by several abnormalities, among them a delay in closure of the *ductus arteriosus.* Despite species differences in closure rate and morphology (Hornblad et al., 1967), presence of PDA in this knockout model and in both the French and the American kindreds, underlies the importance of SM-MHC in this phenomenon. SM-MHC deficient mice were also characterized by a giant thin-walled bladder and alteration of intestinal movement. We did not observe such symptoms, nor hematuria, a sign present in a family with pseudoxanthoma elasticum bearing a large 900 Kb deletion including the *MYH11* gene (Meloni et al., 2001). The phenotypic differences between homozygous SM-MHC deficient mice and the human patients with heterozygous mutations suggest distinct consequences of *MYH11* deletions leading to haploinsufficiency and mutations in the C-terminal rod of SM-MHC likely acting through a dominant-negative effect.

Up to now, inherited arterial diseases have been associated with defects in structural proteins of the extracellular matrix, such as fibrillin and collagen. Human *MYH11* gene mutations are the first example of a direct alteration of the VSMC function leading an inherited arterial disease, for which the inventors propose the term of arteriomyopathy.

### References

Antonarakis et al. (1989), N. Engl. J. Med. 320:153-163 Diagnosis of genetic disorders at the DNA level

Babu, G. J., Warshaw, D. M. & Periasamy, M. Smooth muscle myosin heavy chain isoforms and their role in muscle physiology. Microsc Res Tech 50, 532-40. (2000).

Barbas CF, Bain JD, Hoekstra DM, Lerner RA. (1992), Semisynthetic combinatorial antibody libraries: a chemical solution to the diversity problem. PNAS USA, 89, 4457-4461

Chomocyznski et al., Anal. Biochem., 162:156, 1987

Coady, M. A. et al. Familial patterns of thoracic aortic aneurysms. Arch Surg 134, 361-7. (1999).

Colas et al., 1996,

Cooper et al. (1991) Diagnosis of genetic disease using recombinant DNA, 3rd edition, Hum. Genet, 87:519-560

Dunnen et Antonarakis (2000) Mutation nomenclature extensions and suggestions to describe complex mutations: a discussion. Hum Mutation. 15 :7-12; Erratum in: Hum Mutat 2002 ;20(5):403.

Felgner et al. (1989) Science 337:387-388

Glancy, D. L., Wegmann, M. & Dhurandhar, R. W. Aortic dissection and patent ductus arteriosus in three generations. Am J Cardiol 87, 813-5, A9. (2001).

Golitsina, N. et al. Effects of two familial hypertrophic cardiomyopathy-causing mutations on alpha-tropomyosin structure and function. Biochemistry 36, 4637-42 (1997).

Grompe M. The rapid detection of unknown mutations in nucleic acids (1993) Nat. Genet. 5(2):111-7

Guo, D. et al. Familial thoracic aortic aneurysms and dissections: genetic heterogeneity with a major locus mapping to 5q13-14. Circulation 103, 2461-8. (2001).

Harlow E. et al., Antibodies: A Laboratory Manual, Cold Spring Harbor Laboratory, New York, (1988).

Hasham, S. N. et al. Mapping a locus for familial thoracic aortic aneurysms and dissections (TAAD2) to 3p24-25. Circulation 107, 3184-90. (2003).

Hornblad, P. Y. Studies on closure of the ductus arteriosus. 3. Species differences in closure rate and morphology. Cardiology 51, 262-82. (1967).

Ikebe, M. et al. The tip of the coiled-coil rod determines the filament formation of smooth muscle and nonmuscle myosin. J Biol Chem 276, 30293-300. (2001).

lmamura, S., Nishikawa, T., Hiratsuka, E., Takao, A. & Matsuoka, R. Behavior of smooth muscle cells during arterial ductal closure at birth. J Histochem Cytochem 48, 35-44. (2000).

Jayasena S.D. (1999) Aptamers: an emerging class of molecules that rival antibodies in diagnostics. Clin Chem. 45(9):1628-50.

Khau van Kien, P. et al. Familial thoracic aortic aneurysm/dissection with patent ductus arteriosus: genetic arguments for a particular pathophysiological entity. Eur J Hum Genet Jan 14 [Epub ahead of print] (2004).

Kohler and Milstein (1975) Continuous cultures of fused cells secreting antibody of predefined specificity. Nature ;256, 495-7.

Kruglyak, L., Daly, M. J., Reeve-Daly, M. P. & Lander, E. S. Parametric and Nonparametric Linkage Analysis: A Unified Multipoint Approach. Am J Hum Genet 58, 1347-1363 (1996).

Kuklin et al. Detection of single-nucleotide polymorphisms with the WAVE DNA fragment analysis system Genet. Test (1997-98), 1(3):201-6

Lalande, A. et al. Automatic determination of aortic compliance with cine-magnetic resonance imaging: an application of fuzzy logic theory. Invest Radiol 37, 685-91. (2002).

Lathrop, G. M., Lalouel, J. M., Julier, C. & Ott, J. Strategies for multilocus linkage analysis in humans. Proc Natl Acad Sci U S A 81, 3443-6. (1984).

Little et al. (1996)

Loftus BJ, Kim UJ, Sneddon VP, Kalush F, Brandon R, Fuhrmann J, Mason T, Crosby ML, Barnstead M, Cronin L, Deslattes Mays A, Cao Y, Xu RX, Kang HL, Mitchell S, Eichler EE, Harris PC, Venter JC, Adams MD. Genome duplications and other features in 12 Mb of DNA sequence from human chromosome 16p and 16q. Genomics. 60(3):295-308. (1999)

Mani, A. et al. Finding genetic contributions to sporadic disease: a recessive locus at 12q24 commonly contributes to patent ductus arteriosus. Proc Natl Acad Sci U S A 99, 15054-9. (2002).

Mackey, et al., (1988) Proc. Natl. Acad. Sci. U.S.A. 85:8027-8031

Meloni, I. et al. Pseudoxanthoma elasticum: Point mutations in the ABCC6 gene and a large deletion including also ABCC1 and MYH11. Hum Mutat 18, 85. (2001).

Milewicz, D. M. et al. Reduced penetrance and variable expressivity of familial thoracic aortic aneurysms/dissections. Am J Cardiol 82, 474-9. (1998).

Mitchell, S. C., Korones, S. B. & Berendes, H. W. Congenital heart disease in 56,109 births. Incidence and natural history. Circulation 43, 323-32. (1971).

Morano, I. et al. Smooth-muscle contraction without smooth-muscle myosin. Nat Cell Biol 2, 371-5. (2000).

Nickerson et al., 1990

Nienaber, C. A. & Eagle, K. A. Aortic dissection: new frontiers in diagnosis and management: Part I: from etiology to diagnostic strategies. Circulation 108, 628-35. (2003).

Patel MI, Melrose J, Ghosh P, Appleberg M. Increased synthesis of matrix metalloproteinases by aortic smooth muscle cells is implicated in the etiopathogenesis of abdominal aortic aneurysms. J Vasc Surg. 24(1):82-92. (1996)

Polani, P. E. & Campbell, M. Factors in the causation of persistent ductus arteriosus. Ann Hum Genet 24, 343-57 (1960).

Roman, M. J., Devereux, R. B., Kramer-Fox, R. & O'Loughlin, J. Two-dimensional echocardiographic aortic root dimension in normal children and adults. Am J Cardiol 64, 507-512 (1989).

Rowe VL, Stevens SL, Reddick TT, Freeman MB, Donnell R, Carroll RC, Goldman MH. Vascular smooth muscle cell apoptosis in aneurysmal, occlusive, and normal human aortas. J Vasc Surg. 31 (3):567-76. (2000)

Sambrook, Fritsch & Maniatis, Molecular Cloning: A Laboratory Manual, Second Edition (1989) Cold Spring Harbor Laboratory Press, Cold Spring Harbor, New York

Saiki et al., Science 1988, 239:487

Satoda, M. et al. Mutations in TFAP2B cause Char syndrome, a familial form of patent ductus arteriosus. Nat Genet 25, 42-6. (2000).

Sjoberg, G. et al. A missense mutation in the desmin rod domain is associated with autosomal dominant distal myopathy, and exerts a dominant negative effect on filament formation. Hum Mol Genet 8, 2191-8 (1999).

Slomp, J. et al. Differentiation, dedifferentiation, and apoptosis of smooth muscle cells during the development of the human ductus arteriosus. Arterioscler Thromb Vasc Biol 17, 1003-9. (1997).

Tuerk C. and Gold L. (1990) Systematic evolution of ligands by exponential enrichment: RNA ligands to bacteriophage T4 DNA polymerase. Science. 3;249(4968):505-10.

Vaughan, C. J. et al. Identification of a chromosome 11q23.2-q24 locus for familial aortic aneurysm disease, a genetically heterogeneous disorder. Circulation 103, 2469-75. (2001).

Waterhouse P, Griffiths AD, Johnson KS, Winter G. (1993) Combinatorial infection and in vivo recombination: a strategy for making large phage antibody repertoires. Nucleic Acids Research, 21, 2265-2266.

White, S. L., Zhou, M. Y., Low, R. B. & Periasamy, M. Myosin heavy chain isoform expression in rat smooth muscle development. Am J Physiol 275, C581-9 (1998).

## Claims

**1.** An ex vivo method of diagnosing or predicting an arterial disease, or a risk of arterial disease, in a subject, which method comprises detecting a mutation in the MYH11 gene or protein (myosin heavy polypeptide 11 of smooth muscle cells),
wherein said mutation is indicative of an arterial disease or of a risk of arterial disease.

**2.** The method according to claim 1, wherein said method may comprise the steps consisting of :
(a) obtaining a nucleic acid sample from the subject; and
(b) detecting a MYH11 mutation in said nucleic acid sample;
wherein the presence of a mutation is indicative of an arterial disease or of a risk of arterial disease.

**3.** The method according to claim 1 or 2, wherein said mutation is localised in the region of MYH11 gene that encodes the coiled-coil domain of MYH11 protein which is defined by amino acid residues 844 to 1934 of SEQ ID No.5.

**4.** The method according to any of claims 1 to 3, wherein said MYH11 mutation is selected from the group consisting of a substitution IVS32+1G>T, a substitution 5361G>A, and a deletion 3810-3881del.

**5.** The method according to any one of claims 1 to 4, wherein said nucleic acid sample is contacted with an oligonucleotide which specifically hybridizes to a junction of exon 31 and exon 33 of MYH11 as shown in SEQ ID No.8, wherein hybridization of the oligonucleotide to said nucleic acid sample is indicative of the presence of a substitution IVS32+1 G>T and therefore of an arterial disease or of a risk of arterial disease.

**6.** The method according to any one of claims 1 to 4, wherein said nucleic acid sample is contacted with an oligonucleotide which specifically hybridizes to exon 32 of MYH11 as shown in SEQ ID No.9, wherein non hybridization of the oligonucleotide to said nucleic acid sample is indicative of the presence of a substitution IVS32+1G>T and therefore of an arterial disease or of a risk of arterial disease.

**7.** The method according to any one of claims 1 to 4, wherein said nucleic acid sample is contacted with an oligonucleotide which specifically hybridizes to the junction created by deletion of nucleotides 3810-3881 from MYH11 as shown in SEQ ID No.10, wherein hybridization of the oligonucleotide to said nucleic acid sample is indicative of the presence of a deletion 3810-3881 del and therefore of an arterial disease or of a risk of arterial disease.

**8.** The method according to claim 1, which method comprises:
(a) obtaining a sample from the subject; and
(b) detecting a mutation in a MYH 11 protein contained in said sample;
wherein the presence of a mutation is indicative of an arterial disease or of a risk of arterial disease.

**10.** The method according to claim 1 or 8, wherein said mutation is localised in the coiled-coil domain of MYH11 protein which is defined by amino acid residues 844 to 1934 of SEQ ID No.5.

**11.** The method according to any of claims 1, 8 or 9, wherein said mutation of MYH11 protein is selected from the group consisting of L1456_N1526del, V1242_L264del, and R1758Q.

**12.** An isolated nucleic acid, which comprises a MYH11 (smooth muscle myosin heavy chain) gene sequence that contains a mutation selected from the group consisting of IVS32+1G>T, 5361 G>A and 3810-3881del.

**13.** An isolated oligonucleotide which specifically hybridizes :
a) to MYH11 sequences in the region of the splice-donor site of intron 32, or of the junction of exons 31 and 33 as shown in SEQ ID No.8, or to exon 32 (SEQ ID No.9) or fragments thereof; or
b) to a region of MYH11 nucleic acid that contains the nucleotide in position 5361 according to a sequence such as shown in SEQ ID No. 2 or 3; or
c) to a region of MYH11 nucleic acid comprising nucleotides 3810-3881 (SEQ ID No.11), or to the junction created by deletion of nucleotides 3810-3881 from MYH11 as shown in SEQ ID No. 10.

**14.** An isolated polypeptide which comprises the polypeptide sequence of MYH11 containing a mutation selected from the group consisting of a L1456_N1526 deletion, a deletion V1242_L1264, and a substitution R1758Q.

**15.** An isolated monoclonal or polyclonal antibody that specifically recognizes a MYH11 protein containing a mutation selected from the group consisting of a L1456_N1526 deletion, a deletion V1242_L1264, and a substitution R1758Q.

**16.** Use of a nucleic acid sequence encoding a wild-type MYH11 protein for the manufacture of medicament intended for the treatment of an arterial disease.
